(19) Europäisches Patentamt
European Patent Office
Office européen des brevets

(11) EP 3 461 897 B1

(12) EUROPEAN PATENT SPECIFICATION

(45) Date of publication and mention of the grant of the patent:
**02.12.2020 Bulletin 2020/49**

(51) Int Cl.:
***C12N 15/74*** *(2006.01)*

(21) Application number: **18204455.2**

(22) Date of filing: **20.06.2016**

(54) **A CONSTRUCTION, A VECTOR AND A METHOD PRODUCTION OF A TARGET MOLECULE IN A CYANOBACTERIUM**

KONSTRUKTION, VEKTOR UND VERFAHREN ZUR INTEGRATION EINES ZIELMOLEKÜLS IN EIN CYANOBAKTERIUM

CONSTRUCTION, VECTEUR ET PROCÉDÉ DE PRODUCTION D'UNE MOLÉCULE CIBLE DANS UNE CYANOBACTÉRIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2015 PT 2015108564**

(43) Date of publication of application:
**03.04.2019 Bulletin 2019/14**

(62) Document number(s) of the earlier application(s) in accordance with Art. 76 EPC:
**16175360.3 / 3 106 521**

(73) Proprietor: **Instituto de Biologia Molecular e Celular**
**4200-135 Porto (PT)**

(72) Inventors:
- **VILARINHO PINTO, Filipe José**
  **4250-170 Porto (PT)**
- **SEQUEIRA TAMAGNINI BARBOSA OXELFELT, Paula Maria**
  **4450-649 Matosinhos (PT)**

(74) Representative: **Patentree**
**Edificio Net**
**Rua de Salazares, 842**
**4149-002 Porto (PT)**

(56) References cited:
**CN-A- 103 361 274**

- **KUNERT A ET AL: "Construction of promoter probe vectors for Synechocystis sp. PCC 6803 using the light-emitting reporter systems Gfp and LuxAB", JOURNAL OF MICROBIOLOGICAL METHODS, ELSEVIER, AMSTERDAM, NL, vol. 41, no. 3, 1 August 2000 (2000-08-01) , pages 185-194, XP002763424, ISSN: 0167-7012**
- **BENTLEY FIONA K ET AL: "Heterologous expression of the mevalonic acid pathway in cyanobacteria enhances endogenous carbon partitioning to isoprene", MOLECULAR PLANT JUL 2012,, vol. 7, no. 1, 1 January 2014 (2014-01-01) , pages 71-86, XP002763434, ISSN: 1752-9867**
- **PINTO FILIPE ET AL: "Improving a Synechocystis-based photoautotrophic chassis through systematic genome mapping and validation of neutral sites", DNA RESEARCH, OXFORD UNIVERSITY PRESS, GB, vol. 22, no. 6, 1 December 2015 (2015-12-01), pages 425-437, XP002763423, ISSN: 1756-1663**
- **TAN XIAOMING ET AL: "Role of Rbp1 in the acquired chill-light tolerance of cyanobacteria", JOURNAL OF BACTERIOLOGY : JB, AMERICAN SOCIETY FOR MICROBIOLOGY, US, vol. 193, no. 11, 1 June 2011 (2011-06-01) , pages 2675-2683, XP002763419, ISSN: 1098-5530**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 461 897 B1

- YADA T ET AL: "Gene recognition in cyanobacterium genomic sequence data using the hidden Markov model", PROCEEDINGS / ... INTERNATIONAL CONFERENCE ON INTELLIGENT SYSTEMS FOR MOLECULAR BIOLOGY ; ISMB. INTERNATIONAL CONFERENCE ON INTELLIGENT SYSTEMS FOR MOLECULAR BIOLOGY 1996,, vol. 4, 1 January 1996 (1996-01-01), pages 252-260, XP002763420, ISSN: 1553-0833
- HIHARA YUKAKO ET AL: "DNA microarray analysis of redox-responsive genes in the genome of the cyanobacterium Synechocystis sp. strain PCC 6803", JOURNAL OF BACTERIOLOGY, AMERICAN SOCIETY FOR MICROBIOLOGY, US, vol. 185, no. 5, 1 March 2003 (2003-03-01) , pages 1719-1725, XP002763421, ISSN: 0021-9193
- DÖRRICH ANJA K ET AL: "Deletion of the Synechocystis sp. PCC 6803 kaiAB1C1 gene cluster causes impaired cell growth under light-dark conditions", IDENTIFICATION OF TWO SCYLLO-INOSITOL DEHYDROGENASES IN BACILLUS SUBTILIS, MICROBIOLOGY (READING, ENGLAND) MAY 2010 LNKD-PUBMED:20133360, VOL. 156, NR. PT 5, PAGE(S) 1538 - 1546, vol. 160, no. Pt 11, 1 November 2014 (2014-11-01), pages 2538-2550, XP002763422, ISSN: 1465-2080

## Description

### Technical domain

The present disclosure relates to constructs for the integration of DNA into the genome of cyanobacteria, vectors comprising the constructs, cyanobacteria comprising the constructs or transformed by the vector and a method of transforming cyanobacteria.

The current disclosure described herein employs the use of five previously unidentified neutral sites into which foreign DNA can be integrated into the chromosome of *Synechocystis* without deleterious effects upon the growth and viability of the host cells. It is further described that foreign DNA may be integrated at such neutral sites so that *Synechocystis* expresses genes, which encode proteins that enable production of commercially important chemicals. The identification of a number of neutral sites which are spread throughout the *Synechocystis* chromosome, enables implementation of more complex synthetic circuits i.e. multiple genes may be expressed. New integrative vectors have been produced and are also disclosed herein. The present disclosure therefore relates to constructs for the integration of DNA into the genome of cyanobacteria, vectors comprising the constructs, cyanobacteria comprising the constructs or transformed by the vector and a method of transforming cyanobacteria.

### Technical background

Microorganisms have been widely used in biotechnological applications to produce added-value products (Madigan 2012). The Gram-negative bacterium *Escherichia coli* is the most commonly used, but others such as the Gram-positive bacterium *Bacillus subtilis,* and the eukaryotic yeast *Saccharomyces cerevisiae,* have also been successfully exploited (Harwood, Pohl et al. 2013, Li and Borodina 2014). Heterotrophic organisms require supplementation of the growth medium with external carbon sources increasing production costs therefore autotrophic organisms emerge as a valid alternative (Berla, Saha et al. 2013, Yu, You et al. 2013). In this context, cyanobacteria are promising "low-cost" cell factories since they can use $CO_2$ as carbon source, water as reducing power, light as energy source and some strains are even able to fix atmospheric $N_2$ (nitrogen source). These organisms are found in almost any ecological niche on Earth (Whitton and Potts 2000) which reflects their high degree of metabolic plasticity.

Among cyanobacteria, the unicellular non-$N_2$-fixing *Synechocystis* sp. PCC 6803 is the most studied strain, and the large amount of genomic, proteomic and physiological information generated over the years has allowed the construction of genome-scale models, enabling the prediction of the system behavior (Fu 2009, Montagud, Navarro et al. 2010, Steuer, Knoop et al. 2012). Moreover, it was the first photosynthetic organism to have its genome sequenced (Kaneko, Sato et al. 1996; (http://genome.microbedb.jp/cyanobase/Synechocystis) comprising a 3.6 Mb chromosome and 7 plasmids. It is also naturally transformable, and a number of molecular tools are available for its genetic manipulation (Vermaas 1996, Koksharova and Wolk 2002, Huang, Camsund et al. 2010, Heidorn, Camsund et al. 2011, Berla, Saha et al. 2013, Qi, Yao et al. 2013). Altogether, these characteristics potentially allow the exploitation of *Synechocystis* as a photoautotrophic biotechnological platform. However, in contrast to E. *coli,* for which several replicative plasmids from different compatibility groups are available, for *Synechocystis* these tools are limited (Koksharova and Wolk 2002, Huang, Camsund et al. 2010, Taton, Unglaub et al. 2014). Commonly used tools such as replicative vectors based on *E. coli* plasmids have some disadvantages in *Synechocystis* resulting in them exhibiting some instability.

The preferred approach for *Synechocystis* is for non-replicating suicidal integrative vectors to be used to implement new functionalities by integrating ectopic DNA into the genome, through homologous recombination (Williams 1988). However, the choice of the integration site is critical for use of the organism for biomanufacturing; such loci are required to be 'neutral' in that integration of ectopic DNA into these sites must not affect cellular viability or cause any distinguishable phenotype. A few 'neutral' integration sites in the *Synechocystis* genome have been reported in the literature, for example, to complement knockout mutants (Williams 1988, Burnap, Qian et al. 1994), to test promoters and study transcription regulation (Aoki, Kondo et al. 1995, Kunert, Hagemann et al. 2000), or for the heterologous expression of proteins (Zang, Liu et al. 2007). Although those loci have been described as "not appear to be deleterious" (Aoki, Kondo et al. 1995), "does not otherwise affect any observable functions" (Burnap, Qian et al. 1994), "did not show any detectable phenotype" (Aoki, Goto et al. 2011), or being "a neutral site of the chromosome" (Kunert, Hagemann et al. 2000), they have not been properly characterized as neutral sites - loci that can be disrupted without affecting cellular viability or cause any distinguishable phenotype (Clerico, Ditty et al. 2007).

KUNERT A et al : "Construction of promoter probe vectors for Synechocystis sp. PCC 6803 using the light-emitting reporter systems Gfp and LuxAB.", Journal of Microbiological Methods, Aug 2000, vol. 41, no. 3, and BENTLEY FIONA K et al: "Heterologous expression of the mevalonic acid pathway in cyanobacteria enhances endogenous carbon partitioning to isoprene.", MOLECULAR PLANT JAN 2014, vol. 7, no. 1, both disclose sites for integration of ectopic or heterologous sequences into the Synechocystis genome, which sites are not known to be associated with phenotypic changes following integration. There is a need therefore to identify advantageous neutral sites in the chromosome of *Synechocystis* to enable development of methods and tools for transforming *Synechocystis* so that new functionalities can be introduced. Furthermore, such developments would mean that *Synechocystis* could be exploited as a cost-effective biomanufacturing system.

These facts are disclosed in order to illustrate the technical problem addressed by the present disclosure.

**Brief description**

The current disclosure described herein employs the use of previously unidentified neutral sites into which foreign DNA can be integrated into the chromosome of *Synechocystis* without deleterious effects upon the growth and viability of the host cells. It is further described that foreign DNA may be integrated at such neutral sites so that *Synechocystis* expresses genes, which encode proteins that enable production of commercially important chemicals The identification of a number of neutral sites which are spread throughout the *Synechocystis* chromosome, enables implementation of more complex synthetic circuits i.e. multiple genes may be expressed.

New integrative vectors have been produced and are also disclosed herein which are compatible with widely-available standard tools e.g. BioBrick™ RFC[10] standard (http://www.biobricks.org/) which will make easier to use *Synechocystis* in synthetic biology approaches.

In the present disclosure, foreign DNA was inserted into the chromosome of *Synechocystis,* and the cyanobacteria was transformed to produce a number of model chemicals without affecting the growth and viability of the host cells. Disclosed herein is a method of transforming *Synechocystis* comprising selection of one or more of the identified neutral sites, construction of integrative plasmids that enable foreign DNA to be integrated into the *Synechocystis* chromosome at the neutral sites and generation of mutant strains of *Synechocystis* capable of bioproduction. In addition, the present disclosure provides for vectors for integration at the five different neutral sites.

Further, the present disclosure provides a method of expression of exogenous genes in *Synechocystis* sp. PCC 6803 through use of a T/A cloning vector that contains a synthetic interface used to insulate the multiple cloning sites where ectopic DNA should be cloned for insertion in *Synechocystis* chromosome, whereby the insulation interface is flanked by the two regions for double homologous recombination on one of the neutral sites (n).

The disclosure further comprises a method of constructing integrative plasmids to generate a series of plasmids (pSNn) based upon a T/A cloning vector that contains a synthetic insulation interface flanked by the two regions for double homologous recombination on one of the neutral sites (n).

The disclosure also provides a method of generating a series of plasmids (pSNnKS) by cloning a PCR-amplified kanamycin resistance/sucrose sensitivity cassette, upstream of the insulation interface of each pSNn plasmid. The pSNnKS plasmids series are used to produce *Synechocystis* mutants that will allow the insertion of ectopic DNA cloned into the pSNn plasmids series (delivery vectors), resulting in mutants without selection markers.

The present disclosure relates to a genetic construct for the introduction of ectopic DNA in a cyanobacterium host cell, in particular *Synechocystis,* comprising two sequences selected from

> an upstream and a downstream chromosomal regions of a locus in a cyanobacterium wherein the locus is selected from the following list: SEQ ID No 106, SEQ ID No 107, SEQ ID No 108, SEQ ID No 109, SEQ ID No 110,
> and the sequences length vary between 400-1500 bp, preferably 430-800 bp, more preferably 430-560 bp.

In an embodiment, the genetic construct may comprise one of the following sequences: SEQ ID No 111, SEQ ID No 112, SEQ ID No 113, SEQ ID No 114, SEQ ID No 115.

In an embodiment, the genetic construct may further comprise the SEQ ID NO: 26.

The present disclosure also relates to a vector for the introduction of a genetic construct for the production of a target molecule in a cyanobacterium host cell comprising the genetic construct previously described.

In an embodiment, the vector for the introduction of a genetic construct for the production of a target molecule in a cyanobacterium host cell comprising the genetic construct previously described may further comprise a gene selected from the following list: a gene coding for antibiotic resistance, a gene coding for levansucrase, a gene coding for green fluorescence protein, in particular a gene coding for antibiotic kanamycin resistance.

In an embodiment, said vector may further comprise a constitutive promotor, preferably SEQ ID No 20.

In an embodiment, said vector is an integrative vector.

In an embodiment, said vector may comprise a gene or genes for coding in a host cell: an enzyme or enzymes for production of a target molecule, or a precursor of a target molecule or a target molecule.

In an embodiment, the genetic construct leads to the production of at least a precursor, an enzyme or the target molecule of the following list: a compatible solute, an organic compound, a polymer, an alcohol, a biofuel, an additive, an adhesive, a sealant, a pigment, an antibiotic, a medicament, a protein.

In an embodiment, the genetic construct encodes enzymes for the production of a compatible solute selected from the following list: ectoine, hydroxyectoine, or mixtures thereof.

In an embodiment, the genetic construct encodes enzymes for the production of the organic compound isoprene, glucoglycerol, betaine, or mixtures thereof.

In an embodiment, the genetic construct encodes proteins/enzymes for the production of the pigment phycocyanin.

The present disclosure also relates to a cyanobacterium host cell comprising the genetic construct now disclosed or comprising the vector now disclosed.

In an embodiment, the cyanobacterium host cell is *Synechocystis,* in particular *Synechocystis* sp. PCC6803.

This disclosure further relates to the use of a genetic construct comprising:

two sequences selected from an upstream and downstream chromosomal regions of a locus in a cyanobacterium wherein

the locus is selected from the following list: SEQ ID No 106 - N5 is *sll1476,* SEQ ID No 107 -N8 is *slr0573,* SEQ ID No 108 - N10 is *slr 1396,* SEQ ID No 109 -- N15 is slr0271, SEQ ID No 110 - N16 is slr0397,

and the sequences length vary between 400-1500 bp, preferably 430-800 bp, more preferably 430-560 bp,

as an enhancer of the production of an enzyme for production of the target molecule, or a precursor of a target molecule or a target molecule in a cyanobacterium host cell.

This disclosure also relates to a method for the expression of an enzyme for production of the target molecule, or a precursor of a target molecule or a target molecule with the following steps:

constructing a genetic construct as described in claim 1 - 3;

cloning the genetic construct in an appropriated vector;

cloning the genetic construct for the expression of the precursor, the enzyme or the target molecule, in the vector and obtaining a recombinant vector;

transforming *Synechocystis* host cell with the recombinant vector;

expressing the gene or genes for the production of a precursor, of the enzyme or of the target molecule.

This method is particularly relevant in the production of chemical produts, namely in the cosmetic industry or pharmaceutical industry.

Throughout the description and claims the word "comprise" and variations of the word, are not intended to exclude other technical features, additives, components, or steps. Additional objectives, advantages and features of the solution will become apparent to those skilled in the art upon examination of the description or may be learned by practice of the solution.

**Brief description of the drawings**

For a better understanding of the disclosure, the attached figures are joined, which represent preferred embodiments of the disclosure. The figures provide preferred embodiments for the present disclosure and should not be seen as limiting the scope of the disclosure.

Fig. 1: Chromosomal location and genomic context of the *Synechocystis'* putative neutral sites that were initially considered for transcriptional analysis. (a) In bold and marked with an asterisk are the sites chosen for mutants construction; the other sites fulfilled the neutral sites initial selection criteria, but were subsequently disregarded after additional analyses. **(b)** Genomic context of each putative neutral site (5,000 bp). Black- ORFs corresponding to the neutral sites, Gray-ORFs encoding proteins with assigned functions. tp - putative transposase, '- partial ORFs.

**Fig. 2:** *Synechocystis* growth curves, sample collection and transcription analysis of eight selected loci (N5, N6, N7, N8, N10, N14, N15, N16). **(a)** Growth curves of three independent cultures of *Synechocystis* with sample collection points for RNA extraction indicated by arrows (1 - $OD_{730}$≈0.4, 2-$OD_{730}$≈1.9, 3 - $OD_{730}$≈9.0). (**b**) RT-PCR transcription analysis using total RNA extracted from cells collected at the time points indicated in **a.** These results are representative of three biological replicates and technical duplicates. - negative control (absence of template), + positive control (genomic DNA), and M - GeneRuler DNA ladder (Thermo Fisher Scientific).

**Fig. 3:** Growth curves of *Synechocystis* wild-type and mutants in the five neutral sites (SN5K, SN8K, SN10K, SN15K, SN16K) cultivated under different conditions. 10 mL of culture were grown in 25 mL flasks at 30 °C and $OD_{730}$ values were recorded daily for 10 consecutive days in continuous light (CL) or 12 h light (20 μmol photons/$m^2$/s) / 12 h dark cycles (LD), in autotrophy (G0) or mixotrophy (5 mM glucose, G5).

**Fig. 4:** Detection of GFP expression in *Synechocystis* wild-type (WT) and insertion mutants containing a promoterless GFP generator (SNnK.gfp) or the GFP generator under the control of the constitutive minimal $P_{psba2^*}$ synthetic promoter (SNnK.Cgfp). **(a)** Confocal micrographs of *Synechocystis*: autofluorescence is depicted in the left column, GFP signal in the middle column and the merged signals in the right column. **(b)** Normalized fluorescence of the cultures analyzed in **a.** Measurements were performed in triplicate, using 200 μL of each culture, and fluorescence was normalized to $OD_{790}$. Normalized fluorescence from the wild-type was used as baseline. Bars indicate mean ± SD.

**Fig. 5:** Normalized fold expression of *gfp* (RT-qPCR) in *Synechocystis* mutants harboring a synthetic device with *gfp* under the control of a synthetic constitutive promoter. WT - wild type (control). Data from 3 biological replicates and 3 technical replicates were normalized against three reference genes (16S, *petB,* and *rnpB*) and error bars represent ± SEM.

**Fig. 6:** Chromosomal location and genomic context of the five *Synechocystis'* neutral sites. **(a)** These sites were chosen for mutants construction. **(b)** Genomic context of each of the five neutral sites (5,000 bp). Show in black are the ORFs corresponding to the neutral sites.

**Fig. 7:** List of the 13 putative neutral sites initially identified and then subjected to further analysis. The five neutral sites that comprise this disclosure are identified as being N5, N8, N10, N15 and N16. * d - direct sequence, c - complement sequence.

**Fig. 8:** RT-PCR transcription analysis of thirteen loci for the putative neutral sites (N1, N3, N5, N6, N7, N8, N10, N11, N12, N13, N14, N15, N16). *Synechocystis* wild-type samples for RNA extraction were collected at OD730 ≈ 0.8-0.9. These results are representative of the three biological triplicates and technical duplicates. c - cDNA, - negative control (absence of template), + positive control (genomic DNA), M - GeneRuler DNA ladder (Thermo Fisher Scientific).

**Fig. 9:** List of Forward and Reverse Primers used for transcriptional analysis using RT-PCR of the 13 putative neutral sites

**Fig. 10:** List of other primers used in the present disclosure. *Restriction sites underlined; **Primers used to confirm genomic integrations and mutants full segregation; ***SDM - Site directed mutagenesis.

**Fig. 11:** List of all the mutant organisms and plasmids used or generated in this work

**Fig. 12:** List of plasmids constructed in this work. A range of plasmids were constructed for each of the 5 neutral sites.

**Fig. 13:** Schematic representation of a plasmid (pSN10K.Cgfp) that was constructed in respect of the neutral site identified as N10. Homologous regions flanking the neutral site were amplified by PCR (primers indicated by arrows) and fused by overlap-PCR (1). The fragment containing the two flanking regions was cloned into the pGEM-T easy vector; the restriction sites incompatible with the BioBrick standard RFC[10] were removed by digestion with *Xho*I and Sa*l*I and vector re-circularization, and by site directed mutagenesis (SDM, mutagenic primers indicated by a crossed arrow) (2). The BioBrick-compatible cloning interface, flanked by two double BioBrick transcription terminators (TT), was synthesized and cloned into the *Xma*I site of the vector originating the pSN10 plasmid (3). A selection cassette conferring resistance to kanamycin was amplified by PCR (primers indicated by arrows) from the plasmid pK18mobsacB and cloned into the *Xma*I site (pSN10K) (4). Subsequently, the module containing the GFP encoding gene under the control of the minimal psbA2 promoter ($P_{psbA2^*}$) was excised from the BioBrick vector pSB1A2-Cgfp and cloned into the cloning interface, originating the integrative plasmid pSN10K.Cgfp (5).

**Fig. 14:** ANOVA analysis of *Synechocystis* wild-type growth compared to mutants (SNnK mutants) into which a kanamycin resistance cassette is inserted into each of the five neutral sites. *P*-values of up to second order interactions produced by a three-way ANOVA analysis

**Fig. 15:** List of *Synechocystis* mutants constructed in the present disclosure.

**Fig. 16:** Detection of *gfp* transcripts and GFP in *Synechocystis* mutants harboring a promoterless GFP synthetic device (SN*n*K.gfp) or a device with *gfp* under the control of the synthetic constitutive promoter $P_{psba2*}$ (SN*n*K.Cgfp). *gfp* transcription was assessed by RT-PCR **(a)** and GFP expression was assessed by Western blot **(b).** Wild-type was included as control. M - GeneRuler DNA ladder (Thermo Fisher Scientific); C + - Positive control (wild-type gDNA for 16S rRNA and pSB1A2-E0240 for *gfp*); C - - Negative control (no template); - RT - PCRs performed using RNA as template; + RT - PCRs performed using cDNA as template.

**Fig. 17:** Detection of GFP expression in *Synechocystis* mutants harboring a promoterless GFP synthetic device (SNnK.gfp) or a device with *gfp* under the control of a synthetic constitutive promoter (SNnK.Cgfp). Total cell fluorescence was measured 2, 4, 7, 9 and 11 days after inoculation, using 200 μL of three independent culture replicates. Measurements were performed in triplicate and fluorescence was normalized to $OD_{790}$. Normalized fluorescence from the wild-type was used as baseline. Bars indicate mean ± SD.

**Fig. 18:** PCR confirmation of the full segregation of the *Synechocystis* mutants in the five neutral sites (N5, N8, N10, N15, N16). Schematic representation of the position of the primers used for the wild-type and mutants, exemplified for N10 **(a).** PCR reactions were performed using primers within the ORF corresponding to each neutral site **(b),** a primer external to each site and a primer within the selection cassette **(c),** primer amplifying the kanamycin resistance cassette **(d),** primers amplifying the within the gfp gene (**e**) or primers amplifying the double selection cassette **(f)** (listed in **Fig. 10**). No template controls were always included (c-) and the expected band sizes are within brackets. M - GeneRuler DNA ladder (Thermo Fisher Scientific).

**Fig. 19:** Southern blot confirmation of the full segregation of the *Synechocystis* mutants in the five neutral sites (N5, N8, N10, N15, N16). Expected band sizes are within brackets.

**Fig. 20:** Phenotypic characterization of mutants harboring the double selection cassette conferring kanamycin resistance and sucrose sensitivity (SNnKS). *Synechocystis* wild-type and mutants were grown in agar plates containing BG11 medium (BG11), BG11 medium supplemented with 100 μg/mL kanamycin (BG11 + $Km^{100}$) and BG11 medium supplemented with 5% (m/v) sucrose (BG11 + Suc 5%).

**Fig. 21:** Plasmid map of pSN5 (seq ID NO 1).

**Fig. 22:** Plasmid map of pSN8 (seq ID NO 2).

**Fig. 23:** Plasmid map of pSN10 (seq ID NO 3).

**Fig. 24:** Plasmid map of pSN15 (seq ID NO 4).

**Fig. 25:** Plasmid map of pSN16 (seq ID NO 5).

**Fig. 26:** Plasmid map of pSN5K (seq ID NO 6).

**Fig. 27:** Plasmid map of pSN8K (seq ID NO 7).

**Fig. 28:** Plasmid map of pSN10K (seq ID NO 8).

**Fig. 29:** Plasmid map of pSN15K (seq ID NO 9).

**Fig. 30:** Plasmid map of pSN16K (seq ID NO 10).

**Fig. 31:** Plasmid map of pSN5KS (seq ID NO 11).

**Fig. 32:** Plasmid map of pSN8KS (seq ID NO 12).

**Fig. 33:** Plasmid map of pSN10KS (seq ID NO 13).

**Fig. 34:** Plasmid map of pSN15KS (seq ID NO 14).

**Fig. 35:** Plasmid map of pSN16KS (seq ID NO 15).

**Description of the sequences**

SEQ ID NO: 1: Nucleotide sequence of plasmid for N5 neutral site (pSN5).
SEQ ID NO: 2: Nucleotide sequence of plasmid for neutral site N8 (pSN8).
SEQ ID NO: 3: Nucleotide sequence of plasmid for neutral site N10 (pSN10).
SEQ ID NO: 4: Nucleotide sequence of plasmid for neutral site N15 (pSN15).
SEQ ID NO: 5: Nucleotide sequence of plasmid for neutral site N16 (pSN16).
SEQ ID NO: 6: Nucleotide sequence of plasmid for neutral site N5 containing Kanamycin resistance cassette (pSN5K).
SEQ ID NO: 7: Nucleotide sequence of plasmid for neutral site N8 containing Kanamycin resistance cassette (pSN8K).
SEQ ID NO: 8: Nucleotide sequence of plasmid for neutral site N10 containing Kanamycin resistance cassette (pSN10K).
SEQ ID NO: 9: Nucleotide sequence of plasmid for neutral site N15 containing Kanamycin resistance cassette (pSN15K).
SEQ ID NO: 10: Nucleotide sequence of plasmid for neutral site N16 containing Kanamycin resistance cassette (pSN16K).
SEQ ID NO: 11: Nucleotide sequence of plasmid for neutral site N5 used to produce *Synechocystis* mutants without selection markers (pSN5KS).
SEQ ID NO: 12: Nucleotide sequence of plasmid for neutral site N8 used to produce *Synechocystis* mutants without selection markers (pSN8KS).
SEQ ID NO: 13: Nucleotide sequence of plasmid for neutral site N10 used to produce *Synechocystis* mutants without selection markers (pSN10KS).
SEQ ID NO: 14: Nucleotide sequence of plasmid for neutral site N15 used to produce *Synechocystis* mutants without selection markers (pSN15KS).
SEQ ID NO: 15: Nucleotide sequence of plasmid for neutral site N16 used to produce *Synechocystis* mutants without selection markers (pSN16KS).
SEQ ID NO: 16: Nucleotide sequence of T/A cloning vector (pGEM-T easy).
SEQ ID NO: 17: Nucleotide sequence of PCR amplified kanamycin resistance selection cassette (Km).
SEQ ID NO: 18: Nucleotide sequence of PCR amplified double selection cassette (KmSc).
SEQ ID NO: 19: Nucleotide sequence of GFP generator (BBa_E0840).
SEQ ID NO: 20: Nucleotide sequence of synthetic constitutive promoter (PpsbA2*).
SEQ ID NO: 21: Nucleotide sequence of recombination sequence for site N5 obtained by overlap PCR (N5.OL).
SEQ ID NO: 22: Nucleotide sequence of recombination sequence for site N8 obtained by overlap PCR (N8.OL).
SEQ ID NO: 23: Nucleotide sequence of recombination sequence for site N10 obtained by overlap PCR (N10.OL).
SEQ ID NO: 24: Nucleotide sequence of recombination sequence for site N15 obtained by overlap PCR (N15.OL).
SEQ ID NO: 25: Nucleotide sequence of recombination sequence for site N16 obtained by overlap PCR (N16.OL).
SEQ ID NO: 26: Nucleotide sequence of Bio-Brick compatible cloning interface (FP300).
SEQ ID NO: 27: Nucleotide sequence for DABA acetyltransferase (EctA) of *C. salexigens.*
SEQ ID NO: 28: Nucleotide sequence for diaminobutyric acid (DABA) aminotransferase (EctB) of *C. salexigens.*
SEQ ID NO: 29: Nucleotide sequence for ectoine synthase (EctC) of *C. salexigens.*
SEQ ID NO: 30: Nucleotide sequence for ectoine hydroxylase (EctD) of *C. salexigens.*
SEQ ID NO: 31: Nucleotide sequence for DABA acetyltransferase (EctA) of *M. alcaliphilum.*
SEQ ID NO: 32: Nucleotide sequence for diaminobutyric acid (DABA) aminotransferase (EctB) of *M. alcaliphilum.*
SEQ ID NO: 33: Nucleotide sequence for ectoine synthase (EctC) of *M. alcaliphilum.*
SEQ ID NO: 34: Nucleotide sequence for ectoine hydroxylase (EctD) of *M. alcaliphilum.*
SEQ ID NO: 35: Nucleotide sequence for aspartate kinase isozyme (Ask) of *M. alcaliphilum.*

**Primer Sequences for Transcriptional Analysis of the Putative Neutral Sites**

| Sequence ID Number | Primer name | Sequence 5' → 3' | Amplicon size (bp) |
|---|---|---|---|
| SEQ ID NO: 36: | N1F | CATGGATTGAAGGAAGGG | 100 |
| SEQ ID NO: 37: | N1R | TTTGGTGAACAATTAATGAAC | |
| SEQ ID NO: 38: | N3F | GCAGTCCCTCCTCCAAAC | 129 |
| SEQ ID NO: 39: | N3R | AAGCCGATATTCAGGTAAGATG | |
| SEQ ID NO: 40: | N5F | GGAAGTGTTGTTGCTGTC | 232 |
| SEQ ID NO: 41: | N5R | GTCCTTCGCCGTAGATTG | |
| SEQ ID NO: 42: | N6F | AAGGTAATCAAGCAGAAATG | 217 |
| SEQ ID NO: 43: | N6R | ATTGGTAACAGCACTTCC | |
| SEQ ID NO: 44: | N7F | TGACTGTAAGCAATCTACC | 500 |
| SEQ ID NO: 45: | N7R | ACTAACGATGGAATCTTGG | |
| SEQ ID NO: 46: | N8F | AACTTAACTTCTATTCGTGAG | 231 |
| SEQ ID NO: 47: | N8R | GAAACCTTGATAAGCAGTC | |
| SEQ ID NO: 48: | N10F | GATATTGATGTGTATTGC | 478 |
| SEQ ID NO: 49: | N10R | TTATTATCTTCTGTCTCC | |
| SEQ ID NO: 50: | N11F | TGAATACCACATCTCCCATTGAC | 200 |
| SEQ ID NO: 51: | N11R | TTTAACCTGCTGGCGTGAC | |
| SEQ ID NO: 52: | N12F | TTCTCACCGCTACTGTTAC | 227 |
| SEQ ID NO: 53: | N12R | CACTTCTCGCACTAATTCG | |
| SEQ ID NO: 54: | N13F | CCCAAATGATGACCGAAG | 216 |
| SEQ ID NO: 55: | N13R | CTAGTAACTGCTGTTCCTC | |
| SEQ ID NO: 56: | N14F | AAGTGTCCAAAGCCAAAC | 270 |
| SEQ ID NO: 57: | N14R | TCAACAATGCCATCTTCC | |
| SEQ ID NO: 58: | N15F | TTAATTGCCACTGCCTTAC | 293 |
| SEQ ID NO: 59: | N15R | TAGAGACAGCGGATATGC | |
| SEQ ID NO: 60: | N16F | TGATGTGGACGAGGATAC | 335 |
| SEQ ID NO: 61: | N16R | GGGCTAAGGGAATATGGG | |

**Other Primer Sequences**

| Sequence ID Number | Primer name | Sequence 5' → 3'* | Purpose |
|---|---|---|---|
| SEQ ID NO: 62: | Km.KmScFwd | CTGACCCCGGGTGAATGTCAGCTACTGG | Selection cassettes amplification |
| SEQ ID NO: 63: | KmRev | CAAACCCGGGCGATTTACTTTTCGACCTC | |
| SEQ ID NO: 64: | KmScRev | ACAGACCCGGGCAAGCGGATGGCTGATG | |
| SEQ ID NO: 65: | N5.5O** | GCGGCCTCGAGTGGGGGCATCTGCTAGGCAA TATTTG | |

(continued)

| Sequence ID Number | Primer name | Sequence 5' → 3'* | Purpose |
|---|---|---|---|
| SEQ ID NO: 66: | N5.5I | GATTACACCCGGGTGTAATCGGTTGATTATTT CAGTGGCCCGGCCGATGGATAATTAC | Amplification of the DNA fragments flanking the neutral sites |
| SEQ ID NO: 67: | N5.3O** | GTCCAAATCAGCATTGCTCTGCCAGGTGAGAC | |
| SEQ ID NO: 68: | N5.3I | GATTACACCCGGGTGTAATCCTGAAGCCTTGA CAATCCCCGTTGGTGATTTATACTTAG | |
| SEQ ID NO: 69: | N8.5O** | GAACACTCGAGTGCCAGCAACGACAATG | |
| SEQ ID NO: 70: | N8.5I | GATTACACCCGGGTGTAATCCTTCATTCCCCTA TTGTTATTAATAGTGCTC | |
| SEQ ID NO: 71: | N8.3O** | GATGACCGCTGGCGGAGTTTAGTCCAG | |
| SEQ ID NO: 72: | N8.3I | GATTACACCCGGGTGTAATCTTGTCACCAATT TTTGTAGGGATGTTGGGCTAAATTG | |
| SEQ ID NO: 73: | N10.5O** | GATAACTCGAGTCGCCCGGTGGATTTAATGC | |
| SEQ ID NO: 74: | N10.5I | GATTACACCCGGGTGTAATCCCCATACTCAGC CTTCTAATGCTGAGAGAATG | |
| SEQ ID NO: 75: | N10.3O** | GGACTGACCCAAGATACAGTGGTAG | |
| SEQ ID NO: 76: | N10.3I | GATTACACCCGGGTGTAATCCGAGCGCAAACT ACAATGCGCTTCGTTTGC | |
| SEQ ID NO: 77: | N15.5O** | GGTTCTCGAGCCGCTGAATTTAGTCAACATCG | |
| SEQ ID NO: 78: | N15.5I | GATTACACCCGGGTGTAATCCATTGGGCACGA GAGTTAGTAAGGCAGTG | |
| SEQ ID NO: 79: | N15.3O* | CTAAACTTACGG CATTG G CATCAACG G GAG | |
| SEQ ID NO: 80: | N15.3I | GATTACACCCGGGTGTAATCTCTTTACAATGG CCAGGTCTTTAGGGAGCGGTGAC | |
| SEQ ID NO: 81: | N16.5O** | GAACTCGAGTAGTAACCACAGGCTTTTG | |
| SEQ ID NO: 82: | N16.5I | GATTACACCCGGGTGTAATCGCTGGAGGCGA ACTGGGTGAGAACCAT | |
| SEQ ID NO: 83: | N16.3O** | GTGAGCTTGATGGTGATGGTGGGTAAAG | |
| SEQ ID NO: 84: | N16.3I | GATTACACCCGGGTGTAATCTGCTGGCTTTGT TGCCCTGTCAACCAAAGTTC | |
| SEQ ID NO: 85: | N5_SDM | CGAGGCGATCGCCCAGTTGGAAGAATTGGCC | SDM*** |
| SEQ ID NO: 86: | N10_SDM | CTAAAAAGACAAGTCTGTGGCTAGTTACTATG ACGAGGC | SDM*** |

(continued)

| Sequence ID Number | Primer name | Sequence 5' → 3'* | Purpose |
|---|---|---|---|
| SEQ ID NO: 87: | N5.FO | TCCTGGTAACTCACGCTATC | Mutants confirmation by PCR |
| SEQ ID NO: 88: | N5.FI | AGCCGATCCAGGGAAGTGTTGTTG | |
| SEQ ID NO: 89: | N5.RI | CCATCGTCCTTCGCCGTAGATTGTG | |
| SEQ ID NO: 90: | N8.FO | CCCAGTTAAACTGCGAAAGG | |
| SEQ ID NO: 91: | N8.FI | TCGCCAAGCTTTCAGAAC | |
| SEQ ID NO: 92: | N8.RI | CAAACTCCAGCCGATAAC | Mutants confirmation by PCR |
| SEQ ID NO: 93: | N10.FO | CCGGTTGCCCTTATCGGAACCGATG | |
| SEQ ID NO: 94: | N10.FI | GCTATGGCGTCACTTGTAGC | |
| SEQ ID NO: 95: | N10.RI | TTTGCGACCCATCGGATTGC | |
| SEQ ID NO: 96: | N15.FO | CTCCAAGGCGACTACCTTTC | |
| SEQ ID NO: 97: | N15.FI | CCCAGTGGGAATGCGATCAG | |
| SEQ ID NO: 98: | N15.RI | TAGGAGGGCGATCACCGAAG | |
| SEQ ID NO: 99: | N16.FO | ACCCATTTCTTGGGTGTAGG | |
| SEQ ID NO: 100: | N16.FI | GGCCTTGGTTGCCCTGACTGATGTG | |
| SEQ ID NO: 101: | N16.RI | GACCGATCGCCGCAGTAGTTCTTGG | |
| SEQ ID NO: 102: | GFP.F | TCTTGTTGAATTAGATGGTG | RT-PCR/RT-qPCR |
| SEQ ID NO: 103: | GFP.R | TGTGAGTTATAGTTGTATTCC | |
| *Restriction sites underlined<br>"Primers used to confirm genomic integrations and mutants full segregation.<br>***SDM - Site directed mutagenesis | | | |

SEQ ID NO: 104: Nucleotide sequence for BioBrick part BBa_B0030.

SEQ ID NO: 105: Nucleotide sequence for Ptrc1O (promoter, BioBrick part BBa_J153001).

SEQ ID NO: 106: Nucletotide sequence for N5 (sll1476)

SEQ ID NO: 107: - Nucletotide sequence for N8 (slr0573)

SEQ ID NO 108 - Nucleototide sequence for N10 (slr1396)

SEQ ID NO 109 - Nucleototide sequence for N15 (slr0271)

SEQ ID NO 110 - Nucleototide sequence for N16 (slr0397)

SEQ ID No 111 - Nucleototide sequence for genetic construct comprising the upstream and downstream sequences of site N5 (SEQ ID NO 106)

SEQ ID No 112 - Nucleototide sequence for genetic construct comprising the upstream and downstream sequences of site N8 (SEQ ID NO 107)

SEQ ID No 113- Nucleototide sequence for genetic construct comprising the upstream and downstream sequences of site N10 (SEQ ID NO 108)

SEQ ID No 114 - Nucleototide sequence for genetic construct comprising the upstream and downstream sequences of site N15 (SEQ ID NO 109)

SEQ ID No 115 - Nucleototide sequence for genetic construct comprising the upstream and downstream sequences of site N16 (SEQ ID NO 110)

**Detailed description**

*Synechocystis* is a well-studied cyanobacterium with some molecular tools developed for its genetic manipulation. Replicative plasmids have previously been used to introduce foreign DNA into this organism *via* conjugation or electroporation (Matsunaga and Takeyama 1995, Vioque 2007). These plasmids are usually based on the broad-host-range *E. coli* IncQ plasmid RSF1010, and are not specific for cyanobacteria (Scholz, Haring *et al.* 1989, Sode, Tatara *et al.* 1992, Mermet-Bouvier, Cassier-Chauvat *et al.* 1993, Huang, Camsund *et al.* 2010, Taton, Unglaub *et al.* 2014). Although presenting some advantages, such as being relatively easy to construct and quick to introduce in the cell, such broad-host-range replicative plasmids can exhibit some instability (David, Vielma et al. 1983, Priefer, Simon et al. 1985, Becker and Meyer 1997, De Gelder, Ponciano et al. 2007, Meyer 2009). In addition, (*i*) the use of large native DNA fragments may lead to the recombination of the replicative vectors with the chromosome, (*ii*) removal of selective pressure may lead to loss of the vector, and (*iii*) DNA structure of the vectors may have a strong influence on transcription. Altogether these aspects can compromise system predictions and control. In addition, it has been shown that *Synechocystis'* endogenous plasmids copy number per chromosome vary with nutritional conditions and growth phase (Berla and Pakrasi 2012). Taking into consideration that the number of chromosomes also varies throughout growth (Griese, Lange *et al.* 2011), plasmids copy number calculations may become even more complex. Therefore, stable integration of ectopic DNA into *Synechocystis'* chromosome enabled by this disclosure is the most viable alternative for the implementation of synthetic devices into this photoautotrophic chassis.

To date, only a few chromosomal loci have been used to introduce foreign DNA into *Synechocystis* but they have not been extensively characterized. The locus described by Williams and co-workers (Williams 1988) disrupts the gene *slr0168* that encodes a protein secreted by *Synechocystis* (Sergeyenko and Los 2000). Although its function is unknown, Slr0168 possesses a DUF4114 protein domain, found towards the C-terminal of various bacterial proteins, and suggested to carry enzymatic activity (Marchler-Bauer, Anderson *et al.* 2009). Burnap and co-workers (Burnap, Qian *et al.* 1994) describe a locus in a "nonessential, nontranscribed" region. However, recent RNA-seq works (Mitschke, Georg *et al.* 2011, Kopf, Klähn *et al.* 2014) show that the genes flanking that insertion locus are transcribed. The locus described by Aoki and co-workers (Aoki, Kondo *et al.* 1995) disrupts the *ssl0410* gene, which is located downstream of the gene encoding a subunit of the type I NAD(P)H dehydrogenase complex (*ndhB*) that is important for $CO_2$ uptake in *Synechocystis* (Ohkawa, Price *et al.* 2000). The other locus described by Aoki and co-workers (Aoki, Goto *et al.* 2011) is located in the intergenic region between *slr2030* and *slr2031,* which encodes the regulatory protein RsbU. The latter gene is constitutively transcribed and the respective mutant showed a defect in growth recovery after nitrogen- and sulfur-starvation-induced stationary phase (Huckauf, Nomura *et al.* 2000). Furthermore, *slr2030* and *slr2031* are transcribed as an operon (Huckauf, Nomura *et al.* 2000, Mitschke, Georg *et al.* 2011, Kopf, Klähn *et al.* 2014). Consequently, insertions in that intergenic region may disrupt the transcriptional unit and lead to the same phenotype described for *slr2031* mutants. None of these previously described integration sites are considered to be suitable for use of *Synechocystis* as an effective bioproduction system. Alternative and more advantageous integration loci are employed in this disclosure, described herein.

The method of transforming *Synechocystis* without deleterious effects on cell growth and viability has been enabled by the discovery of five neutral sites into which DNA can be stably integrated into the *Synechocystis* chromosome. In selecting a neutral site, the obvious choice would have been to choose non-coding DNA (ncDNA) regions, however it was further considered that these may possess *cis*-regulatory control sequences, which could affect the transcription profiles of the inserted genes. Therefore Open Reading Frames (ORF) that putatively encode unknown or hypothetical proteins were selected. A total of 16 candidate neutral sites were initially selected, with 13 of these selected for further transcriptional studies.

The cyanobacteria (also known as blue-green algae) in this disclosure preferably comprise a group of prokaryotic microorganisms capable of performing plant type oxygenic photosynthesis. Examples of cyanobacteria include transformable strains of the following strains: *Synechocystis* spp., *Synechococcus* spp., *Microcystis aeruginosa, Prochlorococcus marinus* and *Nostoc* spp.

The use of cyanobacteria for bioproduction may have the following advantages: (1) cyanobacteria are capable of absorbing solar energy and fixing carbon dioxide as carbon source for autotrophic growth, thereby having low cost for culturing; (2) cyanobacteria are ancient microorganisms and have lived on the earth for billions of years, so that they have remarkable adaptability to the environments, and they grow quickly compared to other autotrophic organisms; (3) cyanobacteria are convenient for genetic manipulations, because their genetic background is clear and genomic sequencing of many species of cyanobacteria has been completed which facilitates the genetic engineering of cyanobacteria. *Synechocystis* sp. PCC 6803 is a preferred unicellular cyanobacteria, because for *Synechocystis* sp. PCC 6803 the whole genome has been sequenced, plenty of molecular tools and genome-scale metabolic models are available.

The embodiments of the disclosure provide for a method of transforming *Synechocystis* that can be used to efficiently biomanufacture or bioproduce a range of chemical products. Biomanufacturing includes a whole range of industrial sectors and products that are produced using microbes; including the bioprocessing of proteins and peptides for therapeutic uses, biorefining to convert biomass into fuels, bioremediation to treat environmental waste and the use of microbial fermentation to produce commodity and specialty chemicals.

Further embodiments of the disclosure provide for a method of transforming *Synechocystis* that can be used to bioproduce 'drop-in' chemicals that can be used as direct replacements for petrochemical-based feedstocks. Such products include bioisoprene, a microbially-produced version of isoprene which is used predominantly in the tyre industry as a replacement for rubber. Isoprene is a petrochemical, usually produced as a by-product from the cracking of naphtha (a crude oil product) to produce ethylene or via C4 refinery stream synthesis. However, this requires a lot of crude oil and a greater move towards use of shale gas (ethane) for ethylene production means that the required by-products are no longer produced, resulting in a shortage of isoprene. The high costs and shortages of isoprene have motivated the tyre industry to develop ways to produce isoprene using different feedstocks e.g. biologically-produced isoprene using C5 sugars from renewable sources such as sugar cane or corn. Microbial production in this industry sector has solved the problem of reduced supplies and wildly fluctuating prices of a feedstock. Use of *Synechocystis* would provide for a more cost-effective means to bioproduce isoprene.

Another embodiment of the disclosure provides for a method of transforming *Synechocystis* that can be used to produce biofuels. Production of biofuels on a commercially viable scale has been fraught with difficulties. Examples include farnesene which is microbially-produced from sugar cane and which is converted to farnesane for use as jet-fuel. The major challenge faced by manufacturers has been development of a process that is cost-effective to scale up. Other examples include 'drop-in' fuels such as isobutanol which is used to replace ethanol as it contains more energy per equivalent volume than ethanol and it is less corrosive to pipelines. Currently, isobutanol and ethanol are mostly produced using yeast. Use of *Synechocystis* would provide for a more cost-effective means to bioproduce biofuels such as farnesene, isobutanol and ethanol.

A further embodiment of the disclosure provides for a method of transforming *Synechocystis* that can be used to produce specialty chemicals. Specialty chemicals are high value-added chemicals used in the manufacture of a wide variety of products, including but not limited to fine chemicals, additives, advanced polymers, adhesives, sealants and specialty paints, pigments and coatings. The main difference between specialty chemicals and commodity chemicals is that specialty chemicals are produced on a batch-to-batch basis, typically driven by specific demands from end-users.

Another embodiment of the disclosure provides for a method of transforming *Synechocystis* that can be used to produce phycocyanin. Phycocyanin is a cyanobacterial blue pigment and antioxidant that has applications in biotechnology (e.g. as a reporter dye) and in medicines and foods (as a colouring agent and antioxidant). Phycocyanin is currently commercially produced for use as a food dye in blue-coloured products like ice cream, confectionary and drinks. Phycocyanin is currently produced in *Spirulina plantensis,* another cyanobacterium.

A further embodiment of the disclosure provides for a method of transforming *Synechocystis* that can be used to produce ectoine and hydroxyectoine. Ectoine and hydroxyectoine are compatible solutes which help to protect extremophillic bacteria in high salt conditions, acting as osmolytes. Ectoine is used as a cosmetic ingredient as it it highly UV-protective and prevents skin dryness. However, ectoine cannot be chemically synthesised cost-effectively and at present is produced in halophillic bacteria e.g. *Halomonas elongata.*

Use of *Synechocystis* would potentially provide for the ability to increase the yield of bioproduced specialty chemicals.

Selection of neutral sites

In an embodiment, the selection of neutral sites was carried out as follows. To identify putative neutral sites in the chromosome of *Synechocystis,* the list of annotated open reading frames (ORFs) with the respective encoded proteins was retrieved from CyanoBase (http://genome.kazusa.or.jp/cyanobase). The list of the predicted/annotated ORFs of *Synechocystis* was retrieved from CyanoBase (Distribution of Sequence and Annotated Data Files, ftp://ftp.kazusa.or.jp/pub/CyanoBase/Synechocystis) (Nakamura, Kaneko et al. 1998, Nakamura, Kaneko et al. 2000). From the 3264 ORFs listed, only those putatively encoding unknown or hypothetical proteins were selected. From these, the final selection of 1532 ORFs was based on the following criteria: (*i*) length of the putatively encoded proteins ≤301 amino acids, (*ii*) encoded proteins with no transmembrane domains predicted by the TMHMM Server v. 2.0 (http://www.cbs.dtu.dk/services/TMHMM/), (*iii*) no interaction with other proteins as assessed by the two-hybrid system (CyanoBase data), and (*iv*) no relevant similarities found at the protein sequence level, when comparing with other sequences using BLASTP (http://blast.ncbi.nlm.nih.gov/Blast.cgi). The genomic context of the ORFs was also taken into consideration and sites were disregarded when in the vicinity of genes with assigned putative functions.

This analysis led to the selection of 16 candidate neutral sites - termed N1 to N16 **(Fig. 1** and **Fig 7).** Subsequently, a qualitative analysis of the genomic context of these ORFs was performed **(Fig. 1b)** and revealed that the N2 site superimposes the 5' region of an ORF (*slr0369*) putatively encoding a cation or drug efflux system protein, the N4 site overlaps the 5' region of an ORF (*slr0415*) putatively encoding a $Na^+/H^+$ antiporter, and the N9 site corresponds to the ORF located immediately upstream and in the same direction as an ORF (*sll0182*) putatively encoding an ABC-type transporter. Therefore, these 3 sites were disregarded, and transcription analyses were performed for the other 13 loci.

In an embodiment, the transcriptional analysis was carried out as follows. To assess transcription of the selected ORFs, primers were designed automatically from the genomic sequence (Kaneko, Sato et al. 1996) using the Beacon Designer 6 software (PREMIER Biosoft International). For other purposes, primers were designed manually and analyzed using the Integrated DNA Technologies web resource OligoAnalyzer v3.1 (http://eu.idtdna.com/analyzer/Applications/OligoAnalyzer/).

In an embodiment, to assess transcript levels of the identified loci, RT-PCRs were initially performed using RNA extracted from *Synechocystis* cells collected at the exponential growth phase ($OD_{730}$ of 0.8-0.9) under continuous light at 30 °C. Further transcriptional studies were performed with RNA extracted from samples collected in three different growth phases ($OD_{730}$ of approximately 0.4, 2 and 9, see **Fig. 2a**), from three independent cultures grown under the same conditions. Wild-type and mutants of the cyanobacterium *Synechocystis* sp. PCC 6803 were maintained in BG11 medium (Stanier, Kunisawa et al. 1971) at 25 °C, under a 16 h light /8 h dark regimen. Light intensity was 20 μmol photons/$m^2$/s in all experiments. For solid medium, BG11 was supplemented with 1.5% (w/v) agar noble (Difco), 0.3% (w/v) sodium thiosulfate and 10 mM TES-KOH buffer, pH 8.2. For the selection of mutants, BG11 medium was supplemented with kanamycin (Km, 10-600 μg/mL). For cloning purposes *Escherichia coli* strain DH5α (Stratagene) was used. *E. coli* cells were grown at 37 °C on LB medium (Sambrook and Russell 2001), supplemented with ampicillin (100 μg/mL) or kanamycin (50 μg/mL).

In an embodiment, the cyanobacterial genomic DNA (gDNA) extraction was carried out according to the procedure described previously (Tamagnini, Troshina et al. 1997). For RNA extraction *Synechocystis* cells were collected by centrifugation (10 min at 3850 g), and frozen at -80 °C. RNA was extracted using the TRIzol® Reagent (Invitrogen) according to the method described previously (Leitão, Pereira et al. 2006).

In an embodiment, RT-PCRs were carried out as described elsewhere (Ferreira, Pinto et al. 2009). Briefly, cDNA was synthesized in duplicate from 1 μg of total RNA using the iScript™ Select cDNA Synthesis Kit (Bio-Rad) and the random primers supplied, following the manufacturer's instructions. The two RT reactions from each sample were pooled together and each PCR reaction contained 1.5 μL of cDNA, 0.1 μM of each primer (see **Fig. 9**), 200 μM dNTPs mix, 1X PCR reaction buffer, and 0.025 U of illustra™ *Taq* DNA polymerase (GE Healthcare). The PCR profile was: 2 min at 94 °C followed by 40 cycles of 30 s at 94 °C, 30 s at 50 °C, and 30 s at 72 °C, concluding with a 7 min extension step at 72 °C. RNA contamination by gDNA prior to cDNA synthesis was assessed by PCR using primers specific for the *Synechocystis* 16S rDNA, and the RT reaction efficiency was assessed using the same primers pair. All the RT-PCRs included a negative control (omission of template) and a positive control (*Synechocystis* gDNA).

In an embodiment, PCRs to amplify the regions flanking the target loci contained 7 ng of gDNA, 0.2 μM of each primer, 250 μM dNTPs mix, 1X *Pfu* reaction buffer with $MgSO_4$, and 0.025 U of *Pfu* DNA polymerase (Fermentas). The PCR profile was: 1 min at 94 °C followed by 35 cycles of 30 s at 94 °C, 45 s at 48 °C, and 90 s at 72 °C, concluding with a 7 min extension step at 72 °C. Agarose gel electrophoresis was performed by standard protocols using 1× TAE buffer (Sambrook and Russell 2001), DNA fragments were purified using the GFX™ PCR DNA and Gel Band Purification Kit (GE Healthcare), according to the manufacturer's instructions. "Overlap-PCRs" (see below) contained 80 ng of each purified DNA fragment, 0.125 μM of each primer, 250 μM dNTPs mix, 1X PCR reaction buffer, and 0.025 U of illustra™ *Taq* DNA polymerase (GE Healthcare). The PCR profile was: 5 min at 95 °C followed by 10 cycles of 30 s at 95 °C, 45 s at 48 °C, and 90 s at 72 °C, and 30 cycles of 30 s at 95 °C, 45 s at 55 °C, and 90 s at 72 °C, concluding with a 7 min extension step at 72 °C. The oligonucleotide primers used to amplify the homologous regions are listed in **Fig. 10.**

The results revealed high transcript levels for N3, N11, N12 and N13 sites, low transcript levels for N6, N8, N14 and N15 sites, low primer efficiency for N1 site, and no detectable amplification for the remaining sites (N5, N7, N10 and N16) **(Fig. 8).** Further transcriptional studies were performed collecting cells at different phases (early exponential, exponential and stationary), to evaluate a possible differential transcription of sites N5, N6, N7, N8, N10, N14, N15 and N16 **(Fig. 2).** The results showed that there was residual transcription for N6 and N14 sites throughout the various growth phases and, therefore, these two sites were disregarded. Concerning the other sites, it was possible to detect residual transcription for N8 and N15 sites, but only on samples collected at the early exponential phase. Thus, six sites (N5, N7, N8, N10, N15 and N16) were selected to generate mutants in order to validate their "neutrality". Subsequent cloning difficulties in constructing N7-related plasmids resulted in this site also being disregarded. The final five target sites (N5, N8, N10, N15 and N16) are evenly distributed throughout the chromosome, with intervals in the order of 60-90º (72º corresponds to 1/5 of the chromosome, see **Fig. 1a**).

Construction of integrative plasmids

In an embodiment, the construction of integrative plasmids was carried out as follows. The integrative plasmids used to transform *Synechocystis* and characterize the neutral sites **(Fig. 11** and **Fig. 12)** were constructed following the design represented in **Fig. 13.** Briefly, the chromosomal regions up and downstream of the target loci **(Fig. 11)** were amplified by PCR using the *Pfu* polymerase and primers containing tags with restriction sites for cloning purposes (N*n*.5O/N*n*.5I and Nn.3O/N*n*.3I, **Fig. 10).** The corresponding up and downstream fragments of each locus were fused by "overlap-PCR", using the *Taq* polymerase (see above). The resulting products were cloned into the pGEM®-T easy vector (Promega), according to the manufacturer's instructions, originating the pSN*n** plasmids series (n for 'neutral site number'). The pGEM®-T easy vector restriction sites incompatible with the BioBrick RFC [10] standard were eliminated by digesting the vector with *Xho*I and *Sal*I and recircularizing the vector (originating the pSN*n*** series). The BioBrick RFC[10] standard incompatible restriction sites within the amplified chromosomal regions of pSN5** and pSN10** were eliminated using the QuikChange® Multi Site-Directed Mutagenesis Kit (Stratagene), according to the manufacturer's instructions, using the primers N5_SDM and N10_SDM, respectively **(Fig. 10),** originating the pSN5*** and the pSN10*** plasmids. A BioBrick-compatible cloning interface was synthesized (Epoch Life Science Inc.), containing the *Mun*I*, Xba*I*, Not*I*, Spe*I and *Pst*I restriction sites flanked by two double transcription terminators (based on the registry parts BBa_B1006, BBa_B0062, BBa_B0053 and BBa_B0020). This interface was excised from the delivery vector (pBSK-FP300) using *Xma*I and *Age*I and subsequently cloned into the *Xma*I site of the pSN*n*** or pSN*n**** plasmids, originating the pSNn plasmid series (see **Fig. 13**). Afterwards, two selection cassettes, one containing the *nptII* gene (encoding a neomycin phosphotransferase that confers resistance to neomycin and kanamycin) and the *sacB* gene (encoding a levansucrase that confers sucrose sensitivity), and another containing only the *nptII* gene, were amplified by PCR from the plasmid pK18mobsacB (Schäfer, Tauch et al. 1994), using the primer pairs Km.KmScFwd/KmScRev and Km.KmScFwd/KmRev, respectively **(Fig. 10).** These selection cassettes were cloned into the pGEM-T easy vector, excised with *Xma*I and cloned into the *Xma*I site of the pSN*n* plasmid series. The final plasmid series containing only the Km-resistance cassette was named pSN*n*K and the plasmid series containing the cassette with the two selective markers was named pSN*n*KS. Additionally, based on previous works (Eriksson, Salih et al. 2000, Shibato, Agrawal et al. 2002, Agrawal, Asayama et al. 2003) the minimal promoter sequence required for *Synechocystis psbA2* basal transcription (-38 to +14 bp; +1 corresponding to the transcription start site) was synthesized ($P_{psbA2*}$), meeting the BioBrick RFC[10] standard specifications (DNA 2.0 Inc.). This promoter region was cloned into the BioBrick vector pSB1A2 containing the part BBa_E0240 (composed by the RBS part BBa_B0032, the GFP-coding gene part BBa_E0040 and the double transcription terminator part BBa_B0015), according to the BioBrick standard cloning procedure. The BioBrick composite part BBa_E0240 (promoterless GFP generator) and the part with the synthetic constitutive promoter $P_{psbA2*}$ were digested with *Xba*I and *Spe*I and cloned into the *Xba*I site of both pSNn and pSNnK series, originating the pSNnK.gfp and pSNnK.Cgfp plasmid series (C for 'constitutive promoter'). These plasmids were used to assess proper cloning interface transcriptional insulation (promoterless *gfp*) and functionality of the neutral sites (*gfp* under the control of the constitutive promoter). The vectors were named as follows: p for 'plasmid', S for *'Synechocystis',* N for 'neutral site', n for the 'number of the neutral site' (5, 8, 10, 15 or 16), K for 'kanamycin resistance cassette', KS for the 'double selection cassette ($Km^R/Suc^S$)', C for 'constitutive $P_{psbA2*}$ promoter', and gfp for the 'GFP generator BioBrick part BBa_E0240'. Plasmids DNA was isolated from overnight grown *E. coli* cultures, using the GenElute™ Plasmid Miniprep Kit (Sigma-Aldrich), according to the manufacturer's instructions. All the BioBrick parts can be found at the Registry of Standard Biological Parts website (http://partsregistry.org/).

Generation of mutants

In an embodiment, the generation of mutants was carried out as follows. *Synechocystis* was transformed based on the procedure described previously (Williams 1988). Briefly, *Synechocystis* was grown in standard conditions to an $OD_{730}\approx0.5$. Cells were harvested by centrifugation (10 min at 3850 g) and resuspended in BG11 to a final $OD_{730}\approx2.5$. The purified plasmids were incubated for 5 h with 500 μL of those cells (final plasmid DNA concentration of 20 μg/mL), in light and at 25 °C. Cells were then spread onto Immobilon'"-NC membranes (0.45 μm pore size, 82 mm, Millipore) resting on solid BG11 plates, grown in standard growth conditions, and transferred to selective plates after 24 h (Km, 10 μg/mL). Transformants were observed after 1-2 weeks. For complete segregation, Km-resistant colonies were grown at increasing Km concentrations (25 and 50 μg/mL) and finally transferred into liquid medium. The mutant full segregation was confirmed by PCR, using primers within and external to each site (NnF/NnR or *Nn*.5O/N*n*.3O, respectively; see **Fig. 10),** and by Southern blot with probes labeled with digoxigenin using the DIG DNA labeling kit (Roche Molecular Biochemicals) and following the manufacturer's instructions. The probes were obtained by labeling the PCR-amplified 3' homologous regions for mutants in sites N5, N8, N10 and N16, and the 5' homologous region for mutants in site N15 (see **Fig. 10**). gDNA digestions were performed with *Mun*I*,* with the exception of N8 mutants for which the gDNA was digested with *Spe*I. When necessary antibiotic concentration was increased up to 600 μg/mL to fully segregate the mutants.

To assess the neutrality of the sites, *Synechocystis* mutants were produced exchanging each putative neutral site by a kanamycin resistance cassette, using the pSNnK plasmid series **(Fig. 11** and **Fig. 12).** The successful generation of fully segregated mutants in the five sites showed that the mutations did not affect cell viability under standard growth conditions. The growth of the wild-type and the five mutants was evaluated in standard growth conditions for *Synechocystis*:

For the growth experiments, 50 mL of BG11 medium were inoculated with *Synechocystis* wild-type or SN*n*K mutants to $OD_{730}\approx0.3$. The cultures were grown to $OD_{730}\approx1.0$, under the experimental light regimen of continuous light (20 μmol photons/$m^2$/s; CL) or 12h light (20 μmol photons/$m^2$/s)/12h dark cycles (LD), in autotrophy (no glucose; G0) or mixotrophy (5 mM glucose; G5), and at a constant temperature of 30 °C. The optical density of the cultures ($OD_{730}$) was monitored daily for ten consecutive days and the resulting growth curves for each organism and cultivation conditions are depicted in **Fig. 3** and subsequently diluted to a final $OD_{730}\approx0.1$ in fresh BG11. 10 mL of each diluted culture was transferred to sterile 25 mL borosilicate glass Erlenmeyer-flasks, closed with cellulose stopper, and when necessary, glucose was added to a final concentration of 5 mM. The flasks were incubated at constant temperature (30 °C) and the indicated light regimens. All experiments were performed in triplicate and the $OD_{730}$ of the cultures was recorded daily for ten consecutive days.

In an embodiment, the analysis of growth was carried out as follows. Statistical analysis of variance (ANOVA) of the growth data was performed to compare the wild-type and the mutants, using the specific growth rate. The use of this parameter is reliable since the test duration was the same in all conditions and stationary growth was reached at similar moments. The quantitative analysis of *Synechocystis* wild-type and neutral sites mutant growth in various conditions was based on the OD measurements (see above). For each bin, *i*, a specific growth parameter, representing the exponent of exponential growth within the given time interval was calculated as:

$$\mu_i = \frac{log[x_{i+1}/x_i]}{t_{i+1} - t_i}$$

where $t_i$ is time, in units of days running from day 0 to $t_{imax}$ and $x_i$ is the corresponding measured OD. As test duration was 10 days in all strains and stationary growth was reached at similar moments, the specific growth rate of all the exponential phase of each replica of each organism was considered to be a reliable parameter to assess growth patterns. ANOVA statistical analysis of the growth data was performed to compare the wild-type to each mutant organism, using the software Wolfram's Mathematica© This ANOVA was three-way where a model with all the effects and interactions up to the third order was tested. Values of up to second order interactions can be studied in **Fig. 14.** Mutants SN5K, SN8K showed highly significant *P*-values compared to the wild-type, while for SN10K the *P*-value is approximately 0.04, and for SN15K and SN16K the *P*-values are not statistically significant **(Fig. 14).** Analysis of the growth data has shown that mutant SN5K grows slower than the wild-type (particularly in the presence of glucose), while mutant SN8K grows faster under continuous light (with or without glucose). Taking these results into consideration, the chromosomal loci neutrality may be qualitatively ordered as N15≈N16>N10>N8>>N5, from the most to the least similar to that of the wild-type. For the remaining variables considered in the ANOVA, irradiance and glucose presence were the major parameters affecting growth (highly significant *P*-values).

In an embodiment, the synthetic interface robustness and functionality of neutral sites was analysed. To use the constructed vectors as tools for the integration of synthetic devices, a BioBrick-compatible cloning interface was designed and synthesized. A DNA sequence containing the *Mun*I, *Not*I, *Xba*I, *Spe*I and *Pst*I restriction sites, compatible with the RFC[10] assembly standard, were flanked by two double transcription terminators (BioBricks BBa_B1006 and BBa_B0062 upstream, and BBa_B0053 and BBa_B0020 downstream), to prevent any potential promoter activity from backbone DNA sequences. The interface was synthesized and cloned between the two homologous recombination sequences of each vector. To evaluate the proper transcriptional insulation of the interface, a promoterless green fluorescent protein generator (GFP; BioBrick composite part BBa_E0240) was cloned in the pSNnK plasmid series, and *Synechocystis* mutants were produced (mutants SN*n*K.gfp, see **Fig. 11** and **Fig. 15).** Additionally, to assess the functionality of the neutral sites, *Synechocystis* mutants carrying the GFP generator under the control of a constitutive synthetic promoter (minimal *psbA2* promoter, $P_{psbA2*}$) were constructed (mutants SNnK.Cgfp, see **Fig. 11** and **Fig. 15**). *gfp* transcription was assessed by RT-PCR **(Fig. 16a).**

In an embodiment, for total RNA extraction 400 mL of BG11 medium were inoculated to an $OD_{730}{\approx}0.2$ with pre-cultures of *Synechocystis* wild-type and mutants harboring the *gfp* gene (SNnK.gfp and SN*n*K.Cgfp mutants series). Three independent biological replicates of each strain were grown to an $OD_{730}{\approx}1$, at 28 °C in glass gas washing bottles with aeration, under continuous light. Cells from 100 mL of culture were collected by centrifugation (10 min at 5,000 rpm), pellets were treated with RNAprotect Bacteria Reagent (Qiagen) and stored at -80 °C. Total RNA was extracted as described previously (Pinto, Pacheco et al. 2012) and stored at -80 °C until further analysis. RNA quality and contamination verifications, cDNA synthesis, RT-PCR and RT-qPCR experiments were performed as described in Pinto and colleagues (Pinto, Pacheco et al. 2012) using primers listed in **Fig. 10.** The genes 16S, *petB,* and *rnpB* were amplified using the primers described elsewhere (Pinto, Pacheco et al. 2012) and used as reference genes for data normalization. All RT-qPCR parameters in these experiments were in agreement with the MIQE (minimum information for publication of quantitative real-time PCR experiments) guidelines (Bustin, Benes et al. 2009). RT-qPCR data from 3 biological replicates and 3 technical replicates were analyzed using qbase$^+$ v2.6 (Biogazelle) and the statistical analysis was performed by means of a one-way ANOVA, using GraphPad Prism v6 (GraphPad software Inc.).

In an embodiment, the GFP expression was analyzed by Western blot **(Fig. 16b),** confocal microscopy **(Fig. 4a)** and fluorimetry **(Fig. 4b** and **Fig. 17).**

In an embodiment, a Western blot was carried out as follows. For the Western blot, 20 mL of the same cultures used for *gfp* transcription analyses (by RT-PCR and RT-qPCR) were collected by centrifugation (10 min at 5,000 rpm) and stored at -80 °C until further use. Cells were resuspended in lysis buffer (10 mM HEPES pH 8.0, 10 mM EDTA pH 8.0, 0.5% (v/v) Triton X-100, 2 mM DTT) supplemented with a cocktail of protease inhibitors (cOmplete Mini, EDTA free, Roche) and disrupted by sonication on ice with a Branson Sonifier 250 using 5 cycles of 15 s (50% dutty cycle, output 3) intercalated with 1 min off duty. Cell debris was separated by two rounds of 10 min centrifugation at 10,000 *g* and 4 °C. Protein extracts were stored at -20 °C until further analysis. Protein quantification was performed using the BCA Protein Assay Kit (Thermo Fisher Scientific Inc.) and 15 μg of each extract was separated by electrophoresis on 12% (w/v) SDS-polyacrylamide gels. Protein extracts were visualized with colloidal Coomassie blue staining (Sigma) or transferred to a Hybond™-C extra nitrocellulose membrane (GE Healthcare). After the transfer, membranes were blocked for 3 h with 5% (w/v) milk powder in TTBS (Tris buffered saline (TBS) with 0.05% (v/v) Tween 20) and then incubated overnight at 4 °C with monoclonal mouse anti-GFP (Roche) in fresh blocking buffer. After washing with TTBS, blots were incubated for 1 h with goat-anti-mouse IgG (Invitrogen) linked to horseradish peroxidase. Membranes were washed with TTBS before immunodetection, which was performed by chemiluminescence using ECL Western Blotting Analysis System detection reagents.

In an embodiment, confocal microscopy was carried out as follows. 10 mL of BG11 medium were inoculated with *Synechocystis* wild-type or mutants harboring the *gfp* gene (SN*n*K.gfp and SN*n*K.Cgfp mutants series) to $OD_{730}{\approx}0.15$. The cultures were grown to $OD_{730}{\approx}1.0$, under continuous light at 30 °C and subsequently diluted to a final $OD_{730}{\approx}0.1$ in fresh BG11. 10 mL of each diluted culture were incubated under the same light and temperature conditions as above and grown until $OD_{730}{\approx}1.0$. 200 μL of each culture were collected by centrifugation, resuspended in 20 μL of fresh BG11 and 40 μL of 1% (w/v) low-melting-point agarose dissolved in BG11 medium were added. 10 μL of each mixture were immediately loaded on a glass slide and covered with a coverslip. The GFP emission (collected between 500 nm and 540 nm) was observed when the cells were exposed to a laser beam at 488 nm, using a Leica TCS SP5 II confocal microscope (Leica Microsystems). Cyanobacterial autofluorescence was collected between 640nm and 680 nm after excitation at 633 nm. Images were analyzed with the LAS AF Lite v3.3 software (Leica Microsystems). GFP fluorescence measurements were also performed for these samples as described below.

In an embodiment, GFP fluorescence measurements were carried out as follows. 50 mL of BG11 medium were inoculated with *Synechocystis* wild-type or mutants harboring the *gfp* gene (SN*n*K.gfp and SNnK.Cgfp mutants series) to $OD_{730}{\approx}0.1$. The cultures were grown to $OD_{730}{\approx}2.5$, under continuous light at 30 ºC and subsequently diluted to a final $OD_{730}{\approx}0.2$ in fresh BG11. 10 mL of each diluted culture were transferred to three sterile 25 mL borosilicate glass flasks and closed with cellulose stoppers. The flasks were incubated under the same light and temperature conditions and samples for total fluorescence measurements were collected after 2, 4, 7, 9 and 11 days after inoculation. For fluorescence measurements, three aliquots of 200 μL of each biological replicate were distributed in a Nunc™ MicroWell™ 96-Well Optical-Bottom Plates (Thermo Fisher Scientific Inc.) and GFP fluorescence and $OD_{790}$ were detected using the Synergy 2 Multi-Mode Microplate Reader and the Gen5™ software (BioTek Instruments, Inc.). For fluorescence detection an excitation filter of 485/20 nm and an emission filter of 528/20 nm were used (sensibility set for 110 nm). $OD_{790}$ was measured using an absorbance filter of 790 nm. Wells with BG11 were included for background fluorescence measurement and plate readings were carried out in duplicate. Absolute fluorescence values were normalized to the background values of the BG11 medium and the wild-type was used as fluorescence baseline. Mean GFP fluorescence per cell was normalized to the $OD_{790}$ and is expressed in arbitrary units

In all cases, *gfp* transcripts and GFP levels could only be detected in mutants when the promoter $P_{psbA2*}$ is present (SNnK.Cgfp); in these mutants GFP fluorescence is relatively constant over time (11 days). Moreover, to determine whether the same $P_{psbA2*}$ promoter-*gfp* construction is equivalent in terms of transcription strength at the different sites (chromosomal three-dimensional structure can affect promoter strength) RT-qPCR was performed using RNA extracted from the SNnK.Cgfp mutants (wild-type was used as negative control). All RT-qPCR parameters in these experiments were in agreement with the minimum information for publication of quantitative real-time PCR experiments, MIQE guidelines (Bustin, Benes et al. 2009). **Fig. 5** shows the normalized fold expression of *gfp* in the mutants and there are no statistically significant differences between the five sites, as calculated by one-way ANOVA.

In the present disclosure, a RT-PCR transcription study was performed for the five selected neutral sites throughout growth, and in the *Synechocystis,* in particular *Synechocystis* spp. 6803 strain the transcription levels appeared to be negligible. Moreover, the successful generation of fully segregated mutants, with each selected neutral site replaced by a kanamycin resistance cassette, showed that they do not encode proteins essential to the viability of the cyanobacterium under the growth conditions tested. Several studies reported that genes crucial to the viability of *Synechocystis* cannot be successfully and completely inactivated, resulting in heteroploid mutant cells, containing both chromosomes harboring the wild-type gene and the knockout locus (García-Domínguez, Reyes et al. 2000, Domain, Houot et al. 2004, Ishii and Hihara 2008, Oliveira and Lindblad 2008). Moreover, the neutrality of each site was assessed by analyzing the growth of mutants SN5K, SN8K, SN10K, SN15K and SN16K cultivated under different conditions. The results showed that the N15 and N16 mutants exhibit growth patterns similar to those of the wild-type, while the N5, N8 and N10 site mutants present statistically significant differences. Nonetheless, the mutant on site N8 exhibited growth rates slightly higher than the parental strain under continuous light, a feature that may be advantageous for biotechnological applications. As for the remaining variables considered in the ANOVA, and similarly to the results obtained by Lopo and co-workers (Lopo, Montagud et al. 2012), irradiance and the presence of glucose were the major parameters affecting growth (highly significant *P*-values).

All the integrative plasmids constructed in this disclosure possess a BioBrick-compatible synthetic interface for cloning purposes. The robustness of this synthetic interface was assessed using the promoterless GFP generator (BioBrick BBa_E0240), which showed no leakiness from the chromosomal backbone for each site, since no/negligible levels of *gfp* transcripts were detected by RT-PCR, and no GFP expression was observed by Western blot, confocal microscopy or whole cell fluorescence measurements. In contrast, using the same techniques, *gfp* transcripts or GFP signal could be detected in all the mutants generated bearing the GFP generator under the control of a constitutive synthetic promoter (based on the photosensitive promoter of the *psbA2* gene of *Synechocystis*). This shows that these five sites can effectively be used for the functional integration of synthetic devices. Moreover, the relative quantification of transcripts by RT-qPCR showed that *gfp* transcription is not affected by the position of the neutral site in the chromosome and consequently the five sites may be considered equivalent.

Altogether, these results show that the selected chromosomal sites are functional and suitable to receive ectopic DNA. In addition, the vectors constructed in this disclosure followed a rational design that allows the stable integration of synthetic devices into *Synechocystis.* Moreover, the possibility to generate markerless mutants enables the use of these vectors to introduce more than one synthetic device, and the existence of more than one integration site, spread throughout the chromosome of *Synechocystis,* allows the implementation of complex synthetic circuits. The vectors design also considered the possibility to use different resistance cassettes making the implementation faster. Finally, this disclosure contributes to the field of synthetic biology by allowing the use of *Synechocystis* as a photoautotrophic chassis, providing not only standard BioBrick-compatible vectors to integrate synthetic devices into fully characterized chromosomal neutral sites, but also chassis prone to receive a synthetic device without selective markers.

## DESCRIPTION OF THE PREFERRED EMBODIMENTS

The preferred embodiment comprises an improved method of transforming *Synechocystis* by integrating DNA at newly identified neutral sites (*n*; 5, 8, 10, 15 and 16) within the *Synechocystis* chromosome. The integration of DNA at these 5 neutral sites does not have a deleterious effect on growth of the *Synechocystis* and the inserted genes are insulated from transcriptional control by other promoters and enhancers.

In an embodiment, to transform the *Synechocystis* series of plasmids (pSN*n*; SEQ ID NO: 1:, SEQ ID NO: 2:, SEQ ID NO: 3:, SEQ ID NO: 4:, and SEQ ID NO: 5:) have been generated based on the delivery vector pGEM-T easy and containing the BioBrick compatible interface (FP300) flanked by the two regions for double homologous recombination at the five newly identified neutral sites (*n*).

In an embodiment, pGEM®-T easy (SEQ ID NO: 16:) is a commercially available plasmid (Promega) used for the T/A cloning of the PCR amplified recombination sequences. This is the delivery suicidal vector for all the plasmids used for transformation of *Synechocystis* to integrate DNA at the neutral sites. FP300 (SEQ ID NO: 26) is the synthetic interface used to insulate the multiple cloning site where ectopic DNA should be cloned for insertion in *Synechocystis* chromosome at the neutral sites. This interface contains a BioBrick compatible multiple cloning site, with recognition sequences for the restriction enzymes *Mun*I, *Not*I, *Xba*I, *Spe*I and *Pst*I, and is insulated with the BioBrick transcriptional terminators BBa_B1006 and BBa_B0062 (upstream), and BBa_B0053 and BBa_B020 (downstream). The interface was synthesized by Epoch Life Science Inc. and pBSK is the delivery vector.

Optionally, *Synechocystis* mutants that are resistant to kanamycin can be produced using a series of plasmids that have a kanamycin resistance cassette (Km; SEQ ID NO: 17:) inserted at the neutral sites (pSN*n*K; SEQ ID NO: 6:, SEQ ID NO: 7:, SEQ ID NO: 8:, SEQ ID NO: 9:, and SEQ ID NO: 10:,). The kanamycin resistance plasmids were generated by cloning the PCR-amplified kanamycin resistance cassette, from the plasmid pK18mobsacB, upstream of the BioBrick compatible interface of each pSNn plasmid. Generation of such mutants enables selection of transformed *Synechocystis* that have the cassette by exposure to kanamycin. A double selection cassette (KmSc; SEQ ID NO: 18:) can also be inserted at the neutral sites to confer both resistance to kanamycin (*nptII*) and sensitivity to sucrose (*sacB*). A plasmid series (pSNnKS; SEQ ID NO: 11:, SEQ ID NO: 12:, SEQ ID NO: 13:, SEQ ID NO: 14:, and SEQ ID NO: 15:) was obtained by cloning the PCR-amplified kanamycin resistance/sucrose sensitivity cassette, from the plasmid pK18mobsacB, upstream of the BioBrick compatible interface of each pSNn plasmid. These plasmids are used to produce *Synechocystis* mutants that are resistant to kanamycin and sensitive to sucrose. Such mutants will allow the insertion of ectopic DNA using the pSNn plasmids, resulting in mutants without selection markers (sensitive to kanamycin and tolerant to sucrose). Exposure to sucrose enables selection of transformed *Synechocystis* that lost the KmSc cassette.

In an embodiment, *Synechocystis* mutants that carry a Green Fluorescent Protein (GFP) generator as a reporter can also be generated. The BioBrick™ plasmid (pSB1A2-E0240) contains the GFP generator (BBa_E0240; SEQ ID NO: 19:), which comprises the GFP encoding sequence preceded by a ribosome binding site and followed by transcriptional terminators, and is cloned in the pSB1A2 plasmid (delivery vector). A promoterless series of plasmids (pSN*n*K.gfp) were generated to assess the insulation of the synthetic cloning interface (FP300), by the use of a promoterless GFP generator. The plasmids in this series were obtained by cloning the GFP generator (BioBrick BBa_E0240) in the multiple cloning site of the synthetic interface of each pSN*n*K plasmid. A promoter-controlled series of plasmids (pSN*n*K.Cgfp) were also generated. The promoter (pJ201-$P_{psbA2*}$), comprises a synthetic minimal promoter region based on *Synechocystis* native *psbA2* promoter (termed as $P_{psbA2*}$; SEQ ID NO: 20), and uses pJ201 as the delivery vector. This plasmid series was used to assess the functionality of the neutral sites by insertion of a synthetic construct constitutively promoting GFP expression. For this purpose the GFP generator (BioBrick BBa_E0240) was cloned under the control of the synthetic constitutive promoter $P_{psM2*}$. The plasmids in this series were obtained by cloning this synthetic construct in the multiple cloning site of the synthetic interface of each pSN*n*K plasmid.

Production of ectoine and hydroxyectoine. Ectoine and hydroxyectoine belong to the family of compatible solutes and are among the most abundant osmolytes in nature, protecting and maintaining the native function of biomolecules under non-optimal conditions. Ectoine (1,4,5,6-tetrahydro-2-methyl-4-pyrimidine carboxylic acid) is a widely distributed compatible solute accumulated by halophilic and halotolerant microorganisms to prevent osmotic stress in highly saline environments. Ectoine is a highly water keeping compound stabilizing biomolecules and whole cells and can be used in research, cosmetics, and medicine (Reshetnikov et al., 2011). Although *Synechocystis* does not naturally synthesize ectoine nor its derivative hydroxyectoine, this organism produces L-aspartate 4-semialdehyde, which is the precursor for ectoine/hydroxyectoine. These compounds are produced by several organisms including *Chromohalobacter salexigens* and *Methylomicrobium alcaliphilum,* for which the genome sequences are publically available. *M. alcaliphilum 20Z* is a halotolerant alkaliphilic methanotroph, able to grow using methane as the sole carbon and energy source under conditions of high salinity (up to 10% (w/v) NaCl) and extremely alkaline pH values (pH>9). The halophilic and highly halotolerant bacterium C. *salexigens* is able to grow between 0.9% and 32% (w/v) NaCl in rich media and between 2.9% and 19% (w/v) NaCl in minimal media. These organisms, have the genes coding for the specific enzymes of ectoine biosynthesis:diaminobutyric acid (DABA) aminotransferase (EctB; SEQ ID NO: 28: and SEQ ID NO: 32:), DABA acetyltransferase (EctA; SEQ ID NO: 27: and SEQ ID NO: 31), and ectoine synthase (EctC; SEQ ID NO: 29: and SEQ ID NO: 33). These genes are organized in clusters: *ectABC* in *C. salexigens* and *ectABC-ask* in *M. alcaliphilum. ask* encodes an alternative aspartate kinase isozyme (Ask; SEQ ID NO: 35) that increases ectoine accumulation (Reshetnikov et al., 2011). For the synthesis of hydroxyectoine, the additional enzyme ectoine hydroxylase (EctD; SEQ ID NO: 30: and SEQ ID NO: 34:) is required. The enzymes described above catalyze the following reactions:

1. L-2-amino-4-oxobutanoate + L-glutamate = L-2,4-diaminobutanoate + 2-oxoglutarate (catalyzed by EctA)
2. acetyl-CoA + L-2,4-diaminobutanoate = CoA + N4-acetyl-L-2,4-diaminobutanoate (EctB)

3. 4-N-acetyl-L-2,4-diaminobutanoate = L-ectoine + $H_2O$ (EctC)
4. L-ectoine + 2-Oxoglutarate + $O_2$ = Hydroxyectoine + succinate + $CO_2$ (EctD)

To produce ectoine using the *Synechocystis,* it is necessary to introduce the 3 genes (*ectABC*) into the chassis, and an additional gene is required for hydroxyectoine production (*ectD*). These genes/gene clusters will be preferably introduced in one or more chromosomal neutral sites. In addition, the gene encoding an alternative aspartate kinase (*ask*) present in the *ect* cluster of *M. alcaliphilum* will also be considered, since it is reported to increase ectoine (and possibly hydroxyectoine) accumulation. Both the genes from *C. salexigens* and *M. alcaliphilum* were considered for ectoine and hydroxyectoine production. For this purpose, the original gene sequences were optimized for *Synechocystis* using the software Gene Designer (DNA 2.0 Inc.) and two gene clusters were designed *in silico*: (i) containing *ectABCD* from *C. salexigens* and (ii) containing *ectABCD-ask* from M. *alcaliphilum.* These gene clusters were designed as single operons containing ribosome binding sites for all genes (BioBrick BBa_B0030; SEQ ID NO: 104:) and a double transcription terminator downstream the last gene of the operon (BioBrick BBa_B0015). Restriction sites to remove *ectD* and/or *ask* from the cluster were included (*Avr*II and *Nhe*I). The sequences were adapted to the BioBrick RFC[10] standard, and were synthesized at DNA 2.0 Inc. The synthetic clusters are cloned under the control of two different constitutive promoters ($P_{trc10}$[SEQ ID NO: 105:] and $P_{psba2*}$) and introduced into the chromosomal neutral sites described, using the vectors constructed.

The present disclosure is not, obviously, in any way restricted to the herein described embodiments and a person with average knowledge in the area can predict many possibilities of modification of the same solution and substitutions of technical characteristics by others equivalent, depending on the requirements of each situation, as defined in the appended claims.

The embodiments described above can be combined with each other. The following claims further define the preferred embodiments of the present disclosure.

**Claims**

1. A genetic construct for the introduction of ectopic DNA by double homologous recombination in *Synechocystis* PCC6803, comprising two sequences

   wherein the first sequence is selected from an upstream and the second sequence is selected from a downstream chromosomal regions of a locus in *Synechocystis* sp. PCC6803 wherein the locus is SEQ ID No 109,
   and the sequences length vary between 400-1500 bp, preferably 430-800 bp, more preferably 430-560 bp,
   wherein the genetic construct comprises SEQ ID No 114.

2. The genetic construct according to the preceding claim further comprising the SEQ ID No: 26.

3. A vector for the introduction of a genetic construct for the production of a target molecule in a cyanobacterium host cell comprising the genetic construct described in any one of the previous claims.

4. The vector according to the preceding claim further comprising a gene selected from the following list: a gene coding for antibiotic resistance, a gene coding for levansucrase, a gene coding for green fluorescence protein, in particular a gene coding for antibiotic kanamycin resistance.

5. The vector according to the preceding claims 3-4 further comprising a constitutive promotor, preferably SEQ ID No 20.

6. The vector according to the preceding claims 3-5 wherein the vector is an integrative vector.

7. The vector according to the preceding claims 3-6 further comprising a gene or genes for coding in a host cell: an enzyme or enzymes for production of a target molecule, or a precursor of a target molecule or a target molecule.

8. The vector according to the preceding claims 3-7 wherein the genetic construct leads to the production of at least a precursor, an enzyme or the target molecule of the following list: a compatible solute, an organic compound, a polymer, an alcohol, a biofuel, an additive, an adhesive, a sealant, a pigment, an antibiotic, a medicament, a protein.

9. The vector according to the preceding claims 3-8 wherein the genetic construct encodes enzymes for the production of a compatible solute selected from the following list: ectoine, hydroxyectoine, or mixtures thereof.

10. The vector according to the preceding claims 3-9 wherein the genetic construct encodes enzymes for the production of the organic compound isoprene, glucoglycerol, betaine or mixtures thereof.

11. The vector according to the preceding claims 3-10 wherein the genetic construct encodes proteins/enzymes for the production of the pigment phycocyanin.

12. A cyanobacterium host cell comprising the construct according to any of the claims 1-2 or comprising the vector according to any of the claims 3-11.

13. A use of a genetic construct as described in any of the claims 1-2
as an enhancer of the production of an enzyme for production of the target molecule, or a precursor of a target molecule or a target molecule in a cyanobacterium host cell.

14. A method for the expression of an enzyme for production of the target molecule, or a precursor of a target molecule or a target molecule with the following steps:

constructing a genetic construct as described in claim 1- 2;
cloning the genetic construct in an appropriate vector;
cloning the genetic construct for the expression of the precursor, the enzyme or the target molecule, in the vector and obtaining a recombinant vector;
transforming *Synechocystis* host cell with the recombinant vector;
expressing the gene or genes for the production of a precursor, of the enzyme or of the target molecule.

**Patentansprüche**

1. Ein genetisches Konstrukt zur Einführung ektopischer DNA durch doppelte homologe Rekombination in *Synechocystis* PCC6803, umfassend zwei Sequenzen

wobei die erste Sequenz aus einer stromaufwärts gelegenen und die zweite Sequenz aus einer stromabwärts gelegenen Chromosomenregion eines Locus in *Synechocystis* sp. PCC6803 ausgewählt wird
wobei der Locus SEQ ID NO 109 ist,
und die Sequenzlängen zwischen 400-1500 bp, bevorzugt 430-800 bp, besonders bevorzugt 430-560 bp variiert,
wobei das genetische Konstrukt SEQ ID NO 114 umfasst.

2. Das genetische Konstrukt nach dem vorangehenden Anspruch, ferner umfassend die SEQ ID NO: 26.

3. Ein Vektor zur Einführung eines genetischen Konstrukts zur Herstellung eines Zielmoleküls in eine Cyanobakterium-Wirtszelle, umfassend das in einem der vorangehenden Ansprüche beschriebene genetische Konstrukt.

4. Der Vektor nach dem vorangehenden Anspruch, ferner umfassend ein Gen, ausgewählt aus der folgenden Liste: ein Antibiotika-Resistenz kodierendes Gen, ein Levansucrase kodierendes Gen, ein grünes Fluoreszenzprotein kodierendes Gen, insbesondere ein Kanamycin Antibiotika-Resistenz kodierendes Gen.

5. Der Vektor nach den vorangehenden Ansprüchen 3-4, ferner umfassend einen konstitutiven Promotor, bevorzugt SEQ ID NO 20.

6. Der Vektor nach den vorangehenden Ansprüchen 3-5, wobei der Vektor ein integrativer Vektor ist.

7. Der Vektor nach den vorangehenden Ansprüchen 3-6, ferner umfassend ein Gen oder Gene zur Kodierung in einer Wirtszelle: ein Enzym oder Enzyme zur Herstellung eines Zielmoleküls oder eines Vorläufers eines Zielmoleküls oder eines Zielmolekül.

8. Der Vektor nach den vorangehenden Ansprüchen 3-7, wobei das genetische Konstrukt zur Herstellung mindestens eines Vorläufers, eines Enzyms oder des Zielmoleküls der folgenden Liste führt: ein kompatibler gelöster Stoff, eine organische Verbindung, ein Polymer, ein Alkohol, ein Biobrennstoff, ein Zusatzstoff, ein Klebstoff, ein Dichtstoff, ein Pigment, ein Antibiotikum, ein Medikament, ein Protein.

9. Der Vektor nach den vorangehenden Ansprüchen 3-8, wobei das genetische Konstrukt Enzyme zur Herstellung eines kompatiblen gelösten Stoffes kodiert, ausgewählt aus folgender Liste: Ectoin, Hydroxyectoin oder Mischungen davon.

**10.** Der Vektor nach den vorangehenden Ansprüchen 3-9, wobei das genetische Konstrukt Enzyme zur Herstellung der organischen Verbindung Isopren, Gluco-Glycerol, Betain oder Mischungen davon kodiert.

**11.** Der Vektor nach den vorangehenden Ansprüchen 3-10, wobei das genetische Konstrukt Proteine/Enzyme zur Herstellung des Pigments Phycocyanin kodiert.

**12.** Eine Cyanobakterium-Wirtszelle, umfassend das Konstrukt nach einem der Ansprüche 1-2 oder umfassend den Vektor nach einem der Ansprüche 3-11.

**13.** Eine Verwendung eines genetischen Konstrukts wie in einem der Ansprüche 1-2 beschrieben
als ein Enhancer zur Herstellung eines Enzyms zur Herstellung des Zielmoleküls, oder eines Vorläufers für ein Zielmolekül oder eines Zielmoleküls in einer Cyanobakterium-Wirtszelle.

**14.** Ein Verfahren zur Expression eines Enzyms zur Herstellung des Zielmoleküls oder eines Vorläufers eines Zielmoleküls oder eines Zielmoleküls mit den folgenden Schritten:

Herstellen eines genetischen Konstrukts wie in Anspruch 1 - 2 beschrieben;
Klonen des genetischen Konstrukts in einem geeigneten Vektor;
Klonen des genetischen Konstrukts für die Expression des Vorläufers, des Enzyms oder des Zielmoleküls in dem Vektor und Gewinnung eines rekombinanten Vektors;
Transformation der *Synechocystis*-Wirtszelle mit dem rekombinanten Vektor;
Expression des Gens oder der Gene zur Herstellung eines Vorläufers, des Enzyms oder des Zielmoleküls.

**Revendications**

**1.** Une construction génétique pour l'introduction d'ADN ectopique par double recombinaison homologue de *Synechocystis* PCC6803, comprenant deux séquences

dans laquelle la première séquence est sélectionnée à partir d'une région chromosomique en amont et la seconde séquence est sélectionée à partir d'une région chromosomique en aval d'un locus de *Synechocystis* sp. PCC6803
dans lequel le locus est SEQ ID № 109,
et la longueur des séquences varie entre 400-1500 bp, préférablement 430-800 bp, plus préférablement 430-560 bp,
dans laquelle la construction génétique comprend SEQ ID № 114.

**2.** La construction génétique selon la revendication précédente comprenant également le SEQ ID №: 26.

**3.** Un vecteur pour l'introduction d'une construction génétique pour la production d'une mollécule cible dans une cellule hôte de cyanobactérie comprenant la construction génétique décrite dans l'une quelconque des revendications précédentes.

**4.** Le vecteur selon la revendication précédente comprenant également un gène sélectionné à partir de la liste suivante : une codification de gène pour la résistance antibiotique, une codification de gène pour le lévane-sucrase, une codification de gène pour la protéine de fluorescence verte, en particulier une codification de gène pour une résistance à la kanamycine antibiotique.

**5.** Le vecteur selon les revendications précédentes 3-4 comprenant également un promoteur constitutif, préférablement SEG ID № 20.

**6.** Le vecteur selon les revendications précédentes 3-5 dans lequel le vecteur est un vecteur intégratif.

**7.** Le vecteur selon les revendications précédentes 3-6 comprenant également un gène ou des gènes pour codification dans une cellule hôte : une enzyme ou des enzymes pour la production d'une mollécule cible, ou un précurseur d'une mollécule cible ou une mollécule cible.

**8.** Le vecteur selon les revendications précédentes 3-7 dans lequel la construction génétique mène à la production d'au moins un précurseur, une enzyme ou la mollécule cible de la liste suivante : un soluté compatible, un composé organique, un polymère, un alcool, un biocarburant, un additif, un adhésif, un scellant, un pigment, un antibiotique, un médicament, une protéine.

**9.** Le vecteur selon les revendications précédentes 3-8 dans lequel la construction génétique encode des enzymes pour la production d'un soluté compatible sélectionné à partir de la liste suivante : ectoine, hydroxyectoine, ou des mélanges de ceux-ci.

**10.** Le vecteur selon les revendications précédentes 3-9 dans lequel la construction génétique encode des enzymes pour la production du composé organique isoprène, glucoglycérol, bétaïne ou des mélanges de ceux-ci.

**11.** Le vecteur selon les revendications précédentes 3-10 dans lequel la construction génétique encode des protéines/enzymes pour la production du pigment phycocyanine.

**12.** Une cellule hôte de cyanobactérie comprenant la construction selon l'une quelconque des revendications 1-2 ou comprenant le vecteur selon l'une quelconque des revendications 3-11.

**13.** L'utilisation d'une construction génétique telle que décrite dans l'une quelconque des revendications 1-2
en tant qu'amplificateur de la production d'une enzyme pour la production de la mollécule cible, ou un précurseur d'une mollécule cible ou une mollécule cible dans une cellule hôte de cyanobactérie.

**14.** Un procédé pour l'expression d'une enzyme pour la production de la mollécule cible, ou un précurseur d'une mollécule cible, ou une mollécule cible avec les étapes suivantes :

construire une construction génétique telle que décrite dans la revendication 1-2 ;
clôner la construction génétique dans un vecteur approprié ;
clôner la construction génétique pour l'expression du précurseur, de l'enzyme ou de la mollécule cible, dans le vecteur et obtenir un vecteur recombinant ;
transformer la cellule hôte *Synechocystis* avec le vecteur recombinant ;
exprimer le gène ou les gènes pour la production d'un précurseur, de l'enzyme ou de la mollécule cible.

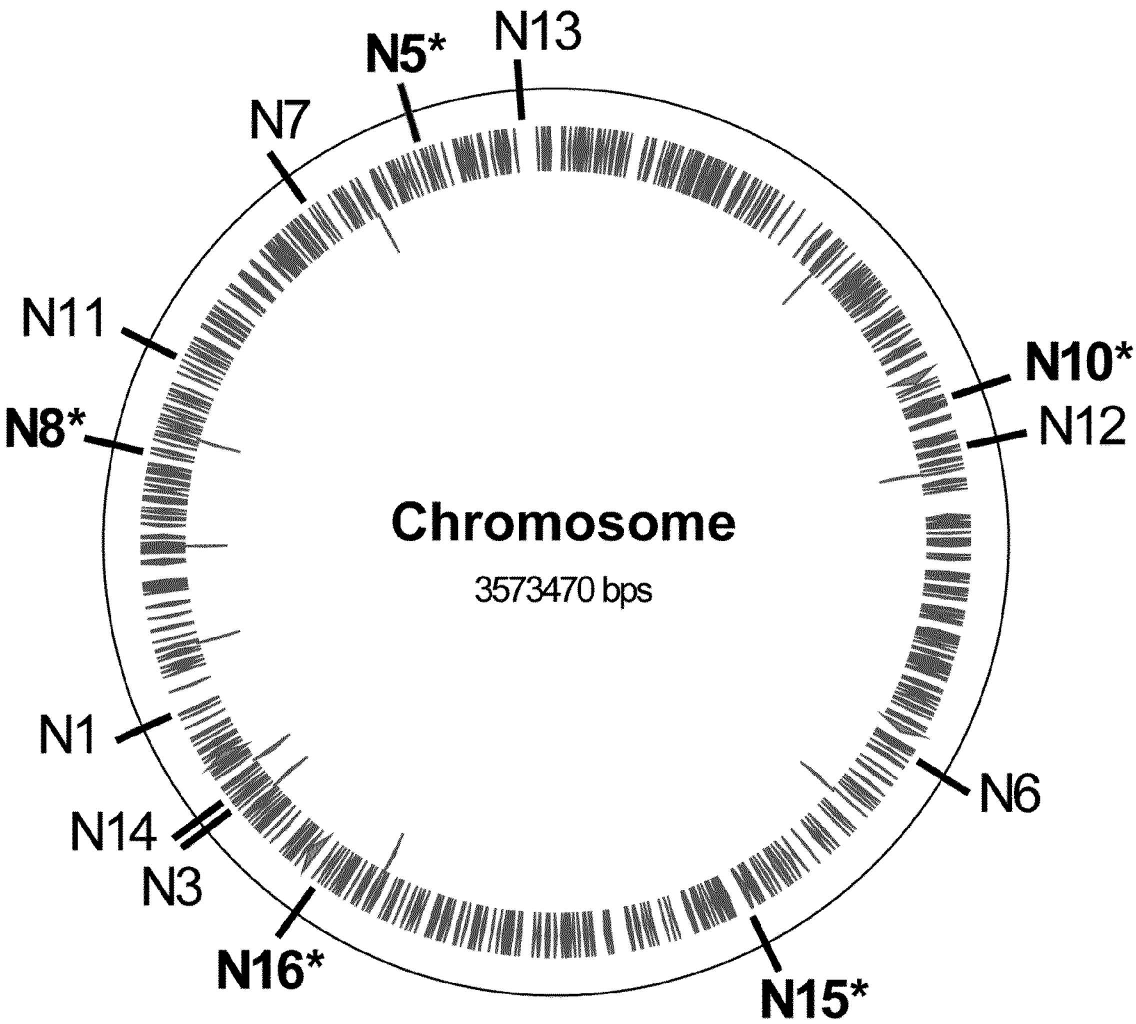
N13
N5*
N7
N11
N10*
N12
N8*
Chromosome
3573470 bps
N1
N6
N14
N3
N16*
N15*

Fig. 1A

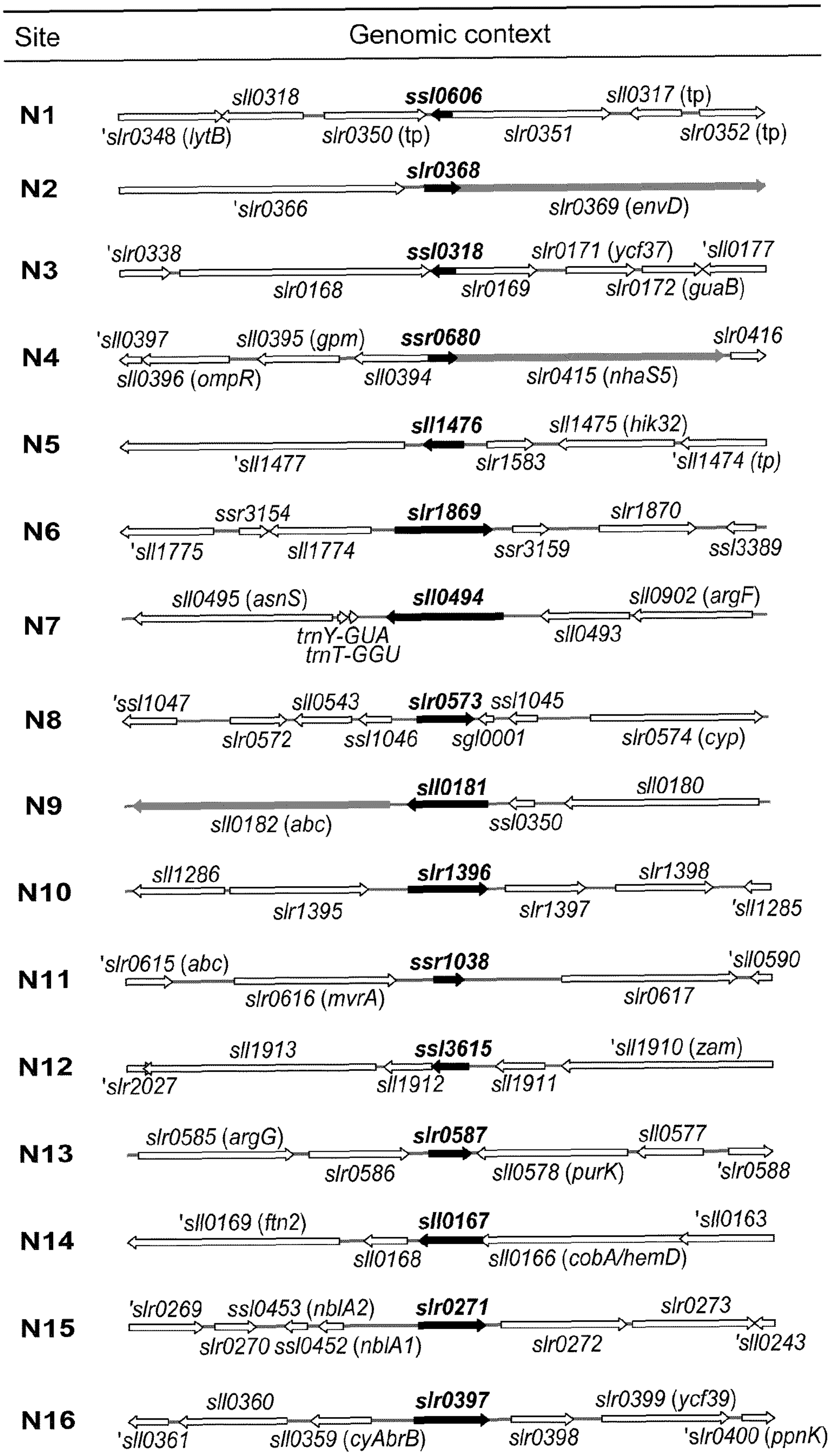

Site
Genomic context
N1
sll0318 ssl0606 sll0317 (tp)
'slr0348 (lytB) slr0350 (tp) slr0351 slr0352 (tp)
N2
slr0368
'slr0366 slr0369 (envD)
N3
'slr0338 ssl0318 slr0171 (ycf37) 'sll0177
slr0168 slr0169 slr0172 (guaB)
N4
'sll0397 sll0395 (gpm) ssr0680 slr0416
sll0396 (ompR) sll0394 slr0415 (nhaS5)
N5
sll1476 sll1475 (hik32)
'sll1477 slr1583 'sll1474 (tp)
N6
ssr3154 slr1869 slr1870
'sll1775 sll1774 ssr3159 ssl3389
N7
sll0495 (asnS) sll0494 sll0902 (argF)
trnY-GUA
trnT-GGU
sll0493
N8
'ssl1047 sll0543 slr0573 ssl1045
slr0572 ssl1046 sgl0001 slr0574 (cyp)
N9
sll0181 sll0180
sll0182 (abc) ssl0350
N10
sll1286 slr1396 slr1398
slr1395 slr1397 'sll1285
N11
'slr0615 (abc) ssr1038 'sll0590
slr0616 (mvrA) slr0617
N12
sll1913 ssl3615 'sll1910 (zam)
'slr2027 sll1912 sll1911
N13
slr0585 (argG) slr0587 sll0577
slr0586 sll0578 (purK) 'slr0588
N14
'sll0169 (ftn2) sll0167 'sll0163
sll0168 sll0166 (cobA/hemD)
N15
'slr0269 ssl0453 (nblA2) slr0271 slr0273
slr0270 ssl0452 (nblA1) slr0272 'sll0243
N16
sll0360 slr0397 slr0399 (ycf39)
'sll0361 sll0359 (cyAbrB) slr0398 'slr0400 (ppnK)


**Fig. 1B**

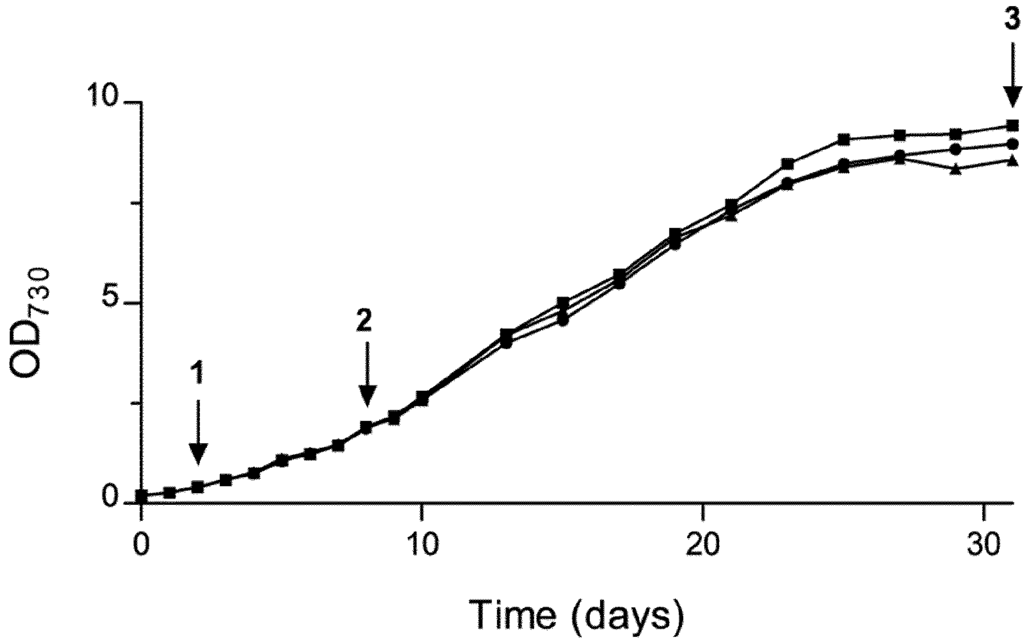
OD730
Time (days)
0
5
10
0
10
20
30
1
2
3

Fig. 2A

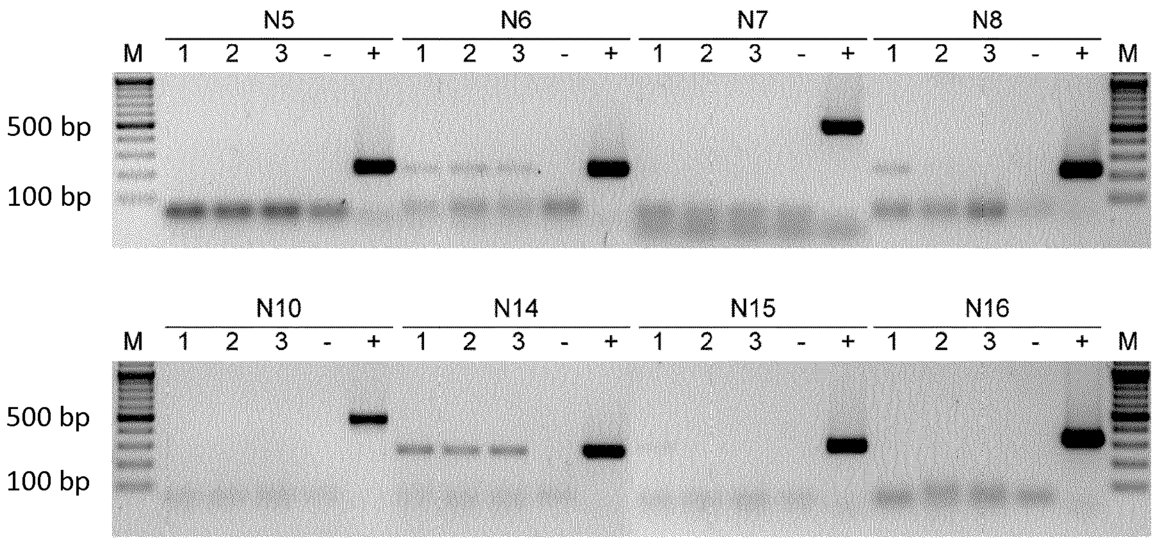
N5
N6
N7
N8
M 1 2 3 - + 1 2 3 - + 1 2 3 - + 1 2 3 - + M
500 bp
100 bp
N10
N14
N15
N16
M 1 2 3 - + 1 2 3 - + 1 2 3 - + 1 2 3 - + M
500 bp
100 bp

Fig. 2B

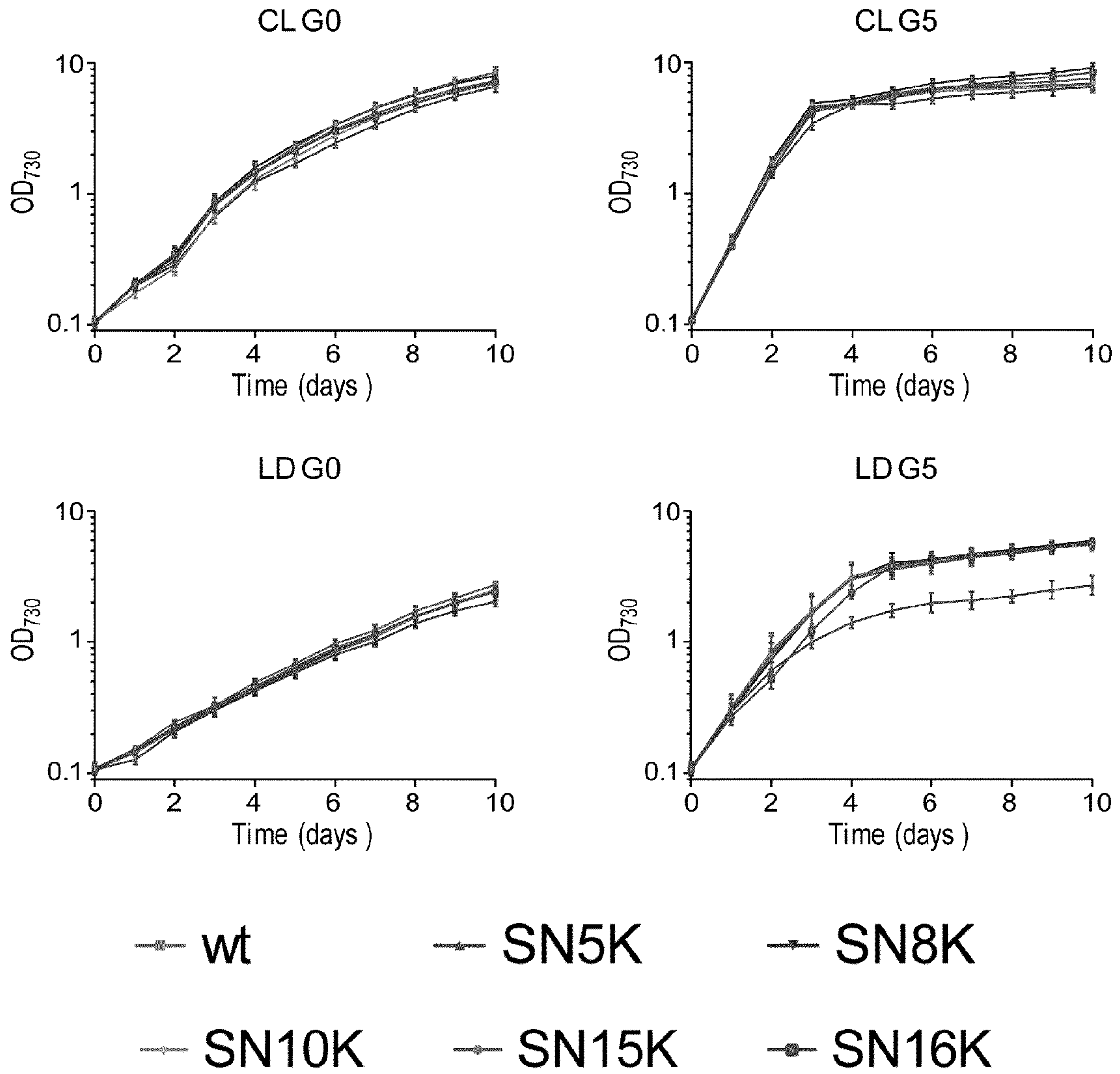
CL G0
CL G5
LD G0
LD G5
OD730
Time (days )
10
1
0.1
0
2
4
6
8
10
wt
SN5K
SN8K
SN10K
SN15K
SN16K

Fig. 3

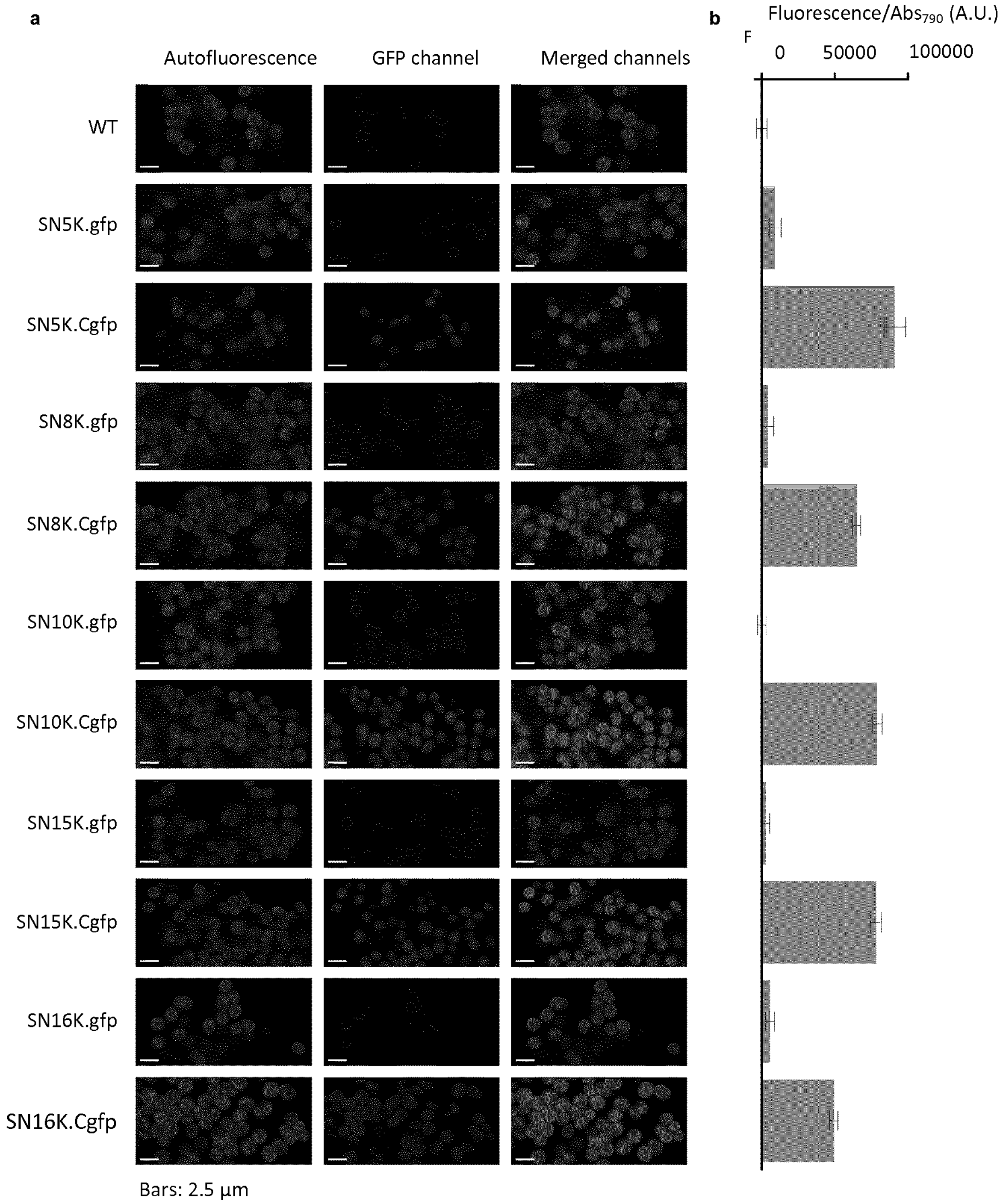
a
b
Autofluorescence
GFP channel
Merged channels
Fluorescence/Abs790 (A.U.)
F
0
50000
100000
WT
SN5K.gfp
SN5K.Cgfp
SN8K.gfp
SN8K.Cgfp
SN10K.gfp
SN10K.Cgfp
SN15K.gfp
SN15K.Cgfp
SN16K.gfp
SN16K.Cgfp
Bars: 2.5 µm

**Fig. 4**

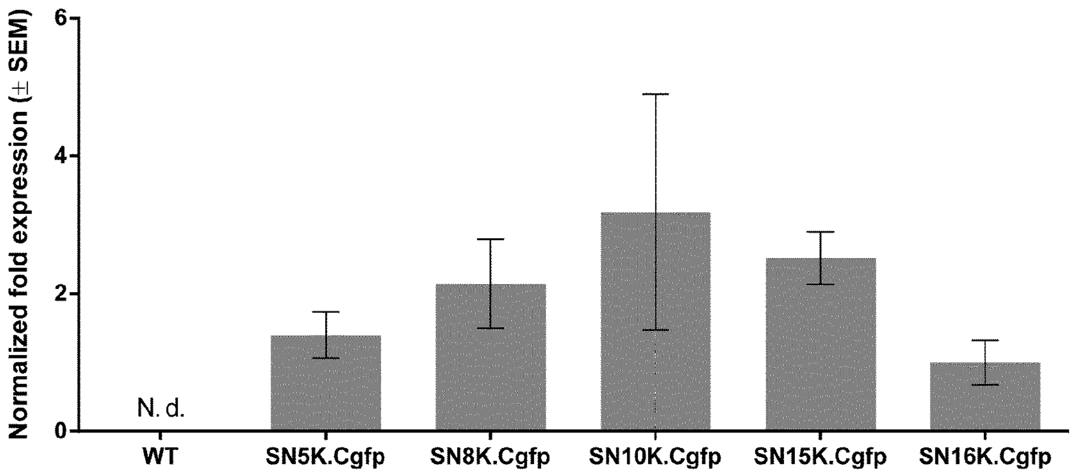
Normalized fold expression (± SEM)
6
4
2
0
N. d.
WT
SN5K.Cgfp
SN8K.Cgfp
SN10K.Cgfp
SN15K.Cgfp
SN16K.Cgfp

Fig. 5

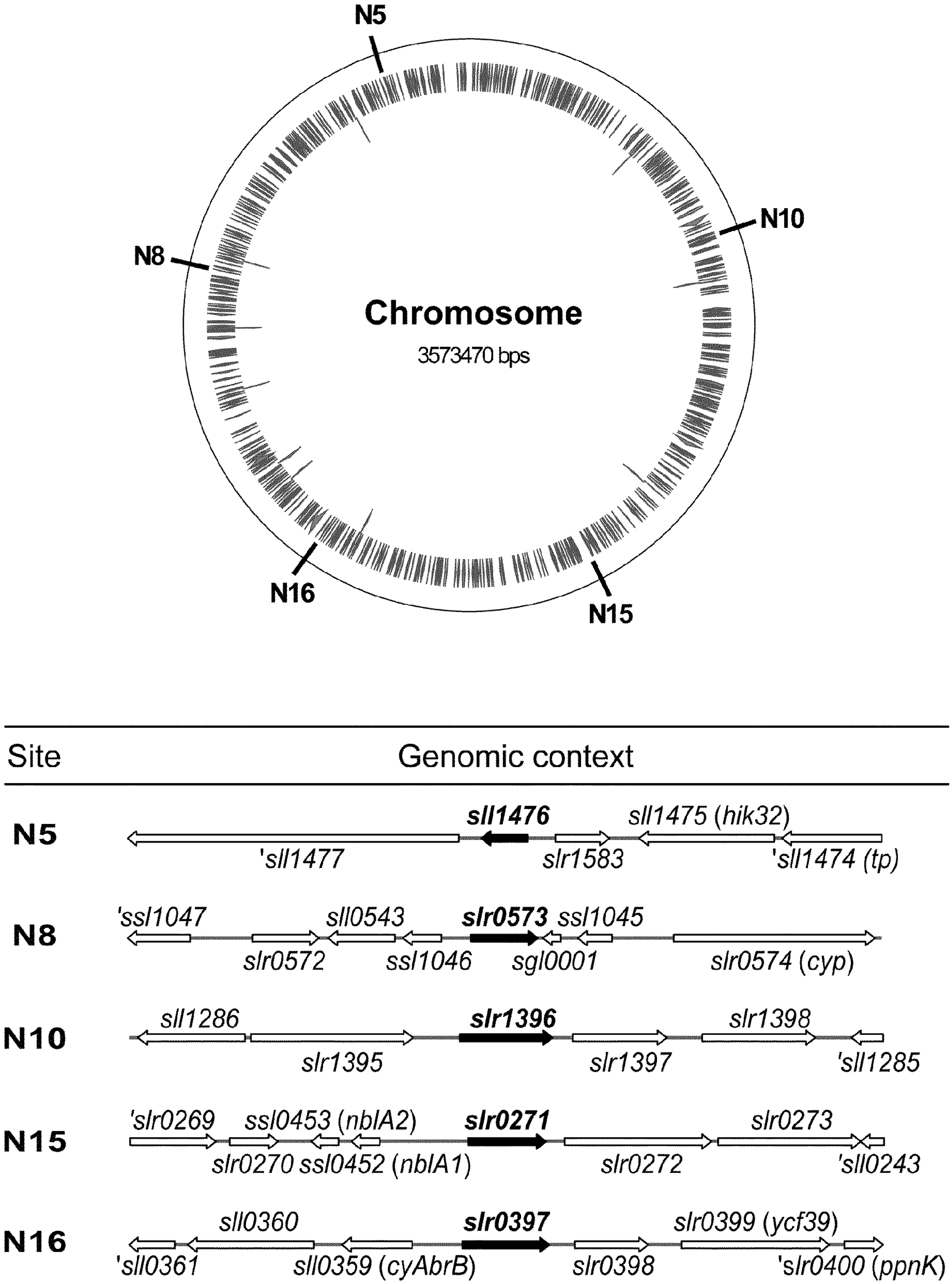
N5
N10
N8
Chromosome
3573470 bps
N16
N15
Site
Genomic context
N5
sll1476
sll1475 (hik32)
'sll1477
slr1583
'sll1474 (tp)
N8
'ssl1047
sll0543
slr0573
ssl1045
slr0572
ssl1046
sgl0001
slr0574 (cyp)
N10
sll1286
slr1396
slr1398
slr1395
slr1397
'sll1285
N15
'slr0269
ssl0453 (nblA2)
slr0271
slr0273
slr0270
ssl0452 (nblA1)
slr0272
'sll0243
N16
sll0360
slr0397
slr0399 (ycf39)
'sll0361
sll0359 (cyAbrB)
slr0398
'slr0400 (ppnK)

Fig. 6

| Site name | ORF ID | Chromosome position | Orientation* | Length of the putatively encoded protein (amino acids) |
|---|---|---|---|---|
| N1 | *ssl0606* | 2441925 - 2442083 | c | 52 |
| N2 | *slr0368* | 2365848 - 2366120 | d | 90 |
| N3 | *ssl0318* | 2302457 - 2302645 | c | 62 |
| N4 | *ssr0680* | 2684766 - 2684990 | d | 74 |
| N5 | *sll1476* | 3398409 - 3398717 | c | 102 |
| N6 | *slr1869* | 1212609 - 1213358 | d | 249 |
| N7 | *sll0494* | 3224429 - 3225334 | c | 301 |
| N8 | *slr0573* | 2816517 - 2816960 | d | 147 |
| N9 | *sll0181* | 2737929 - 2738552 | c | 207 |
| N10 | *slr1396* | 707437 - 708054 | d | 205 |
| N11 | *ssr1038* | 2950107 - 2950343 | d | 78 |
| N12 | *ssl3615* | 776058 - 776342 | c | 94 |
| N13 | *slr0587* | 3536090 - 3536422 | d | 110 |
| N14 | *sll0167* | 2317536 - 2318030 | c | 164 |
| N15 | *slr0271* | 1524568 - 1525086 | d | 172 |
| N16 | *slr0397* | 2146801 - 2147376 | d | 191 |

**Fig. 7**

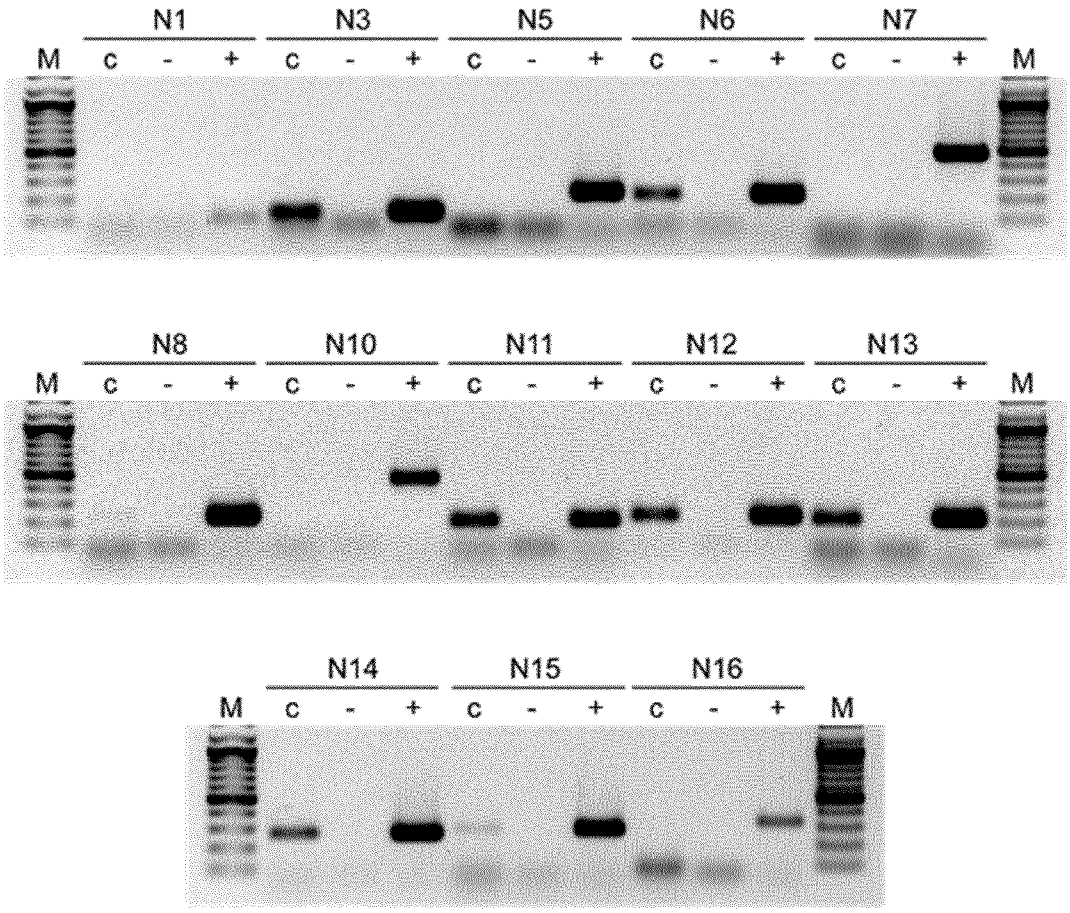
N1
N3
N5
N6
N7
M c - + c - + c - + c - + c - + M
N8
N10
N11
N12
N13
M c - + c - + c - + c - + c - + M
N14
N15
N16
M c - + c - + c - + M

Fig. 8

| Sequence ID Number | Primer name | Sequence 5' → 3' | Amplicon size (bp) |
|---|---|---|---|
| SEQ ID NO: 36: | N1F | CATGGATTGAAGGAAGGG | 100 |
| SEQ ID NO: 37: | N1R | TTTGGTGAACAATTAATGAAC | |
| SEQ ID NO: 38: | N3F | GCAGTCCCTCCTCCAAAC | 129 |
| SEQ ID NO: 39: | N3R | AAGCCGATATTCAGGTAAGATG | |
| SEQ ID NO: 40: | N5F | GGAAGTGTTGTTGCTGTC | 232 |
| SEQ ID NO: 41: | N5R | GTCCTTCGCCGTAGATTG | |
| SEQ ID NO: 42: | N6F | AAGGTAATCAAGCAGAAATG | 217 |
| SEQ ID NO: 43: | N6R | ATTGGTAACAGCACTTCC | |
| SEQ ID NO: 44: | N7F | TGACTGTAAGCAATCTACC | 500 |
| SEQ ID NO: 45: | N7R | ACTAACGATGGAATCTTGG | |
| SEQ ID NO: 46: | N8F | AACTTAACTTCTATTCGTGAG | 231 |
| SEQ ID NO: 47: | N8R | GAAACCTTGATAAGCAGTC | |
| SEQ ID NO: 48: | N10F | GATATTGATGTGTATTGC | 478 |
| SEQ ID NO: 49: | N10R | TTATTATCTTCTGTCTCC | |
| SEQ ID NO: 50: | N11F | TGAATACCACATCTCCCATTGAC | 200 |
| SEQ ID NO: 51: | N11R | TTTAACCTGCTGGCGTGAC | |
| SEQ ID NO: 52: | N12F | TTCTCACCGCTACTGTTAC | 227 |
| SEQ ID NO: 53: | N12R | CACTTCTCGCACTAATTCG | |
| SEQ ID NO: 54: | N13F | CCCAAATGATGACCGAAG | 216 |
| SEQ ID NO: 55: | N13R | CTAGTAACTGCTGTTCCTC | |
| SEQ ID NO: 56: | N14F | AAGTGTCCAAAGCCAAAC | 270 |
| SEQ ID NO: 57: | N14R | TCAACAATGCCATCTTCC | |
| SEQ ID NO: 58: | N15F | TTAATTGCCACTGCCTTAC | 293 |
| SEQ ID NO: 59: | N15R | TAGAGACAGCGGATATGC | |
| SEQ ID NO: 60: | N16F | TGATGTGGACGAGGATAC | 335 |
| SEQ ID NO: 61: | N16R | GGGCTAAGGGAATATGGG | |

**Fig. 9**

| Sequence ID Number | Primer name | Sequence 5' → 3'* | Purpose |
|---|---|---|---|
| SEQ ID NO: 62: | Km.KmScFwd | CTGACCCCGGGTGAATGTCAGCTACTGG | Selection cassettes amplification |
| SEQ ID NO: 63: | KmRev | CAAACCCGGGCGATTTACTTTTCGACCTC | |
| SEQ ID NO: 64: | KmScRev | ACAGACCCGGGCAAGCGGATGGCTGATG | |
| SEQ ID NO: 65: | N5.5O** | GCGGCCTCGAGTGGGGGCATCTGCTAGGCAATATTTG | Amplification of the DNA fragments flanking the neutral sites |
| SEQ ID NO: 66: | N5.5I | GATTACACCCGGGTGTAATCGGTTGATTATTTCAGTGGCCCGGCCGATGGATAATTAC | |
| SEQ ID NO: 67: | N5.3O** | GTCCAAATCAGCATTGCTCTGCCAGGTGAGAC | |
| SEQ ID NO: 68: | N5.3I | GATTACACCCGGGTGTAATCCTGAAGCCTTGACAATCCCCGTTGGTGATTTATACTTAG | |
| SEQ ID NO: 69: | N8.5O** | GAACACTCGAGTGCCAGCAACGACAATG | |
| SEQ ID NO: 70: | N8.5I | GATTACACCCGGGTGTAATCCTTCATTCCCCTATTGTTATTAATAGTGCTC | |
| SEQ ID NO: 71: | N8.3O** | GATGACCGCTGGCGGAGTTTAGTCCAG | |
| SEQ ID NO: 72: | N8.3I | GATTACACCCGGGTGTAATCTTGTCACCAATTTTTGTAGGGATGTTGGGCTAAATTG | |
| SEQ ID NO: 73: | N10.5O** | GATAACTCGAGTCGCCCGGTGGATTTAATGC | |
| SEQ ID NO: 74: | N10.5I | GATTACACCCGGGTGTAATCCCCATACTCAGCCTTCTAATGCTGAGAGAATG | |
| SEQ ID NO: 75: | N10.3O** | GGACTGACCCAAGATACAGTGGTAG | |
| SEQ ID NO: 76: | N10.3I | GATTACACCCGGGTGTAATCCGAGCGCAAACTACAATGCGCTTCGTTTGC | |
| SEQ ID NO: 77: | N15.5O** | GGTTCTCGAGCCGCTGAATTTAGTCAACATCG | |
| SEQ ID NO: 78: | N15.5I | GATTACACCCGGGTGTAATCCATTGGGCACGAGAGTTAGTAAGGCAGTG | |
| SEQ ID NO: 79: | N15.3O* | CTAAACTTACGGCATTGGCATCAACGGGAG | |
| SEQ ID NO: 80: | N15.3I | GATTACACCCGGGTGTAATCTCTTTACAATGGCCAGGTCTTTAGGGAGCGGTGAC | |
| SEQ ID NO: 81: | N16.5O** | GAACTCGAGTAGTAACCACAGGCTTTTG | |
| SEQ ID NO: 82: | N16.5I | GATTACACCCGGGTGTAATCGCTGGAGGCGAACTGGGTGAGAACCAT | |
| SEQ ID NO: 83: | N16.3O** | GTGAGCTTGATGGTGATGGTGGGTAAAG | |
| SEQ ID NO: 84: | N16.3I | GATTACACCCGGGTGTAATCTGCTGGCTTTGTTGCCCTGTCAACCAAAGTTC | |

| | | | |
|---|---|---|---|
| SEQ ID NO: 85: | N5_SDM | CGAGGCGATCGCCCAGTTGGAAGAATT GGCC | SDM*** |
| SEQ ID NO: 86: | N10_SDM | CTAAAAAGACAAGTCTGTGGCTAGTTAC TATGACGAGGC | SDM*** |
| SEQ ID NO: 87: | N5.FO | TCCTGGTAACTCACGCTATC | Mutants confirmation by PCR |
| SEQ ID NO: 88: | N5.FI | AGCCGATCCAGGGAAGTGTTGTTG | |
| SEQ ID NO: 89: | N5.RI | CCATCGTCCTTCGCCGTAGATTGTG | |
| SEQ ID NO: 90: | N8.FO | CCCAGTTAAACTGCGAAAGG | |
| SEQ ID NO: 91: | N8.FI | TCGCCAAGCTTTCAGAAC | |
| SEQ ID NO: 92: | N8.RI | CAAACTCCAGCCGATAAC | Mutants confirmation by PCR |
| SEQ ID NO: 93: | N10.FO | CCGGTTGCCCTTATCGGAACCGATG | |
| SEQ ID NO: 94: | N10.FI | GCTATGGCGTCACTTGTAGC | |
| SEQ ID NO: 95: | N10.RI | TTTGCGACCCATCGGATTGC | |
| SEQ ID NO: 96: | N15.FO | CTCCAAGGCGACTACCTTTC | |
| SEQ ID NO: 97: | N15.FI | CCCAGTGGGAATGCGATCAG | |
| SEQ ID NO: 98: | N15.RI | TAGGAGGGCGATCACCGAAG | |
| SEQ ID NO: 99: | N16.FO | ACCCATTTCTTGGGTGTAGG | |
| SEQ ID NO: 100: | N16.FI | GGCCTTGGTTGCCCTGACTGATGTG | |
| SEQ ID NO: 101: | N16.RI | GACCGATCGCCGCAGTAGTTCTTGG | |
| SEQ ID NO: 102: | GFP.F | TCTTGTTGAATTAGATGGTG | RT-PCR/RT-qPCR |
| SEQ ID NO: 103: | GFP.R | TGTGAGTTATAGTTGTATTCC | |

**Fig. 10**

| **Organisms name*** | **Description** | **Source** |
|---|---|---|
| *Escherichia coli* DH5α | Transformation/cloning strain | Invitrogen |
| *Synechocystis* sp. PCC 6803 | Wild-type strain | Pasteur Culture Collection |
| SN*n*K | *Synechocystis* mutants with replacement of the neutral site N*n* by a kanamycin resistance cassette. | This work |
| SN*n*KS | *Synechocystis* mutants with replacement of the neutral site N*n* by a kanamycin resistance/sucrose sensitivity cassette. | This work |
| SN*n*K.gfp | *Synechocystis* mutants with replacement of the neutral site N*n* by a kanamycin resistance cassette and the promoterless GFP encoding gene. | This work |
| SN*n*K.Cgfp | *Synechocystis* mutants with replacement of the neutral site N*n* by a kanamycin resistance cassette and the GFP encoding gene under the control of a constitutive promoter ($P_{psbA2*}$). | This work |
| **Plamids name*** | **Description** | **Source** |
| pGEM®-T easy | T/A cloning vector | Promega |
| pK18mobsacB | Small mobilizable multi-purpose cloning vector derived from the *E. coli* plasmids pK18, conferring resistance to kanamycin (*nptII*), and sensitivity to sucrose (*sacB*). | National BioResource Project (NIG, Japan): E.coli |
| pSB1A2-E0240 | BioBrick™ vector containing the part BBa_E0240 (GFP generator). | Registry of Standard Biological Parts, MIT |
| pJ201-$P_{psbA2*}$ | Synthesized minimal *psbA2* promoter region, meeting the BioBrick RFC[10]standard specifications, cloned into the delivery vector pJ201 | DNA 2.0 Inc., this work |
| pBSK-FP300 | Synthetic interface containing a BioBrick compatible multiple cloning site( *Mun*I, *Not*I, *Xba*I, *Spe*I, *Not*I, *Pst*I) insulated with the transcriptional terminators BBa_B1006 and BBa_B0062 (upstream), and BBa_B0053 and BBa_B020 (downstream). | Epoch Life Science Inc., this work |
| pSN*n* | pGEM-T easy- based plasmid series containing the BioBrick compatible interface flanked by the two regions for double homologous recombination on neutral site *n*. | This work |

| | | |
|---|---|---|
| pSN*n*K | pSN*n* plasmid series with the kanamycin resistance cassette cloned upstream of the BioBrick compatible interface. | This work |
| pSN*n*KS | pSN*n* plasmid series with the kanamycin resistance/sucrose sensitivity cassette cloned upstream of the BioBrick compatible interface. | This work |
| pSN*n*K.gfp | pSN*n*K plasmid series with the GFP generator BioBrick BBa_E0240 cloned in the BioBrick compatible interface. | This work |
| pSN*n*K.Cgfp | pSN*n*K plasmid series with the GFP generator BioBrick BBa_E0240 under the control of a constitutive promoter ($P_{psbA2*}$), cloned in the BioBrick compatible interface. | This work |

**Fig. 11**

| Plasmid name | Description |
|---|---|
| pSN5 | pGEM-T easy vector containing the BioBrick compatible interface flanked by the two regions for double homologous recombination on neutral site N5. |
| pSN8 | pGEM-T easy vector containing the BioBrick compatible interface flanked by the two regions for double homologous recombination on neutral site N8. |
| pSN10 | pGEM-T easy vector containing the BioBrick compatible interface flanked by the two regions for double homologous recombination on neutral site N10. |
| pSN15 | pGEM-T easy vector containing the BioBrick compatible interface flanked by the two regions for double homologous recombination on neutral site N15. |
| pSN16 | pGEM-T easy vector containing the BioBrick compatible interface flanked by the two regions for double homologous recombination on neutral site N16. |
| pSN5K | pSN5 plasmid with the kanamycin resistance cassette cloned upstream of the BioBrick compatible interface. |
| pSN8K | pSN8 plasmid with the kanamycin resistance cassette cloned upstream of the BioBrick compatible interface. |
| pSN10K | pSN10 plasmid with the kanamycin resistance cassette cloned upstream of the BioBrick compatible interface. |
| pSN15K | pSN15 plasmid with the kanamycin resistance cassette cloned upstream of the BioBrick compatible interface. |
| pSN16K | pSN16 plasmid with the kanamycin resistance cassette cloned upstream of the BioBrick compatible interface. |

| | |
|---|---|
| pSN5KS | pSN5 plasmid with the kanamycin resistance/sucrose sensitivity cassette cloned upstream of the BioBrick compatible interface. |
| pSN8KS | pSN8 plasmid with the kanamycin resistance/sucrose sensitivity cassette cloned upstream of the BioBrick compatible interface. |
| pSN10KS | pSN10 plasmid with the kanamycin resistance/sucrose sensitivity cassette cloned upstream of the BioBrick compatible interface. |
| pSN15KS | pSN15 plasmid with the kanamycin resistance/sucrose sensitivity cassette cloned upstream of the BioBrick compatible interface. |
| pSN16KS | pSN16 plasmid with the kanamycin resistance/sucrose sensitivity cassette cloned upstream of the BioBrick compatible interface. |
| pSN5K.gfp | pSN5K plasmid with the GFP generator BioBrick BBa_E0240 cloned in the BioBrick compatible interface. |
| pSN8K.gfp | pSN8K plasmid with the GFP generator BioBrick BBa_E0240 cloned in the BioBrick compatible interface. |
| pSN10K.gfp | pSN10K plasmid with the GFP generator BioBrick BBa_E0240 cloned in the BioBrick compatible interface. |
| pSN15K.gfp | pSN15K plasmid with the GFP generator BioBrick BBa_E0240 cloned in the BioBrick compatible interface. |
| pSN16K.gfp | pSN16K plasmid with the GFP generator BioBrick BBa_E0240 cloned in the BioBrick compatible interface. |
| pSN5K.Cgfp | pSN5K plasmid with the GFP generator BioBrick BBa_E0240 under the control of a constitutive promoter ($P_{psbA2*}$), cloned in the BioBrick compatible interface. |
| pSN8K.Cgfp | pSN8K plasmid with the GFP generator BioBrick BBa_E0240 under the control of a constitutive promoter ($P_{psbA2*}$), cloned in the BioBrick compatible interface. |
| pSN10K.Cgfp | pSN10K plasmid with the GFP generator BioBrick BBa_E0240 under the control of a constitutive promoter ($P_{psbA2*}$), cloned in the BioBrick compatible interface. |
| pSN15K.Cgfp | pSN15K plasmid with the GFP generator BioBrick BBa_E0240 under the control of a constitutive promoter ($P_{psbA2*}$), cloned in the BioBrick compatible interface. |
| pSN16K.Cgfp | pSN16K plasmid with the GFP generator BioBrick BBa_E0240 under the control of a constitutive promoter ($P_{psbA2*}$), cloned in the BioBrick compatible interface. |

**Fig. 12**

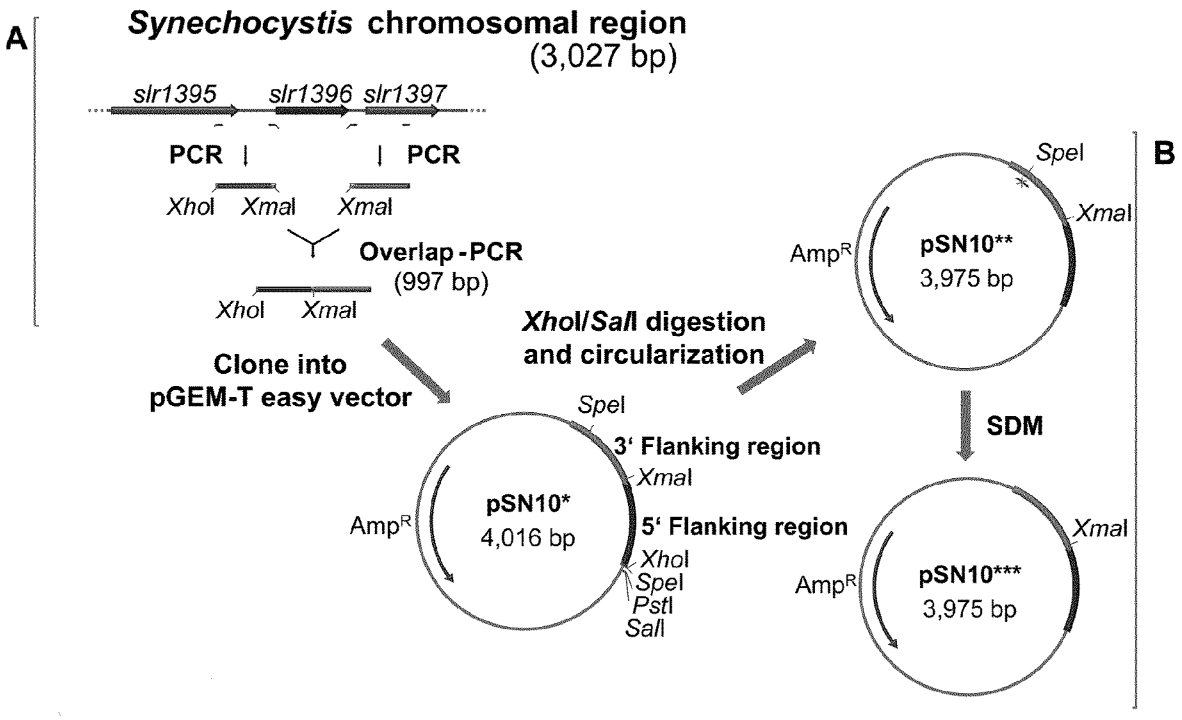


Fig. 13A-B

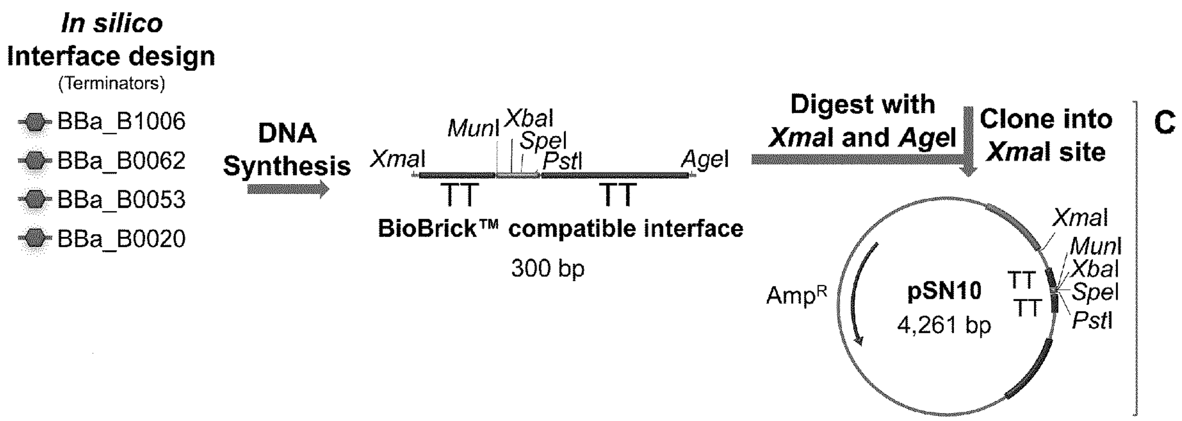


Fig. 13C

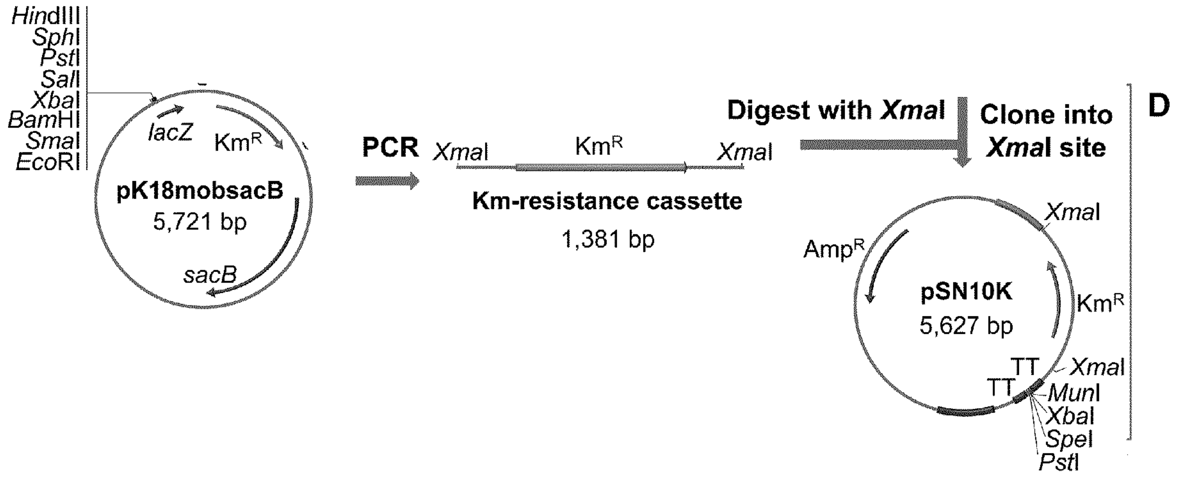


Fig. 13D

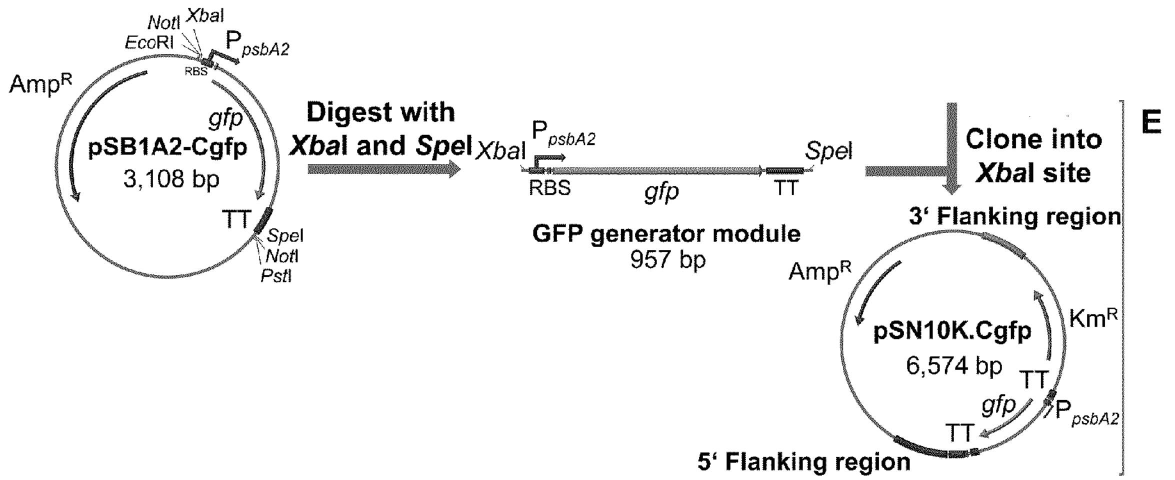


Fig. 13E

| Parameters | Mutants | | | | |
|---|---|---|---|---|---|
| | SN5K | SN8K | SN10K | SN15K | SN16K |
| Light | $1.61x10^{-38}$ | $3.47x10^{-37}$ | $8.70x10^{-34}$ | $1.58x10^{-33}$ | $5.28x10^{-39}$ |
| Glucose | $1.49x10^{-11}$ | $3.62x10^{-22}$ | $8.48x10^{-19}$ | $2.54x10^{-12}$ | $3.50x10^{-25}$ |
| Mutation* | $4.58x10^{-15}$ | $1.24x10^{-04}$ | $8.65x10^{-03}$ | $3.37x10^{-01}$ | $3.48x10^{-01}$ |
| Light + Glucose | $2.57x10^{-10}$ | $1.36x10^{-14}$ | $3.62x10^{-18}$ | $6.46x10^{-20}$ | $9.58x10^{-16}$ |
| Light + Mutation | $1.74x10^{-03}$ | $4.51x10^{-04}$ | $1.04x10^{-01}$ | $5.27x10^{-01}$ | $7.76x10^{-02}$ |
| Glucose + Mutation | $1.74x10^{-07}$ | $1.00x10^{-01}$ | $1.20x10^{-01}$ | $1.44x10^{-04}$ | $1.30x10^{-01}$ |

Fig. 14

| Mutant name | Description |
|---|---|
| SN5K | *Synechocystis* mutant with replacement of the neutral site N5 by a kanamycin resistance cassette. |
| SN8K | *Synechocystis* mutant with replacement of the neutral site N8 by a kanamycin resistance cassette. |
| SN10K | *Synechocystis* mutant with replacement of the neutral site N10 by a kanamycin resistance cassette. |
| SN15K | *Synechocystis* mutant with replacement of the neutral site N15 by a kanamycin resistance cassette. |
| SN16K | *Synechocystis* mutant with replacement of the neutral site N16 by a kanamycin resistance cassette. |
| SN5KS | *Synechocystis* mutant with replacement of the neutral site N5 by a kanamycin resistance/sucrose sensitivity cassette. |
| SN8KS | *Synechocystis* mutant with replacement of the neutral site N8 by a kanamycin resistance/sucrose sensitivity cassette. |
| SN10KS | *Synechocystis* mutant with replacement of the neutral site N10 by a kanamycin resistance/sucrose sensitivity cassette. |
| SN15KS | *Synechocystis* mutant with replacement of the neutral site N15 by a kanamycin resistance/sucrose sensitivity cassette. |
| SN16KS | *Synechocystis* mutant with replacement of the neutral site N16 by a kanamycin resistance/sucrose sensitivity cassette. |
| SN5K.gfp | *Synechocystis* mutant with replacement of the neutral site N5 by a kanamycin resistance cassette and the promoterless GFP encoding gene. |
| SN8K.gfp | *Synechocystis* mutant with replacement of the neutral site N8 by a kanamycin resistance cassette and the promoterless GFP encoding gene. |
| SN10K.gfp | *Synechocystis* mutant with replacement of the neutral site N10 by a kanamycin resistance cassette and the promoterless GFP encoding gene. |
| SN15K.gfp | *Synechocystis* mutant with replacement of the neutral site N15 by a kanamycin resistance cassette and the promoterless GFP encoding gene. |
| SN16K.gfp | *Synechocystis* mutant with replacement of the neutral site N16 by a kanamycin resistance cassette and the promoterless GFP encoding gene. |
| SN5K.Cgfp | *Synechocystis* mutant with replacement of the neutral site N5 by a kanamycin resistance cassette and the GFP encoding gene under the control of a constitutive promoter ($P_{psbA2*}$). |
| SN8K.Cgfp | *Synechocystis* mutant with replacement of the neutral site N8 by a kanamycin resistance cassette and the GFP encoding gene under the control of a constitutive promoter ($P_{psbA2*}$). |
| SN10K.Cgfp | *Synechocystis* mutant with replacement of the neutral site N10 by a kanamycin resistance cassette and the GFP encoding gene under the control of a constitutive promoter ($P_{psbA2*}$). |
| SN15K.Cgfp | *Synechocystis* mutant with replacement of the neutral site N15 by a kanamycin resistance cassette and the GFP encoding gene under the control of a constitutive promoter ($P_{psbA2*}$). |
| SN16K.Cgfp | *Synechocystis* mutant with replacement of the neutral site N16 by a kanamycin resistance cassette and the GFP encoding gene under the control of a constitutive promoter ($P_{psbA2*}$). |

**Fig. 15**

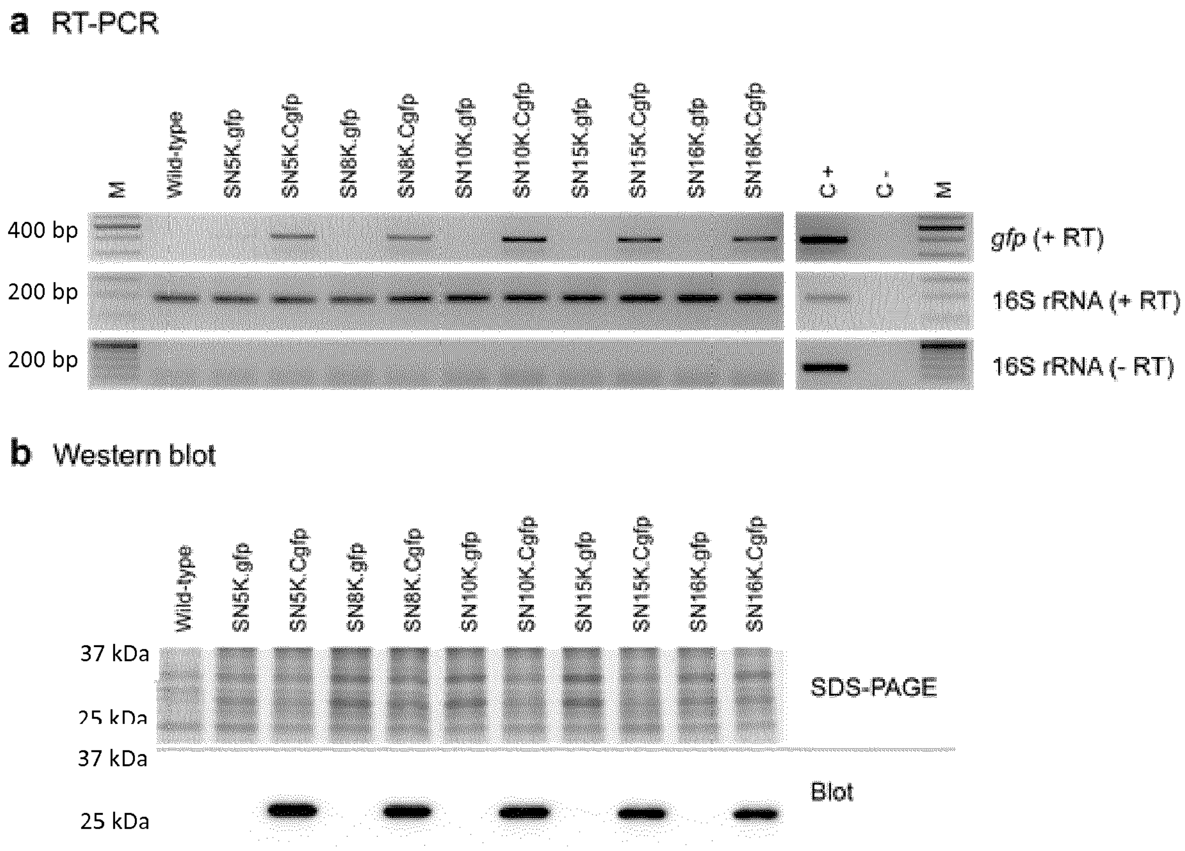
a RT-PCR
M
Wild-type
SN5K.gfp
SN5K.Cgfp
SN8K.gfp
SN8K.Cgfp
SN10K.gfp
SN10K.Cgfp
SN15K.gfp
SN15K.Cgfp
SN16K.gfp
SN16K.Cgfp
C +
C -
M
400 bp
200 bp
200 bp
gfp (+ RT)
16S rRNA (+ RT)
16S rRNA (- RT)
b Western blot
Wild-type
SN5K.gfp
SN5K.Cgfp
SN8K.gfp
SN8K.Cgfp
SN10K.gfp
SN10K.Cgfp
SN15K.gfp
SN15K.Cgfp
SN16K.gfp
SN16K.Cgfp
37 kDa
25 kDa
37 kDa
25 kDa
SDS-PAGE
Blot

**Fig. 16**

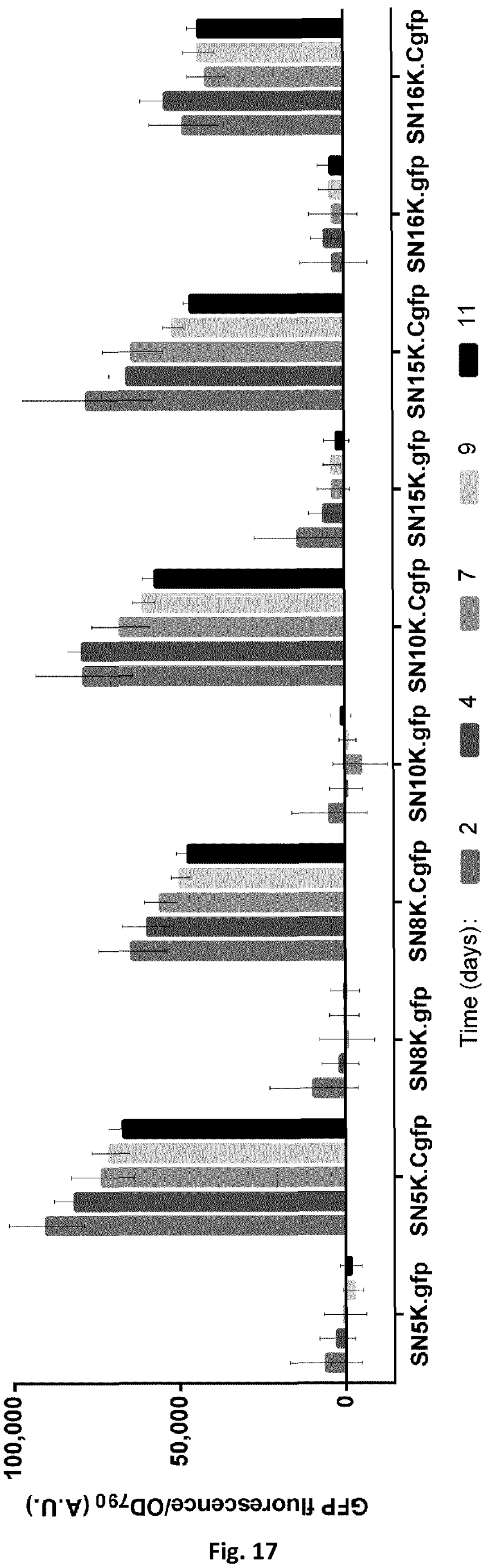
GFP fluorescence/$OD_{790}$ (A.U.)
100,000
50,000
0
SN5K.gfp
SN5K.Cgfp
SN8K.gfp
SN8K.Cgfp
SN10K.gfp
SN10K.Cgfp
SN15K.gfp
SN15K.Cgfp
SN16K.gfp
SN16K.Cgfp
Time (days):
2
4
7
9
11

Fig. 17

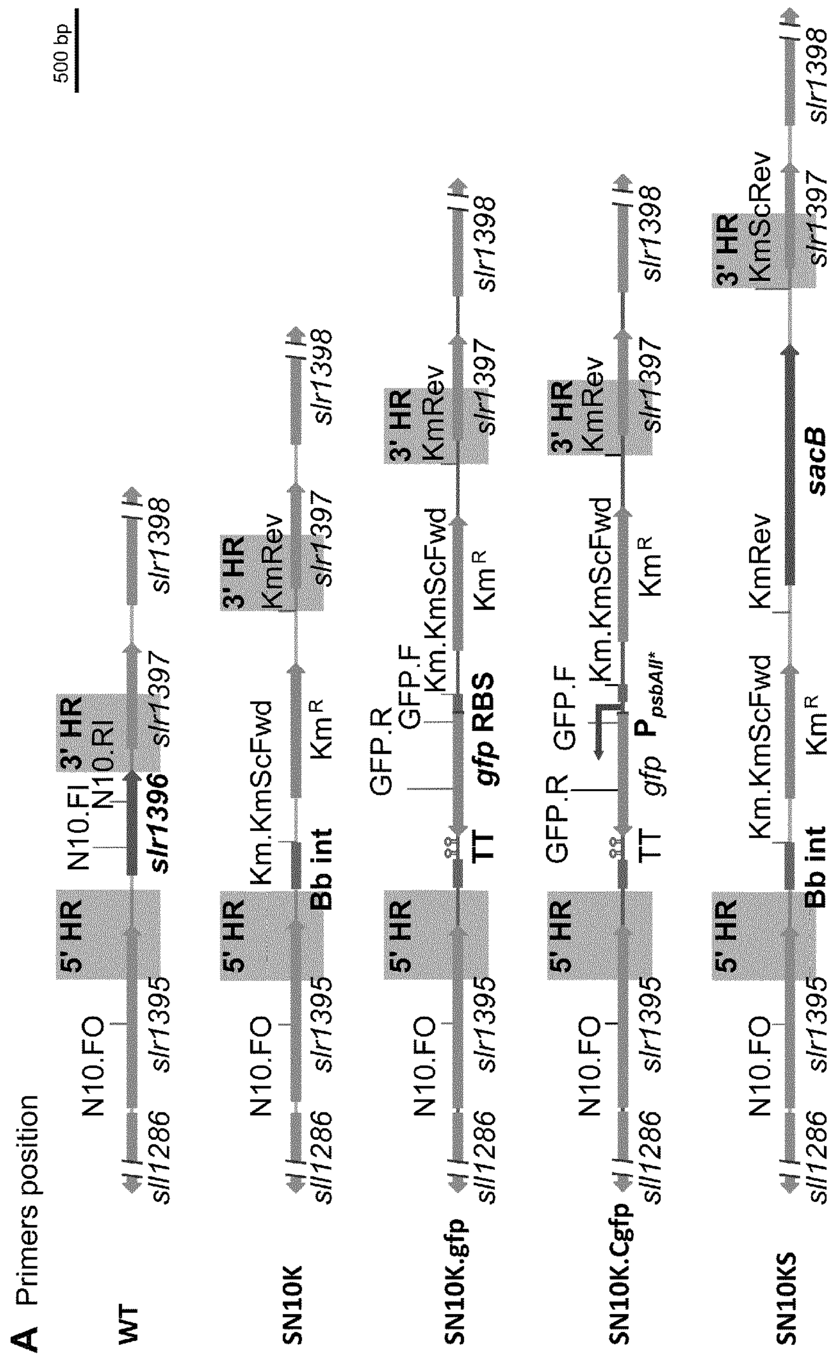
A Primers position
500 bp
WT
SN10K
SN10K.gfp
SN10K.Cgfp
SN10KS
sll1286
slr1395
slr1396
slr1397
slr1398
N10.FO
N10.FI
N10.RI
5' HR
3' HR
Bb int
Km.KmScFwd
KmRev
KmScRev
Km.KmScFwd
GFP.R
GFP.F
TT
gfp
RBS
KmR
PpsbAII*
sacB

Fig. 18 A

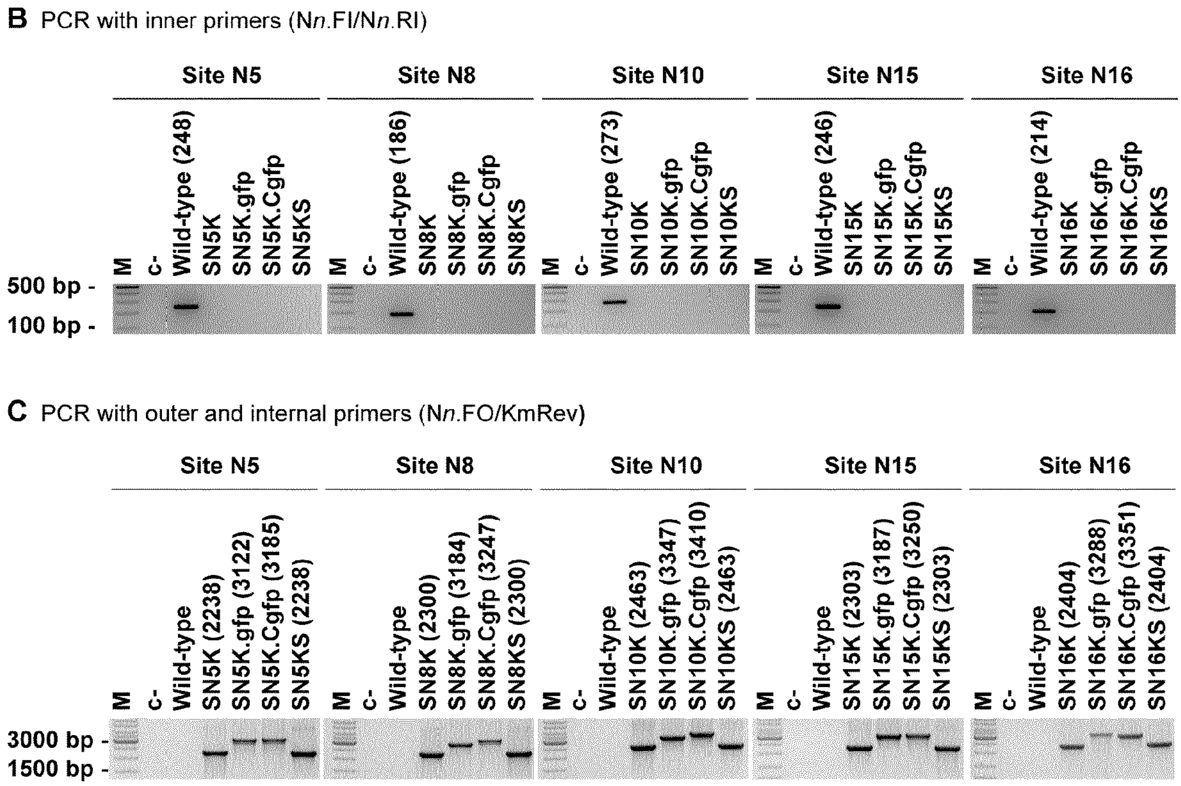


Fig. 18 B-C

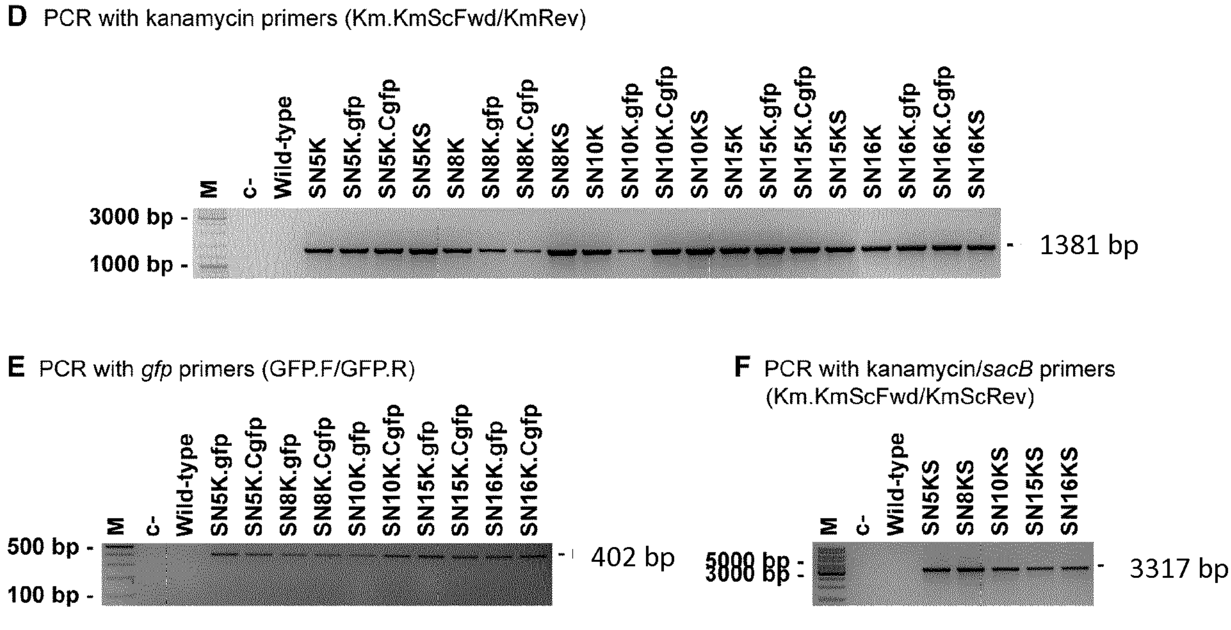


Fig. 18 D-F

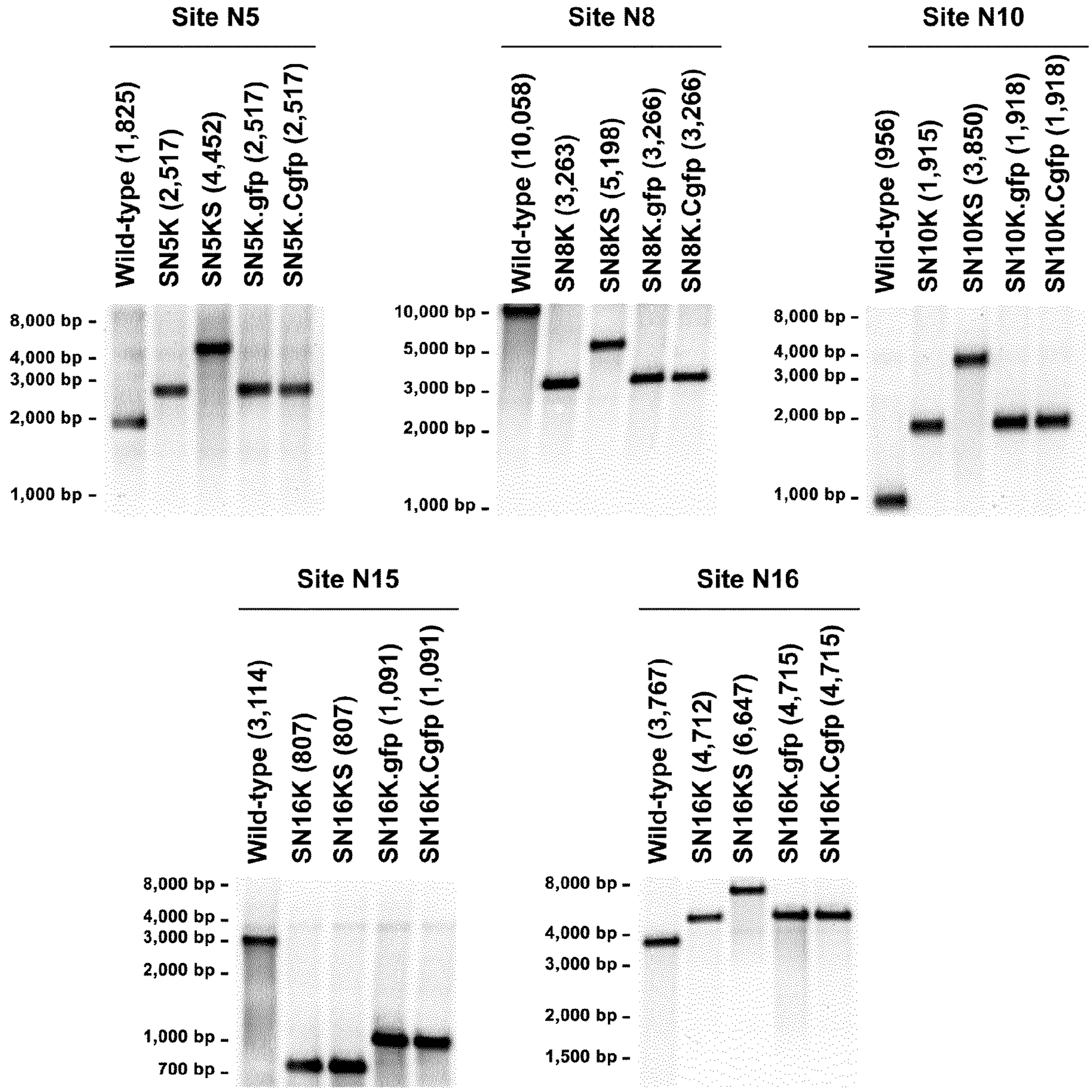
Site N5
Wild-type (1,825)
SN5K (2,517)
SN5KS (4,452)
SN5K.gfp (2,517)
SN5K.Cgfp (2,517)
8,000 bp
4,000 bp
3,000 bp
2,000 bp
1,000 bp
Site N8
Wild-type (10,058)
SN8K (3,263)
SN8KS (5,198)
SN8K.gfp (3,266)
SN8K.Cgfp (3,266)
10,000 bp
5,000 bp
3,000 bp
2,000 bp
1,000 bp
Site N10
Wild-type (956)
SN10K (1,915)
SN10KS (3,850)
SN10K.gfp (1,918)
SN10K.Cgfp (1,918)
8,000 bp
4,000 bp
3,000 bp
2,000 bp
1,000 bp
Site N15
Wild-type (3,114)
SN16K (807)
SN16KS (807)
SN16K.gfp (1,091)
SN16K.Cgfp (1,091)
8,000 bp
4,000 bp
3,000 bp
2,000 bp
1,000 bp
700 bp
Site N16
Wild-type (3,767)
SN16K (4,712)
SN16KS (6,647)
SN16K.gfp (4,715)
SN16K.Cgfp (4,715)
8,000 bp
4,000 bp
3,000 bp
2,000 bp
1,500 bp

Fig. 19

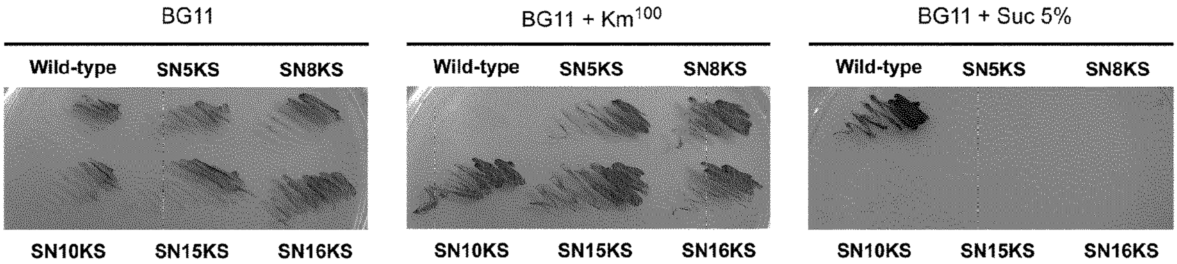
BG11
BG11 + Km100
BG11 + Suc 5%
Wild-type
SN5KS
SN8KS
SN10KS
SN15KS
SN16KS

Fig. 20

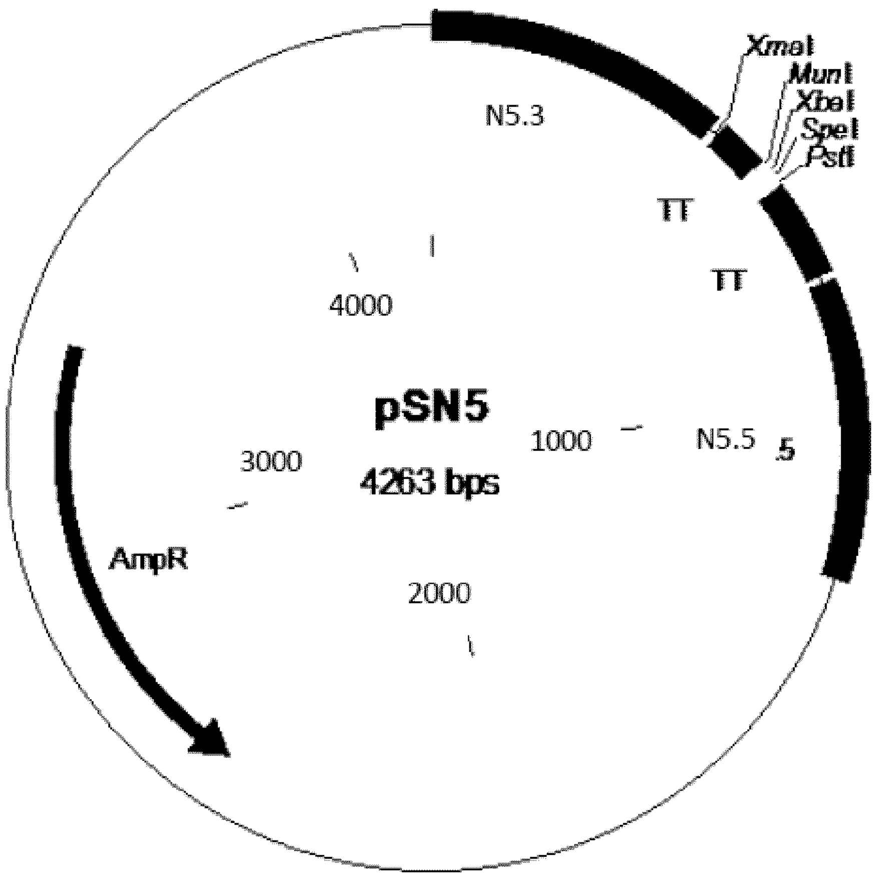
XmaI
MunI
XbaI
SpeI
PstI
N5.3
TT
TT
4000
pSN5
4263 bps
3000
1000
N5.5
AmpR
2000

Fig. 21

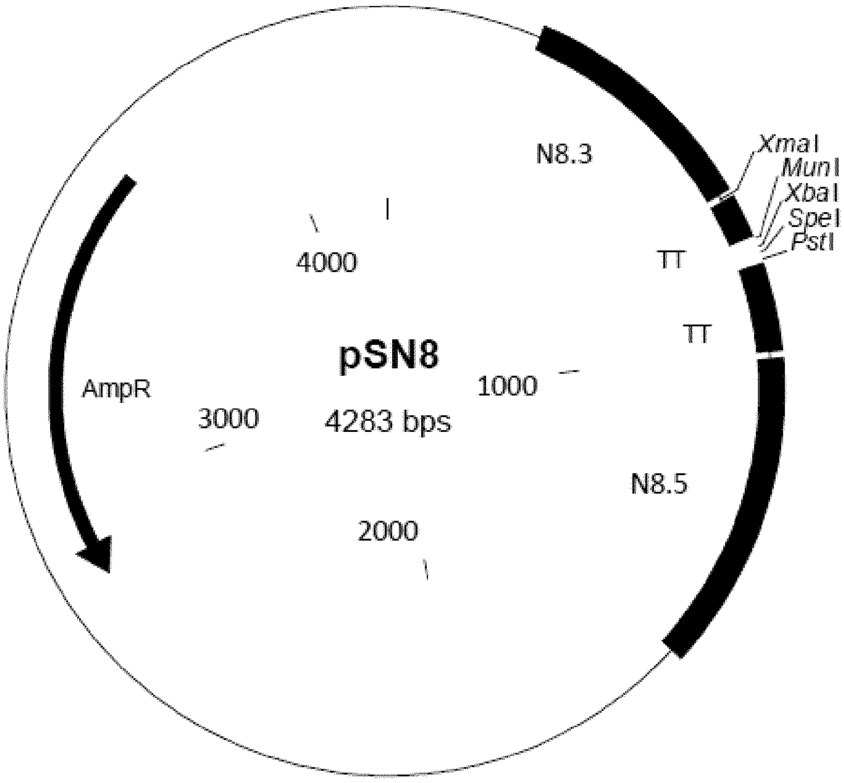
N8.3
XmaI
MunI
XbaI
SpeI
PstI
TT
TT
4000
pSN8
4283 bps
AmpR
3000
1000
N8.5
2000

**Fig. 22**

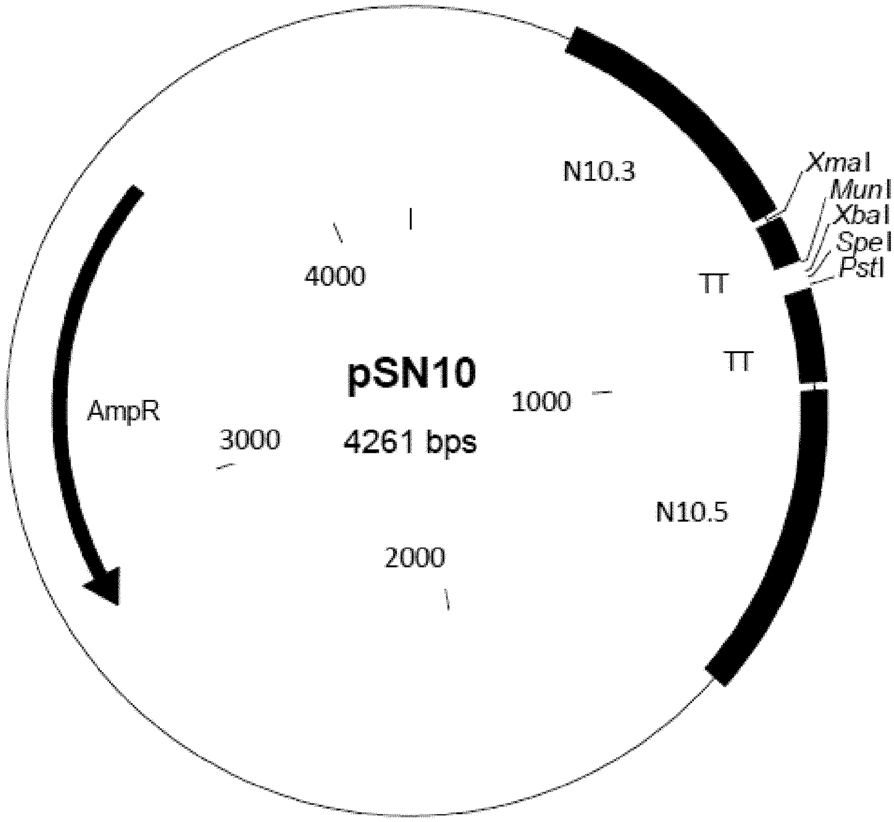
N10.3
XmaI
MunI
XbaI
SpeI
PstI
TT
TT
4000
pSN10
4261 bps
AmpR
3000
1000
N10.5
2000

**Fig. 23**

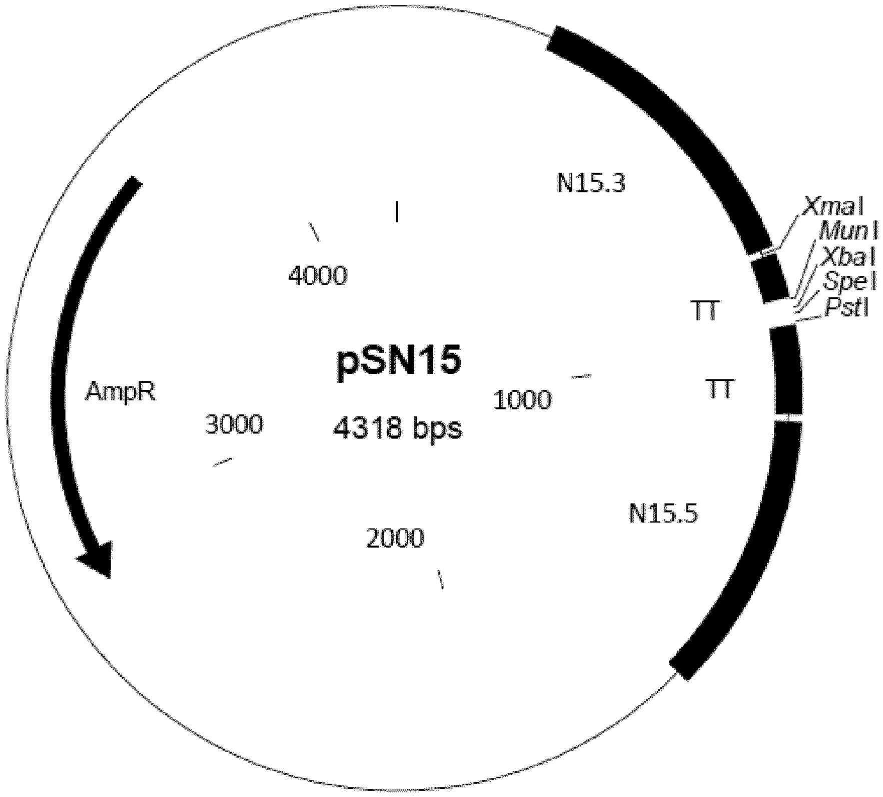
N15.3
XmaI
MunI
XbaI
SpeI
PstI
TT
TT
4000
pSN15
4318 bps
AmpR
3000
1000
2000
N15.5

Fig. 24

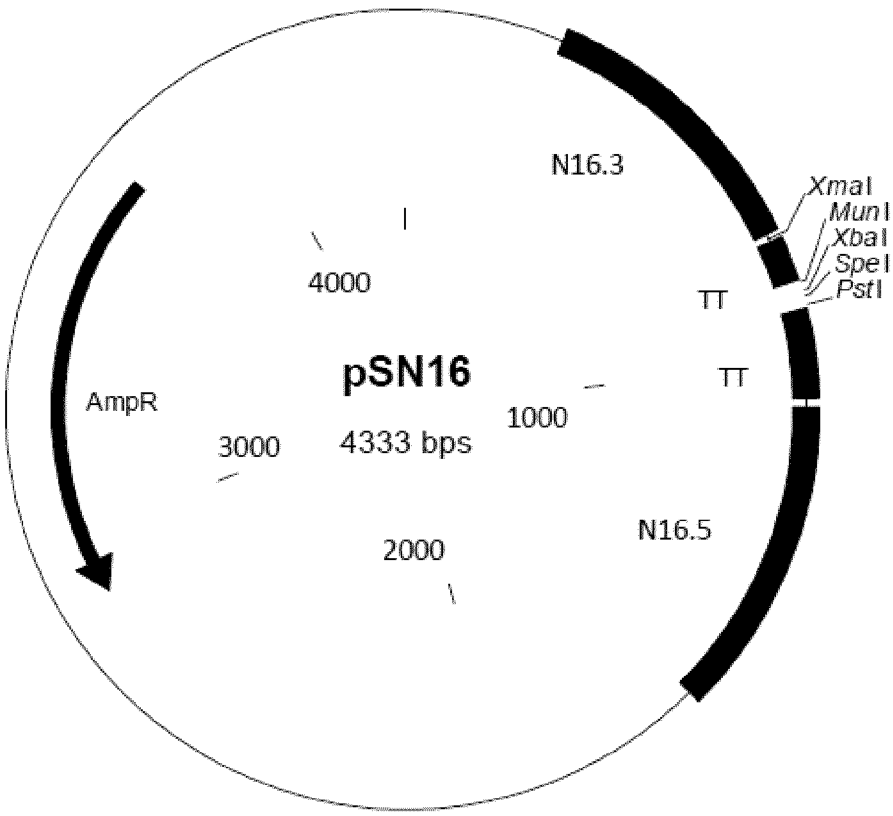
N16.3
XmaI
MunI
XbaI
SpeI
PstI
TT
TT
4000
pSN16
4333 bps
AmpR
3000
1000
2000
N16.5

Fig. 25

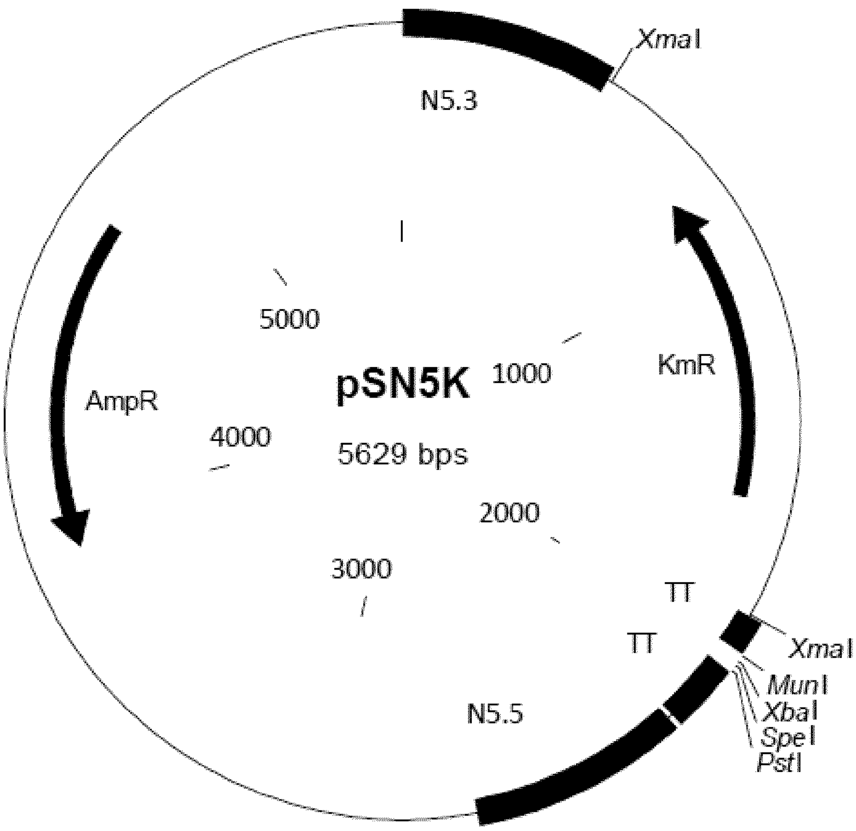
XmaI
N5.3
5000
1000
KmR
AmpR
pSN5K
4000
5629 bps
2000
3000
TT
TT
XmaI
MunI
XbaI
SpeI
PstI
N5.5

Fig. 26

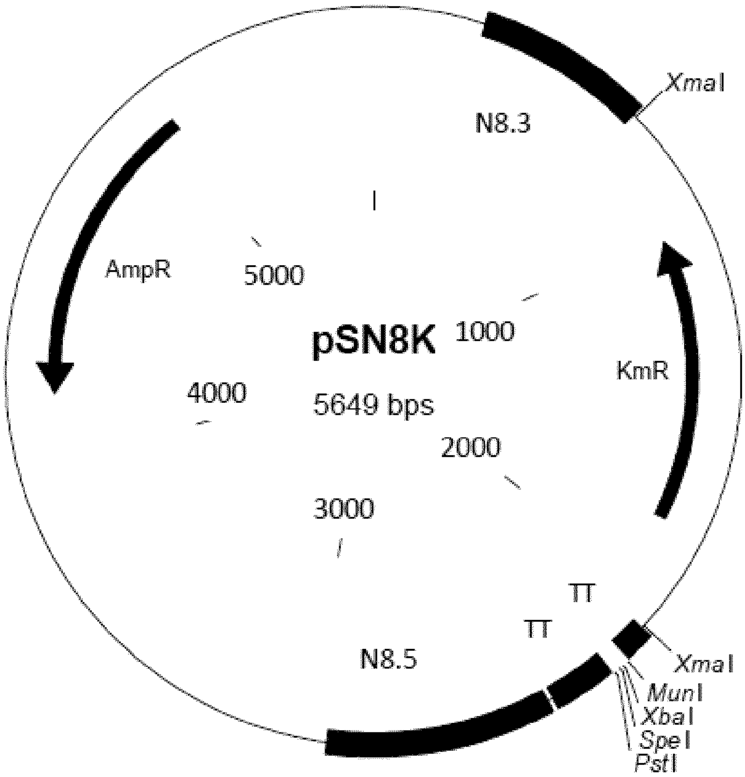
XmaI
N8.3
AmpR
5000
pSN8K
1000
KmR
4000
5649 bps
2000
3000
TT
TT
N8.5
XmaI
MunI
XbaI
SpeI
PstI

Fig. 27

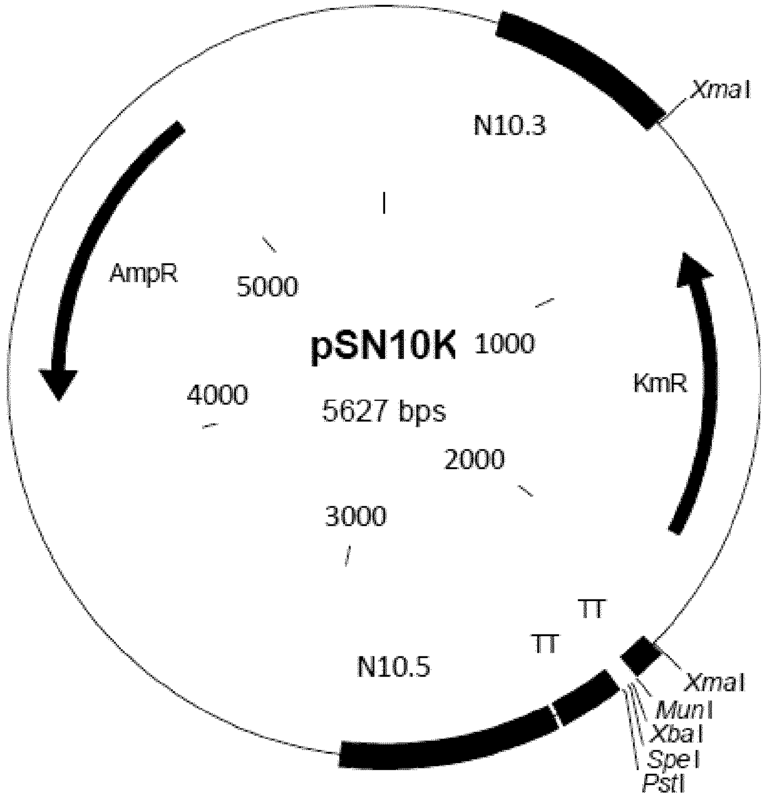
XmaI
N10.3
AmpR
5000
pSN10K
1000
4000
5627 bps
KmR
2000
3000
TT
TT
N10.5
XmaI
MunI
XbaI
SpeI
PstI

Fig. 28

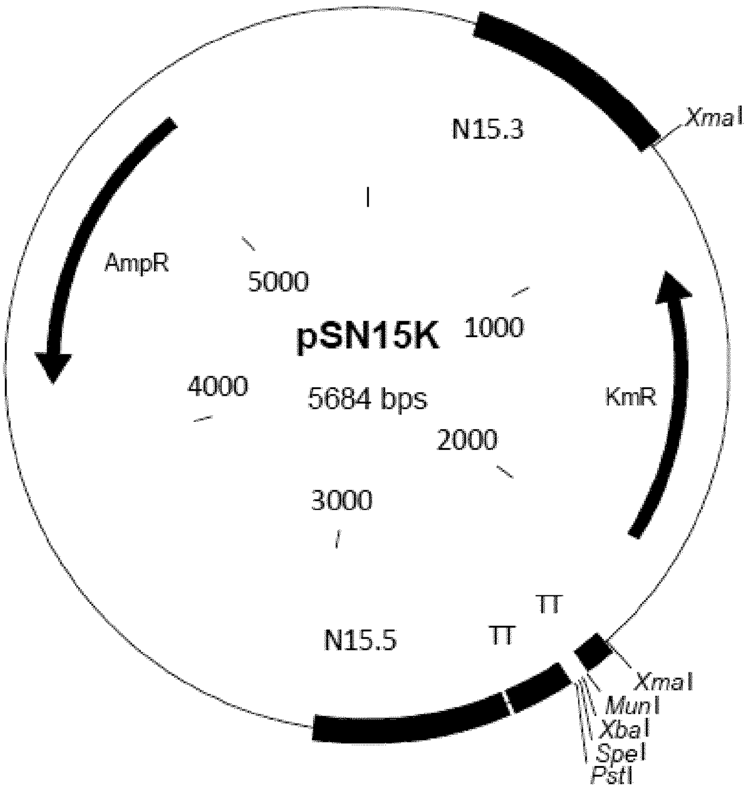
XmaI
N15.3
AmpR
5000
pSN15K
1000
4000
5684 bps
KmR
2000
3000
TT
TT
N15.5
XmaI
MunI
XbaI
SpeI
PstI

Fig. 29

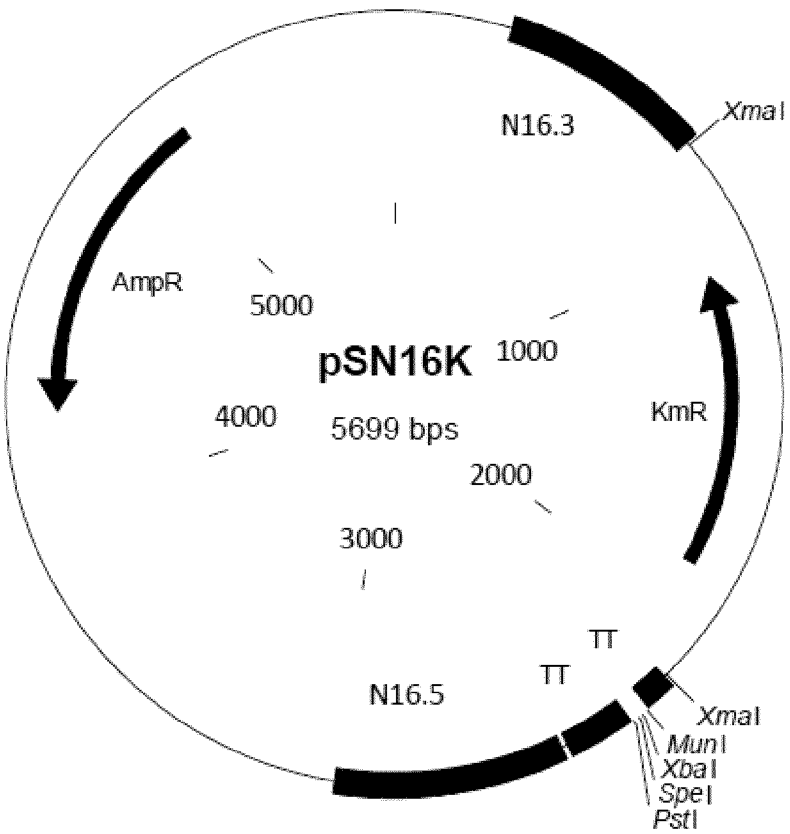
N16.3
XmaI
AmpR
5000
pSN16K
1000
4000
5699 bps
KmR
2000
3000
TT
TT
N16.5
XmaI
MunI
XbaI
SpeI
PstI

Fig. 30

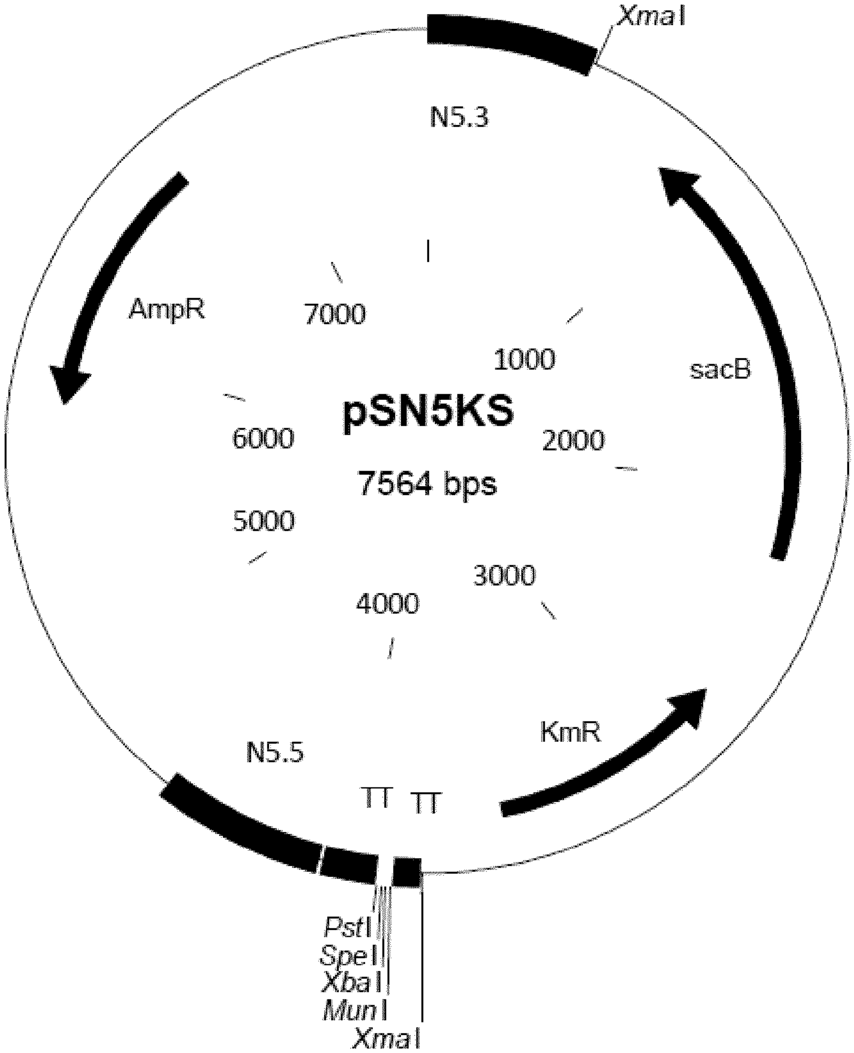
XmaI
N5.3
AmpR
7000
1000
sacB
pSN5KS
6000
2000
7564 bps
5000
3000
4000
KmR
N5.5
TT
TT
PstI
SpeI
XbaI
MunI
XmaI

Fig. 31

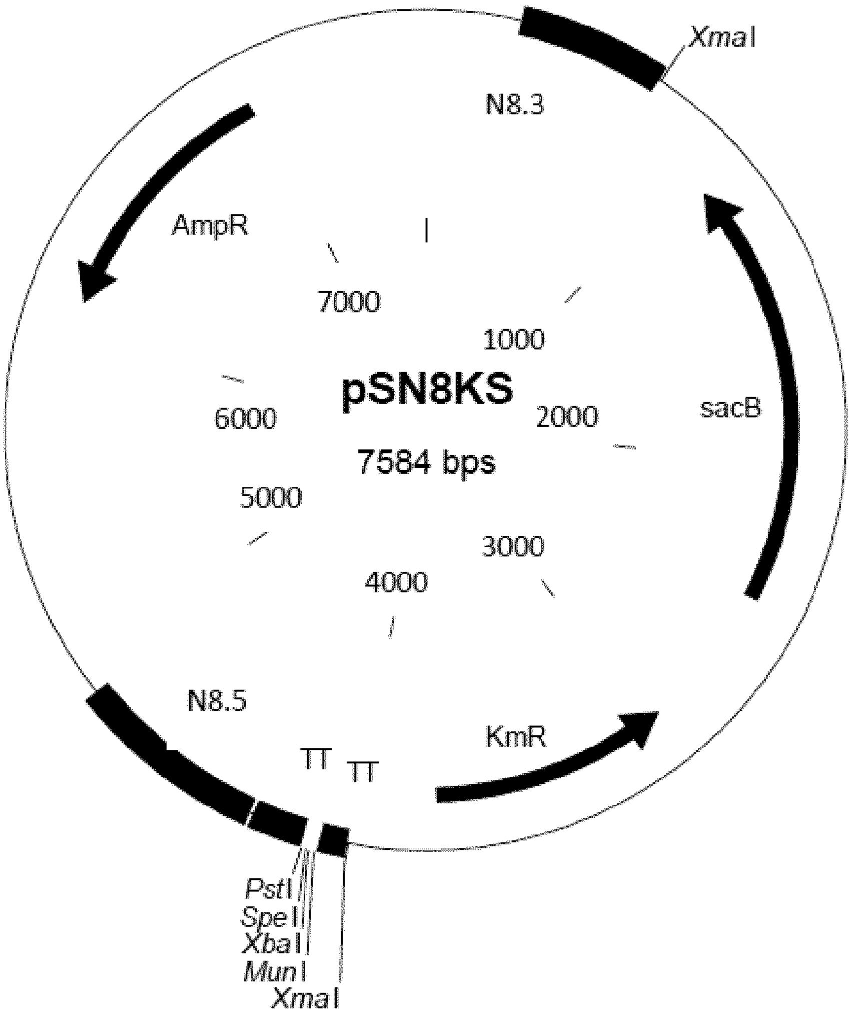
XmaI
N8.3
AmpR
7000
1000
pSN8KS
7584 bps
6000
2000
sacB
5000
3000
4000
N8.5
TT
TT
KmR
PstI
SpeI
XbaI
MunI
XmaI

Fig. 32

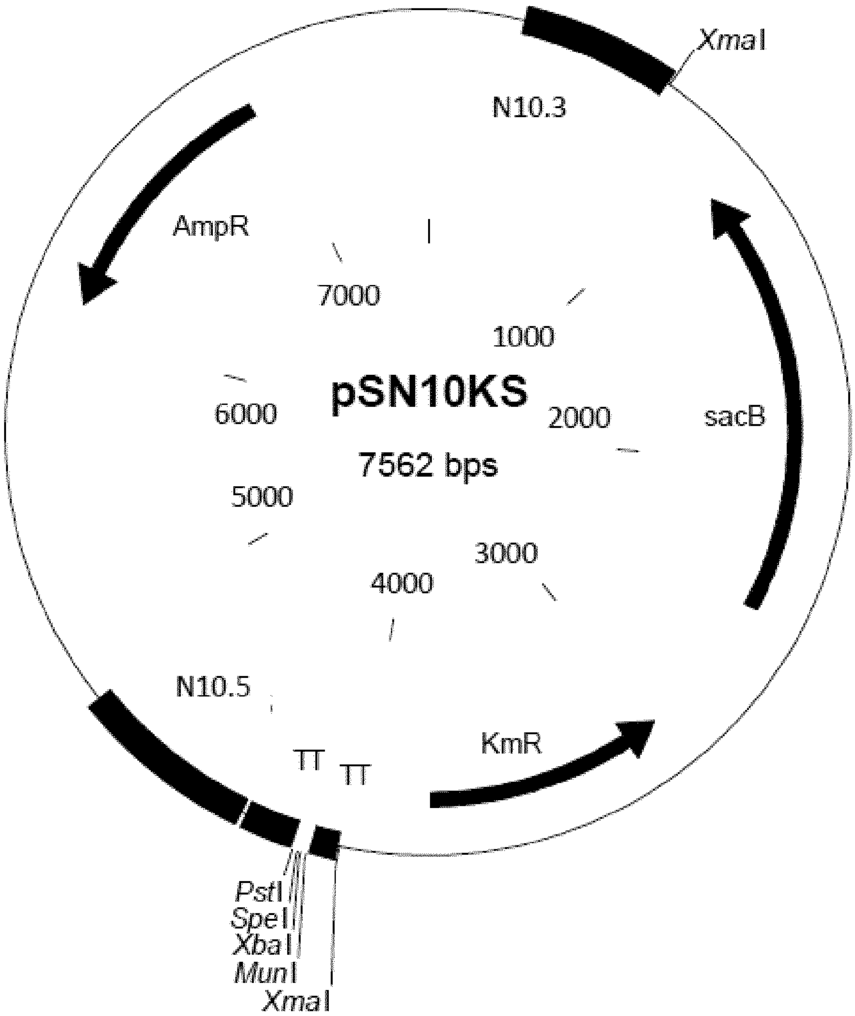
XmaI
N10.3
AmpR
7000
1000
pSN10KS
6000
2000
sacB
7562 bps
5000
3000
4000
N10.5
TT
TT
KmR
PstI
SpeI
XbaI
MunI
XmaI

Fig. 33

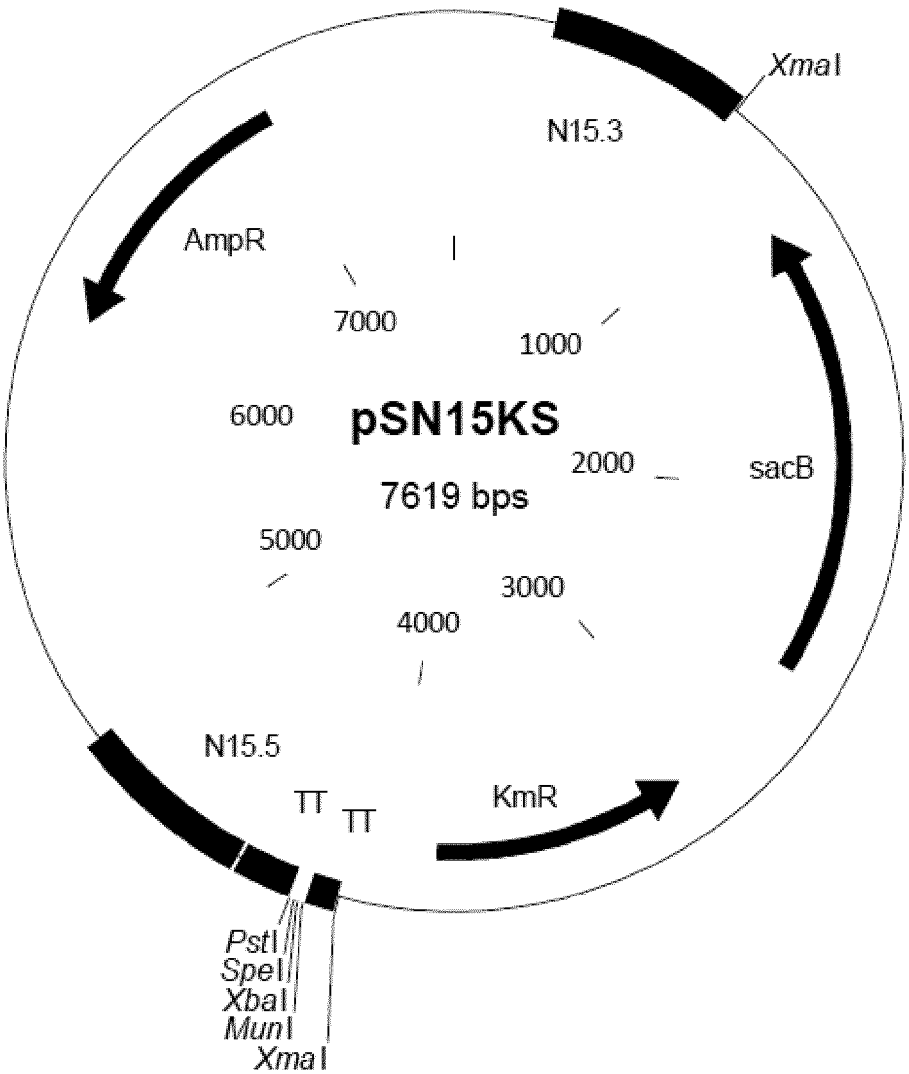
XmaI
N15.3
AmpR
7000
1000
6000
pSN15KS
7619 bps
2000
sacB
5000
3000
4000
N15.5
TT
TT
KmR
PstI
SpeI
XbaI
MunI
XmaI

Fig. 34

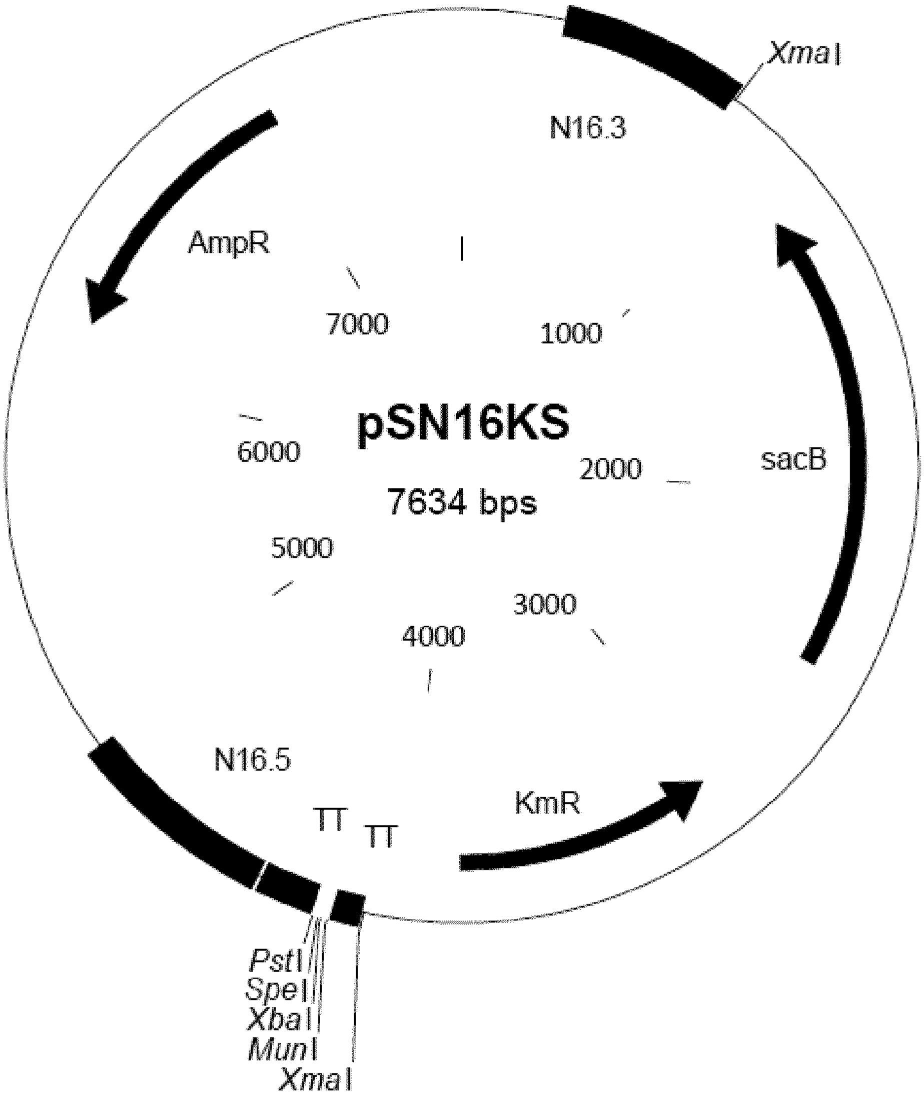
XmaI
N16.3
AmpR
7000
1000
pSN16KS
6000
2000
sacB
7634 bps
5000
3000
4000
N16.5
TT
TT
KmR
PstI
SpeI
XbaI
MunI
XmaI

Fig. 35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- PT 108564


### Non-patent literature cited in the description


- **KUNERT A et al.** Construction of promoter probe vectors for Synechocystis sp. PCC 6803 using the light-emitting reporter systems Gfp and LuxAB. *Journal of Microbiological Methods,* August 2000, vol. 41 (3
- **BENTLEY FIONA K et al.** Heterologous expression of the mevalonic acid pathway in cyanobacteria enhances endogenous carbon partitioning to isoprene. *MOLECULAR PLANT,* January 2014, vol. 7 (1

SEQUENCE LISTING

<110> Instituto de Biologia Molecular e Celular

<120> A CONSTRUCTION, A VECTOR AND A METHOD PRODUCTION OF A TARGET MOLECULE IN A CYANOBACTERIUM

<130> P274.4 EP2

<150> PT108564
<151> 2015-06-19

<160> 115

<170> BiSSAP 1.3.6

<210> 1
<211> 4263
<212> DNA
<213> synthetic construct

<220>
<223> >SEQ ID NO 1

<4> 1

```
tgtccaaatc agcattgctc tgccaggtga gacggagttt ttgtcccact aatccctgtt      60
ggatagaagg cgcttgatac agttccaacc aaacccaatc tcctgtcctg gctttagttt     120
cttccaccgt cggcaggatt aaacgaccca accactctcc caaaggccga taatacgcct     180
catctaaatt ctgttgcaga ggataataat ctacctggtt ggcgggcaac tgactgctaa     240
tttgataatc cgggatgcgg ccagaggttt cctgaggttg aaacagcgta gctagggaaa     300
tcaccaccag agcggcgatc gccaccaata ccatccgcca gcgccattgc ttcatctgtg     360
gactgcccta aaaattgcca taaaaaaaca cctggggcgt ctttccttgt ttcgactcca     420
gatgtttttc taatttcctc acacctgtcc taagtataaa tcaccaacgg ggattgtcaa     480
ggcttcagga ttacacccgg gaaaaaaaaa ccccgcccct gacagggcgg ggtttttttt     540
cagataaaaa aaatccttag ctttcgctaa ggatgatttc tgcaattggc ggccgcttct     600
agaatgcact agtagcggcc gctgcagtcc ggcaaaaaaa cgggcaaggt gtcaccaccc     660
tgcccttttt ctttaaaacc gaaaagatta cttcgcgttg gagagcgttc accgacaaac     720
aacagataaa acgaaaggcc cagtctttcg actgagcctt tcgttttatt tgatgcctgg     780
taccgggtgt aatcggttga ttatttcagt ggcccggccg atggataatt accatggccc     840
gaccgaccgg gatttccaga ccagtttgaa cagtattaaa ttagcctttc cggacaaggc     900
ctctgtgaaa atcttaaaaa agtattaaga ttttaaaagc tagcttattt tcggaggaaa     960
tgtgtttact cgacttgccc agcaacaccg cgatttcgtg aaggatttag tcatgagctt    1020
gagggctttg gccactgtgc tcgaaaatcg gggctacatt gcttcttgct acacctgtgg    1080
```

cgaccaactc aacagcgcct ccttcatggt gagcttgggg gaaaatcatc tgatccgctt 1140
tttggtatcg gactacggca tcacctggac agaaatgcgg gatgaccgag aattaatgaa 1200
attagaagga gccgaggcga tcgcccagtt ggaagaattg gccaatgtgg tcaaatattg 1260
cctagcagat gcccccactc gaccatatgg gagagctccc aacgcgttgg atgcatagct 1320
tgagtattct atagtgtcac ctaaatagct tggcgtaatc atggtcatag ctgtttcctg 1380
tgtgaaattg ttatccgctc acaattccac acaacatacg agccggaagc ataaagtgta 1440
aagcctgggg tgcctaatga gtgagctaac tcacattaat tgcgttgcgc tcactgcccg 1500
ctttccagtc gggaaacctg tcgtgccagc tgcattaatg aatcggccaa cgcgcgggga 1560
gaggcggttt gcgtattggg cgctcttccg cttcctcgct cactgactcg ctgcgctcgg 1620
tcgttcggct gcggcgagcg gtatcagctc actcaaaggc ggtaatacgg ttatccacag 1680
aatcagggga taacgcagga aagaacatgt gagcaaaagg ccagcaaaag gccaggaacc 1740
gtaaaaaggc cgcgttgctg gcgtttttcc ataggctccg cccccctgac gagcatcaca 1800
aaaatcgacg ctcaagtcag aggtggcgaa acccgacagg actataaaga taccaggcgt 1860
ttccccctgg aagctccctc gtgcgctctc ctgttccgac cctgccgctt accggatacc 1920
tgtccgcctt tctcccttcg ggaagcgtgg cgctttctca tagctcacgc tgtaggtatc 1980
tcagttcggt gtaggtcgtt cgctccaagc tgggctgtgt gcacgaaccc cccgttcagc 2040
ccgaccgctg cgccttatcc ggtaactatc gtcttgagtc caacccggta agacacgact 2100
tatcgccact ggcagcagcc actggtaaca ggattagcag agcgaggtat gtaggcggtg 2160
ctacagagtt cttgaagtgg tggcctaact acggctacac tagaagaaca gtatttggta 2220
tctgcgctct gctgaagcca gttaccttcg gaaaaagagt tggtagctct tgatccggca 2280
aacaaaccac cgctggtagc ggtggttttt ttgtttgcaa gcagcagatt acgcgcagaa 2340
aaaaaggatc tcaagaagat cctttgatct tttctacggg gtctgacgct cagtggaacg 2400
aaaactcacg ttaagggatt ttggtcatga gattatcaaa aaggatcttc acctagatcc 2460
ttttaaatta aaaatgaagt tttaaatcaa tctaaagtat atatgagtaa acttggtctg 2520
acagttacca atgcttaatc agtgaggcac ctatctcagc gatctgtcta tttcgttcat 2580
ccatagttgc ctgactcccc gtcgtgtaga taactacgat acgggagggc ttaccatctg 2640
gccccagtgc tgcaatgata ccgcgagacc cacgctcacc ggctccagat ttatcagcaa 2700
taaaccagcc agccggaagg gccgagcgca gaagtggtcc tgcaacttta tccgcctcca 2760
tccagtctat taattgttgc cgggaagcta gagtaagtag ttcgccagtt aatagtttgc 2820
gcaacgttgt tgccattgct acaggcatcg tggtgtcacg ctcgtcgttt ggtatggctt 2880
cattcagctc cggttcccaa cgatcaaggc gagttacatg atcccccatg ttgtgcaaaa 2940
aagcggttag ctccttcggt cctccgatcg ttgtcagaag taagttggcc gcagtgttat 3000

cactcatggt tatggcagca ctgcataatt ctcttactgt catgccatcc gtaagatgct 3060

tttctgtgac tggtgagtac tcaaccaagt cattctgaga atagtgtatg cggcgaccga 3120

gttgctcttg cccggcgtca atacgggata ataccgcgcc acatagcaga actttaaaag 3180

tgctcatcat tggaaaacgt tcttcggggc gaaaactctc aaggatctta ccgctgttga 3240

gatccagttc gatgtaaccc actcgtgcac ccaactgatc ttcagcatct tttactttca 3300

ccagcgtttc tgggtgagca aaaacaggaa ggcaaaatgc cgcaaaaaag ggaataaggg 3360

cgacacggaa atgttgaata ctcatactct tcctttttca atattattga agcatttatc 3420

agggttattg tctcatgagc ggatacatat ttgaatgtat ttagaaaaat aaacaaatag 3480

gggttccgcg cacatttccc cgaaaagtgc cacctgatgc ggtgtgaaat accgcacaga 3540

tgcgtaagga gaaaataccg catcaggaaa ttgtaagcgt taatattttg ttaaaattcg 3600

cgttaaattt ttgttaaatc agctcatttt ttaaccaata ggccgaaatc ggcaaaatcc 3660

cttataaatc aaaagaatag accgagatag ggttgagtgt tgttccagtt tggaacaaga 3720

gtccactatt aaagaacgtg gactccaacg tcaaagggcg aaaaaccgtc tatcagggcg 3780

atggcccact acgtgaacca tcaccctaat caagtttttt ggggtcgagg tgccgtaaag 3840

cactaaatcg gaaccctaaa gggagccccc gatttagagc ttgacgggga aagccggcga 3900

acgtggcgag aaaggaaggg aagaaagcga aaggagcggg cgctagggcg ctggcaagtg 3960

tagcggtcac gctgcgcgta accaccacac ccgccgcgct taatgcgccg ctacagggcg 4020

cgtccattcg ccattcaggc tgcgcaactg ttgggaaggg cgatcggtgc gggcctcttc 4080

gctattacgc cagctggcga aaggggggatg tgctgcaagg cgattaagtt gggtaacgcc 4140

agggttttcc cagtcacgac gttgtaaaac gacggccagt gaattgtaat acgactcact 4200

atagggcgaa ttgggcccga cgtcgcatgc tcccggccgc catggcggcc gcgggaattc 4260

gat 4263

<210> 2
<211> 4283
<212> DNA
<213> synthetic construct

<220>
<223> >SEQ ID NO 2

<400> 2

```
cacccgccgc gcttaatgcg ccgctacagg gcgcgtccat tcgccattca ggctgcgcaa     60
ctgttgggaa gggcgatcgg tgcgggcctc ttcgctatta cgccagctgg cgaaaggggg    120
atgtgctgca aggcgattaa gttgggtaac gccagggttt tcccagtcac gacgttgtaa    180
aacgacggcc agtgaattgt aatacgactc actatagggc gaattgggcc cgacgtcgca    240
```

tgctcccggc cgccatggcg gccgcgggaa ttcgattgat gaccgctggc ggagtttagt 300
ccagggttat ttgtaccgtc aaggttacat tgccacccca gatttaacta cgctgcgagt 360
ccgtaccatt ggcgatcggg cctatctcac cattaagggt aaaaatgcca gcattgcccg 420
cttggatttg agtatgaaat tcttgcagtg gaagccaatt aaattttgac agaactctgt 480
tcaccgcccc tgattgaaaa tatcgttatt gcctcgatta ccatggtaaa acctgagaag 540
tagacgagtt tttgggggat aaccagggtc taattttagc ggaagtggaa ttaacctaca 600
ctggtgaaaa aataagtcta cttccctgga tcggggaaga ggtaacggat gatgcccgct 660
attacaacgt caatttagcc caacatccct acaaaaattg gtgacaagat tacacccggg 720
aaaaaaaaac cccgcccctg acagggcggg gtttttttc agataaaaaa aatccttagc 780
tttcgctaag gatgatttct gcaattggcg gccgcttcta gaatgcacta gtagcggccg 840
ctgcagtccg gcaaaaaaac gggcaaggtg tcaccaccct gccctttttc tttaaaaccg 900
aaaagattac ttcgcgttgg agagcgttca ccgacaaaca acagataaaa cgaaaggccc 960
agtctttcga ctgagccttt cgttttattt gatgcctggt accgggtgta atccttcatt 1020
cccctattgt tattaatagt gctctgaaac taacaaataa gcaagatcaa attgtgaaga 1080
aaacgcaaca aaatgaacca ttaagataat ttctgtctat cctggctggt atattccagt 1140
ctactcagaa atgagtttgt tttatgacaa atcagtatgt accaaattac ttggcgcaat 1200
cgatcctggt aactctattt tgttgcttac ccctgggtat tgtggccatt atcaaagcat 1260
cggaagttaa ttctcgttta gcttcagggg actatgaagg cgctgtaaag gcttccaagg 1320
aagcgaaaaa gttttgttgg tggtcctttg gtgccggcat aattttcatt gccatctatt 1380
ttgtgctagt ggttattgcc gccgtctttg gtcagtaatt aaagttacat tttttgactt 1440
tgccttgttc accattcatt aacgaatacc atgtttagtt tgaaaaatta ttccccatct 1500
ccattactat ccgctaaggc caaggaatat ttagtattca ctttggtaac cctaaccatt 1560
gtcgttgctg gcactcgacc atatgggaga gctcccaacg cgttggatgc atagcttgag 1620
tattctatag tgtcacctaa atagcttggc gtaatcatgg tcatagctgt ttcctgtgtg 1680
aaattgttat ccgctcacaa ttccacacaa catacgagcc ggaagcataa agtgtaaagc 1740
ctggggtgcc taatgagtga gctaactcac attaattgcg ttgcgctcac tgcccgcttt 1800
ccagtcggga aacctgtcgt gccagctgca ttaatgaatc ggccaacgcg cggggagagg 1860
cggtttgcgt attgggcgct cttccgcttc ctcgctcact gactcgctgc gctcggtcgt 1920
tcggctgcgg cgagcggtat cagctcactc aaaggcggta atacggttat ccacagaatc 1980
aggggataac gcaggaaaga acatgtgagc aaaaggccag caaaaggcca ggaaccgtaa 2040
aaaggccgcg ttgctggcgt ttttccatag gctccgcccc cctgacgagc atcacaaaaa 2100

tcgacgctca agtcagaggt ggcgaaaccc gacaggacta taaagatacc aggcgtttcc 2160
ccctggaagc tccctcgtgc gctctcctgt tccgaccctg ccgcttaccg gatacctgtc 2220
cgcctttctc ccttcgggaa gcgtggcgct ttctcatagc tcacgctgta ggtatctcag 2280
ttcggtgtag gtcgttcgct ccaagctggg ctgtgtgcac gaaccccccg ttcagcccga 2340
ccgctgcgcc ttatccggta actatcgtct tgagtccaac ccggtaagac acgacttatc 2400
gccactggca gcagccactg gtaacaggat tagcagagcg aggtatgtag gcggtgctac 2460
agagttcttg aagtggtggc ctaactacgg ctacactaga agaacagtat ttggtatctg 2520
cgctctgctg aagccagtta ccttcggaaa aagagttggt agctcttgat ccggcaaaca 2580
aaccaccgct ggtagcggtg gtttttttgt ttgcaagcag cagattacgc gcagaaaaaa 2640
aggatctcaa gaagatcctt tgatcttttc tacggggtct gacgctcagt ggaacgaaaa 2700
ctcacgttaa gggattttgg tcatgagatt atcaaaaagg atcttcacct agatcctttt 2760
aaattaaaaa tgaagtttta aatcaatcta aagtatatat gagtaaactt ggtctgacag 2820
ttaccaatgc ttaatcagtg aggcacctat ctcagcgatc tgtctatttc gttcatccat 2880
agttgcctga ctccccgtcg tgtagataac tacgatacgg gagggcttac catctggccc 2940
cagtgctgca atgataccgc gagacccacg ctcaccggct ccagatttat cagcaataaa 3000
ccagccagcc ggaagggccg agcgcagaag tggtcctgca actttatccg cctccatcca 3060
gtctattaat tgttgccggg aagctagagt aagtagttcg ccagttaata gtttgcgcaa 3120
cgttgttgcc attgctacag gcatcgtggt gtcacgctcg tcgtttggta tggcttcatt 3180
cagctccggt tcccaacgat caaggcgagt tacatgatcc cccatgttgt gcaaaaaagc 3240
ggttagctcc ttcggtcctc cgatcgttgt cagaagtaag ttggccgcag tgttatcact 3300
catggttatg gcagcactgc ataattctct tactgtcatg ccatccgtaa gatgcttttc 3360
tgtgactggt gagtactcaa ccaagtcatt ctgagaatag tgtatgcggc gaccgagttg 3420
ctcttgcccg gcgtcaatac gggataatac cgcgccacat agcagaactt taaaagtgct 3480
catcattgga aaacgttctt cggggcgaaa actctcaagg atcttaccgc tgttgagatc 3540
cagttcgatg taacccactc gtgcacccaa ctgatcttca gcatctttta ctttcaccag 3600
cgtttctggg tgagcaaaaa caggaaggca aaatgccgca aaaaagggaa taagggcgac 3660
acggaaatgt tgaatactca tactcttcct ttttcaatat tattgaagca tttatcaggg 3720
ttattgtctc atgagcggat acatatttga atgtatttag aaaaataaac aaataggggt 3780
tccgcgcaca tttccccgaa aagtgccacc tgatgcggtg tgaaataccg cacagatgcg 3840
taaggagaaa ataccgcatc aggaaattgt aagcgttaat attttgttaa aattcgcgtt 3900
aaatttttgt taaatcagct cattttttaa ccaataggcc gaaatcggca aaatccctta 3960
taaatcaaaa gaatagaccg agatagggtt gagtgttgtt ccagtttgga acaagagtcc 4020

actattaaag aacgtggact ccaacgtcaa agggcgaaaa accgtctatc agggcgatgg 4080

cccactacgt gaaccatcac cctaatcaag ttttttgggg tcgaggtgcc gtaaagcact 4140

aaatcggaac cctaaaggga gcccccgatt tagagcttga cggggaaagc cggcgaacgt 4200

ggcgagaaag gaagggaaga aagcgaaagg agcgggcgct agggcgctgg caagtgtagc 4260

ggtcacgctg cgcgtaacca cca 4283

<210> 3
<211> 4261
<212> DNA
<213> synthetic construct

<220>
<223> >SEQ ID NO 3

<400> 3

cacccgccgc gcttaatgcg ccgctacagg gcgcgtccat tcgccattca ggctgcgcaa 60
ctgttgggaa gggcgatcgg tgcgggcctc ttcgctatta cgccagctgg cgaaaggggg 120
atgtgctgca aggcgattaa gttgggtaac gccagggttt tcccagtcac gacgttgtaa 180
aacgacggcc agtgaattgt aatacgactc actatagggc gaattgggcc cgacgtcgca 240
tgctcccggc cgccatggcg gccgcgggaa ttcgattgga ctgacccaag atacagtggt 300
agcttcaagt tcgtcaatca accccaatgg aaagctgaga taatctgttg ttgatgcgac 360
cgtcttcatt ttagctaaaa agacaagtct gtggctagtt actatgacga ggccatcggg 420
tctatcccag cggtcttgct gattccaaaa cttggcgatt ttctgaagta gtatcttttc 480
accgcttaag ccgccaaggt cttggatgag cttggtcgaa tcatcttttg atgcctcgac 540
atcgcgaata actttgtaaa cgaccttccc gagcttgaat gcaaagtcgg ttagattatc 600
catcagagtt gaatgccgat aatacagact gctatactat tttaatgaaa agagtgctgg 660
caagcaagcc cgccgaacgt tgaagctgtg cagcgcaaac gaagcgcatt gtagtttgcg 720
ctcggattac acccgggaaa aaaaaacccc gcccctgaca gggcggggtt ttttttcaga 780
taaaaaaaat ccttagcttt cgctaaggat gatttctgca attggcggcc gcttctagaa 840
tgcactagta gcggccgctg cagtccggca aaaaaacggg caaggtgtca ccaccctgcc 900
ctttttcttt aaaaccgaaa agattacttc gcgttggaga gcgttcaccg acaaacaaca 960
gataaaacga aaggcccagt ctttcgactg agcctttcgt tttatttgat gcctggtacc 1020
gggtgtaatc cccatactca gccttctaat gctgagagaa tgcctgaaaa gaaccactaa 1080
gtcaaattat ttggattaag ttttattaag tcaacggagg tgtcagatag catttagatc 1140
gtaaaagcat acggcatgaa caaccatgaa cggcttccca gaggttaacc ccaggtttat 1200
ggtttaccaa aatcccctga ctttttccga gttgggggac aaaatcaact aatttgggcg 1260

acgatttttt ctgctatctg gcgatcgcca tgtttgtcta acttctgatc caacctaggt 1320

ttttgggggc tttccaacaa aaaatcccag ttgccggcga aaaattgctg gtggggcaca 1380

atttggtgct ctccgtagtt ctgcaaacct gccagtaaaa cttgcgcttc cgcaaaacca 1440

tccctggtga gggaaataat cgggacttct aactttaacg cttcagcaaa ggtaccaaaa 1500

ccaggcttgg aaaataccg tccgcaaagg ggcattaaat ccaccgggcg actcgaccat 1560

atgggagagc tcccaacgcg ttggatgcat agcttgagta ttctatagtg tcacctaaat 1620

agcttggcgt aatcatggtc atagctgttt cctgtgtgaa attgttatcc gctcacaatt 1680

ccacacaaca tacgagccgg aagcataaag tgtaaagcct ggggtgccta atgagtgagc 1740

taactcacat taattgcgtt gcgctcactg cccgctttcc agtcgggaaa cctgtcgtgc 1800

cagctgcatt aatgaatcgg ccaacgcgcg gggagaggcg gtttgcgtat tgggcgctct 1860

tccgcttcct cgctcactga ctcgctgcgc tcggtcgttc ggctgcggcg agcggtatca 1920

gctcactcaa aggcggtaat acggttatcc acagaatcag gggataacgc aggaaagaac 1980

atgtgagcaa aaggccagca aaaggccagg aaccgtaaaa aggccgcgtt gctggcgttt 2040

ttccataggc tccgcccccc tgacgagcat cacaaaaatc gacgctcaag tcagaggtgg 2100

cgaaacccga caggactata aagataccag gcgtttcccc ctggaagctc cctcgtgcgc 2160

tctcctgttc cgaccctgcc gcttaccgga tacctgtccg cctttctccc ttcgggaagc 2220

gtggcgcttt ctcatagctc acgctgtagg tatctcagtt cggtgtaggt cgttcgctcc 2280

aagctgggct gtgtgcacga accccccgtt cagcccgacc gctgcgcctt atccggtaac 2340

tatcgtcttg agtccaaccc ggtaagacac gacttatcgc cactggcagc agccactggt 2400

aacaggatta gcagagcgag gtatgtaggc ggtgctacag agttcttgaa gtggtggcct 2460

aactacggct acactagaag aacagtattt ggtatctgcg ctctgctgaa gccagttacc 2520

ttcggaaaaa gagttggtag ctcttgatcc ggcaaacaaa ccaccgctgg tagcggtggt 2580

tttttgtttt gcaagcagca gattacgcgc agaaaaaaag gatctcaaga agatcctttg 2640

atcttttcta cggggtctga cgctcagtgg aacgaaaact cacgttaagg gattttggtc 2700

atgagattat caaaaaggat cttcacctag atccttttaa attaaaaatg aagttttaaa 2760

tcaatctaaa gtatatatga gtaaacttgg tctgacagtt accaatgctt aatcagtgag 2820

gcacctatct cagcgatctg tctatttcgt tcatccatag ttgcctgact ccccgtcgtg 2880

tagataacta cgatacggga gggcttacca tctggcccca gtgctgcaat gataccgcga 2940

gacccacgct caccggctcc agatttatca gcaataaacc agccagccgg aagggccgag 3000

cgcagaagtg gtcctgcaac tttatccgcc tccatccagt ctattaattg ttgccgggaa 3060

gctagagtaa gtagttcgcc agttaatagt ttgcgcaacg ttgttgccat tgctacaggc 3120

atcgtggtgt cacgctcgtc gtttggtatg gcttcattca gctccggttc ccaacgatca 3180

aggcgagtta catgatcccc catgttgtgc aaaaaagcgg ttagctcctt cggtcctccg 3240

atcgttgtca gaagtaagtt ggccgcagtg ttatcactca tggttatggc agcactgcat 3300

aattctctta ctgtcatgcc atccgtaaga tgcttttctg tgactggtga gtactcaacc 3360

aagtcattct gagaatagtg tatgcggcga ccgagttgct cttgcccggc gtcaatacgg 3420

gataataccg cgccacatag cagaacttta aaagtgctca tcattggaaa acgttcttcg 3480

gggcgaaaac tctcaaggat cttaccgctg ttgagatcca gttcgatgta acccactcgt 3540

gcacccaact gatcttcagc atcttttact ttcaccagcg tttctgggtg agcaaaaaca 3600

ggaaggcaaa atgccgcaaa aaagggaata agggcgacac ggaaatgttg aatactcata 3660

ctcttccttt ttcaatatta ttgaagcatt tatcagggtt attgtctcat gagcggatac 3720

atatttgaat gtatttagaa aaataaacaa ataggggttc cgcgcacatt tccccgaaaa 3780

gtgccacctg atgcggtgtg aaataccgca cagatgcgta aggagaaaat accgcatcag 3840

gaaattgtaa gcgttaatat tttgttaaaa ttcgcgttaa atttttgtta aatcagctca 3900

ttttttaacc aataggccga aatcggcaaa atcccttata aatcaaaaga atagaccgag 3960

atagggttga gtgttgttcc agtttggaac aagagtccac tattaaagaa cgtggactcc 4020

aacgtcaaag ggcgaaaaac cgtctatcag ggcgatggcc cactacgtga accatcaccc 4080

taatcaagtt ttttggggtc gaggtgccgt aaagcactaa atcggaaccc taaagggagc 4140

ccccgattta gagcttgacg gggaaagccg gcgaacgtgg cgagaaagga agggaagaaa 4200

gcgaaaggag cgggcgctag ggcgctggca agtgtagcgg tcacgctgcg cgtaaccacc 4260

a 4261

<210> 4
<211> 4318
<212> DNA
<213> synthetic construct

<220>
<223> >SEQ ID NO 4

<400> 4

```
cacccgccgc gcttaatgcg ccgctacagg gcgcgtccat tcgccattca ggctgcgcaa      60
ctgttgggaa gggcgatcgg tgcgggcctc ttcgctatta cgccagctgg cgaaaggggg     120
atgtgctgca aggcgattaa gttgggtaac gccagggttt tcccagtcac gacgttgtaa     180
aacgacggcc agtgaattgt aatacgactc actatagggc gaattgggcc cgacgtcgca     240
tgctcccggc cgccatggcg gccgcgggaa ttcgattcta aacttacggc attggcatca     300
acgggagcca ccgtcggagt aggggaagta acaacggggg aactggtatt ggtgggcaaa     360
```

atttttacta gccattgatt ccattggggc agattgaacg ctcccaacca ccaaagtccc 420

cctaatacaa cgacagaact aaataacagg aaaaaaggaa tccaaggagt tacccctctt 480

ttatgggatg gaacttcatc gacattaaag gtgggagggg gaggaggcaa tggggacaat 540

ggtggtccag aaaaggaagg tggctccgac gtcaaggcaa cgggacatcc acaggattca 600

cagaaacgaa cctgggggct aaggcggttg ccacaattag tacaaaaaac gggagtagtc 660

ataggtgaaa accccgacta tagaattaga aaaatttaac tttttatccg aattttattc 720

gtccatgttc ccccaaataac tatcaaaata attggaaaaa ttaaattatt tggtcgttgg 780

tcaccgctcc cctaaagacct ggccattgta aagagattac acccgggaaa aaaaaacccc 840

gcccctgaca gggcggggtt ttttttcaga taaaaaaaat ccttagcttt cgctaaggat 900

gatttctgca attggcggcc gcttctagaa tgcactagta gcggccgctg cagtccggca 960

aaaaaacggg caaggtgtca ccaccctgcc ctttttcttt aaaaccgaaa agattacttc 1020

gcgttggaga gcgttcaccg acaaacaaca gataaaacga aaggcccagt ctttcgactg 1080

agcctttcgt tttatttgat gcctggtacc gggtgtaatc cattgggcac gagagttagt 1140

aaggcagtgg caattaatag aggcttatgg ttgattcgca ttgttttgct cctgaaattt 1200

tcggcaaata caaatacttc gctcttctag ccctattaac cattttaacg acaaattgat 1260

ggggcaacga ttaacaaata atgaataaat tttatgtttt tcaagatgaa aatttgaaaa 1320

tttgatttcc ttatatttct actatagaag actaatacaa ttagatctaa aatttgcaag 1380

tataaaaatc agcaaatagt tatattgtta ataattcaat gacccaataa ctcgtactgt 1440

tatctacgtg gtgaaagcca aaaagacgaa cagtttagcc tcctcctcct cggcgatcgc 1500

caagcgaaat gtcatgggag atgttcagat tgagcatttt tttctaaaag cccttgctaa 1560

aacaaaccac atgtgcaggg tgtccccgat gttgactaaa ttcagcggct cgaccatatg 1620

ggagagctcc caacgcgttg gatgcatagc ttgagtattc tatagtgtca cctaaatagc 1680

ttggcgtaat catggtcata gctgtttcct gtgtgaaatt gttatccgct cacaattcca 1740

cacaacatac gagccggaag cataaagtgt aaagcctggg gtgcctaatg agtgagctaa 1800

ctcacattaa ttgcgttgcg ctcactgccc gctttccagt cgggaaacct gtcgtgccag 1860

ctgcattaat gaatcggcca acgcgcgggg agaggcggtt tgcgtattgg gcgctcttcc 1920

gcttcctcgc tcactgactc gctgcgctcg gtcgttcggc tgcggcgagc ggtatcagct 1980

cactcaaagg cggtaatacg gttatccaca gaatcagggg ataacgcagg aaagaacatg 2040

tgagcaaaag gccagcaaaa ggccaggaac cgtaaaaagg ccgcgttgct ggcgtttttc 2100

cataggctcc gcccccctga cgagcatcac aaaaatcgac gctcaagtca gaggtggcga 2160

aacccgacag gactataaag ataccaggcg tttccccctg gaagctccct cgtgcgctct 2220

cctgttccga ccctgccgct taccggatac ctgtccgcct ttctcccttc gggaagcgtg 2280

gcgctttctc atagctcacg ctgtaggtat ctcagttcgg tgtaggtcgt tcgctccaag 2340
ctgggctgtg tgcacgaacc ccccgttcag cccgaccgct gcgccttatc cggtaactat 2400
cgtcttgagt ccaacccggt aagacacgac ttatcgccac tggcagcagc cactggtaac 2460
aggattagca gagcgaggta tgtaggcggt gctacagagt tcttgaagtg gtggcctaac 2520
tacggctaca ctagaagaac agtatttggt atctgcgctc tgctgaagcc agttaccttc 2580
ggaaaaagag ttggtagctc ttgatccggc aaacaaacca ccgctggtag cggtggtttt 2640
tttgtttgca agcagcagat tacgcgcaga aaaaaaggat ctcaagaaga tcctttgatc 2700
ttttctacgg ggtctgacgc tcagtggaac gaaaactcac gttaagggat tttggtcatg 2760
agattatcaa aaaggatctt cacctagatc cttttaaatt aaaaatgaag ttttaaatca 2820
atctaaagta tatatgagta aacttggtct gacagttacc aatgcttaat cagtgaggca 2880
cctatctcag cgatctgtct atttcgttca tccatagttg cctgactccc cgtcgtgtag 2940
ataactacga tacgggaggg cttaccatct ggccccagtg ctgcaatgat accgcgagac 3000
ccacgctcac cggctccaga tttatcagca ataaaccagc cagccggaag ggccgagcgc 3060
agaagtggtc ctgcaacttt atccgcctcc atccagtcta ttaattgttg ccgggaagct 3120
agagtaagta gttcgccagt taatagtttg cgcaacgttg ttgccattgc tacaggcatc 3180
gtggtgtcac gctcgtcgtt tggtatggct tcattcagct ccggttccca acgatcaagg 3240
cgagttacat gatcccccat gttgtgcaaa aaagcggtta gctccttcgg tcctccgatc 3300
gttgtcagaa gtaagttggc cgcagtgtta tcactcatgg ttatggcagc actgcataat 3360
tctcttactg tcatgccatc cgtaagatgc ttttctgtga ctggtgagta ctcaaccaag 3420
tcattctgag aatagtgtat gcggcgaccg agttgctctt gcccggcgtc aatacgggat 3480
aataccgcgc cacatagcag aactttaaaa gtgctcatca ttggaaaacg ttcttcgggg 3540
cgaaaactct caaggatctt accgctgttg agatccagtt cgatgtaacc cactcgtgca 3600
cccaactgat cttcagcatc ttttactttc accagcgttt ctgggtgagc aaaaacagga 3660
aggcaaaatg ccgcaaaaaa gggaataagg gcgacacgga aatgttgaat actcatactc 3720
ttcctttttc aatattattg aagcatttat cagggttatt gtctcatgag cggatacata 3780
tttgaatgta tttagaaaaa taaacaaata ggggttccgc gcacatttcc ccgaaaagtg 3840
ccacctgatg cggtgtgaaa taccgcacag atgcgtaagg agaaaatacc gcatcaggaa 3900
attgtaagcg ttaatatttt gttaaaattc gcgttaaatt tttgttaaat cagctcattt 3960
tttaaccaat aggccgaaat cggcaaaatc ccttataaat caaaagaata gaccgagata 4020
gggttgagtg ttgttccagt ttggaacaag agtccactat taaagaacgt ggactccaac 4080
gtcaaagggc gaaaaaccgt ctatcagggc gatggcccac tacgtgaacc atcaccctaa 4140

tcaagttttt tggggtcgag gtgccgtaaa gcactaaatc ggaaccctaa agggagcccc 4200

cgatttagag cttgacgggg aaagccggcg aacgtggcga gaaaggaagg gaagaaagcg 4260

aaaggagcgg gcgctagggc gctggcaagt gtagcggtca cgctgcgcgt aaccacca 4318

<210> 5
<211> 4333
<212> DNA
<213> synthetic construct

<220>
<223> >SEQ ID NO 5

<400> 5

```
cacccgccgc gcttaatgcg ccgctacagg gcgcgtccat tcgccattca ggctgcgcaa      60
ctgttgggaa gggcgatcgg tgcgggcctc ttcgctatta cgccagctgg cgaaaggggg     120
atgtgctgca aggcgattaa gttgggtaac gccagggttt tcccagtcac gacgttgtaa     180
aacgacggcc agtgaattgt aatacgactc actatagggc gaattgggcc cgacgtcgca     240
tgctcccggc cgccatggcg gccgcgggaa ttcgattgtg agcttgatgg tgatggtggg     300
taaagcttca ttgtccatgc gccggagctt gacggtaaaa tccgtgtcct ggggccaatc     360
attgggaacg ctcacacaga taccgttgcg agttaagtcg aaggataccc ccaccaattc     420
cccttccata tcgaacactt ggcaaaaggc caacatccgc tcttcctgcc gtcgctcctg     480
cacccccctgg agatgggcaa tggttagggc ttcttgggag agggaagatg gagaatcttg    540
agccatggag attatttcct cggttaaatt aggtttacct agtaaatggg ccaggttgac     600
cattattgta gtcattcact gggtcgatct tccttccatc agggtcattt ggttaattgt     660
tgatgagaaa tgggaaggag taatccatag atatttgccc agttaactcg atttgagcag     720
aattgggaag gacgtattgg gaactttggt tgacagggca acaaagccag cagattacac     780
ccgggaaaaa aaaaccccgc ccctgacagg gcggggtttt ttttcagata aaaaaaatcc     840
ttagctttcg ctaaggatga tttctgcaat tggcggccgc ttctagaatg cactagtagc     900
ggccgctgca gtccggcaaa aaaacgggca aggtgtcacc accctgccct ttttctttaa     960
aaccgaaaag attacttcgc gttggagagc gttcaccgac aaacaacaga taaaacgaaa    1020
ggcccagtct ttcgactgag cctttcgttt tatttgatgc ctggtaccgg gtgtaatcgc    1080
tggaggcgaa ctgggtgaga accataaaat cttggggaac aggggaatgt taatgaacac    1140
atgacatgca taggatagcg aattgattta aagcaatgat tccccctgag ataaatatat    1200
tttgacccac tgctccgctt aattttggcg ctaatctggg caaaaattcg ttggttttcg    1260
gaaaggtgaa cccgtagcta gcttgtattg cccaaaccta actcatcatt gctccatggc    1320
ggccaaaaat ctttaggaca aaactcgcca gaggtcaagg cttgattttt tcagagggaa    1380
```

aaattcctcg gctaaataat caaaaagttg aaaaaaaaag tttttcagct aaggtttgat 1440

gttatatttt atcttatgta gacttttgaa aaaaacaagt ccccgcaatc aagcatcatt 1500

caacggaaaa aataatccta tgccaaacgc ctccaccgca ctaaccggca aagcattact 1560

caataaagtt aaagaactat cccatctgcc ccgtcgagaa acggcaaaag cctgtggtta 1620

ctactcgacc atatgggaga gctcccaacg cgttggatgc atagcttgag tattctatag 1680

tgtcacctaa atagcttggc gtaatcatgg tcatagctgt ttcctgtgtg aaattgttat 1740

ccgctcacaa ttccacacaa catacgagcc ggaagcataa agtgtaaagc ctggggtgcc 1800

taatgagtga gctaactcac attaattgcg ttgcgctcac tgcccgcttt ccagtcggga 1860

aacctgtcgt gccagctgca ttaatgaatc ggccaacgcg cggggagagg cggtttgcgt 1920

attgggcgct cttccgcttc ctcgctcact gactcgctgc gctcggtcgt tcggctgcgg 1980

cgagcggtat cagctcactc aaaggcggta atacggttat ccacagaatc aggggataac 2040

gcaggaaaga acatgtgagc aaaaggccag caaaaggcca ggaaccgtaa aaaggccgcg 2100

ttgctggcgt ttttccatag gctccgcccc cctgacgagc atcacaaaaa tcgacgctca 2160

agtcagaggt ggcgaaaccc gacaggacta taaagatacc aggcgtttcc ccctggaagc 2220

tccctcgtgc gctctcctgt tccgaccctg ccgcttaccg gatacctgtc cgcctttctc 2280

ccttcgggaa gcgtggcgct ttctcatagc tcacgctgta ggtatctcag ttcggtgtag 2340

gtcgttcgct ccaagctggg ctgtgtgcac gaaccccccg ttcagcccga ccgctgcgcc 2400

ttatccggta actatcgtct tgagtccaac ccggtaagac acgacttatc gccactggca 2460

gcagccactg gtaacaggat tagcagagcg aggtatgtag gcggtgctac agagttcttg 2520

aagtggtggc ctaactacgg ctacactaga agaacagtat ttggtatctg cgctctgctg 2580

aagccagtta ccttcggaaa aagagttggt agctcttgat ccggcaaaca aaccaccgct 2640

ggtagcggtg gtttttttgt ttgcaagcag cagattacgc gcagaaaaaa aggatctcaa 2700

gaagatcctt tgatcttttc tacggggtct gacgctcagt ggaacgaaaa ctcacgttaa 2760

gggattttgg tcatgagatt atcaaaaagg atcttcacct agatcctttt aaattaaaaa 2820

tgaagtttta aatcaatcta aagtatatat gagtaaactt ggtctgacag ttaccaatgc 2880

ttaatcagtg aggcacctat ctcagcgatc tgtctatttc gttcatccat agttgcctga 2940

ctccccgtcg tgtagataac tacgatacgg gagggcttac catctggccc cagtgctgca 3000

atgataccgc gagacccacg ctcaccggct ccagatttat cagcaataaa ccagccagcc 3060

ggaagggccg agcgcagaag tggtcctgca actttatccg cctccatcca gtctattaat 3120

tgttgccggg aagctagagt aagtagttcg ccagttaata gtttgcgcaa cgttgttgcc 3180

attgctacag gcatcgtggt gtcacgctcg tcgtttggta tggcttcatt cagctccggt 3240

tcccaacgat caaggcgagt tacatgatcc cccatgttgt gcaaaaaagc ggttagctcc 3300

ttcggtcctc cgatcgttgt cagaagtaag ttggccgcag tgttatcact catggttatg 3360

gcagcactgc ataattctct tactgtcatg ccatccgtaa gatgcttttc tgtgactggt 3420

gagtactcaa ccaagtcatt ctgagaatag tgtatgcggc gaccgagttg ctcttgcccg 3480

gcgtcaatac gggataatac cgcgccacat agcagaactt taaaagtgct catcattgga 3540

aaacgttctt cggggcgaaa actctcaagg atcttaccgc tgttgagatc cagttcgatg 3600

taacccactc gtgcacccaa ctgatcttca gcatctttta ctttcaccag cgtttctggg 3660

tgagcaaaaa caggaaggca aaatgccgca aaaaagggaa taagggcgac acggaaatgt 3720

tgaatactca tactcttcct ttttcaatat tattgaagca tttatcaggg ttattgtctc 3780

atgagcggat acatatttga atgtatttag aaaaataaac aaataggggt tccgcgcaca 3840

tttccccgaa aagtgccacc tgatgcggtg tgaaataccg cacagatgcg taaggagaaa 3900

ataccgcatc aggaaattgt aagcgttaat attttgttaa aattcgcgtt aaatttttgt 3960

taaatcagct cattttttaa ccaataggcc gaaatcggca aaatccctta taaatcaaaa 4020

gaatagaccg agatagggtt gagtgttgtt ccagtttgga acaagagtcc actattaaag 4080

aacgtggact ccaacgtcaa agggcgaaaa accgtctatc agggcgatgg cccactacgt 4140

gaaccatcac cctaatcaag ttttttgggg tcgaggtgcc gtaaagcact aaatcggaac 4200

cctaaaggga gcccccgatt tagagcttga cggggaaagc cggcgaacgt ggcgagaaag 4260

gaagggaaga aagcgaaagg agcgggcgct agggcgctgg caagtgtagc ggtcacgctg 4320

cgcgtaacca cca 4333

<210> 6
<211> 5629
<212> DNA
<213> synthetic construct

<220>
<223> >SEQ ID NO 6

<400> 6

```
tgtccaaatc agcattgctc tgccaggtga gacggagttt ttgtcccact aatccctgtt      60
ggatagaagg cgcttgatac agttccaacc aaacccaatc tcctgtcctg gctttagttt     120
cttccaccgt cggcaggatt aaacgaccca accactctcc caaaggccga taatacgcct     180
catctaaatt ctgttgcaga ggataataat ctacctggtt ggcgggcaac tgactgctaa     240
tttgataatc cgggatgcgg ccagaggttt cctgaggttg aaacagcgta gctagggaaa     300
tcaccaccag agcggcgatc gccaccaata ccatccgcca gcgccattgc ttcatctgtg     360
gactgcccta aaaattgcca taaaaaaaca cctggggcgt ctttccttgt ttcgactcca     420
gatgtttttc taatttcctc acacctgtcc taagtataaa tcaccaacgg ggattgtcaa     480
```

```
ggcttcagga ttacacccgg gcgatttact tttcgacctc attctattag actctcgttt       540
ggattgcaac tggtctattt tcctcttttg tttgatagaa aatcataaaa ggatttgcag       600
actacgggcc taaagaacta aaaaatctat ctgtttcttt tcattctctg tatttttat        660
agtttctgtt gcatgggcat aaagttgcct ttttaatcac aattcagaaa atatcataat       720
atctcatttc actaaataat agtgaacggc aggtatatgt gatgggttaa aaaggatcga       780
tcctctagcg aaccccagag tcccgctcag aagaactcgt caagaaggcg atagaaggcg       840
atgcgctgcg aatcgggagc ggcgataccg taaagcacga ggaagcggtc agcccattcg       900
ccgccaagct cttcagcaat atcacgggta gccaacgcta tgtcctgata gcggtccgcc       960
acacccagcc ggccacagtc gatgaatcca gaaaagcggc cattttccac catgatattc      1020
ggcaagcagg catcgccatg ggtcacgacg agatcctcgc cgtcgggcat ccgcgccttg      1080
agcctggcga acagttcggc tggcgcgagc ccctgatgct cttcgtccag atcatcctga      1140
tcgacaagac cggcttccat ccgagtacgt gctcgctcga tgcgatgttt cgcttggtgg      1200
tcgaatgggc aggtagccgg atcaagcgta tgcagccgcc gcattgcatc agccatgatg      1260
gatactttct cggcaggagc aaggtgagat gacaggagat cctgccccgg cacttcgccc      1320
aatagcagcc agtcccttcc cgcttcagtg acaacgtcga gcacagctgc gcaaggaacg      1380
cccgtcgtgg ccagccacga tagccgcgct gcctcgtctt ggagttcatt cagggcaccg      1440
gacaggtcgg tcttgacaaa aagaaccggg cgcccctgcg ctgacagccg gaacacggcg      1500
gcatcagagc agccgattgt ctgttgtgcc cagtcatagc cgaatagcct ctccacccaa      1560
gcggccggag aacctgcgtg caatccatct tgttcaatca tgcgaaacga tcctcatcct      1620
gtctcttgat cagatcttga tcccctgcgc catcagatcc ttggcggcaa gaaagccatc      1680
cagtttactt tgcagggctt cccaacctta ccagagggcg ccccagctgg caattccggt      1740
tcgcttgctg tccataaaac cgcccagtct agctatcgcc atgtaagccc actgcaagct      1800
acctgctttc tctttgcgct tgcgttttcc cttgtccaga tagcccagta gctgacattc      1860
acccgggaaa aaaaaacccc gcccctgaca gggcggggtt tttttcaga taaaaaaaat       1920
ccttagcttt cgctaaggat gatttctgca attggcggcc gcttctagaa tgcactagta      1980
gcggccgctg cagtccggca aaaaaacggg caaggtgtca ccaccctgcc ctttttcttt      2040
aaaaccgaaa agattacttc gcgttggaga gcgttcaccg acaaacaaca gataaaacga      2100
aaggcccagt ctttcgactg agcctttcgt tttatttgat gcctggtacc gggtgtaatc      2160
ggttgattat ttcagtggcc cggccgatgg ataattacca tggcccgacc gaccgggatt      2220
tccagaccag tttgaacagt attaaattag cctttccgga caaggcctct gtgaaaatct      2280
taaaaaagta ttaagatttt aaaagctagc ttattttcgg aggaaatgtg tttactcgac      2340
```

```
ttgcccagca acaccgcgat ttcgtgaagg atttagtcat gagcttgagg gctttggcca     2400
ctgtgctcga aaatcggggc tacattgctt cttgctacac ctgtggcgac caactcaaca     2460
gcgcctcctt catggtgagc ttgggggaaa atcatctgat ccgctttttg gtatcggact     2520
acggcatcac ctggacagaa atgcgggatg accgagaatt aatgaaatta gaaggagccg     2580
aggcgatcgc ccagttggaa gaattggcca atgtggtcaa atattgccta gcagatgccc     2640
ccactcgacc atatgggaga gctcccaacg cgttggatgc atagcttgag tattctatag     2700
tgtcacctaa atagcttggc gtaatcatgg tcatagctgt ttcctgtgtg aaattgttat     2760
ccgctcacaa ttccacacaa catacgagcc ggaagcataa agtgtaaagc ctggggtgcc     2820
taatgagtga gctaactcac attaattgcg ttgcgctcac tgcccgcttt ccagtcggga     2880
aacctgtcgt gccagctgca ttaatgaatc ggccaacgcg cggggagagg cggtttgcgt     2940
attgggcgct cttccgcttc ctcgctcact gactcgctgc gctcggtcgt tcggctgcgg     3000
cgagcggtat cagctcactc aaaggcggta atacggttat ccacagaatc aggggataac     3060
gcaggaaaga acatgtgagc aaaaggccag caaaaggcca ggaaccgtaa aaaggccgcg     3120
ttgctggcgt ttttccatag gctccgcccc cctgacgagc atcacaaaaa tcgacgctca     3180
agtcagaggt ggcgaaaccc gacaggacta taaagatacc aggcgtttcc ccctggaagc     3240
tccctcgtgc gctctcctgt tccgaccctg ccgcttaccg gatacctgtc cgcctttctc     3300
ccttcgggaa gcgtggcgct ttctcatagc tcacgctgta ggtatctcag ttcggtgtag     3360
gtcgttcgct ccaagctggg ctgtgtgcac gaaccccccg ttcagcccga ccgctgcgcc     3420
ttatccggta actatcgtct tgagtccaac ccggtaagac acgacttatc gccactggca     3480
gcagccactg gtaacaggat tagcagagcg aggtatgtag gcggtgctac agagttcttg     3540
aagtggtggc ctaactacgg ctacactaga agaacagtat ttggtatctg cgctctgctg     3600
aagccagtta ccttcggaaa aagagttggt agctcttgat ccggcaaaca aaccaccgct     3660
ggtagcggtg gtttttttgt ttgcaagcag cagattacgc gcagaaaaaa aggatctcaa     3720
gaagatcctt tgatcttttc tacggggtct gacgctcagt ggaacgaaaa ctcacgttaa     3780
gggattttgg tcatgagatt atcaaaaagg atcttcacct agatcctttt aaattaaaaa     3840
tgaagtttta aatcaatcta aagtatatat gagtaaactt ggtctgacag ttaccaatgc     3900
ttaatcagtg aggcacctat ctcagcgatc tgtctatttc gttcatccat agttgcctga     3960
ctccccgtcg tgtagataac tacgatacgg gagggcttac catctggccc cagtgctgca     4020
atgataccgc gagacccacg ctcaccggct ccagatttat cagcaataaa ccagccagcc     4080
ggaagggccg agcgcagaag tggtcctgca actttatccg cctccatcca gtctattaat     4140
tgttgccggg aagctagagt aagtagttcg ccagttaata gtttgcgcaa cgttgttgcc     4200
attgctacag gcatcgtggt gtcacgctcg tcgtttggta tggcttcatt cagctccggt     4260
```

tcccaacgat caaggcgagt tacatgatcc cccatgttgt gcaaaaaagc ggttagctcc 4320

ttcggtcctc cgatcgttgt cagaagtaag ttggccgcag tgttatcact catggttatg 4380

gcagcactgc ataattctct tactgtcatg ccatccgtaa gatgcttttc tgtgactggt 4440

gagtactcaa ccaagtcatt ctgagaatag tgtatgcggc gaccgagttg ctcttgcccg 4500

gcgtcaatac gggataatac cgcgccacat agcagaactt taaaagtgct catcattgga 4560

aaacgttctt cggggcgaaa actctcaagg atcttaccgc tgttgagatc cagttcgatg 4620

taacccactc gtgcacccaa ctgatcttca gcatctttta ctttcaccag cgtttctggg 4680

tgagcaaaaa caggaaggca aaatgccgca aaaaagggaa taagggcgac acggaaatgt 4740

tgaatactca tactcttcct ttttcaatat tattgaagca tttatcaggg ttattgtctc 4800

atgagcggat acatatttga atgtatttag aaaaataaac aaataggggt tccgcgcaca 4860

tttccccgaa aagtgccacc tgatgcggtg tgaaataccg cacagatgcg taaggagaaa 4920

ataccgcatc aggaaattgt aagcgttaat attttgttaa aattcgcgtt aaatttttgt 4980

taaatcagct cattttttaa ccaataggcc gaaatcggca aaatccctta taaatcaaaa 5040

gaatagaccg agatagggtt gagtgttgtt ccagtttgga acaagagtcc actattaaag 5100

aacgtggact ccaacgtcaa agggcgaaaa accgtctatc agggcgatgg cccactacgt 5160

gaaccatcac cctaatcaag ttttttgggg tcgaggtgcc gtaaagcact aaatcggaac 5220

cctaaaggga gcccccgatt tagagcttga cggggaaagc cggcgaacgt ggcgagaaag 5280

gaagggaaga aagcgaaagg agcgggcgct agggcgctgg caagtgtagc ggtcacgctg 5340

cgcgtaacca ccacacccgc cgcgcttaat gcgccgctac agggcgcgtc cattcgccat 5400

tcaggctgcg caactgttgg gaagggcgat cggtgcgggc ctcttcgcta ttacgccagc 5460

tggcgaaagg gggatgtgct gcaaggcgat taagttgggt aacgccaggg ttttcccagt 5520

cacgacgttg taaaacgacg gccagtgaat tgtaatacga ctcactatag ggcgaattgg 5580

gcccgacgtc gcatgctccc ggccgccatg gcggccgcgg gaattcgat 5629

<210> 7
<211> 5649
<212> DNA
<213> synthetic construct

<220>
<223> >SEQ ID NO 7

<400> 7

```
cacccgccgc gcttaatgcg ccgctacagg gcgcgtccat tcgccattca ggctgcgcaa        60
ctgttgggaa gggcgatcgg tgcgggcctc ttcgctatta cgccagctgg cgaaaggggg       120
atgtgctgca aggcgattaa gttgggtaac gccagggttt tcccagtcac gacgttgtaa       180
```

aacgacggcc agtgaattgt aatacgactc actatagggc gaattgggcc cgacgtcgca 240
tgctcccggc cgccatggcg gccgcgggaa ttcgattgat gaccgctggc ggagtttagt 300
ccagggttat ttgtaccgtc aaggttacat tgccacccca gatttaacta cgctgcgagt 360
ccgtaccatt ggcgatcggg cctatctcac cattaagggt aaaaatgcca gcattgcccg 420
cttggatttg agtatgaaat tcttgcagtg gaagccaatt aaattttgac agaactctgt 480
tcaccgcccc tgattgaaaa tatcgttatt gcctcgatta ccatggtaaa acctgagaag 540
tagacgagtt tttgggggat aaccagggtc taattttagc ggaagtggaa ttaacctaca 600
ctggtgaaaa aataagtcta cttccctgga tcggggaaga ggtaacggat gatgcccgct 660
attacaacgt caatttagcc caacatccct acaaaaattg gtgacaagat tacacccggg 720
cgatttactt ttcgacctca ttctattaga ctctcgtttg gattgcaact ggtctatttt 780
cctcttttgt ttgatagaaa atcataaaag gatttgcaga ctacgggcct aaagaactaa 840
aaaatctatc tgtttctttt cattctctgt atttttttata gtttctgttg catgggcata 900
aagttgcctt tttaatcaca attcagaaaa tatcataata tctcatttca ctaaataata 960
gtgaacggca ggtatatgtg atgggttaaa aaggatcgat cctctagcga accccagagt 1020
cccgctcaga agaactcgtc aagaaggcga tagaaggcga tgcgctgcga atcgggagcg 1080
gcgataccgt aaagcacgag gaagcggtca gcccattcgc cgccaagctc ttcagcaata 1140
tcacgggtag ccaacgctat gtcctgatag cggtccgcca cacccagccg gccacagtcg 1200
atgaatccag aaaagcggcc attttccacc atgatattcg gcaagcaggc atcgccatgg 1260
gtcacgacga gatcctcgcc gtcgggcatc cgcgccttga gcctggcgaa cagttcggct 1320
ggcgcgagcc cctgatgctc ttcgtccaga tcatcctgat cgacaagacc ggcttccatc 1380
cgagtacgtg ctcgctcgat gcgatgtttc gcttggtggt cgaatgggca ggtagccgga 1440
tcaagcgtat gcagccgccg cattgcatca gccatgatgg atactttctc ggcaggagca 1500
aggtgagatg acaggagatc ctgccccggc acttcgccca atagcagcca gtcccttccc 1560
gcttcagtga caacgtcgag cacagctgcg caaggaacgc ccgtcgtggc cagccacgat 1620
agccgcgctg cctcgtcttg gagttcattc agggcaccgg acaggtcggt cttgacaaaa 1680
agaaccgggc gcccctgcgc tgacagccgg aacacggcgg catcagagca gccgattgtc 1740
tgttgtgccc agtcatagcc gaatagcctc tccacccaag cggccggaga acctgcgtgc 1800
aatccatctt gttcaatcat gcgaaacgat cctcatcctg tctcttgatc agatcttgat 1860
cccctgcgcc atcagatcct tggcggcaag aaagccatcc agtttacttt gcagggcttc 1920
ccaaccttac cagagggcgc cccagctggc aattccggtt cgcttgctgt ccataaaacc 1980
gcccagtcta gctatcgcca tgtaagccca ctgcaagcta cctgctttct ctttgcgctt 2040

gcgttttccc ttgtccagat agcccagtag ctgacattca cccgggaaaa aaaaaccccg 2100
cccctgacag ggcggggttt tttttcagat aaaaaaaatc cttagctttc gctaaggatg 2160
atttctgcaa ttggcggccg cttctagaat gcactagtag cggccgctgc agtccggcaa 2220
aaaaacgggc aaggtgtcac caccctgccc tttttcttta aaaccgaaaa gattacttcg 2280
cgttggagag cgttcaccga caaacaacag ataaaacgaa aggcccagtc tttcgactga 2340
gcctttcgtt ttatttgatg cctggtaccg ggtgtaatcc ttcattcccc tattgttatt 2400
aatagtgctc tgaaactaac aaataagcaa gatcaaattg tgaagaaaac gcaacaaaat 2460
gaaccattaa gataatttct gtctatcctg gctggtatat tccagtctac tcagaaatga 2520
gtttgtttta tgacaaatca gtatgtacca aattacttgg cgcaatcgat cctggtaact 2580
ctattttgtt gcttacccct gggtattgtg gccattatca aagcatcgga agttaattct 2640
cgtttagctt caggggacta tgaaggcgct gtaaaggctt ccaaggaagc gaaaaagttt 2700
tgttggtggt cctttggtgc cggcataatt ttcattgcca tctattttgt gctagtggtt 2760
attgccgccg tctttggtca gtaattaaag ttacattttt tgactttgcc ttgttcacca 2820
ttcattaacg aataccatgt ttagtttgaa aaattattcc ccatctccat tactatccgc 2880
taaggccaag gaatatttag tattcacttt ggtaacccta accattgtcg ttgctggcac 2940
tcgaccatat gggagagctc ccaacgcgtt ggatgcatag cttgagtatt ctatagtgtc 3000
acctaaatag cttggcgtaa tcatggtcat agctgtttcc tgtgtgaaat tgttatccgc 3060
tcacaattcc acacaacata cgagccggaa gcataaagtg taaagcctgg ggtgcctaat 3120
gagtgagcta actcacatta attgcgttgc gctcactgcc cgctttccag tcgggaaacc 3180
tgtcgtgcca gctgcattaa tgaatcggcc aacgcgcggg gagaggcggt ttgcgtattg 3240
ggcgctcttc cgcttcctcg ctcactgact cgctgcgctc ggtcgttcgg ctgcggcgag 3300
cggtatcagc tcactcaaag gcggtaatac ggttatccac agaatcaggg gataacgcag 3360
gaaagaacat gtgagcaaaa ggccagcaaa aggccaggaa ccgtaaaaag gccgcgttgc 3420
tggcgttttt ccataggctc cgcccccctg acgagcatca caaaaatcga cgctcaagtc 3480
agaggtggcg aaacccgaca ggactataaa gataccaggc gtttccccct ggaagctccc 3540
tcgtgcgctc tcctgttccg accctgccgc ttaccggata cctgtccgcc tttctccctt 3600
cgggaagcgt ggcgctttct catagctcac gctgtaggta tctcagttcg gtgtaggtcg 3660
ttcgctccaa gctgggctgt gtgcacgaac cccccgttca gcccgaccgc tgcgccttat 3720
ccggtaacta tcgtcttgag tccaacccgg taagacacga cttatcgcca ctggcagcag 3780
ccactggtaa caggattagc agagcgaggt atgtaggcgg tgctacagag ttcttgaagt 3840
ggtggcctaa ctacggctac actagaagaa cagtatttgg tatctgcgct ctgctgaagc 3900
cagttacctt cggaaaaaga gttggtagct cttgatccgg caaacaaacc accgctggta 3960

gcggtggttt ttttgtttgc aagcagcaga ttacgcgcag aaaaaaagga tctcaagaag 4020
atcctttgat cttttctacg gggtctgacg ctcagtggaa cgaaaactca cgttaaggga 4080
ttttggtcat gagattatca aaaaggatct tcacctagat ccttttaaat taaaaatgaa 4140
gttttaaatc aatctaaagt atatatgagt aaacttggtc tgacagttac caatgcttaa 4200
tcagtgaggc acctatctca gcgatctgtc tatttcgttc atccatagtt gcctgactcc 4260
ccgtcgtgta gataactacg atacgggagg gcttaccatc tggccccagt gctgcaatga 4320
taccgcgaga cccacgctca ccggctccag atttatcagc aataaaccag ccagccggaa 4380
gggccgagcg cagaagtggt cctgcaactt tatccgcctc catccagtct attaattgtt 4440
gccgggaagc tagagtaagt agttcgccag ttaatagttt gcgcaacgtt gttgccattg 4500
ctacaggcat cgtggtgtca cgctcgtcgt ttggtatggc ttcattcagc tccggttccc 4560
aacgatcaag gcgagttaca tgatccccca tgttgtgcaa aaaagcggtt agctccttcg 4620
gtcctccgat cgttgtcaga agtaagttgg ccgcagtgtt atcactcatg gttatggcag 4680
cactgcataa ttctcttact gtcatgccat ccgtaagatg cttttctgtg actggtgagt 4740
actcaaccaa gtcattctga gaatagtgta tgcggcgacc gagttgctct tgcccggcgt 4800
caatacggga taataccgcg ccacatagca gaactttaaa agtgctcatc attggaaaac 4860
gttcttcggg gcgaaaactc tcaaggatct taccgctgtt gagatccagt tcgatgtaac 4920
ccactcgtgc acccaactga tcttcagcat cttttacttt caccagcgtt tctgggtgag 4980
caaaaacagg aaggcaaaat gccgcaaaaa agggaataag ggcgacacgg aaatgttgaa 5040
tactcatact cttccttttt caatattatt gaagcattta tcagggttat tgtctcatga 5100
gcggatacat atttgaatgt atttagaaaa ataaacaaat aggggttccg cgcacatttc 5160
cccgaaaagt gccacctgat gcggtgtgaa ataccgcaca gatgcgtaag gagaaaatac 5220
cgcatcagga aattgtaagc gttaatattt tgttaaaatt cgcgttaaat ttttgttaaa 5280
tcagctcatt ttttaaccaa taggccgaaa tcggcaaaat cccttataaa tcaaaagaat 5340
agaccgagat agggttgagt gttgttccag tttggaacaa gagtccacta ttaaagaacg 5400
tggactccaa cgtcaaaggg cgaaaaaccg tctatcaggg cgatggccca ctacgtgaac 5460
catcacccta atcaagtttt ttggggtcga ggtgccgtaa agcactaaat cggaacccta 5520
aagggagccc ccgatttaga gcttgacggg gaaagccggc gaacgtggcg agaaaggaag 5580
ggaagaaagc gaaaggagcg ggcgctaggg cgctggcaag tgtagcggtc acgctgcgcg 5640
taaccacca 5649

<210> 8
<211> 5627
<212> DNA

<213> synthetic construct

<220>
<223> >SEQ ID NO 8

<400> 8

cacccgccgc gcttaatgcg ccgctacagg gcgcgtccat tcgccattca ggctgcgcaa 60
ctgttgggaa gggcgatcgg tgcgggcctc ttcgctatta cgccagctgg cgaaaggggg 120
atgtgctgca aggcgattaa gttgggtaac gccagggttt tcccagtcac gacgttgtaa 180
aacgacggcc agtgaattgt aatacgactc actatagggc gaattgggcc cgacgtcgca 240
tgctcccggc cgccatggcg gccgcgggaa ttcgattgga ctgacccaag atacagtggt 300
agcttcaagt tcgtcaatca accccaatgg aaagctgaga taatctgttg ttgatgcgac 360
cgtcttcatt ttagctaaaa agacaagtct gtggctagtt actatgacga ggccatcggg 420
tctatcccag cggtcttgct gattccaaaa cttggcgatt ttctgaagta gtatcttttc 480
accgcttaag ccgccaaggt cttggatgag cttggtcgaa tcatcttttg atgcctcgac 540
atcgcgaata actttgtaaa cgaccttccc gagcttgaat gcaaagtcgg ttagattatc 600
catcagagtt gaatgccgat aatacagact gctatactat tttaatgaaa agagtgctgg 660
caagcaagcc cgccgaacgt tgaagctgtg cagcgcaaac gaagcgcatt gtagtttgcg 720
ctcggattac acccgggcga tttacttttc gacctcattc tattagactc tcgtttggat 780
tgcaactggt ctattttcct cttttgtttg atagaaaatc ataaaaggat ttgcagacta 840
cgggcctaaa gaactaaaaa atctatctgt ttcttttcat tctctgtatt ttttatagtt 900
tctgttgcat gggcataaag ttgccttttt aatcacaatt cagaaaatat cataatatct 960
catttcacta aataatagtg aacggcaggt atatgtgatg ggttaaaaag gatcgatcct 1020
ctagcgaacc ccagagtccc gctcagaaga actcgtcaag aaggcgatag aaggcgatgc 1080
gctgcgaatc gggagcggcg ataccgtaaa gcacgaggaa gcggtcagcc cattcgccgc 1140
caagctcttc agcaatatca cgggtagcca acgctatgtc ctgatagcgg tccgccacac 1200
ccagccggcc acagtcgatg aatccagaaa agcggccatt ttccaccatg atattcggca 1260
agcaggcatc gccatgggtc acgacgagat cctcgccgtc gggcatccgc gccttgagcc 1320
tggcgaacag ttcggctggc gcgagcccct gatgctcttc gtccagatca tcctgatcga 1380
caagaccggc ttccatccga gtacgtgctc gctcgatgcg atgtttcgct tggtggtcga 1440
atgggcaggt agccggatca agcgtatgca gccgccgcat tgcatcagcc atgatggata 1500
ctttctcggc aggagcaagg tgagatgaca ggagatcctg ccccggcact tcgcccaata 1560
gcagccagtc ccttcccgct tcagtgacaa cgtcgagcac agctgcgcaa ggaacgcccg 1620
tcgtggccag ccacgatagc cgcgctgcct cgtcttggag ttcattcagg gcaccggaca 1680

ggtcggtctt gacaaaaaga accgggcgcc cctgcgctga cagccggaac acggcggcat 1740

cagagcagcc gattgtctgt tgtgcccagt catagccgaa tagcctctcc acccaagcgg 1800

ccggagaacc tgcgtgcaat ccatcttgtt caatcatgcg aaacgatcct catcctgtct 1860

cttgatcaga tcttgatccc ctgcgccatc agatccttgg cggcaagaaa gccatccagt 1920

ttactttgca gggcttccca accttaccag agggcgcccc agctggcaat tccggttcgc 1980

ttgctgtcca taaaaccgcc cagtctagct atcgccatgt aagcccactg caagctacct 2040

gctttctctt tgcgcttgcg ttttcccttg tccagatagc ccagtagctg acattcaccc 2100

gggaaaaaaa aaccccgccc ctgacagggc ggggtttttt ttcagataaa aaaaatcctt 2160

agctttcgct aaggatgatt tctgcaattg gcggccgctt ctagaatgca ctagtagcgg 2220

ccgctgcagt ccggcaaaaa aacgggcaag gtgtcaccac cctgcccttt ttctttaaaa 2280

ccgaaaagat tacttcgcgt tggagagcgt tcaccgacaa acaacagata aaacgaaagg 2340

cccagtcttt cgactgagcc tttcgtttta tttgatgcct ggtaccgggt gtaatcccca 2400

tactcagcct tctaatgctg agagaatgcc tgaaaagaac cactaagtca aattatttgg 2460

attaagtttt attaagtcaa cggaggtgtc agatagcatt tagatcgtaa aagcatacgg 2520

catgaacaac catgaacggc ttcccagagg ttaaccccag gtttatggtt taccaaaatc 2580

ccctgacttt ttccgagttg ggggacaaaa tcaactaatt tgggcgacga ttttttctgc 2640

tatctggcga tcgccatgtt tgtctaactt ctgatccaac ctaggttttt gggggctttc 2700

caacaaaaaa tcccagttgc cggcgaaaaa ttgctggtgg ggcacaattt ggtgctctcc 2760

gtagttctgc aaacctgcca gtaaaacttg cgcttccgca aaaccatccc tggtgaggga 2820

aataatcggg acttctaact ttaacgcttc agcaaaggta ccaaaaccag gcttggaaaa 2880

tacccgtccg caaaggggca ttaaatccac cgggcgactc gaccatatgg gagagctccc 2940

aacgcgttgg atgcatagct tgagtattct atagtgtcac ctaaatagct tggcgtaatc 3000

atggtcatag ctgtttcctg tgtgaaattg ttatccgctc acaattccac acaacatacg 3060

agccggaagc ataaagtgta aagcctgggg tgcctaatga gtgagctaac tcacattaat 3120

tgcgttgcgc tcactgcccg ctttccagtc gggaaacctg tcgtgccagc tgcattaatg 3180

aatcggccaa cgcgcgggga gaggcggttt gcgtattggg cgctcttccg cttcctcgct 3240

cactgactcg ctgcgctcgg tcgttcggct gcggcgagcg gtatcagctc actcaaaggc 3300

ggtaatacgg ttatccacag aatcagggga taacgcagga aagaacatgt gagcaaaagg 3360

ccagcaaaag gccaggaacc gtaaaaaggc cgcgttgctg gcgtttttcc ataggctccg 3420

cccccctgac gagcatcaca aaaatcgacg ctcaagtcag aggtggcgaa acccgacagg 3480

actataaaga taccaggcgt ttccccctgg aagctccctc gtgcgctctc ctgttccgac 3540

cctgccgctt accggatacc tgtccgcctt tctcccttcg ggaagcgtgg cgctttctca 3600

tagctcacgc tgtaggtatc tcagttcggt gtaggtcgtt cgctccaagc tgggctgtgt 3660

gcacgaaccc cccgttcagc ccgaccgctg cgccttatcc ggtaactatc gtcttgagtc 3720

caacccggta agacacgact tatcgccact ggcagcagcc actggtaaca ggattagcag 3780

agcgaggtat gtaggcggtg ctacagagtt cttgaagtgg tggcctaact acggctacac 3840

tagaagaaca gtatttggta tctgcgctct gctgaagcca gttaccttcg gaaaaagagt 3900

tggtagctct tgatccggca aacaaaccac cgctggtagc ggtggttttt ttgtttgcaa 3960

gcagcagatt acgcgcagaa aaaaaggatc tcaagaagat cctttgatct tttctacggg 4020

gtctgacgct cagtggaacg aaaactcacg ttaagggatt ttggtcatga gattatcaaa 4080

aaggatcttc acctagatcc ttttaaatta aaaatgaagt tttaaatcaa tctaaagtat 4140

atatgagtaa acttggtctg acagttacca atgcttaatc agtgaggcac ctatctcagc 4200

gatctgtcta tttcgttcat ccatagttgc ctgactcccc gtcgtgtaga taactacgat 4260

acgggagggc ttaccatctg gccccagtgc tgcaatgata ccgcgagacc cacgctcacc 4320

ggctccagat ttatcagcaa taaaccagcc agccggaagg gccgagcgca gaagtggtcc 4380

tgcaacttta tccgcctcca tccagtctat taattgttgc cgggaagcta gagtaagtag 4440

ttcgccagtt aatagtttgc gcaacgttgt tgccattgct acaggcatcg tggtgtcacg 4500

ctcgtcgttt ggtatggctt cattcagctc cggttcccaa cgatcaaggc gagttacatg 4560

atcccccatg ttgtgcaaaa aagcggttag ctccttcggt cctccgatcg ttgtcagaag 4620

taagttggcc gcagtgttat cactcatggt tatggcagca ctgcataatt ctcttactgt 4680

catgccatcc gtaagatgct tttctgtgac tggtgagtac tcaaccaagt cattctgaga 4740

atagtgtatg cggcgaccga gttgctcttg cccggcgtca atacgggata ataccgcgcc 4800

acatagcaga actttaaaag tgctcatcat tggaaaacgt tcttcggggc gaaaactctc 4860

aaggatctta ccgctgttga gatccagttc gatgtaaccc actcgtgcac ccaactgatc 4920

ttcagcatct tttactttca ccagcgtttc tgggtgagca aaaacaggaa ggcaaaatgc 4980

cgcaaaaaag ggaataaggg cgacacggaa atgttgaata ctcatactct tcctttttca 5040

atattattga agcatttatc agggttattg tctcatgagc ggatacatat ttgaatgtat 5100

ttagaaaaat aaacaaatag gggttccgcg cacatttccc cgaaaagtgc cacctgatgc 5160

ggtgtgaaat accgcacaga tgcgtaagga gaaaataccg catcaggaaa ttgtaagcgt 5220

taatattttg ttaaaattcg cgttaaattt ttgttaaatc agctcatttt ttaaccaata 5280

ggccgaaatc ggcaaaatcc cttataaatc aaaagaatag accgagatag ggttgagtgt 5340

tgttccagtt tggaacaaga gtccactatt aaagaacgtg gactccaacg tcaaagggcg 5400

aaaaaccgtc tatcagggcg atggcccact acgtgaacca tcaccctaat caagtttttt 5460

```
ggggtcgagg tgccgtaaag cactaaatcg gaaccctaaa gggagccccc gatttagagc     5520
ttgacgggga aagccggcga acgtggcgag aaaggaaggg aagaaagcga aaggagcggg     5580
cgctagggcg ctggcaagtg tagcggtcac gctgcgcgta accacca                   5627
```

<210> 9
<211> 5684
<212> DNA
<213> synthetic construct

<220>
<223> >SEQ ID NO 9

<400> 9

```
cacccgccgc gcttaatgcg ccgctacagg gcgcgtccat tcgccattca ggctgcgcaa      60
ctgttgggaa gggcgatcgg tgcgggcctc ttcgctatta cgccagctgg cgaaaggggg     120
atgtgctgca aggcgattaa gttgggtaac gccagggttt tcccagtcac gacgttgtaa     180
aacgacggcc agtgaattgt aatacgactc actatagggc gaattgggcc cgacgtcgca     240
tgctcccggc cgccatggcg gccgcgggaa ttcgattcta aacttacggc attggcatca     300
acgggagcca ccgtcggagt aggggaagta acaacggggg aactggtatt ggtgggcaaa     360
atttttacta gccattgatt ccattggggc agattgaacg ctcccaacca ccaaagtccc     420
cctaatacaa cgacagaact aaataacagg aaaaaaggaa tccaaggagt taccccctctt     480
ttatgggatg gaacttcatc gacattaaag gtgggagggg gaggaggcaa tggggacaat     540
ggtggtccag aaaaggaagg tggctccgac gtcaaggcaa cgggacatcc acaggattca     600
cagaaacgaa cctgggggct aaggcggttg ccacaattag tacaaaaaac gggagtagtc     660
ataggtgaaa accccgacta tagaattaga aaaatttaac tttttatccg aattttattc     720
gtccatgttc cccaaataac tatcaaaata attggaaaaa ttaaattatt tggtcgttgg     780
tcaccgctcc ctaaagacct ggccattgta aagagattac acccgggcga tttacttttc     840
gacctcattc tattagactc tcgtttggat tgcaactggt ctattttcct cttttgtttg     900
atagaaaatc ataaaaggat ttgcagacta cgggcctaaa gaactaaaaa atctatctgt     960
ttcttttcat tctctgtatt ttttatagtt tctgttgcat gggcataaag ttgccttttt    1020
aatcacaatt cagaaaatat cataatatct catttcacta aataatagtg aacggcaggt    1080
atatgtgatg ggttaaaaag gatcgatcct ctagcgaacc ccagagtccc gctcagaaga    1140
actcgtcaag aaggcgatag aaggcgatgc gctgcgaatc gggagcggcg ataccgtaaa    1200
gcacgaggaa gcggtcagcc cattcgccgc caagctcttc agcaatatca cgggtagcca    1260
acgctatgtc ctgatagcgg tccgccacac ccagccggcc acagtcgatg aatccagaaa    1320
agcggccatt ttccaccatg atattcggca agcaggcatc gccatgggtc acgacgagat    1380
```

cctcgccgtc gggcatccgc gccttgagcc tggcgaacag ttcggctggc gcgagcccct 1440
gatgctcttc gtccagatca tcctgatcga caagaccggc ttccatccga gtacgtgctc 1500
gctcgatgcg atgtttcgct tggtggtcga atgggcaggt agccggatca agcgtatgca 1560
gccgccgcat tgcatcagcc atgatggata ctttctcggc aggagcaagg tgagatgaca 1620
ggagatcctg ccccggcact tcgcccaata gcagccagtc ccttcccgct tcagtgacaa 1680
cgtcgagcac agctgcgcaa ggaacgcccg tcgtggccag ccacgatagc cgcgctgcct 1740
cgtcttggag ttcattcagg gcaccggaca ggtcggtctt gacaaaaaga accgggcgcc 1800
cctgcgctga cagccggaac acggcggcat cagagcagcc gattgtctgt tgtgcccagt 1860
catagccgaa tagcctctcc acccaagcgg ccggagaacc tgcgtgcaat ccatcttgtt 1920
caatcatgcg aaacgatcct catcctgtct cttgatcaga tcttgatccc ctgcgccatc 1980
agatccttgg cggcaagaaa gccatccagt ttactttgca gggcttccca accttaccag 2040
agggcgcccc agctggcaat tccggttcgc ttgctgtcca taaaaccgcc cagtctagct 2100
atcgccatgt aagcccactg caagctacct gctttctctt tgcgcttgcg ttttcccttg 2160
tccagatagc ccagtagctg acattcaccc gggaaaaaaa aacccccgccc ctgacagggc 2220
ggggtttttt ttcagataaa aaaaatcctt agctttcgct aaggatgatt tctgcaattg 2280
gcggccgctt ctagaatgca ctagtagcgg ccgctgcagt ccggcaaaaa aacgggcaag 2340
gtgtcaccac cctgcccttt ttctttaaaa ccgaaaagat tacttcgcgt tggagagcgt 2400
tcaccgacaa acaacagata aaacgaaagg cccagtcttt cgactgagcc tttcgtttta 2460
tttgatgcct ggtaccgggt gtaatccatt gggcacgaga gttagtaagg cagtggcaat 2520
taatagaggc ttatggttga ttcgcattgt tttgctcctg aaattttcgg caaatacaaa 2580
tacttcgctc ttctagccct attaaccatt ttaacgacaa attgatgggg caacgattaa 2640
caaataatga ataaatttta tgtttttcaa gatgaaaatt tgaaaatttg atttccttat 2700
atttctacta tagaagacta atacaattag atctaaaatt tgcaagtata aaaatcagca 2760
aatagttata ttgttaataa ttcaatgacc caataactcg tactgttatc tacgtggtga 2820
aagccaaaaa gacgaacagt ttagcctcct cctcctcggc gatcgccaag cgaaatgtca 2880
tgggagatgt tcagattgag catttttttc taaaagccct tgctaaaaca aaccacatgt 2940
gcagggtgtc cccgatgttg actaaattca gcggctcgac catatgggag agctcccaac 3000
gcgttggatg catagcttga gtattctata gtgtcaccta aatagcttgg cgtaatcatg 3060
gtcatagctg tttcctgtgt gaaattgtta tccgctcaca attccacaca acatacgagc 3120
cggaagcata aagtgtaaag cctggggtgc ctaatgagtg agctaactca cattaattgc 3180
gttgcgctca ctgcccgctt tccagtcggg aaacctgtcg tgccagctgc attaatgaat 3240
cggccaacgc gcggggagag gcggtttgcg tattgggcgc tcttccgctt cctcgctcac 3300

tgactcgctg cgctcggtcg ttcggctgcg gcgagcggta tcagctcact caaaggcggt 3360
aatacggtta tccacagaat caggggataa cgcaggaaag aacatgtgag caaaaggcca 3420
gcaaaaggcc aggaaccgta aaaaggccgc gttgctggcg tttttccata ggctccgccc 3480
ccctgacgag catcacaaaa atcgacgctc aagtcagagg tggcgaaacc cgacaggact 3540
ataaagatac caggcgtttc cccctggaag ctccctcgtg cgctctcctg ttccgaccct 3600
gccgcttacc ggatacctgt ccgcctttct cccttcggga agcgtggcgc tttctcatag 3660
ctcacgctgt aggtatctca gttcggtgta ggtcgttcgc tccaagctgg gctgtgtgca 3720
cgaacccccc gttcagcccg accgctgcgc cttatccggt aactatcgtc ttgagtccaa 3780
cccggtaaga cacgacttat cgccactggc agcagccact ggtaacagga ttagcagagc 3840
gaggtatgta ggcggtgcta cagagttctt gaagtggtgg cctaactacg gctacactag 3900
aagaacagta tttggtatct gcgctctgct gaagccagtt accttcggaa aaagagttgg 3960
tagctcttga tccggcaaac aaaccaccgc tggtagcggt ggtttttttg tttgcaagca 4020
gcagattacg cgcagaaaaa aaggatctca agaagatcct ttgatctttt ctacggggtc 4080
tgacgctcag tggaacgaaa actcacgtta agggattttg gtcatgagat tatcaaaaag 4140
gatcttcacc tagatccttt taaattaaaa atgaagtttt aaatcaatct aaagtatata 4200
tgagtaaact tggtctgaca gttaccaatg cttaatcagt gaggcaccta tctcagcgat 4260
ctgtctattt cgttcatcca tagttgcctg actccccgtc gtgtagataa ctacgatacg 4320
ggagggctta ccatctggcc ccagtgctgc aatgataccg cgagacccac gctcaccggc 4380
tccagattta tcagcaataa accagccagc cggaagggcc gagcgcagaa gtggtcctgc 4440
aactttatcc gcctccatcc agtctattaa ttgttgccgg gaagctagag taagtagttc 4500
gccagttaat agtttgcgca acgttgttgc cattgctaca ggcatcgtgg tgtcacgctc 4560
gtcgtttggt atggcttcat tcagctccgg ttcccaacga tcaaggcgag ttacatgatc 4620
ccccatgttg tgcaaaaaag cggttagctc cttcggtcct ccgatcgttg tcagaagtaa 4680
gttggccgca gtgttatcac tcatggttat ggcagcactg cataattctc ttactgtcat 4740
gccatccgta agatgctttt ctgtgactgg tgagtactca accaagtcat tctgagaata 4800
gtgtatgcgg cgaccgagtt gctcttgccc ggcgtcaata cgggataata ccgcgccaca 4860
tagcagaact ttaaaagtgc tcatcattgg aaaacgttct tcggggcgaa aactctcaag 4920
gatcttaccg ctgttgagat ccagttcgat gtaacccact cgtgcaccca actgatcttc 4980
agcatctttt actttcacca gcgtttctgg gtgagcaaaa acaggaaggc aaaatgccgc 5040
aaaaaaggga ataagggcga cacggaaatg ttgaatactc atactcttcc tttttcaata 5100
ttattgaagc atttatcagg gttattgtct catgagcgga tacatatttg aatgtattta 5160

gaaaaataaa caaatagggg ttccgcgcac atttccccga aaagtgccac ctgatgcggt 5220

gtgaaatacc gcacagatgc gtaaggagaa aataccgcat caggaaattg taagcgttaa 5280

tattttgtta aaattcgcgt taaatttttg ttaaatcagc tcatttttta accaataggc 5340

cgaaatcggc aaaatccctt ataaatcaaa agaatagacc gagatagggt tgagtgttgt 5400

tccagtttgg aacaagagtc cactattaaa gaacgtggac tccaacgtca aagggcgaaa 5460

aaccgtctat cagggcgatg gcccactacg tgaaccatca ccctaatcaa gttttttggg 5520

gtcgaggtgc cgtaaagcac taaatcggaa ccctaaaggg agcccccgat ttagagcttg 5580

acggggaaag ccggcgaacg tggcgagaaa ggaagggaag aaagcgaaag gagcgggcgc 5640

tagggcgctg gcaagtgtag cggtcacgct gcgcgtaacc acca 5684

<210> 10
<211> 5699
<212> DNA
<213> synthetic construct

<220>
<223> >SEQ ID NO 10

<400> 10

```
cacccgccgc gcttaatgcg ccgctacagg gcgcgtccat tcgccattca ggctgcgcaa      60
ctgttgggaa gggcgatcgg tgcgggcctc ttcgctatta cgccagctgg cgaaaggggg     120
atgtgctgca aggcgattaa gttgggtaac gccagggttt tcccagtcac gacgttgtaa     180
aacgacggcc agtgaattgt aatacgactc actatagggc gaattgggcc cgacgtcgca     240
tgctcccggc cgccatggcg gccgcgggaa ttcgattgtg agcttgatgg tgatggtggg     300
taaagcttca ttgtccatgc gccggagctt gacggtaaaa tccgtgtcct ggggccaatc     360
attgggaacg ctcacacaga taccgttgcg agttaagtcg aaggataccc ccaccaattc     420
cccttccata tcgaacactt ggcaaaaggc caacatccgc tcttcctgcc gtcgctcctg     480
cacccccctgg agatgggcaa tggttagggc ttcttgggag agggaagatg gagaatcttg     540
agccatggag attatttcct cggttaaatt aggtttacct agtaaatggg ccaggttgac     600
cattattgta gtcattcact gggtcgatct tccttccatc agggtcattt ggttaattgt     660
tgatgagaaa tgggaaggag taatccatag atatttgccc agttaactcg atttgagcag     720
aattgggaag gacgtattgg gaactttggt tgacagggca acaaagccag cagattacac     780
ccgggcgatt tacttttcga cctcattcta ttagactctc gtttggattg caactggtct     840
attttcctct tttgtttgat agaaaatcat aaaaggattt gcagactacg ggcctaaaga     900
actaaaaaat ctatctgttt cttttcattc tctgtatttt ttatagtttc tgttgcatgg     960
gcataaagtt gcctttttaa tcacaattca gaaaatatca taatatctca tttcactaaa    1020
```

taatagtgaa cggcaggtat atgtgatggg ttaaaaagga tcgatcctct agcgaacccc 1080
agagtcccgc tcagaagaac tcgtcaagaa ggcgatagaa ggcgatgcgc tgcgaatcgg 1140
gagcggcgat accgtaaagc acgaggaagc ggtcagccca ttcgccgcca agctcttcag 1200
caatatcacg ggtagccaac gctatgtcct gatagcggtc cgccacaccc agccggccac 1260
agtcgatgaa tccagaaaag cggccatttt ccaccatgat attcggcaag caggcatcgc 1320
catgggtcac gacgagatcc tcgccgtcgg gcatccgcgc cttgagcctg gcgaacagtt 1380
cggctggcgc gagcccctga tgctcttcgt ccagatcatc ctgatcgaca agaccggctt 1440
ccatccgagt acgtgctcgc tcgatgcgat gtttcgcttg gtggtcgaat gggcaggtag 1500
ccggatcaag cgtatgcagc cgccgcattg catcagccat gatggatact ttctcggcag 1560
gagcaaggtg agatgacagg agatcctgcc ccggcacttc gcccaatagc agccagtccc 1620
ttcccgcttc agtgacaacg tcgagcacag ctgcgcaagg aacgcccgtc gtggccagcc 1680
acgatagccg cgctgcctcg tcttggagtt cattcagggc accggacagg tcggtcttga 1740
caaaaagaac cgggcgcccc tgcgctgaca gccggaacac ggcggcatca gagcagccga 1800
ttgtctgttg tgcccagtca tagccgaata gcctctccac ccaagcggcc ggagaacctg 1860
cgtgcaatcc atcttgttca atcatgcgaa acgatcctca tcctgtctct tgatcagatc 1920
ttgatcccct gcgccatcag atccttggcg gcaagaaagc catccagttt actttgcagg 1980
gcttcccaac cttaccagag ggcgccccag ctggcaattc cggttcgctt gctgtccata 2040
aaaccgccca gtctagctat cgccatgtaa gcccactgca agctacctgc tttctctttg 2100
cgcttgcgtt ttcccttgtc cagatagccc agtagctgac attcacccgg gaaaaaaaaa 2160
ccccgcccct gacagggcgg ggtttttttt cagataaaaa aaatccttag ctttcgctaa 2220
ggatgatttc tgcaattggc ggccgcttct agaatgcact agtagcggcc gctgcagtcc 2280
ggcaaaaaaa cgggcaaggt gtcaccaccc tgcccttttt ctttaaaacc gaaaagatta 2340
cttcgcgttg gagagcgttc accgacaaac aacagataaa acgaaaggcc cagtctttcg 2400
actgagcctt tcgttttatt tgatgcctgg taccgggtgt aatcgctgga ggcgaactgg 2460
gtgagaacca taaaatcttg gggaacaggg gaatgttaat gaacacatga catgcatagg 2520
atagcgaatt gatttaaagc aatgattccc cctgagataa atatattttg acccactgct 2580
ccgcttaatt ttggcgctaa tctgggcaaa aattcgttgg ttttcggaaa ggtgaacccg 2640
tagctagctt gtattgccca aacctaactc atcattgctc catggcggcc aaaaatcttt 2700
aggacaaaac tcgccagagg tcaaggcttg attttttcag agggaaaaat tcctcggcta 2760
aataatcaaa aagttgaaaa aaaaagtttt tcagctaagg tttgatgtta tattttatct 2820
tatgtagact tttgaaaaaa acaagtcccc gcaatcaagc atcattcaac ggaaaaaata 2880
atcctatgcc aaacgcctcc accgcactaa ccggcaaagc attactcaat aaagttaaag 2940

aactatccca tctgccccgt cgagaaacgg caaaagcctg tggttactac tcgaccatat 3000

gggagagctc ccaacgcgtt ggatgcatag cttgagtatt ctatagtgtc acctaaatag 3060

cttggcgtaa tcatggtcat agctgtttcc tgtgtgaaat tgttatccgc tcacaattcc 3120

acacaacata cgagccggaa gcataaagtg taaagcctgg ggtgcctaat gagtgagcta 3180

actcacatta attgcgttgc gctcactgcc cgctttccag tcgggaaacc tgtcgtgcca 3240

gctgcattaa tgaatcggcc aacgcgcggg gagaggcggt ttgcgtattg ggcgctcttc 3300

cgcttcctcg ctcactgact cgctgcgctc ggtcgttcgg ctgcggcgag cggtatcagc 3360

tcactcaaag gcggtaatac ggttatccac agaatcaggg gataacgcag gaaagaacat 3420

gtgagcaaaa ggccagcaaa aggccaggaa ccgtaaaaag gccgcgttgc tggcgttttt 3480

ccataggctc cgcccccctg acgagcatca caaaaatcga cgctcaagtc agaggtggcg 3540

aaacccgaca ggactataaa gataccaggc gtttccccct ggaagctccc tcgtgcgctc 3600

tcctgttccg accctgccgc ttaccggata cctgtccgcc tttctccctt cgggaagcgt 3660

ggcgctttct catagctcac gctgtaggta tctcagttcg gtgtaggtcg ttcgctccaa 3720

gctgggctgt gtgcacgaac cccccgttca gcccgaccgc tgcgccttat ccggtaacta 3780

tcgtcttgag tccaacccgg taagacacga cttatcgcca ctggcagcag ccactggtaa 3840

caggattagc agagcgaggt atgtaggcgg tgctacagag ttcttgaagt ggtggcctaa 3900

ctacggctac actagaagaa cagtatttgg tatctgcgct ctgctgaagc cagttacctt 3960

cggaaaaaga gttggtagct cttgatccgg caaacaaacc accgctggta gcggtggttt 4020

ttttgtttgc aagcagcaga ttacgcgcag aaaaaaagga tctcaagaag atcctttgat 4080

cttttctacg gggtctgacg ctcagtggaa cgaaaactca cgttaaggga ttttggtcat 4140

gagattatca aaaaggatct tcacctagat ccttttaaat taaaaatgaa gttttaaatc 4200

aatctaaagt atatatgagt aaacttggtc tgacagttac caatgcttaa tcagtgaggc 4260

acctatctca gcgatctgtc tatttcgttc atccatagtt gcctgactcc ccgtcgtgta 4320

gataactacg atacgggagg gcttaccatc tggccccagt gctgcaatga taccgcgaga 4380

cccacgctca ccggctccag atttatcagc aataaaccag ccagccggaa gggccgagcg 4440

cagaagtggt cctgcaactt tatccgcctc catccagtct attaattgtt gccgggaagc 4500

tagagtaagt agttcgccag ttaatagttt gcgcaacgtt gttgccattg ctacaggcat 4560

cgtggtgtca cgctcgtcgt ttggtatggc ttcattcagc tccggttccc aacgatcaag 4620

gcgagttaca tgatccccca tgttgtgcaa aaaagcggtt agctccttcg gtcctccgat 4680

cgttgtcaga agtaagttgg ccgcagtgtt atcactcatg gttatggcag cactgcataa 4740

ttctcttact gtcatgccat ccgtaagatg cttttctgtg actggtgagt actcaaccaa 4800

gtcattctga gaatagtgta tgcggcgacc gagttgctct tgcccggcgt caatacggga 4860
taataccgcg ccacatagca gaactttaaa agtgctcatc attggaaaac gttcttcggg 4920
gcgaaaactc tcaaggatct taccgctgtt gagatccagt tcgatgtaac ccactcgtgc 4980
acccaactga tcttcagcat cttttacttt caccagcgtt tctgggtgag caaaaacagg 5040
aaggcaaaat gccgcaaaaa agggaataag ggcgacacgg aaatgttgaa tactcatact 5100
cttccttttt caatattatt gaagcattta tcagggttat tgtctcatga gcggatacat 5160
atttgaatgt atttagaaaa ataaacaaat aggggttccg cgcacatttc cccgaaaagt 5220
gccacctgat gcggtgtgaa ataccgcaca gatgcgtaag gagaaaatac cgcatcagga 5280
aattgtaagc gttaatattt tgttaaaatt cgcgttaaat ttttgttaaa tcagctcatt 5340
ttttaaccaa taggccgaaa tcggcaaaat cccttataaa tcaaaagaat agaccgagat 5400
agggttgagt gttgttccag tttggaacaa gagtccacta ttaaagaacg tggactccaa 5460
cgtcaaaggg cgaaaaaccg tctatcaggg cgatggccca ctacgtgaac catcacccta 5520
atcaagtttt ttggggtcga ggtgccgtaa agcactaaat cggaacccta aagggagccc 5580
ccgatttaga gcttgacggg gaaagccggc gaacgtggcg agaaaggaag ggaagaaagc 5640
gaaaggagcg ggcgctaggg cgctggcaag tgtagcggtc acgctgcgcg taaccacca 5699

<210> 11
<211> 7564
<212> DNA
<213> synthetic construct

<220>
<223> >SEQ ID NO 11

<400> 11

```
tgtccaaatc agcattgctc tgccaggtga gacggagttt ttgtcccact aatccctgtt      60
ggatagaagg cgcttgatac agttccaacc aaacccaatc tcctgtcctg gctttagttt     120
cttccaccgt cggcaggatt aaacgaccca accactctcc caaaggccga taatacgcct     180
catctaaatt ctgttgcaga ggataataat ctacctggtt ggcgggcaac tgactgctaa     240
tttgataatc cgggatgcgg ccagaggttt cctgaggttg aaacagcgta gctagggaaa     300
tcaccaccag agcggcgatc gccaccaata ccatccgcca gcgccattgc ttcatctgtg     360
gactgcccta aaaattgcca taaaaaaaca cctggggcgt ctttccttgt ttcgactcca     420
gatgtttttc taatttcctc acacctgtcc taagtataaa tcaccaacgg ggattgtcaa     480
ggcttcagga ttacacccgg gcaagcggat ggctgatgaa accaagccaa ccaggaaggg     540
cagcccacct atcaaggtgt actgccttcc agacgaacga agagcgattg aggaaaaggc     600
ggcggcggcc ggcatgagcc tgtcggccta cctgctggcc gtcggccagg gctacaaaat     660
```

cacgggcgtc gtggactatg agcacgtccg cgagggcgtc ccggaaaacg attccgaagc 720
ccaacctttc atagaaggcg gcggtggaat cgaaatctcg tgatggcagg ttgggcgtcg 780
cttggtcggt catttcgctc ggtaccatcg gcattttctt ttgcgttttt atttgttaac 840
tgttaattgt ccttgttcaa ggatgctgtc tttgacaaca gatgttttct tgcctttgat 900
gttcagcagg aagctcggcg caaacgttga ttgtttgtct gcgtagaatc ctctgtttgt 960
catatagctt gtaatcacga cattgtttcc tttcgcttga ggtacagcga agtgtgagta 1020
agtaaaggtt acatcgttag gatcaagatc catttttaac acaaggccag ttttgttcag 1080
cggcttgtat gggccagtta aagaattaga aacataacca agcatgtaaa tatcgttaga 1140
cgtaatgccg tcaatcgtca tttttgatcc gcgggagtca gtgaacaggt accatttgcc 1200
gttcatttta aagacgttcg cgcgttcaat ttcatctgtt actgtgttag atgcaatcag 1260
cggtttcatc acttttttca gtgtgtaatc atcgtttagc tcaatcatac cgagagcgcc 1320
gtttgctaac tcagccgtgc gtttttttatc gctttgcaga agtttttgac tttcttgacg 1380
gaagaatgat gtgcttttgc catagtatgc tttgttaaat aaagattctt cgccttggta 1440
gccatcttca gttccagtgt ttgcttcaaa tactaagtat ttgtggcctt tatcttctac 1500
gtagtgagga tctctcagcg tatggttgtc gcctgagctg tagttgcctt catcgatgaa 1560
ctgctgtaca ttttgatacg tttttccgtc accgtcaaag attgatttat aatcctctac 1620
accgttgatg ttcaaagagc tgtctgatgc tgatacgtta acttgtgcag ttgtcagtgt 1680
ttgtttgccg taatgtttac cggagaaatc agtgtagaat aaacggattt ttccgtcaga 1740
tgtaaatgtg gctgaacctg accattcttg tgtttggtct tttaggatag aatcatttgc 1800
atcgaatttg tcgctgtctt taaagacgcg gccagcgttt ttccagctgt caatagaagt 1860
ttcgccgact ttttgataga acatgtaaat cgatgtgtca tccgcatttt taggatctcc 1920
ggctaatgca aagacgatgt ggtagccgtg atagtttgcg acagtgccgt cagcgttttg 1980
taatggccag ctgtcccaaa cgtccaggcc ttttgcagaa gagatatttt taattgtgga 2040
cgaatcaaat tcagaaactt gatatttttc attttttgc tgttcaggga tttgcagcat 2100
atcatggcgt gtaatatggg aaatgccgta tgtttcctta tatggctttt ggttcgtttc 2160
tttcgcaaac gcttgagttg cgcctcctgc cagcagtgcg gtagtaaagg ttaatactgt 2220
tgcttgtttt gcaaactttt tgatgttcat cgttcatgtc tcctttttta tgtactgtgt 2280
tagcggtctg cttcttccag ccctcctgtt tgaagatggc aagttagtta cgcacaataa 2340
aaaaagacct aaaatatgta aggggtgacg ccaaagtata cactttgccc tttacacatt 2400
ttaggtcttg cctgctttat cagtaacaaa cccgcgcgat ttacttttcg acctcattct 2460
attagactct cgtttggatt gcaactggtc tattttcctc ttttgtttga tagaaaatca 2520
taaaaggatt tgcagactac gggcctaaag aactaaaaa tctatctgtt tcttttcatt 2580

ctctgtattt tttatagttt ctgttgcatg ggcataaagt tgccttttta atcacaattc 2640
agaaaatatc ataatatctc atttcactaa ataatagtga acggcaggta tatgtgatgg 2700
gttaaaaagg atcgatcctc tagcgaaccc cagagtcccg ctcagaagaa ctcgtcaaga 2760
aggcgataga aggcgatgcg ctgcgaatcg ggagcggcga taccgtaaag cacgaggaag 2820
cggtcagccc attcgccgcc aagctcttca gcaatatcac gggtagccaa cgctatgtcc 2880
tgatagcggt ccgccacacc cagccggcca cagtcgatga atccagaaaa gcggccattt 2940
tccaccatga tattcggcaa gcaggcatcg ccatgggtca cgacgagatc ctcgccgtcg 3000
ggcatccgcg ccttgagcct ggcgaacagt tcggctggcg cgagcccctg atgctcttcg 3060
tccagatcat cctgatcgac aagaccggct tccatccgag tacgtgctcg ctcgatgcga 3120
tgtttcgctt ggtggtcgaa tgggcaggta gccggatcaa gcgtatgcag ccgccgcatt 3180
gcatcagcca tgatggatac tttctcggca ggagcaaggt gagatgacag gagatcctgc 3240
cccggcactt cgcccaatag cagccagtcc cttcccgctt cagtgacaac gtcgagcaca 3300
gctgcgcaag gaacgcccgt cgtggccagc cacgatagcc gcgctgcctc gtcttggagt 3360
tcattcaggg caccggacag gtcggtcttg acaaaaagaa ccgggcgccc ctgcgctgac 3420
agccggaaca cggcggcatc agagcagccg attgtctgtt gtgcccagtc atagccgaat 3480
agcctctcca cccaagcggc cggagaacct gcgtgcaatc catcttgttc aatcatgcga 3540
aacgatcctc atcctgtctc ttgatcagat cttgatcccc tgcgccatca gatccttggc 3600
ggcaagaaag ccatccagtt tactttgcag ggcttcccaa ccttaccaga gggcgcccca 3660
gctggcaatt ccggttcgct tgctgtccat aaaaccgccc agtctagcta tcgccatgta 3720
agcccactgc aagctacctg ctttctcttt gcgcttgcgt ttcccttgt ccagatagcc 3780
cagtagctga cattcacccg ggaaaaaaaa accccgcccc tgacagggcg gggttttttt 3840
tcagataaaa aaaatcctta gctttcgcta aggatgattt ctgcaattgg cggccgcttc 3900
tagaatgcac tagtagcggc cgctgcagtc cggcaaaaaa acgggcaagg tgtcaccacc 3960
ctgccctttt tctttaaaac cgaaaagatt acttcgcgtt ggagagcgtt caccgacaaa 4020
caacagataa aacgaaaggc ccagtctttc gactgagcct ttcgttttat ttgatgcctg 4080
gtaccgggtg taatcggttg attatttcag tggcccggcc gatggataat taccatggcc 4140
cgaccgaccg ggatttccag accagtttga acagtattaa attagccttt ccggacaagg 4200
cctctgtgaa aatcttaaaa aagtattaag attttaaaag ctagcttatt ttcggaggaa 4260
atgtgtttac tcgacttgcc cagcaacacc gcgatttcgt gaaggattta gtcatgagct 4320
tgagggcttt ggccactgtg ctcgaaaatc ggggctacat tgcttcttgc tacacctgtg 4380
gcgaccaact caacagcgcc tccttcatgg tgagcttggg ggaaaatcat ctgatccgct 4440

ttttggtatc ggactacggc atcacctgga cagaaatgcg ggatgaccga gaattaatga 4500

aattagaagg agccgaggcg atcgcccagt tggaagaatt ggccaatgtg gtcaaatatt 4560

gcctagcaga tgcccccact cgaccatatg ggagagctcc caacgcgttg gatgcatagc 4620

ttgagtattc tatagtgtca cctaaatagc ttggcgtaat catggtcata gctgtttcct 4680

gtgtgaaatt gttatccgct cacaattcca cacaacatac gagccggaag cataaagtgt 4740

aaagcctggg gtgcctaatg agtgagctaa ctcacattaa ttgcgttgcg ctcactgccc 4800

gctttccagt cgggaaacct gtcgtgccag ctgcattaat gaatcggcca acgcgcgggg 4860

agaggcggtt tgcgtattgg gcgctcttcc gcttcctcgc tcactgactc gctgcgctcg 4920

gtcgttcggc tgcggcgagc ggtatcagct cactcaaagg cggtaatacg gttatccaca 4980

gaatcagggg ataacgcagg aaagaacatg tgagcaaaag gccagcaaaa ggccaggaac 5040

cgtaaaaagg ccgcgttgct ggcgtttttc cataggctcc gcccccctga cgagcatcac 5100

aaaaatcgac gctcaagtca gaggtggcga aacccgacag gactataaag ataccaggcg 5160

tttccccctg gaagctccct cgtgcgctct cctgttccga ccctgccgct taccggatac 5220

ctgtccgcct ttctcccttc gggaagcgtg gcgctttctc atagctcacg ctgtaggtat 5280

ctcagttcgg tgtaggtcgt tcgctccaag ctgggctgtg tgcacgaacc ccccgttcag 5340

cccgaccgct gcgccttatc cggtaactat cgtcttgagt ccaacccggt aagacacgac 5400

ttatcgccac tggcagcagc cactggtaac aggattagca gagcgaggta tgtaggcggt 5460

gctacagagt tcttgaagtg gtggcctaac tacggctaca ctagaagaac agtatttggt 5520

atctgcgctc tgctgaagcc agttaccttc ggaaaaagag ttggtagctc ttgatccggc 5580

aaacaaacca ccgctggtag cggtggtttt tttgtttgca agcagcagat tacgcgcaga 5640

aaaaaaggat ctcaagaaga tcctttgatc ttttctacgg ggtctgacgc tcagtggaac 5700

gaaaactcac gttaagggat tttggtcatg agattatcaa aaaggatctt cacctagatc 5760

cttttaaatt aaaaatgaag ttttaaatca atctaaagta tatatgagta aacttggtct 5820

gacagttacc aatgcttaat cagtgaggca cctatctcag cgatctgtct atttcgttca 5880

tccatagttg cctgactccc cgtcgtgtag ataactacga tacgggaggg cttaccatct 5940

ggccccagtg ctgcaatgat accgcgagac ccacgctcac cggctccaga tttatcagca 6000

ataaaccagc cagccggaag ggccgagcgc agaagtggtc ctgcaacttt atccgcctcc 6060

atccagtcta ttaattgttg ccgggaagct agagtaagta gttcgccagt taatagtttg 6120

cgcaacgttg ttgccattgc tacaggcatc gtggtgtcac gctcgtcgtt tggtatggct 6180

tcattcagct ccggttccca acgatcaagg cgagttacat gatcccccat gttgtgcaaa 6240

aaagcggtta gctccttcgg tcctccgatc gttgtcagaa gtaagttggc cgcagtgtta 6300

tcactcatgg ttatggcagc actgcataat tctcttactg tcatgccatc cgtaagatgc 6360

ttttctgtga ctggtgagta ctcaaccaag tcattctgag aatagtgtat gcggcgaccg 6420

agttgctctt gcccggcgtc aatacgggat aataccgcgc cacatagcag aactttaaaa 6480

gtgctcatca ttggaaaacg ttcttcgggg cgaaaactct caaggatctt accgctgttg 6540

agatccagtt cgatgtaacc cactcgtgca cccaactgat cttcagcatc ttttactttc 6600

accagcgttt ctgggtgagc aaaaacagga aggcaaaatg ccgcaaaaaa gggaataagg 6660

gcgacacgga aatgttgaat actcatactc ttcctttttc aatattattg aagcatttat 6720

cagggttatt gtctcatgag cggatacata tttgaatgta tttagaaaaa taaacaaata 6780

ggggttccgc gcacatttcc ccgaaaagtg ccacctgatg cggtgtgaaa taccgcacag 6840

atgcgtaagg agaaaatacc gcatcaggaa attgtaagcg ttaatatttt gttaaaattc 6900

gcgttaaatt tttgttaaat cagctcattt tttaaccaat aggccgaaat cggcaaaatc 6960

ccttataaat caaaagaata gaccgagata gggttgagtg ttgttccagt ttggaacaag 7020

agtccactat taaagaacgt ggactccaac gtcaaagggc gaaaaaccgt ctatcagggc 7080

gatggcccac tacgtgaacc atcaccctaa tcaagttttt tggggtcgag gtgccgtaaa 7140

gcactaaatc ggaaccctaa agggagcccc cgatttagag cttgacgggg aaagccggcg 7200

aacgtggcga gaaaggaagg gaagaaagcg aaaggagcgg gcgctagggc gctggcaagt 7260

gtagcggtca cgctgcgcgt aaccaccaca cccgccgcgc ttaatgcgcc gctacagggc 7320

gcgtccattc gccattcagg ctgcgcaact gttgggaagg gcgatcggtg cgggcctctt 7380

cgctattacg ccagctggcg aaagggggat gtgctgcaag gcgattaagt tgggtaacgc 7440

cagggttttc ccagtcacga cgttgtaaaa cgacggccag tgaattgtaa tacgactcac 7500

tatagggcga attgggcccg acgtcgcatg ctcccggccg ccatggcggc cgcgggaatt 7560

cgat 7564

<210> 12
<211> 7584
<212> DNA
<213> synthetic construct

<220>
<223> >SEQ ID NO 12

<400> 12

```
cacccgccgc gcttaatgcg ccgctacagg gcgcgtccat tcgccattca ggctgcgcaa      60
ctgttgggaa gggcgatcgg tgcgggcctc ttcgctatta cgccagctgg cgaaaggggg     120
atgtgctgca aggcgattaa gttgggtaac gccagggttt tcccagtcac gacgttgtaa     180
aacgacggcc agtgaattgt aatacgactc actatagggc gaattgggcc cgacgtcgca     240
tgctcccggc cgccatggcg gccgcgggaa ttcgattgat gaccgctggc ggagtttagt     300
```

ccagggttat ttgtaccgtc aaggttacat tgccacccca gatttaacta cgctgcgagt 360

ccgtaccatt ggcgatcggg cctatctcac cattaagggt aaaaatgcca gcattgcccg 420

cttggatttg agtatgaaat tcttgcagtg gaagccaatt aaattttgac agaactctgt 480

tcaccgcccc tgattgaaaa tatcgttatt gcctcgatta ccatggtaaa acctgagaag 540

tagacgagtt tttgggggat aaccagggtc taattttagc ggaagtggaa ttaacctaca 600

ctggtgaaaa aataagtcta cttccctgga tcggggaaga ggtaacggat gatgcccgct 660

attacaacgt caatttagcc caacatccct acaaaaattg gtgacaagat tacacccggg 720

caagcggatg gctgatgaaa ccaagccaac caggaagggc agcccaccta tcaaggtgta 780

ctgccttcca gacgaacgaa gagcgattga ggaaaaggcg gcggcggccg gcatgagcct 840

gtcggcctac ctgctggccg tcggccaggg ctacaaaatc acgggcgtcg tggactatga 900

gcacgtccgc gagggcgtcc cggaaaacga ttccgaagcc caacctttca tagaaggcgg 960

cggtggaatc gaaatctcgt gatggcaggt tgggcgtcgc ttggtcggtc atttcgctcg 1020

gtaccatcgg cattttcttt tgcgttttta tttgttaact gttaattgtc cttgttcaag 1080

gatgctgtct ttgacaacag atgttttctt gcctttgatg ttcagcagga agctcggcgc 1140

aaacgttgat tgtttgtctg cgtagaatcc tctgtttgtc atatagcttg taatcacgac 1200

attgtttcct ttcgcttgag gtacagcgaa gtgtgagtaa gtaaaggtta catcgttagg 1260

atcaagatcc atttttaaca caaggccagt tttgttcagc ggcttgtatg ggccagttaa 1320

agaattagaa acataaccaa gcatgtaaat atcgttagac gtaatgccgt caatcgtcat 1380

ttttgatccg cgggagtcag tgaacaggta ccatttgccg ttcattttaa agacgttcgc 1440

gcgttcaatt tcatctgtta ctgtgttaga tgcaatcagc ggtttcatca ctttttttcag 1500

tgtgtaatca tcgtttagct caatcatacc gagagcgccg tttgctaact cagccgtgcg 1560

ttttttatcg ctttgcagaa gtttttgact ttcttgacgg aagaatgatg tgcttttgcc 1620

atagtatgct ttgttaaata aagattcttc gccttggtag ccatcttcag ttccagtgtt 1680

tgcttcaaat actaagtatt tgtggccttt atcttctacg tagtgaggat ctctcagcgt 1740

atggttgtcg cctgagctgt agttgccttc atcgatgaac tgctgtacat tttgatacgt 1800

ttttccgtca ccgtcaaaga ttgatttata atcctctaca ccgttgatgt tcaaagagct 1860

gtctgatgct gatacgttaa cttgtgcagt tgtcagtgtt tgtttgccgt aatgtttacc 1920

ggagaaatca gtgtagaata aacggatttt tccgtcagat gtaaatgtgg ctgaacctga 1980

ccattcttgt gtttggtctt ttaggataga atcatttgca tcgaatttgt cgctgtcttt 2040

aaagacgcgg ccagcgtttt tccagctgtc aatagaagtt tcgccgactt tttgatagaa 2100

catgtaaatc gatgtgtcat ccgcattttt aggatctccg gctaatgcaa agacgatgtg 2160

gtagccgtga tagtttgcga cagtgccgtc agcgttttgt aatggccagc tgtcccaaac 2220
gtccaggcct tttgcagaag agatattttt aattgtggac gaatcaaatt cagaaacttg 2280
atatttttca tttttttgct gttcagggat ttgcagcata tcatggcgtg taatatggga 2340
aatgccgtat gtttccttat atggcttttg gttcgtttct ttcgcaaacg cttgagttgc 2400
gcctcctgcc agcagtgcgg tagtaaaggt taatactgtt gcttgttttg caaacttttt 2460
gatgttcatc gttcatgtct cctttttat gtactgtgtt agcggtctgc ttcttccagc 2520
cctcctgttt gaagatggca agttagttac gcacaataaa aaaagaccta aaatatgtaa 2580
ggggtgacgc caaagtatac actttgccct ttacacattt taggtcttgc ctgctttatc 2640
agtaacaaac ccgcgcgatt tacttttcga cctcattcta ttagactctc gtttggattg 2700
caactggtct attttcctct tttgtttgat agaaaatcat aaaaggattt gcagactacg 2760
ggcctaaaga actaaaaaat ctatctgttt cttttcattc tctgtatttt ttatagtttc 2820
tgttgcatgg gcataaagtt gcctttttaa tcacaattca gaaaatatca taatatctca 2880
tttcactaaa taatagtgaa cggcaggtat atgtgatggg ttaaaaagga tcgatcctct 2940
agcgaacccc agagtcccgc tcagaagaac tcgtcaagaa ggcgatagaa ggcgatgcgc 3000
tgcgaatcgg gagcggcgat accgtaaagc acgaggaagc ggtcagccca ttcgccgcca 3060
agctcttcag caatatcacg ggtagccaac gctatgtcct gatagcggtc cgccacaccc 3120
agccggccac agtcgatgaa tccagaaaag cggccatttt ccaccatgat attcggcaag 3180
caggcatcgc catgggtcac gacgagatcc tcgccgtcgg gcatccgcgc cttgagcctg 3240
gcgaacagtt cggctggcgc gagcccctga tgctcttcgt ccagatcatc ctgatcgaca 3300
agaccggctt ccatccgagt acgtgctcgc tcgatgcgat gtttcgcttg gtggtcgaat 3360
gggcaggtag ccggatcaag cgtatgcagc cgccgcattg catcagccat gatggatact 3420
ttctcggcag gagcaaggtg agatgacagg agatcctgcc ccggcacttc gcccaatagc 3480
agccagtccc ttcccgcttc agtgacaacg tcgagcacag ctgcgcaagg aacgcccgtc 3540
gtggccagcc acgatagccg cgctgcctcg tcttggagtt cattcagggc accggacagg 3600
tcggtcttga caaaaagaac cgggcgcccc tgcgctgaca gccggaacac ggcggcatca 3660
gagcagccga ttgtctgttg tgcccagtca tagccgaata gcctctccac ccaagcggcc 3720
ggagaacctg cgtgcaatcc atcttgttca atcatgcgaa acgatcctca tcctgtctct 3780
tgatcagatc ttgatcccct gcgccatcag atccttggcg gcaagaaagc catccagttt 3840
actttgcagg gcttcccaac cttaccagag ggcgccccag ctggcaattc cggttcgctt 3900
gctgtccata aaaccgccca gtctagctat cgccatgtaa gcccactgca agctacctgc 3960
tttctctttg cgcttgcgtt ttcccttgtc cagatagccc agtagctgac attcacccgg 4020
gaaaaaaaaa ccccgcccct gacagggcgg ggtttttttt cagataaaaa aaatccttag 4080

ctttcgctaa ggatgatttc tgcaattggc ggccgcttct agaatgcact agtagcggcc 4140
gctgcagtcc ggcaaaaaaa cgggcaaggt gtcaccaccc tgcccttttt ctttaaaacc 4200
gaaaagatta cttcgcgttg gagagcgttc accgacaaac aacagataaa acgaaaggcc 4260
cagtctttcg actgagcctt tcgttttatt tgatgcctgg taccgggtgt aatccttcat 4320
tcccctattg ttattaatag tgctctgaaa ctaacaaata agcaagatca aattgtgaag 4380
aaaacgcaac aaaatgaacc attaagataa tttctgtcta tcctggctgg tatattccag 4440
tctactcaga aatgagtttg ttttatgaca aatcagtatg taccaaatta cttggcgcaa 4500
tcgatcctgg taactctatt ttgttgctta cccctgggta ttgtggccat tatcaaagca 4560
tcggaagtta attctcgttt agcttcaggg gactatgaag gcgctgtaaa ggcttccaag 4620
gaagcgaaaa agtttgttgg gtggtccttt ggtgccggca taattttcat tgccatctat 4680
tttgtgctag tggttattgc cgccgtcttt ggtcagtaat taaagttaca ttttttgact 4740
ttgccttgtt caccattcat taacgaatac catgtttagt ttgaaaaatt attccccatc 4800
tccattacta tccgctaagg ccaaggaata tttagtattc actttggtaa ccctaaccat 4860
tgtcgttgct ggcactcgac catatgggag agctcccaac gcgttggatg catagcttga 4920
gtattctata gtgtcaccta aatagcttgg cgtaatcatg gtcatagctg tttcctgtgt 4980
gaaattgtta tccgctcaca attccacaca acatacgagc cggaagcata aagtgtaaag 5040
cctggggtgc ctaatgagtg agctaactca cattaattgc gttgcgctca ctgcccgctt 5100
tccagtcggg aaacctgtcg tgccagctgc attaatgaat cggccaacgc gcggggagag 5160
gcggtttgcg tattgggcgc tcttccgctt cctcgctcac tgactcgctg cgctcggtcg 5220
ttcggctgcg gcgagcggta tcagctcact caaaggcggt aatacggtta tccacagaat 5280
caggggataa cgcaggaaag aacatgtgag caaaaggcca gcaaaaggcc aggaaccgta 5340
aaaaggccgc gttgctggcg tttttccata ggctccgccc ccctgacgag catcacaaaa 5400
atcgacgctc aagtcagagg tggcgaaacc cgacaggact ataaagatac caggcgtttc 5460
cccctggaag ctccctcgtg cgctctcctg ttccgaccct gccgcttacc ggatacctgt 5520
ccgcctttct cccttcggga agcgtggcgc tttctcatag ctcacgctgt aggtatctca 5580
gttcggtgta ggtcgttcgc tccaagctgg gctgtgtgca cgaacccccc gttcagcccg 5640
accgctgcgc cttatccggt aactatcgtc ttgagtccaa cccggtaaga cacgacttat 5700
cgccactggc agcagccact ggtaacagga ttagcagagc gaggtatgta ggcggtgcta 5760
cagagttctt gaagtggtgg cctaactacg gctacactag aagaacagta tttggtatct 5820
gcgctctgct gaagccagtt accttcggaa aaagagttgg tagctcttga tccggcaaac 5880
aaaccaccgc tggtagcggt ggtttttttg tttgcaagca gcagattacg cgcagaaaaa 5940

aaggatctca agaagatcct ttgatctttt ctacggggtc tgacgctcag tggaacgaaa 6000

actcacgtta agggattttg gtcatgagat tatcaaaaag gatcttcacc tagatccttt 6060

taaattaaaa atgaagtttt aaatcaatct aaagtatata tgagtaaact tggtctgaca 6120

gttaccaatg cttaatcagt gaggcaccta tctcagcgat ctgtctattt cgttcatcca 6180

tagttgcctg actccccgtc gtgtagataa ctacgatacg ggagggctta ccatctggcc 6240

ccagtgctgc aatgataccg cgagacccac gctcaccggc tccagattta tcagcaataa 6300

accagccagc cggaagggcc gagcgcagaa gtggtcctgc aactttatcc gcctccatcc 6360

agtctattaa ttgttgccgg gaagctagag taagtagttc gccagttaat agtttgcgca 6420

acgttgttgc cattgctaca ggcatcgtgg tgtcacgctc gtcgtttggt atggcttcat 6480

tcagctccgg ttcccaacga tcaaggcgag ttacatgatc ccccatgttg tgcaaaaaag 6540

cggttagctc cttcggtcct ccgatcgttg tcagaagtaa gttggccgca gtgttatcac 6600

tcatggttat ggcagcactg cataattctc ttactgtcat gccatccgta agatgctttt 6660

ctgtgactgg tgagtactca accaagtcat tctgagaata gtgtatgcgg cgaccgagtt 6720

gctcttgccc ggcgtcaata cgggataata ccgcgccaca tagcagaact ttaaaagtgc 6780

tcatcattgg aaaacgttct tcggggcgaa aactctcaag gatcttaccg ctgttgagat 6840

ccagttcgat gtaacccact cgtgcaccca actgatcttc agcatctttt actttcacca 6900

gcgtttctgg gtgagcaaaa acaggaaggc aaaatgccgc aaaaaaggga ataagggcga 6960

cacggaaatg ttgaatactc atactcttcc tttttcaata ttattgaagc atttatcagg 7020

gttattgtct catgagcgga tacatatttg aatgtattta gaaaaataaa caaatagggg 7080

ttccgcgcac atttccccga aaagtgccac ctgatgcggt gtgaaatacc gcacagatgc 7140

gtaaggagaa aataccgcat caggaaattg taagcgttaa tattttgtta aaattcgcgt 7200

taaatttttg ttaaatcagc tcatttttta accaataggc cgaaatcggc aaaatccctt 7260

ataaatcaaa agaatagacc gagatagggt tgagtgttgt tccagtttgg aacaagagtc 7320

cactattaaa gaacgtggac tccaacgtca aagggcgaaa aaccgtctat cagggcgatg 7380

gcccactacg tgaaccatca ccctaatcaa gtttttgggg gtcgaggtgc cgtaaagcac 7440

taaatcggaa ccctaaaggg agcccccgat ttagagcttg acggggaaag ccggcgaacg 7500

tggcgagaaa ggaagggaag aaagcgaaag gagcgggcgc tagggcgctg gcaagtgtag 7560

cggtcacgct gcgcgtaacc acca 7584

<210> 13
<211> 7562
<212> DNA
<213> synthetic construct

<220>
<223> >SEQ ID NO 13

<400> 13

cacccgccgc gcttaatgcg ccgctacagg gcgcgtccat tcgccattca ggctgcgcaa 60

ctgttgggaa gggcgatcgg tgcgggcctc ttcgctatta cgccagctgg cgaaaggggg 120

atgtgctgca aggcgattaa gttgggtaac gccagggttt tcccagtcac gacgttgtaa 180

aacgacggcc agtgaattgt aatacgactc actatagggc gaattgggcc cgacgtcgca 240

tgctcccggc cgccatggcg gccgcgggaa ttcgattgga ctgacccaag atacagtggt 300

agcttcaagt tcgtcaatca accccaatgg aaagctgaga taatctgttg ttgatgcgac 360

cgtcttcatt ttagctaaaa agacaagtct gtggctagtt actatgacga ggccatcggg 420

tctatcccag cggtcttgct gattccaaaa cttggcgatt ttctgaagta gtatcttttc 480

accgcttaag ccgccaaggt cttggatgag cttggtcgaa tcatcttttg atgcctcgac 540

atcgcgaata actttgtaaa cgaccttccc gagcttgaat gcaaagtcgg ttagattatc 600

catcagagtt gaatgccgat aatacagact gctatactat tttaatgaaa agagtgctgg 660

caagcaagcc cgccgaacgt tgaagctgtg cagcgcaaac gaagcgcatt gtagtttgcg 720

ctcggattac acccgggcaa gcggatggct gatgaaacca agccaaccag gaagggcagc 780

ccacctatca aggtgtactg ccttccagac gaacgaagag cgattgagga aaaggcggcg 840

gcggccggca tgagcctgtc ggcctacctg ctggccgtcg gccagggcta caaaatcacg 900

ggcgtcgtgg actatgagca cgtccgcgag ggcgtcccgg aaaacgattc cgaagcccaa 960

cctttcatag aaggcggcgg tggaatcgaa atctcgtgat ggcaggttgg gcgtcgcttg 1020

gtcggtcatt tcgctcggta ccatcggcat ttcttttgc gtttttattt gttaactgtt 1080

aattgtcctt gttcaaggat gctgtctttg acaacagatg ttttcttgcc tttgatgttc 1140

agcaggaagc tcggcgcaaa cgttgattgt ttgtctgcgt agaatcctct gtttgtcata 1200

tagcttgtaa tcacgacatt gtttcctttc gcttgaggta cagcgaagtg tgagtaagta 1260

aaggttacat cgttaggatc aagatccatt tttaacacaa ggccagtttt gttcagcggc 1320

ttgtatgggc cagttaaaga attagaaaca taaccaagca tgtaaatatc gttagacgta 1380

atgccgtcaa tcgtcatttt tgatccgcgg gagtcagtga acaggtacca tttgccgttc 1440

attttaaaga cgttcgcgcg ttcaatttca tctgttactg tgttagatgc aatcagcggt 1500

ttcatcactt ttttcagtgt gtaatcatcg tttagctcaa tcataccgag agcgccgttt 1560

gctaactcag ccgtgcgttt tttatcgctt tgcagaagtt tttgactttc ttgacggaag 1620

aatgatgtgc ttttgccata gtatgctttg ttaaataaag attcttcgcc ttggtagcca 1680

tcttcagttc cagtgtttgc ttcaaatact aagtatttgt ggcctttatc ttctacgtag 1740

tgaggatctc tcagcgtatg gttgtcgcct gagctgtagt tgccttcatc gatgaactgc 1800

tgtacatttt gatacgtttt tccgtcaccg tcaaagattg atttataatc ctctacaccg 1860
ttgatgttca aagagctgtc tgatgctgat acgttaactt gtgcagttgt cagtgtttgt 1920
ttgccgtaat gtttaccgga gaaatcagtg tagaataaac ggatttttcc gtcagatgta 1980
aatgtggctg aacctgacca ttcttgtgtt tggtctttta ggatagaatc atttgcatcg 2040
aatttgtcgc tgtctttaaa gacgcggcca gcgtttttcc agctgtcaat agaagtttcg 2100
ccgacttttt gatagaacat gtaaatcgat gtgtcatccg catttttagg atctccggct 2160
aatgcaaaga cgatgtggta gccgtgatag tttgcgacag tgccgtcagc gttttgtaat 2220
ggccagctgt cccaaacgtc caggcctttt gcagaagaga tatttttaat tgtggacgaa 2280
tcaaattcag aaacttgata tttttcattt ttttgctgtt cagggatttg cagcatatca 2340
tggcgtgtaa tatgggaaat gccgtatgtt tccttatatg gcttttggtt cgtttctttc 2400
gcaaacgctt gagttgcgcc tcctgccagc agtgcggtag taaaggttaa tactgttgct 2460
tgttttgcaa actttttgat gttcatcgtt catgtctcct ttttatgta ctgtgttagc 2520
ggtctgcttc ttccagccct cctgtttgaa gatggcaagt tagttacgca caataaaaaa 2580
agacctaaaa tatgtaaggg gtgacgccaa agtatacact ttgcccttta cacattttag 2640
gtcttgcctg ctttatcagt aacaaacccg cgcgatttac ttttcgacct cattctatta 2700
gactctcgtt tggattgcaa ctggtctatt ttcctctttt gtttgataga aaatcataaa 2760
aggatttgca gactacgggc ctaaagaact aaaaaatcta tctgtttctt ttcattctct 2820
gtatttttta tagtttctgt tgcatgggca taaagttgcc tttttaatca caattcagaa 2880
aatatcataa tatctcattt cactaaataa tagtgaacgg caggtatatg tgatgggtta 2940
aaaaggatcg atcctctagc gaaccccaga gtcccgctca gaagaactcg tcaagaaggc 3000
gatagaaggc gatgcgctgc gaatcgggag cggcgatacc gtaaagcacg aggaagcggt 3060
cagcccattc gccgccaagc tcttcagcaa tatcacgggt agccaacgct atgtcctgat 3120
agcggtccgc cacacccagc cggccacagt cgatgaatcc agaaaagcgg ccattttcca 3180
ccatgatatt cggcaagcag gcatcgccat gggtcacgac gagatcctcg ccgtcgggca 3240
tccgcgcctt gagcctggcg aacagttcgg ctggcgcgag cccctgatgc tcttcgtcca 3300
gatcatcctg atcgacaaga ccggcttcca tccgagtacg tgctcgctcg atgcgatgtt 3360
tcgcttggtg gtcgaatggg caggtagccg gatcaagcgt atgcagccgc cgcattgcat 3420
cagccatgat ggatactttc tcggcaggag caaggtgaga tgacaggaga tcctgccccg 3480
gcacttcgcc caatagcagc cagtcccttc ccgcttcagt gacaacgtcg agcacagctg 3540
cgcaaggaac gcccgtcgtg gccagccacg atagccgcgc tgcctcgtct tggagttcat 3600
tcagggcacc ggacaggtcg gtcttgacaa aaagaaccgg gcgcccctgc gctgacagcc 3660

ggaacacggc ggcatcagag cagccgattg tctgttgtgc ccagtcatag ccgaatagcc 3720
tctccaccca agcggccgga gaacctgcgt gcaatccatc ttgttcaatc atgcgaaacg 3780
atcctcatcc tgtctcttga tcagatcttg atcccctgcg ccatcagatc cttggcggca 3840
agaaagccat ccagtttact ttgcagggct tcccaacctt accagagggc gccccagctg 3900
gcaattccgg ttcgcttgct gtccataaaa ccgcccagtc tagctatcgc catgtaagcc 3960
cactgcaagc tacctgcttt ctctttgcgc ttgcgttttc ccttgtccag atagcccagt 4020
agctgacatt cacccgggaa aaaaaaaccc cgcccctgac agggcggggt ttttttcag 4080
ataaaaaaaa tccttagctt tcgctaagga tgatttctgc aattggcggc cgcttctaga 4140
atgcactagt agcggccgct gcagtccggc aaaaaaacgg gcaaggtgtc accaccctgc 4200
cctttttctt taaaaccgaa aagattactt cgcgttggag agcgttcacc gacaaacaac 4260
agataaaacg aaaggcccag tctttcgact gagcctttcg ttttatttga tgcctggtac 4320
cgggtgtaat ccccatactc agccttctaa tgctgagaga atgcctgaaa agaaccacta 4380
agtcaaatta tttggattaa gttttattaa gtcaacggag gtgtcagata gcatttagat 4440
cgtaaaagca tacggcatga acaaccatga acggcttccc agaggttaac cccaggttta 4500
tggtttacca aaatcccctg actttttccg agttggggga caaaatcaac taatttgggc 4560
gacgattttt tctgctatct ggcgatcgcc atgtttgtct aacttctgat ccaacctagg 4620
tttttggggg ctttccaaca aaaaatccca gttgccggcg aaaaattgct ggtggggcac 4680
aatttggtgc tctccgtagt tctgcaaacc tgccagtaaa acttgcgctt ccgcaaaacc 4740
atccctggtg agggaaataa tcgggacttc taactttaac gcttcagcaa aggtaccaaa 4800
accaggcttg gaaaataccc gtccgcaaag gggcattaaa tccaccgggc gactcgacca 4860
tatgggagag ctcccaacgc gttggatgca tagcttgagt attctatagt gtcacctaaa 4920
tagcttggcg taatcatggt catagctgtt tcctgtgtga aattgttatc cgctcacaat 4980
tccacacaac atacgagccg gaagcataaa gtgtaaagcc tggggtgcct aatgagtgag 5040
ctaactcaca ttaattgcgt tgcgctcact gcccgctttc cagtcgggaa acctgtcgtg 5100
ccagctgcat taatgaatcg gccaacgcgc ggggagaggc ggtttgcgta ttgggcgctc 5160
ttccgcttcc tcgctcactg actcgctgcg ctcggtcgtt cggctgcggc gagcggtatc 5220
agctcactca aaggcggtaa tacggttatc cacagaatca ggggataacg caggaaagaa 5280
catgtgagca aaaggccagc aaaaggccag gaaccgtaaa aaggccgcgt tgctggcgtt 5340
tttccatagg ctccgccccc ctgacgagca tcacaaaaat cgacgctcaa gtcagaggtg 5400
gcgaaacccg acaggactat aaagatacca ggcgtttccc cctggaagct ccctcgtgcg 5460
ctctcctgtt ccgaccctgc cgcttaccgg atacctgtcc gcctttctcc cttcgggaag 5520
cgtggcgctt tctcatagct cacgctgtag gtatctcagt tcggtgtagg tcgttcgctc 5580

caagctgggc tgtgtgcacg aaccccccgt tcagcccgac cgctgcgcct tatccggtaa 5640
ctatcgtctt gagtccaacc cggtaagaca cgacttatcg ccactggcag cagccactgg 5700
taacaggatt agcagagcga ggtatgtagg cggtgctaca gagttcttga agtggtggcc 5760
taactacggc tacactagaa gaacagtatt tggtatctgc gctctgctga agccagttac 5820
cttcggaaaa agagttggta gctcttgatc cggcaaacaa accaccgctg gtagcggtgg 5880
tttttttgtt tgcaagcagc agattacgcg cagaaaaaaa ggatctcaag aagatccttt 5940
gatcttttct acggggtctg acgctcagtg gaacgaaaac tcacgttaag ggattttggt 6000
catgagatta tcaaaaagga tcttcaccta gatcctttta aattaaaaat gaagttttaa 6060
atcaatctaa agtatatatg agtaaacttg gtctgacagt taccaatgct taatcagtga 6120
ggcacctatc tcagcgatct gtctatttcg ttcatccata gttgcctgac tccccgtcgt 6180
gtagataact acgatacggg agggcttacc atctggcccc agtgctgcaa tgataccgcg 6240
agacccacgc tcaccggctc cagatttatc agcaataaac cagccagccg gaagggccga 6300
gcgcagaagt ggtcctgcaa ctttatccgc ctccatccag tctattaatt gttgccggga 6360
agctagagta agtagttcgc cagttaatag tttgcgcaac gttgttgcca ttgctacagg 6420
catcgtggtg tcacgctcgt cgtttggtat ggcttcattc agctccggtt cccaacgatc 6480
aaggcgagtt acatgatccc ccatgttgtg caaaaaagcg gttagctcct tcggtcctcc 6540
gatcgttgtc agaagtaagt tggccgcagt gttatcactc atggttatgg cagcactgca 6600
taattctctt actgtcatgc catccgtaag atgcttttct gtgactggtg agtactcaac 6660
caagtcattc tgagaatagt gtatgcggcg accgagttgc tcttgcccgg cgtcaatacg 6720
ggataatacc gcgccacata gcagaacttt aaaagtgctc atcattggaa aacgttcttc 6780
ggggcgaaaa ctctcaagga tcttaccgct gttgagatcc agttcgatgt aacccactcg 6840
tgcacccaac tgatcttcag catcttttac tttcaccagc gtttctgggt gagcaaaaac 6900
aggaaggcaa aatgccgcaa aaaagggaat aagggcgaca cggaaatgtt gaatactcat 6960
actcttcctt tttcaatatt attgaagcat ttatcagggt tattgtctca tgagcggata 7020
catatttgaa tgtatttaga aaaataaaca aataggggtt ccgcgcacat ttccccgaaa 7080
agtgccacct gatgcggtgt gaaataccgc acagatgcgt aaggagaaaa taccgcatca 7140
ggaaattgta agcgttaata ttttgttaaa attcgcgtta aatttttgtt aaatcagctc 7200
atttttaac caataggccg aaatcggcaa aatcccttat aaatcaaaag aatagaccga 7260
gatagggttg agtgttgttc cagtttggaa caagagtcca ctattaaaga acgtggactc 7320
caacgtcaaa gggcgaaaaa ccgtctatca gggcgatggc ccactacgtg aaccatcacc 7380
ctaatcaagt tttttggggt cgaggtgccg taaagcacta aatcggaacc ctaaagggag 7440

cccccgattt agagcttgac ggggaaagcc ggcgaacgtg gcgagaaagg aagggaagaa 7500

agcgaaagga gcgggcgcta gggcgctggc aagtgtagcg gtcacgctgc gcgtaaccac 7560

ca 7562

<210> 14
<211> 7619
<212> DNA
<213> synthetic construct

<220>
<223> >SEQ ID NO 14

<400> 14

cacccgccgc gcttaatgcg ccgctacagg gcgcgtccat tcgccattca ggctgcgcaa 60
ctgttgggaa gggcgatcgg tgcgggcctc ttcgctatta cgccagctgg cgaaaggggg 120
atgtgctgca aggcgattaa gttgggtaac gccagggttt tcccagtcac gacgttgtaa 180
aacgacggcc agtgaattgt aatacgactc actatagggc gaattgggcc cgacgtcgca 240
tgctcccggc cgccatggcg gccgcgggaa ttcgattcta aacttacggc attggcatca 300
acgggagcca ccgtcggagt aggggaagta acaacggggg aactggtatt ggtgggcaaa 360
atttttacta gccattgatt ccattggggc agattgaacg ctcccaacca ccaaagtccc 420
cctaatacaa cgacagaact aaataacagg aaaaaaggaa tccaaggagt taccccctctt 480
ttatgggatg gaacttcatc gacattaaag gtgggagggg gaggaggcaa tggggacaat 540
ggtggtccag aaaaggaagg tggctccgac gtcaaggcaa cgggacatcc acaggattca 600
cagaaacgaa cctgggggct aaggcggttg ccacaattag tacaaaaaac gggagtagtc 660
ataggtgaaa accccgacta tagaattaga aaaatttaac tttttatccg aattttattc 720
gtccatgttc cccaaataac tatcaaaata attggaaaaa ttaaattatt tggtcgttgg 780
tcaccgctcc ctaaagacct ggccattgta aagagattac acccgggcaa gcggatggct 840
gatgaaacca agccaaccag gaagggcagc ccacctatca aggtgtactg ccttccagac 900
gaacgaagag cgattgagga aaaggcggcg gcggccggca tgagcctgtc ggcctacctg 960
ctggccgtcg gccagggcta caaaatcacg ggcgtcgtgg actatgagca cgtccgcgag 1020
ggcgtcccgg aaaacgattc cgaagcccaa cctttcatag aaggcggcgg tggaatcgaa 1080
atctcgtgat ggcaggttgg gcgtcgcttg gtcggtcatt tcgctcggta ccatcggcat 1140
tttcttttgc gtttttattt gttaactgtt aattgtcctt gttcaaggat gctgtctttg 1200
acaacagatg ttttcttgcc tttgatgttc agcaggaagc tcggcgcaaa cgttgattgt 1260
ttgtctgcgt agaatcctct gtttgtcata tagcttgtaa tcacgacatt gtttcctttc 1320
gcttgaggta cagcgaagtg tgagtaagta aaggttacat cgttaggatc aagatccatt 1380

tttaacacaa ggccagtttt gttcagcggc ttgtatgggc cagttaaaga attagaaaca 1440

taaccaagca tgtaaatatc gttagacgta atgccgtcaa tcgtcatttt tgatccgcgg 1500

gagtcagtga acaggtacca tttgccgttc attttaaaga cgttcgcgcg ttcaatttca 1560

tctgttactg tgttagatgc aatcagcggt ttcatcactt ttttcagtgt gtaatcatcg 1620

tttagctcaa tcataccgag agcgccgttt gctaactcag ccgtgcgttt tttatcgctt 1680

tgcagaagtt tttgactttc ttgacggaag aatgatgtgc ttttgccata gtatgctttg 1740

ttaaataaag attcttcgcc ttggtagcca tcttcagttc cagtgtttgc ttcaaatact 1800

aagtatttgt ggcctttatc ttctacgtag tgaggatctc tcagcgtatg gttgtcgcct 1860

gagctgtagt tgccttcatc gatgaactgc tgtacatttt gatacgtttt tccgtcaccg 1920

tcaaagattg atttataatc ctctacaccg ttgatgttca aagagctgtc tgatgctgat 1980

acgttaactt gtgcagttgt cagtgtttgt ttgccgtaat gtttaccgga gaaatcagtg 2040

tagaataaac ggatttttcc gtcagatgta aatgtggctg aacctgacca ttcttgtgtt 2100

tggtctttta ggatagaatc atttgcatcg aatttgtcgc tgtctttaaa gacgcggcca 2160

gcgtttttcc agctgtcaat agaagtttcg ccgacttttt gatagaacat gtaaatcgat 2220

gtgtcatccg catttttagg atctccggct aatgcaaaga cgatgtggta gccgtgatag 2280

tttgcgacag tgccgtcagc gttttgtaat ggccagctgt cccaaacgtc caggcctttt 2340

gcagaagaga tatttttaat tgtggacgaa tcaaattcag aaacttgata tttttcattt 2400

ttttgctgtt cagggatttg cagcatatca tggcgtgtaa tatgggaaat gccgtatgtt 2460

tccttatatg gcttttggtt cgtttctttc gcaaacgctt gagttgcgcc tcctgccagc 2520

agtgcggtag taaaggttaa tactgttgct tgttttgcaa actttttgat gttcatcgtt 2580

catgtctcct tttttatgta ctgtgttagc ggtctgcttc ttccagccct cctgtttgaa 2640

gatggcaagt tagttacgca caataaaaaa agacctaaaa tatgtaaggg gtgacgccaa 2700

agtatacact ttgcccttta cacattttag gtcttgcctg ctttatcagt aacaaacccg 2760

cgcgatttac ttttcgacct cattctatta gactctcgtt tggattgcaa ctggtctatt 2820

ttcctctttt gtttgataga aaatcataaa aggatttgca gactacgggc ctaaagaact 2880

aaaaaatcta tctgtttctt ttcattctct gtattttttta tagtttctgt tgcatgggca 2940

taaagttgcc tttttaatca caattcagaa aatatcataa tatctcattt cactaaataa 3000

tagtgaacgg caggtatatg tgatgggtta aaaaggatcg atcctctagc gaaccccaga 3060

gtcccgctca gaagaactcg tcaagaaggc gatagaaggc gatgcgctgc gaatcgggag 3120

cggcgatacc gtaaagcacg aggaagcggt cagcccattc gccgccaagc tcttcagcaa 3180

tatcacgggt agccaacgct atgtcctgat agcggtccgc cacacccagc cggccacagt 3240

cgatgaatcc agaaaagcgg ccattttcca ccatgatatt cggcaagcag gcatcgccat 3300

gggtcacgac gagatcctcg ccgtcgggca tccgcgcctt gagcctggcg aacagttcgg 3360
ctggcgcgag cccctgatgc tcttcgtcca gatcatcctg atcgacaaga ccggcttcca 3420
tccgagtacg tgctcgctcg atgcgatgtt tcgcttggtg gtcgaatggg caggtagccg 3480
gatcaagcgt atgcagccgc cgcattgcat cagccatgat ggatactttc tcggcaggag 3540
caaggtgaga tgacaggaga tcctgccccg gcacttcgcc caatagcagc cagtcccttc 3600
ccgcttcagt gacaacgtcg agcacagctg cgcaaggaac gcccgtcgtg gccagccacg 3660
atagccgcgc tgcctcgtct tggagttcat tcagggcacc ggacaggtcg gtcttgacaa 3720
aaagaaccgg gcgcccctgc gctgacagcc ggaacacggc ggcatcagag cagccgattg 3780
tctgttgtgc ccagtcatag ccgaatagcc tctccaccca agcggccgga gaacctgcgt 3840
gcaatccatc ttgttcaatc atgcgaaacg atcctcatcc tgtctcttga tcagatcttg 3900
atcccctgcg ccatcagatc cttggcggca agaaagccat ccagtttact ttgcagggct 3960
tcccaacctt accagagggc gccccagctg gcaattccgg ttcgcttgct gtccataaaa 4020
ccgcccagtc tagctatcgc catgtaagcc cactgcaagc tacctgcttt ctctttgcgc 4080
ttgcgttttc ccttgtccag atagcccagt agctgacatt cacccgggaa aaaaaaaccc 4140
cgcccctgac agggcggggt ttttttttcag ataaaaaaaa tccttagctt tcgctaagga 4200
tgatttctgc aattggcggc cgcttctaga atgcactagt agcggccgct gcagtccggc 4260
aaaaaaacgg gcaaggtgtc accaccctgc cctttttctt taaaaccgaa aagattactt 4320
cgcgttggag agcgttcacc gacaaacaac agataaaacg aaaggcccag tctttcgact 4380
gagcctttcg ttttatttga tgcctggtac cgggtgtaat ccattgggca cgagagttag 4440
taaggcagtg gcaattaata gaggcttatg gttgattcgc attgttttgc tcctgaaatt 4500
ttcggcaaat acaaatactt cgctcttcta gccctattaa ccattttaac gacaaattga 4560
tggggcaacg attaacaaat aatgaataaa ttttatgttt ttcaagatga aaatttgaaa 4620
atttgatttc cttatatttc tactatagaa gactaataca attagatcta aaatttgcaa 4680
gtataaaaat cagcaaatag ttatattgtt aataattcaa tgacccaata actcgtactg 4740
ttatctacgt ggtgaaagcc aaaaagacga acagtttagc ctcctcctcc tcggcgatcg 4800
ccaagcgaaa tgtcatggga gatgttcaga ttgagcattt ttttctaaaa gcccttgcta 4860
aaacaaacca catgtgcagg gtgtccccga tgttgactaa attcagcggc tcgaccatat 4920
gggagagctc ccaacgcgtt ggatgcatag cttgagtatt ctatagtgtc acctaaatag 4980
cttggcgtaa tcatggtcat agctgtttcc tgtgtgaaat tgttatccgc tcacaattcc 5040
acacaacata cgagccggaa gcataaagtg taaagcctgg ggtgcctaat gagtgagcta 5100
actcacatta attgcgttgc gctcactgcc cgctttccag tcgggaaacc tgtcgtgcca 5160

gctgcattaa tgaatcggcc aacgcgcggg gagaggcggt ttgcgtattg ggcgctcttc 5220
cgcttcctcg ctcactgact cgctgcgctc ggtcgttcgg ctgcggcgag cggtatcagc 5280
tcactcaaag gcggtaatac ggttatccac agaatcaggg gataacgcag gaaagaacat 5340
gtgagcaaaa ggccagcaaa aggccaggaa ccgtaaaaag gccgcgttgc tggcgttttt 5400
ccataggctc cgcccccctg acgagcatca caaaaatcga cgctcaagtc agaggtggcg 5460
aaacccgaca ggactataaa gataccaggc gtttccccct ggaagctccc tcgtgcgctc 5520
tcctgttccg accctgccgc ttaccggata cctgtccgcc tttctccctt cgggaagcgt 5580
ggcgctttct catagctcac gctgtaggta tctcagttcg gtgtaggtcg ttcgctccaa 5640
gctgggctgt gtgcacgaac cccccgttca gcccgaccgc tgcgccttat ccggtaacta 5700
tcgtcttgag tccaacccgg taagacacga cttatcgcca ctggcagcag ccactggtaa 5760
caggattagc agagcgaggt atgtaggcgg tgctacagag ttcttgaagt ggtggcctaa 5820
ctacggctac actagaagaa cagtatttgg tatctgcgct ctgctgaagc cagttacctt 5880
cggaaaaaga gttggtagct cttgatccgg caaacaaacc accgctggta gcggtggttt 5940
ttttgtttgc aagcagcaga ttacgcgcag aaaaaaagga tctcaagaag atcctttgat 6000
cttttctacg gggtctgacg ctcagtggaa cgaaaactca cgttaaggga ttttggtcat 6060
gagattatca aaaaggatct tcacctagat ccttttaaat taaaaatgaa gttttaaatc 6120
aatctaaagt atatatgagt aaacttggtc tgacagttac caatgcttaa tcagtgaggc 6180
acctatctca gcgatctgtc tatttcgttc atccatagtt gcctgactcc ccgtcgtgta 6240
gataactacg atacgggagg gcttaccatc tggccccagt gctgcaatga taccgcgaga 6300
cccacgctca ccggctccag atttatcagc aataaaccag ccagccggaa gggccgagcg 6360
cagaagtggt cctgcaactt tatccgcctc catccagtct attaattgtt gccgggaagc 6420
tagagtaagt agttcgccag ttaatagttt gcgcaacgtt gttgccattg ctacaggcat 6480
cgtggtgtca cgctcgtcgt ttggtatggc ttcattcagc tccggttccc aacgatcaag 6540
gcgagttaca tgatccccca tgttgtgcaa aaaagcggtt agctccttcg gtcctccgat 6600
cgttgtcaga agtaagttgg ccgcagtgtt atcactcatg gttatggcag cactgcataa 6660
ttctcttact gtcatgccat ccgtaagatg cttttctgtg actggtgagt actcaaccaa 6720
gtcattctga gaatagtgta tgcggcgacc gagttgctct tgcccggcgt caatacggga 6780
taataccgcg ccacatagca gaactttaaa agtgctcatc attggaaaac gttcttcggg 6840
gcgaaaactc tcaaggatct taccgctgtt gagatccagt tcgatgtaac ccactcgtgc 6900
acccaactga tcttcagcat cttttacttt caccagcgtt tctgggtgag caaaaacagg 6960
aaggcaaaat gccgcaaaaa agggaataag ggcgacacgg aaatgttgaa tactcatact 7020
cttccttttt caatattatt gaagcattta tcagggttat tgtctcatga gcggatacat 7080

atttgaatgt atttagaaaa ataaacaaat aggggttccg cgcacatttc cccgaaaagt 7140

gccacctgat gcggtgtgaa ataccgcaca gatgcgtaag gagaaaatac cgcatcagga 7200

aattgtaagc gttaatattt tgttaaaatt cgcgttaaat ttttgttaaa tcagctcatt 7260

ttttaaccaa taggccgaaa tcggcaaaat cccttataaa tcaaaagaat agaccgagat 7320

agggttgagt gttgttccag tttggaacaa gagtccacta ttaaagaacg tggactccaa 7380

cgtcaaaggg cgaaaaaccg tctatcaggg cgatggccca ctacgtgaac catcacccta 7440

atcaagtttt ttggggtcga ggtgccgtaa agcactaaat cggaacccta aagggagccc 7500

ccgatttaga gcttgacggg gaaagccggc gaacgtggcg agaaaggaag ggaagaaagc 7560

gaaaggagcg ggcgctaggg cgctggcaag tgtagcggtc acgctgcgcg taaccacca 7619

<210> 15
<211> 7634
<212> DNA
<213> synthetic construct

<220>
<223> >SEQ ID NO 15

<400> 15

```
cacccgccgc gcttaatgcg ccgctacagg gcgcgtccat tcgccattca ggctgcgcaa      60
ctgttgggaa gggcgatcgg tgcgggcctc ttcgctatta cgccagctgg cgaaaggggg     120
atgtgctgca aggcgattaa gttgggtaac gccagggttt tcccagtcac gacgttgtaa     180
aacgacggcc agtgaattgt aatacgactc actatagggc gaattgggcc cgacgtcgca     240
tgctcccggc cgccatggcg gccgcgggaa ttcgattgtg agcttgatgg tgatggtggg     300
taaagcttca ttgtccatgc gccggagctt gacggtaaaa tccgtgtcct ggggccaatc     360
attgggaacg ctcacacaga taccgttgcg agttaagtcg aaggataccc ccaccaattc     420
cccttccata tcgaacactt ggcaaaaggc caacatccgc tcttcctgcc gtcgctcctg     480
cacccccctgg agatgggcaa tggttagggc ttcttgggag agggaagatg gagaatcttg     540
agccatggag attatttcct cggttaaatt aggtttacct agtaaatggg ccaggttgac     600
cattattgta gtcattcact gggtcgatct tccttccatc agggtcattt ggttaattgt     660
tgatgagaaa tgggaaggag taatccatag atatttgccc agttaactcg atttgagcag     720
aattgggaag gacgtattgg gaactttggt tgacagggca acaaagccag cagattacac     780
ccgggcaagc ggatggctga tgaaaccaag ccaaccagga agggcagccc acctatcaag     840
gtgtactgcc ttccagacga acgaagagcg attgaggaaa aggcggcggc ggccggcatg     900
agcctgtcgg cctacctgct ggccgtcggc cagggctaca aaatcacggg cgtcgtggac     960
tatgagcacg tccgcgaggg cgtcccggaa aacgattccg aagcccaacc tttcatagaa    1020
```

ggcggcggtg gaatcgaaat ctcgtgatgg caggttgggc gtcgcttggt cggtcatttc 1080
gctcggtacc atcggcattt tcttttgcgt ttttatttgt taactgttaa ttgtccttgt 1140
tcaaggatgc tgtctttgac aacagatgtt ttcttgcctt tgatgttcag caggaagctc 1200
ggcgcaaacg ttgattgttt gtctgcgtag aatcctctgt ttgtcatata gcttgtaatc 1260
acgacattgt ttcctttcgc ttgaggtaca gcgaagtgtg agtaagtaaa ggttacatcg 1320
ttaggatcaa gatccatttt taacacaagg ccagttttgt tcagcggctt gtatgggcca 1380
gttaaagaat tagaaacata accaagcatg taaatatcgt tagacgtaat gccgtcaatc 1440
gtcatttttg atccgcggga gtcagtgaac aggtaccatt tgccgttcat tttaaagacg 1500
ttcgcgcgtt caatttcatc tgttactgtg ttagatgcaa tcagcggttt catcactttt 1560
ttcagtgtgt aatcatcgtt tagctcaatc ataccgagag cgccgtttgc taactcagcc 1620
gtgcgttttt tatcgctttg cagaagtttt tgactttctt gacggaagaa tgatgtgctt 1680
ttgccatagt atgctttgtt aaataaagat tcttcgcctt ggtagccatc ttcagttcca 1740
gtgtttgctt caaatactaa gtatttgtgg cctttatctt ctacgtagtg aggatctctc 1800
agcgtatggt tgtcgcctga gctgtagttg ccttcatcga tgaactgctg tacattttga 1860
tacgtttttc cgtcaccgtc aaagattgat ttataatcct ctacaccgtt gatgttcaaa 1920
gagctgtctg atgctgatac gttaacttgt gcagttgtca gtgtttgttt gccgtaatgt 1980
ttaccggaga aatcagtgta gaataaacgg atttttccgt cagatgtaaa tgtggctgaa 2040
cctgaccatt cttgtgtttg gtcttttagg atagaatcat ttgcatcgaa tttgtcgctg 2100
tctttaaaga cgcggccagc gtttttccag ctgtcaatag aagtttcgcc gactttttga 2160
tagaacatgt aaatcgatgt gtcatccgca tttttaggat ctccggctaa tgcaaagacg 2220
atgtggtagc cgtgatagtt tgcgacagtg ccgtcagcgt tttgtaatgg ccagctgtcc 2280
caaacgtcca ggccttttgc agaagagata tttttaattg tggacgaatc aaattcagaa 2340
acttgatatt tttcattttt ttgctgttca gggatttgca gcatatcatg gcgtgtaata 2400
tgggaaatgc cgtatgtttc cttatatggc ttttggttcg tttctttcgc aaacgcttga 2460
gttgcgcctc ctgccagcag tgcggtagta aaggttaata ctgttgcttg ttttgcaaac 2520
tttttgatgt tcatcgttca tgtctccttt tttatgtact gtgttagcgg tctgcttctt 2580
ccagccctcc tgtttgaaga tggcaagtta gttacgcaca ataaaaaaag acctaaaata 2640
tgtaaggggt gacgccaaag tatacacttt gccctttaca cattttaggt cttgcctgct 2700
ttatcagtaa caaacccgcg cgatttactt ttcgacctca ttctattaga ctctcgtttg 2760
gattgcaact ggtctatttt cctcttttgt ttgatagaaa atcataaaag gatttgcaga 2820
ctacgggcct aaagaactaa aaaatctatc tgtttctttt cattctctgt atttttata 2880

gtttctgttg catgggcata aagttgcctt tttaatcaca attcagaaaa tatcataata 2940

tctcatttca ctaaataata gtgaacggca ggtatatgtg atgggttaaa aaggatcgat 3000

cctctagcga accccagagt cccgctcaga agaactcgtc aagaaggcga tagaaggcga 3060

tgcgctgcga atcgggagcg gcgataccgt aaagcacgag gaagcggtca gcccattcgc 3120

cgccaagctc ttcagcaata tcacgggtag ccaacgctat gtcctgatag cggtccgcca 3180

cacccagccg gccacagtcg atgaatccag aaaagcggcc attttccacc atgatattcg 3240

gcaagcaggc atcgccatgg gtcacgacga gatcctcgcc gtcgggcatc cgcgccttga 3300

gcctggcgaa cagttcggct ggcgcgagcc cctgatgctc ttcgtccaga tcatcctgat 3360

cgacaagacc ggcttccatc cgagtacgtg ctcgctcgat gcgatgtttc gcttggtggt 3420

cgaatgggca ggtagccgga tcaagcgtat gcagccgccg cattgcatca gccatgatgg 3480

atactttctc ggcaggagca aggtgagatg acaggagatc ctgccccggc acttcgccca 3540

atagcagcca gtcccttccc gcttcagtga caacgtcgag cacagctgcg caaggaacgc 3600

ccgtcgtggc cagccacgat agccgcgctg cctcgtcttg gagttcattc agggcaccgg 3660

acaggtcggt cttgacaaaa agaaccgggc gcccctgcgc tgacagccgg aacacggcgg 3720

catcagagca gccgattgtc tgttgtgccc agtcatagcc gaatagcctc tccacccaag 3780

cggccggaga acctgcgtgc aatccatctt gttcaatcat gcgaaacgat cctcatcctg 3840

tctcttgatc agatcttgat cccctgcgcc atcagatcct tggcggcaag aaagccatcc 3900

agtttacttt gcagggcttc ccaaccttac cagagggcgc cccagctggc aattccggtt 3960

cgcttgctgt ccataaaacc gcccagtcta gctatcgcca tgtaagccca ctgcaagcta 4020

cctgctttct ctttgcgctt gcgttttccc ttgtccagat agcccagtag ctgacattca 4080

cccgggaaaa aaaaaccccg cccctgacag ggcggggttt tttttcagat aaaaaaaatc 4140

cttagctttc gctaaggatg atttctgcaa ttggcggccg cttctagaat gcactagtag 4200

cggccgctgc agtccggcaa aaaaacgggc aaggtgtcac caccctgccc tttttcttta 4260

aaaccgaaaa gattacttcg cgttggagag cgttcaccga caaacaacag ataaaacgaa 4320

aggcccagtc tttcgactga gcctttcgtt ttatttgatg cctggtaccg ggtgtaatcg 4380

ctggaggcga actgggtgag aaccataaaa tcttggggaa caggggaatg ttaatgaaca 4440

catgacatgc ataggatagc gaattgattt aaagcaatga ttcccccctga gataaatata 4500

ttttgaccca ctgctccgct taattttggc gctaatctgg gcaaaaattc gttggttttc 4560

ggaaaggtga acccgtagct agcttgtatt gcccaaacct aactcatcat tgctccatgg 4620

cggccaaaaa tctttaggac aaaactcgcc agaggtcaag gcttgatttt ttcagaggga 4680

aaaattcctc ggctaaataa tcaaaaagtt gaaaaaaaaa gtttttcagc taaggtttga 4740

tgttatattt tatcttatgt agacttttga aaaaaacaag tccccgcaat caagcatcat 4800

tcaacggaaa aaataatcct atgccaaacg cctccaccgc actaaccggc aaagcattac 4860

tcaataaagt taaagaacta tcccatctgc cccgtcgaga aacggcaaaa gcctgtggtt 4920

actactcgac catatgggag agctcccaac gcgttggatg catagcttga gtattctata 4980

gtgtcaccta aatagcttgg cgtaatcatg gtcatagctg tttcctgtgt gaaattgtta 5040

tccgctcaca attccacaca acatacgagc cggaagcata aagtgtaaag cctggggtgc 5100

ctaatgagtg agctaactca cattaattgc gttgcgctca ctgcccgctt tccagtcggg 5160

aaacctgtcg tgccagctgc attaatgaat cggccaacgc gcggggagag gcggtttgcg 5220

tattgggcgc tcttccgctt cctcgctcac tgactcgctg cgctcggtcg ttcggctgcg 5280

gcgagcggta tcagctcact caaaggcggt aatacggtta tccacagaat caggggataa 5340

cgcaggaaag aacatgtgag caaaaggcca gcaaaaggcc aggaaccgta aaaaggccgc 5400

gttgctggcg tttttccata ggctccgccc ccctgacgag catcacaaaa atcgacgctc 5460

aagtcagagg tggcgaaacc cgacaggact ataaagatac caggcgtttc cccctggaag 5520

ctccctcgtg cgctctcctg ttccgaccct gccgcttacc ggatacctgt ccgcctttct 5580

cccttcggga agcgtggcgc tttctcatag ctcacgctgt aggtatctca gttcggtgta 5640

ggtcgttcgc tccaagctgg gctgtgtgca cgaacccccc gttcagcccg accgctgcgc 5700

cttatccggt aactatcgtc ttgagtccaa cccggtaaga cacgacttat cgccactggc 5760

agcagccact ggtaacagga ttagcagagc gaggtatgta ggcggtgcta cagagttctt 5820

gaagtggtgg cctaactacg gctacactag aagaacagta tttggtatct gcgctctgct 5880

gaagccagtt accttcggaa aaagagttgg tagctcttga tccggcaaac aaaccaccgc 5940

tggtagcggt ggtttttttg tttgcaagca gcagattacg cgcagaaaaa aaggatctca 6000

agaagatcct ttgatctttt ctacggggtc tgacgctcag tggaacgaaa actcacgtta 6060

agggattttg gtcatgagat tatcaaaaag gatcttcacc tagatccttt taaattaaaa 6120

atgaagtttt aaatcaatct aaagtatata tgagtaaact tggtctgaca gttaccaatg 6180

cttaatcagt gaggcaccta tctcagcgat ctgtctattt cgttcatcca tagttgcctg 6240

actccccgtc gtgtagataa ctacgatacg ggagggctta ccatctggcc ccagtgctgc 6300

aatgataccg cgagacccac gctcaccggc tccagattta tcagcaataa accagccagc 6360

cggaagggcc gagcgcagaa gtggtcctgc aactttatcc gcctccatcc agtctattaa 6420

ttgttgccgg gaagctagag taagtagttc gccagttaat agtttgcgca acgttgttgc 6480

cattgctaca ggcatcgtgg tgtcacgctc gtcgtttggt atggcttcat tcagctccgg 6540

ttcccaacga tcaaggcgag ttacatgatc ccccatgttg tgcaaaaaag cggttagctc 6600

cttcggtcct ccgatcgttg tcagaagtaa gttggccgca gtgttatcac tcatggttat 6660

ggcagcactg cataattctc ttactgtcat gccatccgta agatgctttt ctgtgactgg 6720
tgagtactca accaagtcat tctgagaata gtgtatgcgg cgaccgagtt gctcttgccc 6780
ggcgtcaata cgggataata ccgcgccaca tagcagaact ttaaaagtgc tcatcattgg 6840
aaaacgttct tcggggcgaa aactctcaag gatcttaccg ctgttgagat ccagttcgat 6900
gtaacccact cgtgcaccca actgatcttc agcatctttt actttcacca gcgtttctgg 6960
gtgagcaaaa acaggaaggc aaaatgccgc aaaaaaggga ataagggcga cacggaaatg 7020
ttgaatactc atactcttcc tttttcaata ttattgaagc atttatcagg gttattgtct 7080
catgagcgga tacatatttg aatgtattta gaaaaataaa caaatagggg ttccgcgcac 7140
atttccccga aaagtgccac ctgatgcggt gtgaaatacc gcacagatgc gtaaggagaa 7200
aataccgcat caggaaattg taagcgttaa tattttgtta aaattcgcgt taaatttttg 7260
ttaaatcagc tcatttttta accaataggc cgaaatcggc aaaatccctt ataaatcaaa 7320
agaatagacc gagatagggt tgagtgttgt tccagtttgg aacaagagtc cactattaaa 7380
gaacgtggac tccaacgtca aagggcgaaa aaccgtctat cagggcgatg gcccactacg 7440
tgaaccatca ccctaatcaa gtttttggg gtcgaggtgc cgtaaagcac taaatcggaa 7500
ccctaaaggg agcccccgat ttagagcttg acggggaaag ccggcgaacg tggcgagaaa 7560
ggaagggaag aaagcgaaag gagcgggcgc tagggcgctg gcaagtgtag cggtcacgct 7620
gcgcgtaacc acca 7634

<210> 16
<211> 3017
<212> DNA
<213> synthetic construct

<220>
<223> >SEQ ID NO 16

<400> 16

```
aatcactagt gaattcgcgg ccgcctgcag gtcgaccata tgggagagct cccaacgcgt      60
tggatgcata gcttgagtat tctatagtgt cacctaaata gcttggcgta atcatggtca     120
tagctgtttc ctgtgtgaaa ttgttatccg ctcacaattc cacacaacat acgagccgga     180
agcataaagt gtaaagcctg gggtgcctaa tgagtgagct aactcacatt aattgcgttg     240
cgctcactgc ccgctttcca gtcgggaaac ctgtcgtgcc agctgcatta atgaatcggc     300
caacgcgcgg ggagaggcgg tttgcgtatt gggcgctctt ccgcttcctc gctcactgac     360
tcgctgcgct cggtcgttcg gctgcggcga gcggtatcag ctcactcaaa ggcggtaata     420
cggttatcca cagaatcagg ggataacgca ggaaagaaca tgtgagcaaa aggccagcaa     480
aaggccagga accgtaaaaa ggccgcgttg ctggcgtttt tccataggct ccgcccccct     540
```

gacgagcatc acaaaaatcg acgctcaagt cagaggtggc gaaacccgac aggactataa 600

agataccagg cgtttccccc tggaagctcc ctcgtgcgct ctcctgttcc gaccctgccg 660

cttaccggat acctgtccgc ctttctccct tcgggaagcg tggcgctttc tcatagctca 720

cgctgtaggt atctcagttc ggtgtaggtc gttcgctcca agctgggctg tgtgcacgaa 780

ccccccgttc agcccgaccg ctgcgcctta tccggtaact atcgtcttga gtccaacccg 840

gtaagacacg acttatcgcc actggcagca gccactggta acaggattag cagagcgagg 900

tatgtaggcg gtgctacaga gttcttgaag tggtggccta actacggcta cactagaaga 960

acagtatttg gtatctgcgc tctgctgaag ccagttacct tcggaaaaag agttggtagc 1020

tcttgatccg gcaaacaaac caccgctggt agcggtggtt tttttgtttg caagcagcag 1080

attacgcgca gaaaaaaagg atctcaagaa gatcctttga tcttttctac ggggtctgac 1140

gctcagtgga acgaaaactc acgttaaggg attttggtca tgagattatc aaaaaggatc 1200

ttcacctaga tccttttaaa ttaaaaatga agttttaaat caatctaaag tatatatgag 1260

taaacttggt ctgacagtta ccaatgctta atcagtgagg cacctatctc agcgatctgt 1320

ctatttcgtt catccatagt tgcctgactc cccgtcgtgt agataactac gatacgggag 1380

ggcttaccat ctggccccag tgctgcaatg ataccgcgag acccacgctc accggctcca 1440

gatttatcag caataaacca gccagccgga agggccgagc gcagaagtgg tcctgcaact 1500

ttatccgcct ccatccagtc tattaattgt tgccgggaag ctagagtaag tagttcgcca 1560

gttaatagtt tgcgcaacgt tgttgccatt gctacaggca tcgtggtgtc acgctcgtcg 1620

tttggtatgg cttcattcag ctccggttcc caacgatcaa ggcgagttac atgatccccc 1680

atgttgtgca aaaaagcggt tagctccttc ggtcctccga tcgttgtcag aagtaagttg 1740

gccgcagtgt tatcactcat ggttatggca gcactgcata attctcttac tgtcatgcca 1800

tccgtaagat gcttttctgt gactggtgag tactcaacca agtcattctg agaatagtgt 1860

atgcggcgac cgagttgctc ttgcccggcg tcaatacggg ataataccgc gccacatagc 1920

agaactttaa aagtgctcat cattggaaaa cgttcttcgg ggcgaaaact ctcaaggatc 1980

ttaccgctgt tgagatccag ttcgatgtaa cccactcgtg cacccaactg atcttcagca 2040

tcttttactt tcaccagcgt ttctgggtga gcaaaaacag gaaggcaaaa tgccgcaaaa 2100

aagggaataa gggcgacacg gaaatgttga atactcatac tcttcctttt tcaatattat 2160

tgaagcattt atcagggtta ttgtctcatg agcggataca tatttgaatg tatttagaaa 2220

aataaacaaa taggggttcc gcgcacattt ccccgaaaag tgccacctga tgcggtgtga 2280

aataccgcac agatgcgtaa ggagaaaata ccgcatcagg aaattgtaag cgttaatatt 2340

ttgttaaaat tcgcgttaaa tttttgttaa atcagctcat tttttaacca ataggccgaa 2400

atcggcaaaa tcccttataa atcaaaagaa tagaccgaga tagggttgag tgttgttcca 2460

gtttggaaca agagtccact attaaagaac gtggactcca acgtcaaagg gcgaaaaacc 2520

gtctatcagg gcgatggccc actacgtgaa ccatcaccct aatcaagttt tttggggtcg 2580

aggtgccgta aagcactaaa tcggaaccct aaagggagcc cccgatttag agcttgacgg 2640

ggaaagccgg cgaacgtggc gagaaaggaa gggaagaaag cgaaaggagc gggcgctagg 2700

gcgctggcaa gtgtagcggt cacgctgcgc gtaaccacca cacccgccgc gcttaatgcg 2760

ccgctacagg gcgcgtccat tcgccattca ggctgcgcaa ctgttgggaa gggcgatcgg 2820

tgcgggcctc ttcgctatta cgccagctgg cgaaaggggg atgtgctgca aggcgattaa 2880

gttgggtaac gccagggttt tcccagtcac gacgttgtaa aacgacggcc agtgaattgt 2940

aatacgactc actatagggc gaattgggcc cgacgtcgca tgctcccggc cgccatggcg 3000

gccgcgggaa ttcgatt 3017

<210> 17
<211> 1372
<212> DNA
<213> Streptomyces kanamyceticus

<220>
<223> >SEQ ID NO 17

<400> 17

cccgggtgaa tgtcagctac tgggctatct ggacaaggga aaacgcaagc gcaaagagaa 60
agcaggtagc ttgcagtggg cttacatggc gatagctaga ctgggcggtt ttatggacag 120
caagcgaacc ggaattgcca gctggggcgc cctctggtaa ggttgggaag ccctgcaaag 180
taaactggat ggctttcttg ccgccaagga tctgatggcg caggggatca agatctgatc 240
aagagacagg atgaggatcg tttcgcatga ttgaacaaga tggattgcac gcaggttctc 300
cggccgcttg ggtggagagg ctattcggct atgactgggc acaacagaca atcggctgct 360
ctgatgccgc cgtgttccgg ctgtcagcgc aggggcgccc ggttcttttt gtcaagaccg 420
acctgtccgg tgccctgaat gaactccaag acgaggcagc gcggctatcg tggctggcca 480
cgacgggcgt tccttgcgca gctgtgctcg acgttgtcac tgaagcggga agggactggc 540
tgctattggg cgaagtgccg gggcaggatc tcctgtcatc tcaccttgct cctgccgaga 600
aagtatccat catggctgat gcaatgcggc ggctgcatac gcttgatccg gctacctgcc 660
cattcgacca ccaagcgaaa catcgcatcg agcgagcacg tactcggatg gaagccggtc 720
ttgtcgatca ggatgatctg gacgaagagc atcaggggct cgcgccagcc gaactgttcg 780
ccaggctcaa ggcgcggatg cccgacggcg aggatctcgt cgtgacccat ggcgatgcct 840
gcttgccgaa tatcatggtg gaaaatggcc gcttttctgg attcatcgac tgtggccggc 900
tgggtgtggc ggaccgctat caggacatag cgttggctac ccgtgatatt gctgaagagc 960

ttggcggcga atgggctgac cgcttcctcg tgctttacgg tatcgccgct cccgattcgc 1020
agcgcatcgc cttctatcgc cttcttgacg agttcttctg agcgggactc tggggttcgc 1080
tagaggatcg atccttttta acccatcaca tatacctgcc gttcactatt atttagtgaa 1140
atgagatatt atgatatttt ctgaattgtg attaaaaagg caactttatg cccatgcaac 1200
agaaactata aaaaatacag agaatgaaaa gaaacagata gatttttag ttctttaggc 1260
ccgtagtctg caaatccttt tatgattttc tatcaaacaa aagaggaaaa tagaccagtt 1320
gcaatccaaa cgagagtcta atagaatgag gtcgaaaagt aaatcgcccg gg 1372

<210> 18
<211> 3301
<212> DNA
<213> synthetic construct

<220>
<223> >SEQ ID NO 18

<400> 18

```
ccgggtgaat gtcagctact gggctatctg gacaagggaa aacgcaagcg caaagagaaa      60
gcaggtagct tgcagtgggc ttacatggcg atagctagac tgggcggttt tatggacagc     120
aagcgaaccg gaattgccag ctggggcgcc ctctggtaag gttgggaagc cctgcaaagt     180
aaactggatg gctttcttgc cgccaaggat ctgatggcgc aggggatcaa gatctgatca     240
agagacagga tgaggatcgt ttcgcatgat tgaacaagat ggattgcacg caggttctcc     300
ggccgcttgg gtggagaggc tattcggcta tgactgggca caacagacaa tcggctgctc     360
tgatgccgcc gtgttccggc tgtcagcgca ggggcgcccg gttctttttg tcaagaccga     420
cctgtccggt gccctgaatg aactccaaga cgaggcagcg cggctatcgt ggctggccac     480
gacgggcgtt ccttgcgcag ctgtgctcga cgttgtcact gaagcgggaa gggactggct     540
gctattgggc gaagtgccgg ggcaggatct cctgtcatct caccttgctc ctgccgagaa     600
agtatccatc atggctgatg caatgcggcg gctgcatacg cttgatccgg ctacctgccc     660
attcgaccac caagcgaaac atcgcatcga gcgagcacgt actcggatgg aagccggtct     720
tgtcgatcag gatgatctgg acgaagagca tcaggggctc gcgccagccg aactgttcgc     780
caggctcaag gcgcggatgc ccgacggcga ggatctcgtc gtgacccatg gcgatgcctg     840
cttgccgaat atcatggtgg aaaatggccg cttttctgga ttcatcgact gtggccggct     900
gggtgtggcg gaccgctatc aggacatagc gttggctacc cgtgatattg ctgaagagct     960
tggcggcgaa tgggctgacc gcttcctcgt gctttacggt atcgccgctc ccgattcgca    1020
gcgcatcgcc ttctatcgcc ttcttgacga gttcttctga gcgggactct ggggttcgct    1080
agaggatcga tcctttttaa cccatcacat atacctgccg ttcactatta tttagtgaaa    1140
```

tgagatatta tgatattttc tgaattgtga ttaaaaaggc aactttatgc ccatgcaaca 1200
gaaactataa aaaatacaga gaatgaaaag aaacagatag atttttagt tctttaggcc 1260
cgtagtctgc aaatcctttt atgattttct atcaaacaaa agaggaaaat agaccagttg 1320
caatccaaac gagagtctaa tagaatgagg tcgaaaagta aatcgcgcgg gtttgttact 1380
gataaagcag gcaagaccta aaatgtgtaa agggcaaagt gtatactttg gcgtcacccc 1440
ttacatattt taggtctttt tttattgtgc gtaactaact tgccatcttc aaacaggagg 1500
gctggaagaa gcagaccgct aacacagtac ataaaaaagg agacatgaac gatgaacatc 1560
aaaaagtttg caaaacaagc aacagtatta acctttacta ccgcactgct ggcaggaggc 1620
gcaactcaag cgtttgcgaa agaaacgaac caaaagccat ataaggaaac atacggcatt 1680
tcccatatta cacgccatga tatgctgcaa atccctgaac agcaaaaaaa tgaaaaatat 1740
caagtttctg aatttgattc gtccacaatt aaaaatatct cttctgcaaa aggcctggac 1800
gtttgggaca gctggccatt acaaaacgct gacggcactg tcgcaaacta tcacggctac 1860
cacatcgtct ttgcattagc cggagatcct aaaaatgcgg atgacacatc gatttacatg 1920
ttctatcaaa aagtcggcga aacttctatt gacagctgga aaaacgctgg ccgcgtcttt 1980
aaagacagcg acaaattcga tgcaaatgat tctatcctaa aagaccaaac acaagaatgg 2040
tcaggttcag ccacatttac atctgacgga aaaatccgtt tattctacac tgatttctcc 2100
ggtaaacatt acggcaaaca aacactgaca actgcacaag ttaacgtatc agcatcagac 2160
agctctttga acatcaacgg tgtagaggat tataaatcaa tctttgacgg tgacggaaaa 2220
acgtatcaaa atgtacagca gttcatcgat gaaggcaact acagctcagg cgacaaccat 2280
acgctgagag atcctcacta cgtagaagat aaaggccaca aatacttagt atttgaagca 2340
aacactggaa ctgaagatgg ctaccaaggc gaagaatctt tatttaacaa agcatactat 2400
ggcaaaagca catcattctt ccgtcaagaa agtcaaaaac ttctgcaaag cgataaaaaa 2460
cgcacggctg agttagcaaa cggcgctctc ggtatgattg agctaaacga tgattacaca 2520
ctgaaaaaag tgatgaaacc gctgattgca tctaacacag taacagatga aattgaacgc 2580
gcgaacgtct ttaaaatgaa cggcaaatgg tacctgttca ctgactcccg cggatcaaaa 2640
atgacgattg acggcattac gtctaacgat atttacatgc ttggttatgt ttctaattct 2700
ttaactggcc catacaagcc gctgaacaaa actggccttg tgttaaaaat ggatcttgat 2760
cctaacgatg taacctttac ttactcacac ttcgctgtac ctcaagcgaa aggaaacaat 2820
gtcgtgatta caagctatat gacaaacaga ggattctacg cagacaaaca atcaacgttt 2880
gcgccgagct tcctgctgaa catcaaaggc aagaaaacat ctgttgtcaa agacagcatc 2940
cttgaacaag gacaattaac agttaacaaa taaaaacgca aaagaaaatg ccgatggtac 3000

cgagcgaaat gaccgaccaa gcgacgccca acctgccatc acgagatttc gattccaccg 3060

ccgccttcta tgaaaggttg ggcttcggaa tcgttttccg ggacgccctc gcggacgtgc 3120

tcatagtcca cgacgcccgt gattttgtag ccctggccga cggccagcag gtaggccgac 3180

aggctcatgc cggccgccgc cgccttttcc tcaatcgctc ttcgttcgtc tggaaggcag 3240

tacaccttga taggtgggct gcccttcctg gttggcttgg tttcatcagc catccgcttg 3300

c 3301

<210> 19
<211> 878
<212> DNA
<213> synthetic construct

<220>
<223> >SEQ ID NO 19

<400> 19

attaaagagg agaaatacta gatgcgtaaa ggagaagaac ttttcactgg agttgtccca 60

attcttgttg aattagatgg tgatgttaat gggcacaaat tttctgtcag tggagagggt 120

gaaggtgatg caacatacgg aaaacttacc cttaaattta tttgcactac tggaaaacta 180

cctgttccat ggccaacact tgtcactact ttcggttatg gtgttcaatg ctttgcgaga 240

tacccagatc atatgaaaca gcatgacttt ttcaagagtg ccatgcccga aggttatgta 300

caggaaagaa ctatattttt caaagatgac gggaactaca agacacgtgc tgaagtcaag 360

tttgaaggtg atacccttgt taatagaatc gagttaaaag gtattgattt taaagaagat 420

ggaaacattc ttggacacaa attggaatac aactataact cacacaatgt atacatcatg 480

gcagacaaac aaaagaatgg aatcaaagtt aacttcaaaa ttagacacaa cattgaagat 540

ggaagcgttc aactagcaga ccattatcaa caaaatactc caattggcga tggccctgtc 600

cttttaccag acaaccatta cctgtccaca caatctgccc tttcgaaaga tcccaacgaa 660

aagagagacc acatggtcct tcttgagttt gtaacagctg ctgggattac acatggcatg 720

gatgaactat acaaataata atactagagc caggcatcaa ataaaacgaa aggctcagtc 780

gaaagactgg gcctttcgtt ttatctgttg tttgtcggtg aacgctctct actagagtca 840

cactggctca ccttcgggtg ggcctttctg cgtttata 878

<210> 20
<211> 55
<212> DNA
<213> Synechocystis sp. PCC 6803

<220>
<223> >SEQ ID NO 20

<400> 20
agctttacaa aactctcatt aatcctttag actaagttta gtcagttcca atctg 55

<210> 21
<211> 1001
<212> DNA
<213> Synechocystis sp. PCC 6803

<220>
<223> >SEQ ID NO 21

<400> 21

```
gcggcctcga gtgggggcat ctgctaggca atatttgacc acattggcca attcttccaa      60
ttgggcgatc gcctcggctc cttctaattt cattaattct cggtcatccc gcatttctgt     120
ccaggtgatg ccgtagtccg ataccaaaaa gcggatcaga tgattttccc ccaagctcac     180
catgaaggag gcgctgttga gttggtcgcc acaggtgtag caagaagcaa tgtagccccg     240
attttcgagc acagtggcca aagccctcaa gctcatgact aaatccttca cgaaatcgcg     300
gtgttgctgg gcaagtcgag taaacacatt tcctccgaaa ataagctagc ttttaaaatc     360
ttaatacttt tttaagattt tcacagaggc cttgtccgga aaggctaatt taatactgtt     420
caaactggtc tggaaatccc ggtcggtcgg gccatggtaa ttatccatcg gccgggccac     480
tgaaataatc aaccgattac acccgggtgt aatcctgaag ccttgacaat ccccgttggt     540
gatttatact taggacaggt gtgaggaaat tagaaaaaca tctggagtcg aaacaaggaa     600
agacgcccca ggtgtttttt tatggcaatt tttagggcag tccacagatg aagcaatggc     660
gctggcggat ggtattggtg gcgatcgccg ctctggtggt gatttcccta gctacgctgt     720
ttcaacctca ggaaacctct ggccgcatcc cggattatca aattagcagt cagttgcccg     780
ccaaccaggt agattattat cctctgcaac agaatttaga tgaggcgtat tatcggcctt     840
tgggagagtg gttgggtcgt ttaatcctgc cgacggtgga agaaactaaa gccaggacag     900
gagattgggt ttggttggaa ctgtatcaag cgccttctat ccaacaggga ttagtgggac     960
aaaaactccg tctcacctgg cagagcaatg ctgatttgga c                        1001
```

<210> 22
<211> 1021
<212> DNA
<213> Synechocystis sp. PCC 6803

<220>
<223> >SEQ ID NO 22

<400> 22

```
gaacactcga gtgccagcaa cgacaatggt tagggttacc aaagtgaata ctaaatattc      60
cttggcctta gcggatagta atggagatgg ggaataattt ttcaaactaa acatggtatt     120
cgttaatgaa tggtgaacaa ggcaaagtca aaaaatgtaa ctttaattac tgaccaaaga     180
cggcggcaat aaccactagc acaaaataga tggcaatgaa aattatgccg gcaccaaagg     240
accaccaaca aaactttttc gcttccttgg aagcctttac agcgccttca tagtcccctg     300
aagctaaacg agaattaact tccgatgctt tgataatggc cacaataccc aggggtaagc     360
aacaaaatag agttaccagg atcgattgcg ccaagtaatt tggtacatac tgatttgtca     420
taaaacaaac tcatttctga gtagactgga atataccagc caggatagac agaaattatc     480
ttaatggttc attttgttgc gttttcttca caatttgatc ttgcttattt gttagtttca     540
gagcactatt aataacaata ggggaatgaa ggattacacc cgggtgtaat cttgtcacca     600
atttttgtag ggatgttggg ctaaattgac gttgtaatag cgggcatcat ccgttacctc     660
ttccccgatc cagggaagta gacttatttt ttcaccagtg taggttaatt ccacttccgc     720
taaaattaga ccctggttat cccccaaaaa ctcgtctact tctcaggttt taccatggta     780
atcgaggcaa taacgatatt ttcaatcagg ggcggtgaac agagttctgt caaaatttaa     840
ttggcttcca ctgcaagaat ttcatactca aatccaagcg ggcaatgctg gcatttttac     900
ccttaatggt gagataggcc cgatcgccaa tggtacggac tcgcagcgta gttaaatctg     960
gggtggcaat gtaaccttga cggtacaaat aaccctggac taaactccgc cagcggtcat    1020
c                                                                    1021
```

<210> 23
<211> 999
<212> DNA
<213> Synechocystis sp. PCC 6803

<220>
<223> >SEQ ID NO 23

<400> 23

```
gataactcga gtcgcccggt ggatttaatg cccctttgcg gacgggtatt ttccaagcct      60
ggttttggta cctttgctga agcgttaaag ttagaagtcc cgattatttc cctcaccagg     120
gatggttttg cggaagcgca agttttactg gcaggtttgc agaactacgg agagcaccaa     180
attgtgcccc accagcaatt tttcgccggc aactgggatt ttttgttgga aagcccccaa     240
aaacctaggt tggatcagaa gttagacaaa catggcgatc gccagatagc agaaaaaatc     300
gtcgcccaaa ttagttgatt ttgtccccca actcggaaaa agtcagggga ttttggtaaa     360
ccataaacct ggggttaacc tctgggaagc cgttcatggt tgttcatgcc gtatgctttt     420
acgatctaaa tgctatctga cacctccgtt gacttaataa aacttaatcc aaataatttg     480
acttagtggt tcttttcagg cattctctca gcattagaag gctgagtatg gggattacac     540
ccgggtgtaa tccgagcgca aactacaatg cgcttcgttt gcgctgcaca gcttcaacgt     600
tcggcgggct tgcttgccag cactcttttc attaaaatag tatagcagtc tgtattatcg     660
gcattcaact ctgatggata atctaaccga ctttgcattc aagctcggga aggtcgttta     720
caaagttatt cgcgatgtcg aggcatcaaa agatgattcg accaagctca tccaagacct     780
tggcggctta agcggtgaaa agatactact tcagaaaatc gccaagtttt ggaatcagca     840
agaccgctgg gatagacccg atggcctcgt catagtaact agtcacagac ttgtcttttt     900
agctaaaatg aagacggtcg catcaacaac agattatctc agctttccat tggggttgat     960
tgacgaactt gaagctacca ctgtatcttg ggtcagtcc                            999
```

<210> 24
<211> 1055
<212> DNA
<213> Synechocystis sp. PCC 6803

<220>
<223> >SEQ ID NO 24

<400> 24

```
ggttctcgag ccgctgaatt tagtcaacat cggggacacc ctgcacatgt ggtttgtttt      60
agcaagggct tttagaaaaa aatgctcaat ctgaacatct cccatgacat ttcgcttggc     120
gatcgccgag gaggaggagg ctaaactgtt cgtctttttg gctttcacca cgtagataac     180
agtacgagtt attgggtcat tgaattatta acaatataac tatttgctga tttttatact     240
tgcaaatttt agatctaatt gtattagtct tctatagtag aaatataagg aaatcaaatt     300
ttcaaatttt catcttgaaa aacataaaat ttattcatta tttgttaatc gttgccccat     360
caatttgtcg ttaaaatggt taatagggct agaagagcga agtatttgta tttgccgaaa     420
atttcaggag caaaacaatg cgaatcaacc ataagcctct attaattgcc actgccttac     480
taactctcgt gcccaatgga ttacacccgg gtgtaatctc tttacaatgg ccaggtcttt     540
agggagcggt gaccaacgac caaataattt aatttttcca attattttga tagttatttg     600
gggaacatgg acgaataaaa ttcggataaa aagttaaatt tttctaattc tatagtcggg     660
gttttcacct atgactactc ccgtttttttg tactaattgt ggcaaccgcc ttagccccca     720
ggttcgtttc tgtgaatcct gtggatgtcc cgttgccttg acgtcggagc caccttcctt     780
ttctggacca ccattgtccc cattgcctcc tccccctccc acctttaatg tcgatgaagt     840
tccatcccat aaaagagggg taactccttg gattcctttt ttcctgttat ttagttctgt     900
cgttgtatta gggggacttt ggtggttggg agcgttcaat ctgccccaat ggaatcaatg     960
gctagtaaaa attttgccca ccaataccag ttcccccgtt gttacttccc ctactccgac    1020
ggtggctccc gttgatgcca atgccgtaag tttag                               1055
```

<210> 25
<211> 1069
<212> DNA
<213> Synechocystis sp. PCC 6803

<220>
<223> >SEQ ID NO 25

<400> 25

gaactcgagt agtaaccaca ggcttttgcc gtttctcgac ggggcagatg ggatagttct 60
ttaactttat tgagtaatgc tttgccggtt agtgcggtgg aggcgtttgg cataggatta 120
ttttttccgt tgaatgatgc ttgattgcgg ggacttgttt ttttcaaaag tctacataag 180
ataaaatata acatcaaacc ttagctgaaa aacttttttt ttcaactttt tgattattta 240
gccgaggaat ttttccctct gaaaaaatca agccttgacc tctggcgagt tttgtcctaa 300
agatttttgg ccgccatgga gcaatgatga gttaggtttg ggcaatacaa gctagctacg 360
ggttcacctt tccgaaaacc aacgaatttt tgcccagatt agcgccaaaa ttaagcggag 420
cagtgggtca aaatatattt atctcagggg gaatcattgc tttaaatcaa ttcgctatcc 480
tatgcatgtc atgtgttcat taacattccc ctgttcccca agattttatg gttctcaccc 540
agttcgcctc cagcgattac acccgggtgt aatctgctgg ctttgttgcc ctgtcaacca 600
aagttcccaa tacgtccttc ccaattctgc tcaaatcgag ttaactgggc aaatatctat 660
ggattactcc ttcccatttc tcatcaacaa ttaaccaaat gaccctgatg gaaggaagat 720
cgacccagtg aatgactaca ataatggtca acctggccca tttactaggt aaacctaatt 780
taaccgagga aataatctcc atggctcaag attctccatc ttccctctcc caagaagccc 840
taaccattgc ccatctccag ggggtgcagg agcgacggca ggaagagcgg atgttggcct 900
tttgccaagt gttcgatatg gaaggggaat tggtgggggt atccttcgac ttaactcgca 960
acggtatctg tgtgagcgtt cccaatgatt ggccccagga cacggatttt accgtcaagc 1020
tccggcgcat ggacaatgaa gctttaccca ccatcaccat caagctcac 1069

<210> 26
<211> 300
<212> DNA
<213> synthetic construct

<220>
<223> >SEQ ID NO 26

<400> 26

atgccccggg aaaaaaaaac cccgcccctg acagggcggg gtttttttc agataaaaa 60
aatccttagc tttcgctaag gatgatttct gcaattggcg gccgcttcta gaatgcacta 120
gtagcggccg ctgcagtccg gcaaaaaaac gggcaaggtg tcaccaccct gcccttttc 180
tttaaaaccg aaaagattac ttcgcgttgg agagcgttca ccgacaaaca acagataaaa 240

cgaaaggccc agtctttcga ctgagccttt cgttttattt gatgcctggt accggtatgc 300

<210> 27
<211> 582
<212> DNA

<213> Chromohalobacter salexigens

<220>
<223> >SEQ ID NO 27

<400> 27

```
atgaccccaa ccactgagaa ctttacccca agtgccgatt tagcccgccc ctcggtggcg      60
gacaccgtga ttggttccgc taaaaaaact ttatttattc gcaaacccac cactgacgat     120
ggttggggta tttacgaact ggttaaagcg tgtccacctt tagacgtgaa ctctggctac     180
gcttaccttt tgctcgccac ccaatttcgt gacacctgtg cggtggcgac cgatgaggag     240
ggggaaattg ttggttttgt gagtggttat gtaaagcgta acgcgcccga tacctatttc     300
ttgtggcaag tggcggtggg ggaaaaagca cggggaaccg ggctcgcccg gagattagtg     360
gaagctgtgc taatgcgccc cggaatgggg gatgttcgcc atctcgaaac gactattacc     420
ccggacaacg aagctagttg gggcttttt aaaagattgg ccgatcgctg gcaggcccca     480
ctgaatagtc gcgaatattt ttccacaggt cagctaggcg gcgaacatga tcctgaaaac     540
ctggtacgga taggaccctt cgaaccccag caaatctaat aa                        582
```

<210> 28
<211> 1275
<212> DNA
<213> Chromohalobacter salexigens

<220>
<223> >SEQ ID NO 28

<400> 28

```
atgcagacgc aaatcttaga acgtatggag agtgaagtgc gtacctattc tcgttctttc      60
cccaccgtat ttaccgaggc caaaggtgcg cggttgcacg ccgaggacgg caatcagtac     120
attgattttt tagcgggggc tggaactcta aattatggtc acaaccaccc caaattgaag     180
caagccttgg ccgattatat cgcctctgat ggtattgttc atggcctgga catgtggtcc     240
gccgctaaac gcgactattt agaaacatta gaagaagtta ttctaaaacc gcgcgggtta     300
gactacaaag tgcatttgcc cggccccacc gggactaatg cggtggaagc cgccatccgc     360
ttggccagaa atgccaaagg cagacataat attgtcacct ttaccaatgg gttccacggt     420
gttactatgg gcgcactcgc caccacaggc aaccgcaagt ttcgggaagc tactggcggc     480
attccaactc aaggagcctc ttttatgccc ttcgacggtt atatgggtga aggggtagac     540
```

accttatcct attttgaaaa gctcttgggc gataattccg ggggtttaga cgttcccgcc 600
gccgtaatta tcgagactgt ccaaggggaa gggggcataa atccggctgg tattccttgg 660
ttgcaaagac tagaaaagat ttgtcgtgac catgatatgc tacttattgt agacgacatt 720
caagcgggct gtggtcgtac cggcaagttt tttagttttg agcatgcggg cattacccct 780
gacattgtga cgaacagcaa gtccctatcc gggtttggtt tgccatttgc tcatgtgctt 840
atgagaccag aactggatat ttggaaacca ggtcagtata atgggacctt tagaggcttc 900
aacttggcct ttgttaccgc tgcggcggcc atgcgacatt tctggtccga cgacactttt 960
gaacgcgacg tgcaacgaaa agggcgtgtc gtggaagata gatttcagaa actggcgagt 1020
tttatgacgg aaaaaggcca cccagcttcc gagcgaggcc gtggcttgat gcgggggtta 1080
gatgttggcg atggcgatat ggccgataag attactgcgc aggcatttaa aaatgggtta 1140
attattgaaa cgtccggtca ttccggccag gtcattaagt gtctgtgccc cttgacaatt 1200
accgacgaag atctagtcgg tggcttagat attttggaac agagtgtgaa agaagtgttt 1260
ggtcaagcgt aataa 1275

<210> 29
<211> 396
<212> DNA
<213> Chromohalobacter salexigens

<220>
<223> >SEQ ID NO 29

<400> 29

atgattgttc gcaacctgga agaatgtcgg aaaactgagc ggttcgtgga ggccgagaat 60
gggaattggg acagtacccg gctcgtgtta gccgacgata atgtgggttt ttcgttcaac 120
attacccgta tacatccagg taccgaaaca catattcact acaagcatca ctttgaagcg 180
gtgttctgct atgaaggcga aggagaggtg gaaaccttgg ccgatggtaa gattcacccc 240
atcaaggccg gagatatgta tctactggac cagcatgatg aacatctctt gcggggtaaa 300
gaaaaaggca tgacagtcgc ctgcgtgttc aacccagctc tgaccggccg cgaagtccat 360
cgtgaagacg ggtcctacgc tcccgtggat taataa 396

<210> 30
<211> 948
<212> DNA
<213> Chromohalobacter salexigens

<220>
<223> >SEQ ID NO 30

<400> 30

```
atgtctgtgc aatcagcttt taggcatgaa gatgttacgt ttgggaacgc ttttgacgcc      60

tatcctactc ggctctctga cgccacgggc tccctgtggc aggatcgaaa agatgcagta     120
gtcaaaggta gggccgaaga tggcccgttg tcccgcgatc aactagcccg ctttgagcgt     180
gacgggtttc tttttgagcc ccacttcctg gccgatgatg aagtcgggga attacgtaga     240
gaacttgccg ccctgctaga acgcgacgat tttcggggtc gtgacttctc cattacggaa     300
cccgattctc aagagatccg gtcattgttt gcagtgcatt ttttatccga gcggtttcgt     360
agattggccg aagatcccag gttgaccggg cgtgcacgac aaattgtcgg tggggacgtg     420
tatgtacatc aaagtcgtat taactataaa ccgggctttc acggcaaggg gttcaactgg     480
cattctgact tcgaaacctg gcatgcggaa gatggcatgc ccgaaatgca cgcggtgtcc     540
gcctcgattg tattaaccga caatcatcat tataatgggc ccttaatgtt gatcccaggt     600
tcccataaag tattcgtaca ctgcttaggt gaaaccccag aagatcacca caagcagagt     660
ttgaagtccc aagagttcgg agtgcctagt cacgaagcct tgcgccgctt aatcggcgct     720
aatgggattg aagcgcccac cggggcagcg ggagggctgt tattattcga ttgcaatacc     780
ctacacggaa gcaatgccaa tatgagcccg gacccgcgca gtaatgcctt ctttgtgtat     840
aaccgcagag ataatgcctg ccacgcacca tacggcgcaa aacgccctcg acccagcttt     900
ttagcccatg cccccgacga agtgtggaca cctgataccc attaataa                  948
```

<210> 31
<211> 522
<212> DNA
<213> Methylomicrobium alcaliphilum

<220>
<223> >SEQ ID NO 31

<400> 31

```
atgcttcctg ataaaacagc cttgcctatt attaccctta gccaaccaac cgcggaagtc      60
ggtgcgcaag tgcatcgact gatctccaaa tgtccaccct tagatccgaa ctccatgtac     120
tgcaatctgc ttcaaagcag tcacttctca gaaactgccg tggctgccaa aattggagat     180
gaattagttg gcttcgtttc aggatatcgt attccccaga ggcccgacac tttatttgtg     240
tggcaagtag ccgttgggga aaaggcgcgg ggccaggggt tagccactcg tatgctgaaa     300
gccattttag ccaggcccgt caaccaggac attaaccgaa ttgaaacgac cattaccccc     360
aataataaag cgtcgtgggc tctgtttgaa gggctggcca aaaagttgga cacacagatt     420
ggtagcgctg ttatgtttga taaaactcgt cactttgcag atcaacatga gaccgaaatg     480
ctcgttaaag tgggtccatt taaagcagtg caagcataat aa                        522
```

<210> 32
<211> 1335
<212> DNA
<213> Methylomicrobium alcaliphilum

<220>
<223> >SEQ ID NO 32

<400> 32

```
atgaagattt ttgaggagat tgaaagtgaa gttaggtcct acgcccgcgc cttcccgcgc      60
gtatttgatc gggctcaggg cgctaagatt tacgaccacg aaggaaacga atacctggat     120
tttttggctg gtgcgggcag cctgaattat gggcataacc atcccgtgtt taaggaaaag     180
ttactagaat atattcaaaa tgatggcatt acccagggtc ttgatctgca tacgcgcgcg     240
aaaggggagt tcttagaatc gtttaatgaa cacattctca agccccgtaa cttggactat     300
atcgtgatgt ttactggacc gacagggact aatgctgttg aagccgccct gaaaatagcg     360
cgcaaaaaaa cagggcggga gaatattatt tccttcacca acggttggca tgggcaaacg     420
ctcggcagtt taagcgtgac cggaaactca acccaccggg gcggcgctgg aattgctttg     480
catggaagta cgcgaatacc ctatgatggt tacctgggtg atgattttga taccactaat     540
ctattagaca agatgctgtc agatagcagt tccggagtgg ataaacccgc ggctgtgatc     600
gttgaaactg tgcaggggga gggtggcatt aacgctgcca gcatgacctg gttacgatcg     660
ctatccgaaa tctgtaagcg gcatgatgtg ctccttatca ttgacgatat acaggctggc     720
tgcggaagaa ctggtacctt tttttccttt gaggaagccg gtatttaccc cgatatcgta     780
acgttgagta aaagtttatc cggatatggg ctgcccttag ccatcgtgtt aatgaagcca     840
gaaatagacc aatggtcccc aggcgaacat aatggtactt ttcgagggaa taatcacgcc     900
tttgtgaccg ccaaagctgc gattgatcat ttctggaaag acgattcctt tgcgaaagat     960
gtgcaacgga aagggcgata cattgccgac cgtgttgatg taattgttgc caagtatgga    1020
gagggaaact ttaattcaca cggccgaggc atgtttcgtg gcattaactg tgtgtccggc    1080
gatttggcgg ggcaaatcac gcgcagatgt ttccaaaagg gcctcatcat tgaaacctcc    1140
ggtgctgatg atcatgtggt taagttcctg tgtcccctta ttattaccga tgaggaactc    1200
gaaaaaggta ttgatatttt ggaacaggca attaaggaag tgtgcgataa ggcagacacg    1260
attccggaag gtaaggattt cttcgaaggg gattatagcg ttagcaaaga agtgagcaaa    1320
gctggacact aataa                                                     1335
```

<210> 33
<211> 408
<212> DNA
<213> Methylomicrobium alcaliphilum

<220>
<223> >SEQ ID NO 33

<400> 33

atgattgtgc gccaactcca agacgcccaa caaaccaacc gccgcattgt gtctcccgat 60

gggaattggg aatccacaag attgttacta aaggatgatg gcatgggtta ctcctttcat 120

attaccacca tttttgcagg agctgatttt cgcatgcact atcaaaatca cttagaaagc 180

gtatattgta tctccggcga gggcgaagta gagacccttg acgatggcaa gaaatatcct 240

attactcctg gcaccttgta taatctcgac cagcacgaca ggcacatctt gcgggccttt 300

aaagaacttc aactcgcctg cgttttcaat cctcccctaa atggcaagga agtacacaac 360

gccgaaggcg catacgaatt agaagcggaa actgtgtcag attaataa 408

<210> 34
<211> 963
<212> DNA
<213> Methylomicrobium alcaliphilum

<220>
<223> >SEQ ID NO 34

<400> 34

atgattaccg aaaatgccgc acaatccgaa cagagtgaag atttttatca gtcccggaat 60

ggaagcaaac ccaaaattat tcctcgcgtc gatcctgtgg tctatgcgca gacggcaaat 120

cccggactaa tcgccgagga cttgcaggcc cgttacgaac agcaaggttt tttggttatt 180

gataatgtgt ttaacgaacg cgaagtggat tgtttcaaac aagaattaaa gcggttgaac 240

gatgatgaaa agatcaaagc cagcgccgaa gccattacag agctgtcgtc cgatgaatta 300

cgctccctat ttaaaataca tgaagtgtca cctgtgttca aacgcctggc cgccgataac 360

cgtctggccg gtctcgctca acacttatta aatgatcgag tatacattca ccaaagccgt 420

ttgaattaca aacccggttt ccgtggcaag gaattttatt ggcactccga ctttgaaacc 480

tggcacgtag aggacggtat gcctcgcatg cgcgctttat ctatgagtat cattttgacc 540

gaaaacgatc aacataacgg accactgatg ttggtgcccg gcagtcacaa gaaatttgtc 600

gtgtgcgaag aagaaactcc cgaaaaccat tattcagtta gtttaaaaaa acaagaatat 660

gggattccct cagacgaatg tttagcctca ttggttgccg atggagggat agtaagcgcc 720

aatggcaaac ctggtagtgt gcttattttt gatagtaacg tgatgcatgg ttccaattca 780

aacattaccc cgtggcctcg ctccaactta ttctttgtgt ataacgccat aaataatcgt 840

gttacctggc ccttttgcgg tctgttaccc cgcccagaat acttgtgtag ccggaaaaat 900

attcgagtaa tcgaacctag accctttata gccgccgcgg accaactaat ttacgcctaa 960

taa 963

<210> 35
<211> 1446

<212> DNA
<213> Methylomicrobium alcaliphilum

<220>
<223> >SEQ ID NO 35

<400> 35

```
atgacgttcc ataccgtaga aaagatcgga ggcacctcta tgagtgacta cgtggctgtt      60
cgtgacaaca ttatcttgaa acctgtccac cgcgaagatc tgtaccaacg tatttttgtg     120
gtgtccgcgt acggcggaat taccgacatg ttattggagc ataaaaaaaa tgggcaagcc     180
gggatttatg gacagtttgc gaatagtgtg aatgacgacg gatggcaagc cagcctcgtt     240
gctttaaaac aagccatgtt tgccattaat gccaatattt tcgataccgc agatgccatt     300
aaaaaagccg atacatttat tggcgagcgg ttagatgatg cggaacggtg tttagctgat     360
ctgcaacact tgtgtcgtca cgggcatttc tcgttagatg cgcacttgtc caccgtgagg     420
gagatgctcg ctagtcttgg ggaagcgcat agcgcctgga acacagtgca actcttgcaa     480
caagatggta ttaacgccgt ttttgtggat ctaaccgggt ggcaaaccga taaacacatg     540
accttagatg aacgtattaa aaatgctttt gcagaaattg acttacgtca gcaactgccg     600
atcgccacag gctatgctca cagcattgac ggtctgatga gtaccttcga ccggggttat     660
tcagaaatga cctttagccg tatcgcagtc ctgaccaagg ctgatgaggc tatcattcat     720
aaggaatttc acttgagtag tgccgatcct cggttggtcg gagaagataa agccgttccc     780
attggtcgca ccaattatga tgtagcggat caactcgcca acctgggtat ggaggctatc     840
caccccaaag ccgcgaaggg cctccgccaa aacgatatag ccctacgtgt gcgtaatacg     900
tttgaacccg atcatgcagg gacactaatt accggagatt acgtgtccag taaaccatgt     960
gttgaaatta tagcaggctg taaaggggtc tatgcgttcg aagttttcga ccaaaacatg    1020
gccggtgaaa tacacaatta cgatcgcgag attctgggcc agattcgccg ttttcgcgct    1080
catattgtaa gtaaggacat taatgcaaac acgattacac actacttatc tgcctcatta    1140
aagacgattc gtcggatccg agatgccttg caagagattt atccagacgc ggaaatcaac    1200
caacaaaagg tgagtattgt gagtgccatt gggtctgata tgaagatccc cggtattcta    1260
gccaaaaccg tgagtgctct ggctgagaaa caaatctcgg ttttggccat gcatcaatcc    1320
atgcgtcagg ttgatatgca gtttgtaatt gatgaggacg cgtacactga tgccatgaaa    1380
agtttacatt gtcacttggt ggaagttcat gatcatggga ttgcgatttg cctagcgagc    1440
taataa                                                                1446
```

<210> 36
<211> 18
<212> DNA

<213> synthetic construct

<220>
<223> >SEQ ID NO 36

<400> 36
catggattga aggaaggg 18

<210> 37
<211> 21
<212> DNA
<213> synthetic construct

<220>
<223> >SEQ ID NO 37

<400> 37
tttggtgaac aattaatgaa c 21

<210> 38
<211> 18
<212> DNA
<213> synthetic construct

<220>
<223> >SEQ ID NO 38

<400> 38
gcagtccctc ctccaaac 18

<210> 39
<211> 22
<212> DNA
<213> synthetic construct

<220>
<223> >SEQ ID NO 39

<400> 39
aagccgatat tcaggtaaga tg 22

<210> 40
<211> 18
<212> DNA
<213> synthetic construct

<220>
<223> >SEQ ID NO 40

<400> 40
ggaagtgttg ttgctgtc 18

<210> 41
<211> 18
<212> DNA
<213> synthetic construct

<220>

<223> >SEQ ID NO 41

<400> 41
gtccttcgcc gtagattg 18

<210> 42
<211> 20
<212> DNA
<213> synthetic construct

<220>
<223> >SEQ ID NO 42

<400> 42
aaggtaatca agcagaaatg 20

<210> 43
<211> 18
<212> DNA
<213> synthetic construct

<220>
<223> >SEQ ID NO 43

<400> 43
attggtaaca gcacttcc 18

<210> 44
<211> 19
<212> DNA
<213> synthetic construct

<220>
<223> >SEQ ID NO 44

<400> 44
tgactgtaag caatctacc 19

<210> 45
<211> 19
<212> DNA
<213> synthetic construct

<220>
<223> >SEQ ID NO 45

<400> 45
actaacgatg gaatcttgg 19

<210> 46
<211> 21
<212> DNA
<213> synthetic construct

<220>
<223> >SEQ ID NO 46

<400> 46

aacttaactt ctattcgtga g 21

<210> 47
<211> 19
<212> DNA
<213> synthetic construct

<220>
<223> >SEQ ID NO 47

<400> 47
gaaaccttga taagcagtc 19

<210> 48
<211> 18
<212> DNA
<213> synthetic construct

<220>
<223> >SEQ ID NO 48

<400> 48
gatattgatg tgtattgc 18

<210> 49
<211> 18
<212> DNA
<213> synthetic construct

<220>
<223> >SEQ ID NO 49

<400> 49
ttattatctt ctgtctcc 18

<210> 50
<211> 23
<212> DNA
<213> synthetic construct

<220>
<223> >SEQ ID NO 50

<400> 50
tgaataccac atctcccatt gac 23

<210> 51
<211> 19
<212> DNA
<213> synthetic construct

<220>
<223> >SEQ ID NO 51

<400> 51
tttaacctgc tggcgtgac 19

<210> 52

<211> 19
<212> DNA
<213> synthetic construct

<220>
<223> >SEQ ID NO 52

<400> 52
ttctcaccgc tactgttac 19

<210> 53
<211> 19
<212> DNA
<213> synthetic construct

<220>
<223> >SEQ ID NO 53

<400> 53
cacttctcgc actaattcg 19

<210> 54
<211> 18
<212> DNA
<213> synthetic construct

<220>
<223> >SEQ ID NO 54

<400> 54
cccaaatgat gaccgaag 18

<210> 55
<211> 19
<212> DNA
<213> synthetic construct

<220>
<223> >SEQ ID NO 55

<400> 55
ctagtaactg ctgttcctc 19

<210> 56
<211> 18
<212> DNA
<213> synthetic construct

<220>
<223> >SEQ ID NO 56

<400> 56
aagtgtccaa agccaaac 18

<210> 57
<211> 18
<212> DNA
<213> synthetic construct

<220>
<223> >SEQ ID NO 57

<400> 57
tcaacaatgc catcttcc 18

<210> 58
<211> 19
<212> DNA
<213> synthetic construct

<220>
<223> >SEQ ID NO 58

<400> 58
ttaattgcca ctgccttac 19

<210> 59
<211> 18
<212> DNA
<213> synthetic construct

<220>
<223> >SEQ ID NO 59

<400> 59
tagagacagc ggatatgc 18

<210> 60
<211> 18
<212> DNA
<213> synthetic construct

<220>
<223> >SEQ ID NO 60

<400> 60
tgatgtggac gaggatac 18

<210> 61
<211> 18
<212> DNA
<213> synthetic construct

<220>
<223> >SEQ ID NO 61

<400> 61
gggctaaggg aatatggg 18

<210> 62
<211> 28
<212> DNA
<213> synthetic construct

<220>
<223> >SEQ ID NO 62

<400> 62
ctgaccccgg gtgaatgtca gctactgg 28

<210> 63
<211> 29
<212> DNA
<213> synthetic construct

<220>
<223> >SEQ ID NO 63

<400> 63
caaacccggg cgatttactt ttcgacctc 29

<210> 64
<211> 28
<212> DNA
<213> synthetic construct

<220>
<223> >SEQ ID NO 64

<400> 64
acagacccgg gcaagcggat ggctgatg 28

<210> 65
<211> 37
<212> DNA
<213> synthetic construct

<220>
<223> >SEQ ID NO 65

<400> 65
gcggcctcga gtgggggcat ctgctaggca atatttg 37

<210> 66
<211> 58
<212> DNA
<213> synthetic construct

<220>
<223> >SEQ ID NO 66

<400> 66
gattacaccc gggtgtaatc ggttgattat ttcagtggcc cggccgatgg ataattac 58

<210> 67
<211> 32
<212> DNA
<213> synthetic construct

<220>
<223> >SEQ ID NO 67

<400> 67
gtccaaatca gcattgctct gccaggtgag ac 32

<210> 68
<211> 59
<212> DNA
<213> synthetic construct

<220>
<223> >SEQ ID NO 68

<400> 68
gattacaccc gggtgtaatc ctgaagcctt gacaatcccc gttggtgatt tatacttag 59

<210> 69
<211> 28
<212> DNA
<213> synthetic construct

<220>
<223> >SEQ ID NO 69

<400> 69
gaacactcga gtgccagcaa cgacaatg 28

<210> 70
<211> 51
<212> DNA
<213> synthetic construct

<220>
<223> >SEQ ID NO 70

<400> 70
gattacaccc gggtgtaatc cttcattccc ctattgttat taatagtgct c 51

<210> 71
<211> 27
<212> DNA
<213> synthetic construct

<220>
<223> >SEQ ID NO 71

<400> 71
gatgaccgct ggcggagttt agtccag 27

<210> 72
<211> 57
<212> DNA
<213> synthetic construct

<220>
<223> >SEQ ID NO 72

<400> 72
gattacaccc gggtgtaatc ttgtcaccaa tttttgtagg gatgttgggc taaattg 57

<210> 73
<211> 31
<212> DNA

<213> synthetic construct

<220>
<223> >SEQ ID NO 73

<400> 73
gataactcga gtcgcccggt ggatttaatg c 31

<210> 74
<211> 52
<212> DNA
<213> synthetic construct

<220>
<223> >SEQ ID NO 74

<400> 74
gattacaccc gggtgtaatc cccatactca gccttctaat gctgagagaa tg 52

<210> 75
<211> 25
<212> DNA
<213> synthetic construct

<220>
<223> >SEQ ID NO 75

<400> 75
ggactgaccc aagatacagt ggtag 25

<210> 76
<211> 50
<212> DNA
<213> synthetic construct

<220>
<223> >SEQ ID NO 76

<400> 76
gattacaccc gggtgtaatc cgagcgcaaa ctacaatgcg cttcgtttgc 50

<210> 77
<211> 32
<212> DNA
<213> synthetic construct

<220>
<223> >SEQ ID NO 77

<400> 77
ggttctcgag ccgctgaatt tagtcaacat cg 32

<210> 78
<211> 49
<212> DNA
<213> synthetic construct

<220>

<223> >SEQ ID NO 78

<400> 78
gattacaccc gggtgtaatc cattgggcac gagagttagt aaggcagtg 49

<210> 79
<211> 30
<212> DNA
<213> synthetic construct

<220>
<223> >SEQ ID NO 79

<400> 79
ctaaacttac ggcattggca tcaacgggag 30

<210> 80
<211> 55
<212> DNA
<213> synthetic construct

<220>
<223> >SEQ ID NO 80

<400> 80
gattacaccc gggtgtaatc tctttacaat ggccaggtct ttagggagcg gtgac 55

<210> 81
<211> 28
<212> DNA
<213> synthetic construct

<220>
<223> >SEQ ID NO 81

<400> 81
gaactcgagt agtaaccaca ggcttttg 28

<210> 82
<211> 47
<212> DNA
<213> synthetic construct

<220>
<223> >SEQ ID NO 82

<400> 82
gattacaccc gggtgtaatc gctggaggcg aactgggtga gaaccat 47

<210> 83
<211> 28
<212> DNA
<213> synthetic construct

<220>
<223> >SEQ ID NO 83

<400> 83

gtgagcttga tggtgatggt gggtaaag 28

<210> 84
<211> 52
<212> DNA
<213> synthetic construct

<220>
<223> >SEQ ID NO 84

<400> 84
gattacaccc gggtgtaatc tgctggcttt gttgccctgt caaccaaagt tc 52

<210> 85
<211> 31
<212> DNA
<213> synthetic construct

<220>
<223> >SEQ ID NO 85

<400> 85
cgaggcgatc gcccagttgg aagaattggc c 31

<210> 86
<211> 39
<212> DNA
<213> synthetic construct

<220>
<223> >SEQ ID NO 86

<400> 86
ctaaaaagac aagtctgtgg ctagttacta tgacgaggc 39

<210> 87
<211> 20
<212> DNA
<213> synthetic construct

<220>
<223> >SEQ ID NO 87

<400> 87
tcctggtaac tcacgctatc 20

<210> 88
<211> 24
<212> DNA
<213> synthetic construct

<220>
<223> >SEQ ID NO 88

<400> 88
agccgatcca gggaagtgtt gttg 24

<210> 89

<211> 25
<212> DNA
<213> synthetic construct

<220>
<223> >SEQ ID NO 89

<400> 89
ccatcgtcct tcgccgtaga ttgtg 25

<210> 90
<211> 20
<212> DNA
<213> synthetic construct

<220>
<223> >SEQ ID NO 90

<400> 90
cccagttaaa ctgcgaaagg 20

<210> 91
<211> 18
<212> DNA
<213> synthetic construct

<220>
<223> >SEQ ID NO 91

<400> 91
tcgccaagct ttcagaac 18

<210> 92
<211> 18
<212> DNA
<213> synthetic construct

<220>
<223> >SEQ ID NO 92

<400> 92
caaactccag ccgataac 18

<210> 93
<211> 25
<212> DNA
<213> synthetic construct

<220>
<223> >SEQ ID NO 93

<400> 93
ccggttgccc ttatcggaac cgatg 25

<210> 94
<211> 20
<212> DNA
<213> Synechocystis sp. PCC 6803

<220>
<223> >SEQ ID NO 94

<400> 94
gctatggcgt cacttgtagc 20

<210> 95
<211> 20
<212> DNA
<213> synthetic construct

<220>
<223> >SEQ ID NO 95

<400> 95
tttgcgaccc atcggattgc 20

<210> 96
<211> 20
<212> DNA
<213> synthetic construct

<220>
<223> >SEQ ID NO 96

<400> 96
ctccaaggcg actacctttc 20

<210> 97
<211> 20
<212> DNA
<213> synthetic construct

<220>
<223> >SEQ ID NO 97

<400> 97
cccagtggga atgcgatcag 20

<210> 98
<211> 20
<212> DNA
<213> synthetic construct

<220>
<223> >SEQ ID NO 98

<400> 98
taggagggcg atcaccgaag 20

<210> 99
<211> 20
<212> DNA
<213> synthetic construct

<220>
<223> >SEQ ID NO 99

<400> 99
acccatttct tgggtgtagg 20

<210> 100
<211> 25
<212> DNA
<213> synthetic construct

<220>
<223> >SEQ ID NO 100

<400> 100
ggccttggtt gccctgactg atgtg 25

<210> 101
<211> 25
<212> DNA
<213> synthetic construct

<220>
<223> >SEQ ID NO 101

<400> 101
gaccgatcgc cgcagtagtt cttgg 25

<210> 102
<211> 20
<212> DNA
<213> synthetic construct

<220>
<223> >SEQ ID NO 102

<400> 102
tcttgttgaa ttagatggtg 20

<210> 103
<211> 21
<212> DNA
<213> synthetic construct

<220>
<223> >SEQ ID NO 103

<400> 103
tgtgagttat agttgtattc c 21

<210> 104
<211> 15
<212> DNA
<213> synthetic construct

<220>
<223> >SEQ ID NO 104

<400> 104
attaaagagg agaaa 15

<210> 105
<211> 63
<212> DNA
<213> synthetic construct

<220>
<223> >SEQ ID NO 105

<400> 105

ttgacaatta atcatccggc tcgtataatg tgtggaattg tgagcggata acaatttcac 60

aca 63

<210> 106
<211> 309
<212> DNA
<213> Synechocystis sp. PCC 6803

<220>
<223> >SEQ ID NO 106

<400> 106

atggggaaag gcaaagccga tccagggaag tgttgttgct gtccaaaaaa gtcaccaatc 60

agcctgggct ttttgccaat cctccgtgat cttagcgtga ttattaaaat ggcgatggcc 120

tttatacaac tcttaaccga aaacaatccg agtggggaat ttgtttttgg tctatggagt 180

ggtgctgggc caggggaaac cctgattgaa gaatttttaa aaatcttaga aaaaattcac 240

aatctacggc gaaggacgat ggaaaaagct tcggagttct gctttaggtc aaagtttctt 300

gaaaattaa 309

<210> 107
<211> 444
<212> DNA
<213> Synechocystis sp. PCC 6803

<220>
<223> >SEQ ID NO 107

<400> 107

atgattttta tgaaatacaa agctgaacaa tttatcagct cttctgaatt tagtcctagt 60

ttcgccaagc tttcagaaca cggcaaaaac tctttgtcat gcaaaatagt cagaaattac 120

tatgtgatgg cttctacaaa cttaacttct attcgtgagt ctcttaggag atgtcgcgat 180

gagagtggac agccacttag cttaagccct agcgaattaa aaccatcaag ttatcggctg 240

gagtttgaac tagtagcact aattgcttca aatttgacac agatgccaga tttagagaat 300

tttatcaaag atgataaaga aactactaat gaagataaat taattttgtt gactgcttat 360

caaggtttca aaaataaagt tgatggattg tttaaattat ttggttggcc atgctttgga 420

tttaaacaag aaaaagaaac ttaa 444

<210> 108
<211> 618
<212> DNA
<213> Synechocystis sp. PCC 6803

<220>
<223> >SEQ ID NO 108

<400> 108

atgaaaaaac cagatattga tgtgtattgc aatgtaatgg aagaaatcaa gcgacgaaca 60

ggcgttatcg atttttttct atctggtaat ggtcacgctt tatgtgaacc tacaacactg 120

gaatcaattg cgctgcaaat caggaaaatc ctagagctaa tagctatggc gtcacttgta 180

gcaaacgaaa aagaatacaa aaaggtatat tccaattttg ccagcgcatg gaatgcagaa 240

tatttactaa aagacttagg cagaattaat cctgattttt accctaaacc agtcgtagag 300

aaactttctg atgaccctaa agtaaaaaat cgcctagcag atcgtgacga agactatcta 360

accaaaaatg aatttataaa agtttacaag aaatgtggcg caatcatgca tgcgagcaat 420

ccgatgggtc gcaaaatagg ctatgaatac tacaaaaaga acattccaga atggagacag 480

aagataataa acctcctaaa caaccaccaa attaagttat ttaatcactc tggcttttat 540

ctaattcata tgaaagaaga tcgtgatgat aaggtacatt tttatgaatt cgaccttgtg 600

caaaccacaa gcagctaa 618

<210> 109
<211> 519
<212> DNA
<213> Synechocystis sp. PCC 6803

<220>
<223> >SEQ ID NO 109

<400> 109

atgcgaatca accataagcc tctattaatt gccactgcct tactaactct cgtgcccaat 60

gttgtttggt cttctccctt tgatgaagat caggagattt gtccccagtg ggaatgcgat 120

caggcgggaa attgccgcaa acttgatctt acctgcggtt cttccccaga ttcttctggt 180

```
gctcagggct atcaattata ttttgatggg gaattagtgt ctggccccga cgcccagggt     240
tatacatccc aacaagcgaa agaaaattgt acttggaata tgcaaactaa accgcaaatg     300
catatccgct gtctctacaa tggccaggtc ttcggtgatc gccctcctag taactctcct     360
gggtatgaac tatattttga tggggaatta gtgtctggcg ccgacgccca aggttatacg     420
ttccaacaag cggaagaaaa ttgcatttgg aatatgcaaa ccaaaccgca aattcacatt     480
cgctgtcttt acaatggcca ggtctttagg gagcggtga                            519
```

<210> 110
<211> 576
<212> DNA
<213> Synechocystis sp. PCC 6803

<220>
<223> >SEQ ID NO 110

<400> 110

```
atggttctca cccagttcgc ctccagccca gctcctcccc agtcctttgc tgttgccccc      60
aaccgtcaac aattgcgaca aatgaattgc tacctggacc agcttttgtt ggccttggtt     120
gccctgactg atgtggacga ggatacttgg acggcaatcc tagaaaaaat ttcgtcatct     180
acgacaacag gccaatcttt gactaggaaa taccccattc ctgctgttgc ccccggccag     240
ctagatgtgg agggaattcg acttttggcc atggcgatcg cctatttagg ggcccattac     300
caagaactac tgcggcgatc ggtcagttta gttgaacaga ccaaggaaca gggtaaaaat     360
cctttacaaa ccgtactttt aagcgagtac gcccgaaaat ttacctcagc ttggcaagcc     420
tatggagaga aacaaggggg agaagcccat attcccttag cccccattac ctctgatcgc     480
ctgctttctt tgctgacaga attatttttc tttagcagtc aagacggccc ccgccgactc     540
tggggagtat tggccaccat ggtgtccccc gcttag                               576
```

<210> 111
<211> 1001
<212> DNA
<213> Synechocystis sp. PCC 6803

<220>
<223> >SEQ ID No 111

<400> 111

```
gtccaaatca gcattgctct gccaggtgag acggagtttt tgtcccacta atccctgttg      60
gatagaaggc gcttgataca gttccaacca aacccaatct cctgtcctgg ctttagtttc     120
ttccaccgtc ggcaggatta aacgacccaa ccactctccc aaaggccgat aatacgcctc     180
atctaaattc tgttgcagag gataataatc tacctggttg gcgggcaact gactgctaat     240
ttgataatcc gggatgcggc cagaggtttc ctgaggttga aacagcgtag ctagggaaat     300
caccaccaga gcggcgatcg ccaccaatac catccgccag cgccattgct tcatctgtgg     360
actgccctaa aaattgccat aaaaaaacac ctggggcgtc tttccttgtt tcgactccag     420
atgtttttct aatttcctca cacctgtcct aagtataaat caccaacggg gattgtcaag     480
gcttcaggat tacacccggg tgtaatcggt tgattatttc agtggcccgg ccgatggata     540
attaccatgg cccgaccgac cgggatttcc agaccagttt gaacagtatt aaattagcct     600
ttccggacaa ggcctctgtg aaaatcttaa aaaagtatta agattttaaa agctagctta     660
ttttcggagg aaatgtgttt actcgacttg cccagcaaca ccgcgatttc gtgaaggatt     720
tagtcatgag cttgagggct ttggccactg tgctcgaaaa tcggggctac attgcttctt     780
gctacacctg tggcgaccaa ctcaacagcg cctccttcat ggtgagcttg ggggaaaatc     840
atctgatccg ctttttggta tcggactacg gcatcacctg gacagaaatg cgggatgacc     900
gagaattaat gaaattagaa ggagccgagg cgatcgccca attggaagaa ttggccaatg     960
tggtcaaata ttgcctagca gatgccccca ctcgaggccg c                        1001
```

<210> 112
<211> 1021
<212> DNA
<213> Synechocystis sp. PCC 6803

<220>
<223> >SEQ ID No 112

<400> 112

```
gatgaccgct ggcggagttt agtccagggt tatttgtacc gtcaaggtta cattgccacc      60
ccagatttaa ctacgctgcg agtccgtacc attggcgatc gggcctatct caccattaag     120
ggtaaaaatg ccagcattgc ccgcttggat ttgagtatga aattcttgca gtggaagcca     180
attaaatttt gacagaactc tgttcaccgc ccctgattga aaatatcgtt attgcctcga     240
ttaccatggt aaaacctgag aagtagacga gtttttgggg gataaccagg gtctaatttt     300
agcggaagtg gaattaacct acactggtga aaaaataagt ctacttccct ggatcgggga     360
agaggtaacg gatgatgccc gctattacaa cgtcaattta gcccaacatc cctacaaaaa     420
ttggtgacaa gattacaccc gggtgtaatc cttcattccc ctattgttat taatagtgct     480
ctgaaactaa caaataagca agatcaaatt gtgaagaaaa cgcaacaaaa tgaaccatta     540
agataatttc tgtctatcct ggctggtata ttccagtcta ctcagaaatg agtttgtttt     600
atgacaaatc agtatgtacc aaattacttg gcgcaatcga tcctggtaac tctattttgt     660
tgcttacccc tgggtattgt ggccattatc aaagcatcgg aagttaattc tcgtttagct     720
tcaggggact atgaaggcgc tgtaaaggct tccaaggaag cgaaaaagtt ttgttggtgg     780
tcctttggtg ccggcataat tttcattgcc atctattttg tgctagtggt tattgccgcc     840

gtctttggtc agtaattaaa gttacatttt ttgactttgc cttgttcacc attcattaac     900
gaataccatg tttagtttga aaaattattc cccatctcca ttactatccg ctaaggccaa     960
ggaatattta gtattcactt tggtaaccct aaccattgtc gttgctggca ctcgagtgtt    1020
c                                                                    1021
```

<210> 113
<211> 999
<212> DNA
<213> Synechocystis sp. PCC 6803

<220>
<223> >SEQ ID No 113

<400> 113

ggactgaccc aagatacagt ggtagcttca agttcgtcaa tcaaccccaa tggaaagctg 60
agataatctg ttgttgatgc gaccgtcttc attttagcta aaaagacaag tctgtgacta 120
gttactatga cgaggccatc gggtctatcc cagcggtctt gctgattcca aaacttggcg 180
attttctgaa gtagtatctt ttcaccgctt aagccgccaa ggtcttggat gagcttggtc 240
gaatcatctt ttgatgcctc gacatcgcga ataactttgt aaacgacctt cccgagcttg 300
aatgcaaagt cggttagatt atccatcaga gttgaatgcc gataatacag actgctatac 360
tattttaatg aaaagagtgc tggcaagcaa gcccgccgaa cgttgaagct gtgcagcgca 420
aacgaagcgc attgtagttt gcgctcggat tacacccggg tgtaatcccc atactcagcc 480
ttctaatgct gagagaatgc ctgaaaagaa ccactaagtc aaattatttg gattaagttt 540
tattaagtca acggaggtgt cagatagcat ttagatcgta aaagcatacg gcatgaacaa 600
ccatgaacgg cttcccagag gttaacccca ggtttatggt ttaccaaaat cccctgactt 660
tttccgagtt gggggacaaa atcaactaat ttgggcgacg atttttctg ctatctggcg 720
atcgccatgt ttgtctaact tctgatccaa cctaggtttt tgggggcttt ccaacaaaaa 780
atcccagttg ccggcgaaaa attgctggtg gggcacaatt tggtgctctc cgtagttctg 840
caaacctgcc agtaaaactt gcgcttccgc aaaaccatcc ctggtgaggg aaataatcgg 900
gacttctaac tttaacgctt cagcaaaggt accaaaacca ggcttggaaa atacccgtcc 960
gcaaaggggc attaaatcca ccgggcgact cgagttatc 999

<210> 114
<211> 1055
<212> DNA
<213> Synechocystis sp. PCC 6803

<220>
<223> >SEQ ID No 114

<400> 114

ctaaacttac ggcattggca tcaacgggag ccaccgtcgg agtaggggaa gtaacaacgg 60
gggaactggt attggtgggc aaaattttta ctagccattg attccattgg ggcagattga 120
acgctcccaa ccaccaaagt ccccctaata caacgacaga actaaataac aggaaaaaag 180
gaatccaagg agttacccct cttttatggg atggaacttc atcgacatta aaggtgggag 240
ggggaggagg caatggggac aatggtggtc cagaaaagga aggtggctcc gacgtcaagg 300
caacgggaca tccacaggat tcacagaaac gaacctgggg gctaaggcgg ttgccacaat 360
tagtacaaaa aacgggagta gtcataggtg aaaaccccga ctatagaatt agaaaaattt 420
aactttttat ccgaatttta ttcgtccatg ttccccaaat aactatcaaa ataattggaa 480
aaattaaatt atttggtcgt tggtcaccgc tccctaaaga cctggccatt gtaaagagat 540
tacacccggg tgtaatccat tgggcacgag agttagtaag gcagtggcaa ttaatagagg 600
cttatggttg attcgcattg ttttgctcct gaaattttcg gcaaatacaa atacttcgct 660
cttctagccc tattaaccat tttaacgaca aattgatggg gcaacgatta acaaataatg 720
aataaatttt atgtttttca agatgaaaat ttgaaaattt gatttcctta tatttctact 780
atagaagact aatacaatta gatctaaaat ttgcaagtat aaaaatcagc aaatagttat 840
attgttaata attcaatgac ccaataactc gtactgttat ctacgtggtg aaagccaaaa 900
agacgaacag tttagcctcc tcctcctcgg cgatcgccaa gcgaaatgtc atgggagatg 960
ttcagattga gcattttttt ctaaaagccc ttgctaaaac aaaccacatg tgcagggtgt 1020
ccccgatgtt gactaaattc agcggctcga gaacc 1055

<210> 115
<211> 1032
<212> DNA
<213> Synechocystis sp. PCC 6803

<220>
<223> >SEQ ID No 115

<400> 115

gtgagcttga tggtgatggt gggtaaagct tcattgtcca tgcgccggag cttgacggta 60
aaatccgtgt cctggggcca atcattggga acgctcacac agataccgtt gcgagttaag 120
tcgaaggata cccccaccaa ttccccttcc atatcgaaca cttggcaaaa ggccaacatc 180
cgctcttcct gccgtcgctc ctgcaccccc tggagatggg caatggttag ggcttcttgg 240
gagagggaag atggagaatc ttgagccatg gagattattt cctcggttaa attaggttta 300
cctagtaaat gggccaggtt gaccattatt gtagtcattc actgggtcga tcttccttcc 360
atcagggtca tttggttaat tgttgatgag aaatgggaag gagtaatcca tagatatttg 420

```
cccagttaac tcgatttgag cagaattggg aaggacgtat tgggaacttt ggttgacagg     480
gcaacaaagc cagcagatta cacccgggtg taatcgctgg aggcgaactg ggtgagaacc     540
ataaaatctt ggggaacagg ggaatgttaa tgaacacatg acatgcatag gatagcgaat     600
tgatttaaag caatgattcc ccctgagata aatatatttt gacccactgc tccgcttaat     660
tttggcgcta atctgggcaa aaattcgttg gttttcggaa aggtgaaccc gtagctagct     720
tgtattgccc aaacctaact catcattgct ccatggcggc caaaaatctt taggacaaaa     780
ctcgccagag gtcaaggctt gattttttca gagggaaaaa ttcctcggct aaataatcaa     840
aaagttgaaa aaaaaagttt ttcagctaag gtttgatgtt atattttatc ttatgtagac     900
ttttgaaaaa aacaagtccc cgcaatcaag catcattcaa cggaaaaaat aatcctatgc     960
caaacgcctc caccgcacta accggcaaag cattactcaa taaagttaaa gaactatccc    1020
atctgccccg tc                                                        1032
```